# EUROPEAN PATENT APPLICATION

(11) **EP 4 480 966 A1**
(43) Date of publication of application: **25.12.2024**
(21) Application number: 23755846.5
(22) Date of filing: 16.02.2023
(51) Int. Cl.: C07K 16/28, C07K 16/46, C12N 15/13, C12N 15/63, C12N 5/10, A61K 39/395, A61P 35/00

(54) **HUMANIZED ANTI-CD28 ANTIBODY AND BISPECIFIC ANTIBODY THEREOF WITH ANTI-CD40 ANTIBODY**

(30) Priority: 16.02.2022 WO PCT/CN2022/076503; 16.02.2022 WO PCT/CN2022/076504; 16.02.2022 WO PCT/CN2022/076506
(71) Applicant: UTC Therapeutics (Shanghai) Co., Ltd., Shanghai 201318 (CN)
(72) Inventor: ZHAO, Yangbing, Shanghai 201318 (CN); LIU, Xiaojun, Shanghai 201318 (CN); CAO, Qingjuan, Shanghai 201318 (CN)
(74) Representative: Symbiosis IP Limited
(86) International application number: PCT/CN2023/076535
(87) International publication number: WO 2023/155845

(57) **Abstract**

The present invention relates to a humanized anti-CD28 antibody, a multispecific antibody comprising the humanized anti-CD28 antibody, especially a bispecific antibody comprising the humanized anti-CD28 antibody and an anti-CD40 antibody, a polynucleotide encoding these antibodies, a vector comprising the polynucleotide, a host cell comprising the polynucleotide or the vector, and a pharmaceutical composition comprising same. The present invention also relates to use of the humanized anti-CD28 antibody in inducing T cell proliferation and use of the bispecific antibody in enhancing the killing effect of immune cells on a target cell.

## Description

### CROSS-REFERENCE TO RELATED APPLICATIONS

The present application claims priority to PCT application No. PCT/CN2022/076506 filed on February 16, 2022, PCT application No. PCT/CN2022/076503 filed on February 16, 2022, and PCT application No. PCT/CN2022/076504 filed on February 16, 2022, which are incorporated herein by reference in their entireties.

### TECHNICAL FIELD

The present invention relates to antibodies, and in particular, the present invention relates to a humanized anti-CD28 antibody and a bispecific antibody thereof with an anti-CD40 antibody.

### BACKGROUND

When tissues are damaged or infected, the immune system is activated, and B and T lymphocytes, macrophages, eosinophils, neutrophils, etc., are recruited and activated. Antigen presenting cells (APCs), such as macrophages or dendritic cells, capture an exogenous protein and display it on the surface of the APC cells. T-helper cells (T_{H} cells) express immunoglobulins on their surface and are activated upon recognition of the antigen displayed by APCs. Activated T_{H} cells in turn activate certain B cell clones to proliferate and differentiate into plasma cells, which then produce and secrete an antibody against the exogenous protein. The antibody binds to cells expressing the exogenous protein and thus targets these cells to allow the killing of these cells by immune effector cells[1].

Stimulation of resting T lymphocytes for activation, proliferation, and functional differentiation requires binding to 2 receptors: (1) a receptor capable of specifically recognizing an antigen; (2) a CD28 molecule. Cluster of differentiation 28 (CD28) is a membrane protein expressed on the vast majority of resting T cells, is 44 kDa, and is capable of binding to CD80 (B7.1) and CD86 (B7.2) proteins[2] on the surface of APC cells to provide costimulatory signals for T cell activation and proliferation. In the classical costimulatory system, antigen receptor binding nor CD28 molecule binding alone cannot induce T cell proliferation. CD28 is an essential costimulatory receptor for T cell clonal expansion, differentiation, and effector function. CD28 binding lowers the T cell activation threshold and results in the enhancement of TCR signaling events necessary for efficient cytokine production (by enhancing transcriptional activity and messenger RNA stability), cell cycle progression, survival, metabolic regulation, and T cell response. CD28 is a key factor in the immune synapse (IS) tissue, in which CD28 enhances intimate contact between T cells and APCs.

However, the CD28-specific monoclonal antibody (mAb) 9.3 can induce T cell proliferation without co-stimulation, i.e., the CD28-specific mAb, which is independent of antigen receptor binding, activates resting T lymphocytes[3, 4]. The 9.3 antibody is derived from mouse hybridoma cells[4] and has high immunogenicity in humans. Philip Tan et al. used CDR grafting and pairing of a hypervariable loop region with a human V gene to humanize the 9.3 antibody[5] and grafted the CDR of the murine antibody onto a human framework, thereby greatly reducing the immunogenicity of the chimeric antibody. The humanized 9.3 chimeric antibody has a reduced ability to bind to an antigen.

When tissues are damaged or infected, the immune system is activated, and B and T lymphocytes, macrophages, eosinophils, neutrophils, etc., are recruited and activated. Antigen presenting cells (APCs), such as macrophages or dendritic cells, capture an exogenous protein and display it on the surface of the APC cells. T-helper cells (T_{H} cells) express immunoglobulins on their surface and are activated upon recognition of the antigen displayed by APCs. Activated T_{H} cells in turn activate certain B cell clones to proliferate and differentiate into plasma cells, which then produce and secrete an antibody against the exogenous protein. The antibody binds to cells expressing the exogenous protein and thus targets these cells to allow the killing of these cells by immune effector cells[1]. Contact of T_{H} cells with B cells stimulates B cell proliferation and the conversion of immunoglobulin (Ig) from IgM to IgG, IgA or IgE. The CD40/CD40L receptor-ligand interaction plays an important role in mediating the contact of T_{H} cells with B cells.

CD40 is a costimulatory member of the tumor necrosis factor receptor (TNFR) superfamily. Human CD40 is a type I transmembrane glycoprotein, consists of 277 amino acids, has a molecular weight of 30,600, and is structurally divided into a signal peptide, an extracellular domain, a transmembrane domain, and an intracellular domain. Like other members of the TNFR superfamily, CD40 forms trimers and has higher-order protein clustering properties required for optimal signaling. Human CD40L is a member of the tumor necrosis factor (TNF) superfamily, belongs to a type II transmembrane glycoprotein, and has an extracellular domain, a transmembrane domain, and an intracellular domain from the C-terminus to the N-terminus. CD40L was originally found on the surface of activated CD4+ T cells, and the CD40-CD40L interaction between B and T cells is also critical for the germinal center (GC) response. Functions of CD40 on B cells. CD40 as a co-stimulator, together with cytokines or other stimuli, promotes B cell activation and proliferation. CD40 stimulation causes up-regulation of CD80 and CD86 on human peripheral blood B cells and naive, memory, and germinal center B cells. CD40 signaling also induces up-regulation of CD95/Fas and major histocompatibility complex class II (MHCII) on human B cells. In addition to early activation, CD40 signaling also promotes further progression in activation through cell cycle and B cell expansion. CD40, when binding to an anti-CD20 antibody, an anti-IL-4 antibody, or an anti-IL-21 antibody, can also stimulate rapid proliferation of circulating and tissue-resident B cells.

In addition to B cells, CD40 is also expressed on other hematopoietic cells, such as monocytes and dendritic cells, and can promote growth of these cells and cytokine release therefrom, as well as release signals to induce activation, proliferation and cytokine production of CD4+ T cells expressing CD40L. The CD40 signaling pathway also affects the function of non-hematopoietic cells, including endothelial cells, epithelial cells, fibroblasts, and neural cells. CD40L is also widely expressed on a variety of cells, including mast cells, basophils, B cells, NK cells, macrophages, megakaryocytes, and platelets[6]. The CD40 signaling pathway has a wide range of effects in cell biology. Although CD40 is considered to be the most important binding protein for CD40L, CD40L also binds to other proteins of the integrin family.

CD40 plays a broad role in immune regulation by mediating T cell interactions with B cells as well as other cells. An agonistic CD40 antibody can be used as an immunostimulant to enhance the immune response in an individual. Several anti-CD40 antibodies currently being developed have low activity.

The tumor microenvironment (TME) is composed of blood vessels, myeloid-derived suppressor cells (MDSCs), antigen presenting cells (APCs), lymphocytes, neutrophils, tumor-associated macrophages (TAMs), fibroblasts, extracellular matrix, cytokines, growth factors, etc., and is closely related to the generation and metastasis of tumors. The tumor microenvironment is involved in expelling T cells outside the tumor edges, inhibiting the accumulation of T cells in the tumor and inhibiting the proliferation of T cells[7].

A bispecific antibody (diabody or BsAb for short) can simultaneously bind to two different antigen epitopes, bridge two kinds of cells carrying different antigens, redirect effector T cells to tumor cells, and promote the function of the effector T cells. The diabody herein is formed by an scFv targeting CD28 and an scFv targeting CD40 in tandem, and such a diabody is also referred to as BiTE (bispecific T-cell engager or bispecific T-cell redirecting antibody). The CD28-scFv acts as an agonist for the CD28 molecule, and the CD40-scFv is an agonist for the CD40 molecule. Therefore, the CD28-CD40 diabody can simultaneously activate CD28 and CD40 signaling pathways and play an important role in immune response.

The CD40 signaling pathway affects the differentiation of CD4⁺ T cells into effector cells. During the early activation stage of CD4⁺ T cells, CD4⁺ T cells transiently express CD40L (CD40 ligand), CD40L interacts with CD40 on other cells to cause degradation of CD40L, and soluble CD40L is released outside the cell. In the late stage of activation, expression of CD40L is dependent on the costimulatory molecule CD28. CD28 binds to its ligands CD80 and CD86 before it can induce complete activation and differentiation of T cells. CD40 expressed by APCs binds to CD40L of T cells, promoting the expression of CD80 and CD86 on APCs. At the same time, CD28's binding to CD80/CD86 up-regulates CD40L, thereby producing a positive feedback effect. The two synergistically drive T cell activation and dendritic cell maturation[6]. The CD40 signaling pathway affects dendritic cells and macrophages. Dendritic cells, as antigen presenting cells, present an antigen to T cells via the interaction between the major histocompatibility complex (MHC) and the T cell receptor (TCR), thus allowing effector T cells to function. The CD28-CD40 diabody activates CD40 on dendritic cells and thereby up-regulates expression of MHC and costimulatory molecule, thus resulting in maturation and activation of the dendritic cells and pre-activation of CD8 T cells. The downstream signaling pathway of CD40 in turn activates the MAPK and NFKb signaling pathways via TRAF6, thereby promoting survival and maturation of dendritic cells and cytokine release therefrom. In the tumor microenvironment, macrophages may enhance the immune response by secreting IL-10, IDO, and TGF-beta.

The CD40 signaling pathway affects NK cells. NK cells can be directly activated through the CD40L/CD40 interaction of itself, thereby promoting proliferation, activation of NK cells and killing of target cells. In the tumor microenvironment, the activation of the CD40 pathway can increase the killing effect of NK cells. [8] CD40 signaling pathway granulocytes. Granulocytes also express CD40 under different circumstances. In the above, the CD40 pathway promotes the survival of human peripheral blood eosinophils and the release of macrophage colony-stimulating factor (GMCSF). Eosinophils can also act as antigen presenting cells, activating numerous T cell helper responses (pro-inflammation and inhibition of inflammation). Therefore, the CD28-CD40 diabody not only provides a costimulatory signal for T cell activation, but also can stimulate various immune cells (such as dendritic cells, macrophages, NK cells, granulocytes, etc.) to activate and function, and thus the immune response is remarkably enhanced.

### SUMMARY

In one aspect, the present invention provides a humanized anti-CD28 antibody or an antigen-binding fragment thereof, wherein the humanized anti-CD28 antibody comprises a heavy chain variable region comprising an HCDR1, an HCDR2 and an HCDR3 and a light chain variable region comprising an LCDR1, an LCDR2 and an LCDR3, wherein,
the HCDR1 comprises SEQ ID NO: 1,
the HCDR2 comprises a sequence selected from SEQ ID NOs: 2, 17 and 18,
the HCDR3 comprises SEQ ID NO: 3,
the LCDR1 comprises SEQ ID NO: 9,
the LCDR2 comprises SEQ ID NO: 10, and
the LCDR3 comprises SEQ ID NO: 11;
the humanized anti-CD28 antibody has an EC50 of 0.008 µg/mL to 0.016 µg/mL for binding to human CD28 as determined by ELISA; or the humanized anti-CD28 antibody has an equilibrium dissociation constant KD of 6.1 × 10⁻⁹ M to 1.2 × 10⁻⁸ M for binding to human CD28 as determined by biolayer interferometry (BLI).

In some embodiments, the heavy chain variable region of the humanized anti-CD28 antibody comprises an HFR1, an HFR2, an HFR3, and an HFR4, and the light chain variable region of the humanized anti-CD28 antibody comprises an LFR1, an LFR2, an LFR3, and an LFR4, wherein,
the HFR1 comprises a sequence selected from SEQ ID NOs: 19-22,
the HFR2 comprises a sequence selected from SEQ ID NOs: 23-26,
the HFR3 comprises a sequence selected from SEQ ID NOs: 27-30,
the HFR4 comprises the sequence set forth in SEQ ID NO: 7,
the LFR1 comprises a sequence selected from SEQ ID NOs: 36-37,
the LFR2 comprises a sequence selected from SEQ ID NOs: 38-39,
the LFR3 comprises a sequence selected from SEQ ID NOs: 40-42, and the LFR4 comprises the sequence set forth in SEQ ID NO: 15.

In some embodiments, the HFR1, the HFR2, the HFR3, and the HFR4 comprised in the heavy chain variable region of the humanized anti-CD28 antibody are selected from the group consisting of:
(1) an HFR1 comprising the sequence set forth in SEQ ID NO: 19, an HFR2 comprising the sequence set forth in SEQ ID NO: 23, an HFR3 comprising the sequence set forth in SEQ ID NO: 27, and an HFR4 comprising the sequence set forth in SEQ ID NO: 7;
(2) an HFR1 comprising the sequence set forth in SEQ ID NO: 20, an HFR2 comprising the sequence set forth in SEQ ID NO: 24, an HFR3 comprising the sequence set forth in SEQ ID NO: 28, and an HFR4 comprising the sequence set forth in SEQ ID NO: 7;
(3) an HFR1 comprising the sequence set forth in SEQ ID NO: 21, an HFR2 comprising the sequence set forth in SEQ ID NO: 25, an HFR3 comprising the sequence set forth in SEQ ID NO: 29, and an HFR4 comprising the sequence set forth in SEQ ID NO: 7;
(4) an HFR1 comprising the sequence set forth in SEQ ID NO: 21, an HFR2 comprising the sequence set forth in SEQ ID NO: 26, an HFR3 comprising the sequence set forth in SEQ ID NO: 30, and an HFR4 comprising the sequence set forth in SEQ ID NO: 7; and
(5) an HFR1 comprising the sequence set forth in SEQ ID NO: 22, an HFR2 comprising the sequence set forth in SEQ ID NO: 26, an HFR3 comprising the sequence set forth in SEQ ID NO: 30, and an HFR4 comprising the sequence set forth in SEQ ID NO: 7.

In some embodiments, the LFR1, the LFR2, the LFR3, and the LFR4 comprised in the light chain variable region of the humanized anti-CD28 antibody are selected from the group consisting of:
(1) an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(2) an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15; and
(3) an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15.

In some embodiments, the HFR1, the HFR2, the HFR3, and the HFR4 comprised in the heavy chain variable region and the LFR1, the LFR2, the LFR3, and the LFR4 comprised in the light chain variable region of the humanized anti-CD28 antibody are selected from the group consisting of:
(1) an HFR1 comprising the sequence set forth in SEQ ID NO: 19, an HFR2 comprising the sequence set forth in SEQ ID NO: 23, an HFR3 comprising the sequence set forth in SEQ ID NO: 27, an HFR4 comprising the sequence set forth in SEQ ID NO: 7, an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(2) an HFR1 comprising the sequence set forth in SEQ ID NO: 19, an HFR2 comprising the sequence set forth in SEQ ID NO: 23, an HFR3 comprising the sequence set forth in SEQ ID NO: 27, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(3) an HFR1 comprising the sequence set forth in SEQ ID NO: 20, an HFR2 comprising the sequence set forth in SEQ ID NO: 24, an HFR3 comprising the sequence set forth in SEQ ID NO: 28, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(4) an HFR1 comprising the sequence set forth in SEQ ID NO: 20, an HFR2 comprising the sequence set forth in SEQ ID NO: 24, an HFR3 comprising the sequence set forth in SEQ ID NO: 28, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(5) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(6) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(7) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(8) an HFR1 comprising the sequence set forth in SEQ ID NO: 21, an HFR2 comprising the sequence set forth in SEQ ID NO: 26, an HFR3 comprising the sequence set forth in SEQ ID NO: 30, an HFR4 comprising the sequence set forth in SEQ ID NO: 7, (2) an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(9) an HFR1 comprising the sequence set forth in SEQ ID NO: 21, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(10) an HFR1 comprising the sequence set forth in SEQ ID NO: 22, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15; and
(11) an HFR1 comprising the sequence set forth in SEQ ID NO: 22, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15.

In some embodiments, the heavy chain variable region of the humanized anti-CD28 antibody comprises a sequence selected from SEQ ID NOs: 31-35.

In some embodiments, the light chain variable region of the humanized anti-CD28 antibody comprises a sequence selected from SEQ ID NOs: 43-45.

In some embodiments, the heavy chain variable region and the light chain variable region of the humanized anti-CD28 antibody are selected from the following combinations:
(1) a heavy chain variable region comprising the sequence set forth in SEQ ID NO: 31 and a light chain variable region comprising the sequence set forth in SEQ ID NO: 43;
(2) a heavy chain variable region comprising the sequence set forth in SEQ ID NO: 31 and a light chain variable region comprising the sequence set forth in SEQ ID NO: 44;
(3) a heavy chain variable region comprising the sequence set forth in SEQ ID NO: 32 and a light chain variable region comprising the sequence set forth in SEQ ID NO: 44;
(4) a heavy chain variable region comprising the sequence set forth in SEQ ID NO: 32 and a light chain variable region comprising the sequence set forth in SEQ ID NO: 45;
(5) a heavy chain variable region comprising the sequence set forth in SEQ ID NO: 33 and a light chain variable region comprising the sequence set forth in SEQ ID NO: 43;
(6) a heavy chain variable region comprising the sequence set forth in SEQ ID NO: 33 and a light chain variable region comprising the sequence set forth in SEQ ID NO: 44;
(7) a heavy chain variable region comprising the sequence set forth in SEQ ID NO: 33 and a light chain variable region comprising the sequence set forth in SEQ ID NO: 45;
(8) a heavy chain variable region comprising the sequence set forth in SEQ ID NO: 34 and a light chain variable region comprising the sequence set forth in SEQ ID NO: 44;
(9) a heavy chain variable region comprising the sequence set forth in SEQ ID NO: 34 and a light chain variable region comprising the sequence set forth in SEQ ID NO: 45;
(10) a heavy chain variable region comprising the sequence set forth in SEQ ID NO: 35 and a light chain variable region comprising the sequence set forth in SEQ ID NO: 44; and
(11) a heavy chain variable region comprising the sequence set forth in SEQ ID NO: 35 and a light chain variable region comprising the sequence set forth in SEQ ID NO: 45.

In some embodiments, the heavy chain variable region of the humanized anti-CD28 antibody is fused to an immunoglobulin heavy chain constant region and/or the light chain variable region of the humanized anti-CD28 antibody is fused to an immunoglobulin light chain constant region.

In some embodiments, the immunoglobulin heavy chain constant region comprises SEQ ID NO: 81 and the immunoglobulin light chain constant region comprises SEQ ID NO: 82.

In some embodiments, the humanized anti-CD28 antibody is an scFv.

In some embodiments, the humanized anti-CD28 scFv comprises a heavy chain variable region and a light chain variable region linked via a peptide linker.

In some embodiments, the sequence of the peptide linker in the humanized anti-CD28 scFv comprises SEQ ID NO: 80.

In some embodiments, the heavy chain variable region in the humanized anti-CD28 scFv is located at the N-terminus or C-terminus of the light chain variable region.

Another aspect of the present invention provides a multispecific antibody, which comprises at least a first antigen-binding moiety and a second antigen-binding moiety, wherein the first antigen-binding moiety comprises the aforementioned humanized anti-CD28 antibody or antigen-binding fragment thereof, and the second antigen-binding moiety and the first antigen-binding moiety bind to different antigens.

In some embodiments, the second antigen-binding moiety specifically binds to a tumor antigen or is capable of stimulating an immune cell.

In some embodiments, the second antigen-binding moiety is an anti-CD40 antibody.

In some embodiments, the first antigen-binding moiety is an scFv and the second antigen-binding moiety is an scFv.

In some embodiments, the first antigen-binding moiety and the second antigen-binding moiety are linked via a peptide linker.

In some embodiments, the peptide linker comprises the sequence of SEQ ID NO: 79.

In some embodiments, the first antigen-binding moiety is located at the N-terminus or C-terminus of the second antigen-binding moiety.

In some embodiments, the multispecific antibody is a bispecific antibody.

In some embodiments, the bispecific antibody comprises the first antigen-binding moiety and the second antigen-binding moiety, wherein the second antigen-binding moiety is an anti-CD40 antibody or an antigen-binding fragment thereof, wherein the anti-CD40 antibody has an EC50 of less than 0.04919 µg/mL for binding to human CD40 as determined by ELISA, or the anti-CD40 antibody has an EC50 of less than 3.44 × 10⁻⁷ M for binding to human CD40 as determined by ELISA.

In some embodiments, the anti-CD40 antibody is obtained by affinity maturation of a parent antibody and exhibits greater binding affinity for CD40 than the parent antibody; the parent antibody has a heavy chain variable region and a light chain variable region, wherein the heavy chain variable region comprises an HCDR1 comprising SEQ ID NO: 46, an HCDR2 comprising SEQ ID NO: 47, and an HCDR3 comprising SEQ ID NO: 48, and the light chain variable region comprises an LCDR1 comprising SEQ ID NO: 54, an LCDR2 comprising SEQ ID NO: 55, and an LCDR3 comprising SEQ ID NO: 56.

In some embodiments, a heavy chain variable region of the anti-CD40 antibody comprises an HCDR1 comprising a sequence selected from SEQ ID NO: 46 and SEQ ID NO: 62, an HCDR2 comprising SEQ ID NO: 47, and an HCDR3 comprising SEQ ID NO: 48.

In some embodiments, the heavy chain variable region of the anti-CD40 antibody comprises:
(1) an HCDR1 comprising SEQ ID NO: 46, an HCDR2 comprising SEQ ID NO: 47, and an HCDR3 comprising SEQ ID NO: 48; or
(2) an HCDR1 comprising SEQ ID NO: 62, an HCDR2 comprising SEQ ID NO: 47, and an HCDR3 comprising SEQ ID NO: 48.

In some embodiments, a light chain variable region of the anti-CD40 antibody comprises: an LCDR1 comprising SEQ ID NO: 54, an LCDR2 comprising a sequence selected from SEQ ID NOs: 55 and 64-66, and an LCDR3 comprising a sequence selected from SEQ ID NOs: 56 and 67-68.

In some embodiments, the light chain variable region of the anti-CD40 antibody comprises:
(1) an LCDR1 comprising SEQ ID NO: 54, an LCDR2 comprising SEQ ID NO: 64, and an LCDR3 comprising SEQ ID NO: 56;
(2) an LCDR1 comprising SEQ ID NO: 54, an LCDR2 comprising SEQ ID NO: 55, and an LCDR3 comprising SEQ ID NO: 67;
(3) an LCDR1 comprising SEQ ID NO: 54, an LCDR2 comprising SEQ ID NO: 55, and an LCDR3 comprising SEQ ID NO: 68;
(4) an LCDR1 comprising SEQ ID NO: 54, an LCDR2 comprising SEQ ID NO: 65, and an LCDR3 comprising SEQ ID NO: 56;
(5) an LCDR1 comprising SEQ ID NO: 54, an LCDR2 comprising SEQ ID NO: 66, and an LCDR3 comprising SEQ ID NO: 56.

In some embodiments, the heavy chain variable region and the light chain variable region of the anti-CD40 antibody comprise:
(1) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 64, and an LCDR3 comprising SEQ ID NO: 56;
(2) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 67;
(3) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 68;
(4) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 65, and an LCDR3 comprising SEQ ID NO: 56;
(5) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 66, and an LCDR3 comprising SEQ ID NO: 56.

In some embodiments, the heavy chain variable region of the anti-CD40 antibody comprises:
(1) an HFR1 comprising SEQ ID NO: 49, an HFR2 comprising SEQ ID NO: 50, an HFR3 comprising SEQ ID NO: 51, and an HFR4 comprising SEQ ID NO: 52;
(2) an HFR1 comprising SEQ ID NO: 63, an HFR2 comprising SEQ ID NO: 50, an HFR3 comprising SEQ ID NO: 51, and an HFR4 comprising SEQ ID NO: 52.

In some embodiments, the light chain variable region of the anti-CD40 antibody comprises an LFR1 comprising SEQ ID NO: 57, an LFR2 comprising SEQ ID NO: 58, an LFR3 comprising SEQ ID NO: 59, and an LFR4 comprising SEQ ID NO: 60.

In some embodiments, the heavy chain variable region of the anti-CD40 antibody has at least 70% sequence identity to a sequence selected from SEQ ID NOs: 69-73.

In some embodiments, the heavy chain variable region of the anti-CD40 antibody comprises a sequence selected from SEQ ID NOs: 69-73.

In some embodiments, the light chain variable region of the anti-CD40 antibody has at least 70% sequence identity to a sequence selected from SEQ ID NOs: 74-78.

In some embodiments, the light chain variable region of the anti-CD40 antibody comprises a sequence selected from SEQ ID NOs: 74-78.

In some embodiments, the anti-CD40 antibody is an scFv.

In some embodiments, the anti-CD40 scFv comprises a heavy chain variable region and a light chain variable region linked via a peptide linker.

In some embodiments, the sequence of the peptide linker in the anti-CD40 scFv comprises SEQ ID NO: 80.

In some embodiments, the heavy chain variable region in the anti-CD40 scFv is located at the N-terminus or C-terminus of the light chain variable region.

Another aspect of the present invention provides a polynucleotide encoding the aforementioned humanized anti-CD28 antibody or antigen-binding fragment thereof or the aforementioned multispecific antibody or bispecific antibody.

Another aspect of the present invention provides a vector comprising the aforementioned polynucleotide.

In some embodiments, the vector is a plasmid vector, a viral vector, a circular RNA comprising the polynucleotide, a precursor RNA from which the circular RNA can be obtained by circularization, or a vector comprising a DNA template for the precursor RNA, wherein the circular RNA comprises, in sequence, an internal ribosome entry site (IRES) element, the polynucleotide, and a polyA.

In some embodiments, the polyA is at least 45 nucleotides in length; preferably, the polyA is at least 70 nucleotides in length.

Another aspect of the present invention provides a host cell comprising the aforementioned polynucleotide or the aforementioned vector.

Another aspect of the present invention provides a pharmaceutical composition comprising the aforementioned humanized anti-CD28 antibody or antigen-binding fragment thereof, the aforementioned multispecific antibody, the aforementioned bispecific antibody, the aforementioned polynucleotide or the aforementioned vector and a pharmaceutically acceptable carrier. Another aspect of the present invention provides a pharmaceutical combination, which comprises a combination of: a) the aforementioned humanized anti-CD28 antibody or antigen-binding fragment thereof, the aforementioned multispecific antibody, the aforementioned bispecific antibody, or the aforementioned polynucleotide, or the aforementioned vector; and b) an immune cell; or provides an immune cell expressing the aforementioned humanized anti-CD28 antibody or antigen-binding fragment thereof, the aforementioned multispecific antibody, or the aforementioned bispecific antibody.

In some embodiments, the immune cell is a T cell, an NK cell, an NKT cell, a macrophage, a neutrophil, or a granulocyte.

In some embodiments, the immune cell recombinantly expresses a chimeric antigen receptor (CAR), a T cell receptor (TCR), or a bispecific T cell engager (BiTE), wherein the CAR, the TCR, or the BiTE binds to a tumor antigen or a viral antigen. In some embodiments, the immune cell is a CAR-T cell.

In some embodiments, the CAR targets mesothelin, CD123, BCMA, HER2, II,13Ra2, CD19, or B7H3.

In some embodiments, the CAR targeting mesothelin, CD123, BCMA, HER2, IL13Ra2, CD19, or B7H3 is as defined herein.

Another aspect of the present invention provides a method for enhancing the killing effect of an immune cell on a target cell in a subject, which comprises administering to the subject receiving the immune cell the aforementioned humanized anti-CD28 antibody or antigen-binding fragment thereof, the aforementioned multispecific antibody, the aforementioned bispecific antibody, the aforementioned polynucleotide, the aforementioned vector, or the aforementioned pharmaceutical composition. In some embodiments, the immune cell is as defined above.

The present invention also provides use of the aforementioned humanized anti-CD28 antibody or antigen-binding fragment thereof, the aforementioned multispecific antibody, the aforementioned bispecific antibody, the aforementioned polynucleotide, the aforementioned vector, or the aforementioned pharmaceutical composition in enhancing the killing effect of an immune cell on a target cell in a subject or in preparing a medicament for enhancing the killing effect of an immune cell on a target cell in a subject. The immune cell is as defined above.

Another aspect of the present invention provides a method for killing a target cell in a subject, which comprises administering to the subject the aforementioned pharmaceutical combination or the aforementioned immune cell.

In some embodiments, the target cell is a tumor cell. In some embodiments, the tumor cell expresses mesothelin, CD123, BCMA, HER2, IL13Ra2, CD19, or B7H3.

In some embodiments, the immune cell is a CAR-T cell.

The present invention also provides use of the aforementioned pharmaceutical combination or the aforementioned immune cell in killing a target cell in a subject or in preparing a medicament for killing a target cell in a subject.

Another aspect of the present invention provides a method for treating a disease in a subject in need thereof, which comprises administering to the subject a therapeutically effective amount of the aforementioned pharmaceutical combination or the aforementioned immune cell.

In some embodiments, the disease is a tumor, a cancer, a viral infection, or an autoimmune disease.

In some embodiments, the cancer expresses mesothelin, CD123, BCMA, HER2, IL13Ra2, or B7H3.

In some embodiments, the cancer is a solid tumor or a hematologic cancer.

In some embodiments, the cancer is acute myeloid leukemia (AML), B-type acute lymphocytic leukemia (B-ALL), T-type acute lymphocytic leukemia (T-ALL), B-cell precursor acute lymphocytic leukemia (BCP-ALL), or blastic placytoid dendritic cell neoplasm (BPDCN), non-Hodgkin's lymphoma, chronic lymphocytic leukemia, acute lymphocytic leukemia, human B-cell precursor leukemia, multiple myeloma, or malignant lymphoma.

In some embodiments, the cancer is mesothelioma, pancreatic cancer, ovarian cancer, lung cancer, breast cancer, gastric cancer, cervical cancer, urothelial cancer, esophageal cancer, bladder cancer, colorectal cancer, endometrial cancer, renal cancer, head and neck cancer, sarcoma, glioblastoma, prostate cancer, thyroid cancer, or glioma. Another aspect of the present invention provides use of the aforementioned pharmaceutical combination or the aforementioned immune cell in treating the aforementioned diseases or in preparing a medicament for treating the aforementioned diseases.

Another aspect of the present invention provides a method for inducing T cell proliferation, which comprises contacting the aforementioned humanized anti-CD28 antibody or antigen-binding fragment thereof, the aforementioned multispecific antibody, the aforementioned bispecific antibody, the aforementioned polynucleotide, or the aforementioned vector with a T cell.

In some embodiments, the method further comprises introducing the aforementioned polynucleotide or the aforementioned vector into the T cell and allowing the T cell to express the aforementioned humanized anti-CD28 antibody or antigen-binding fragment thereof or the aforementioned multispecific antibody.

In some embodiments, the T cell expresses a chimeric antigen receptor (CAR). Another aspect of the present invention provides use of the aforementioned humanized anti-CD28 antibody or antigen-binding fragment thereof, the aforementioned multispecific antibody, the aforementioned bispecific antibody, the aforementioned polynucleotide, or the aforementioned vector in inducing T cell proliferation or in preparing a medicament for inducing T cell proliferation.

### BRIEF DESCRIPTION OF THE DRAWINGS

FIG. 1: the cartoon representation of the structure model of the variable region of the CD28 antibody 9.3. The left chain represents the heavy chain and the right chain represents the light chain.
FIG. 2A: the heavy chain mutated sequence of a humanized antibody. "·" indicates the same amino acid, and the CDR regions are underlined.
FIG. 2B: the light chain mutated sequence of a humanized antibody. "·" indicates the same amino acid, and the CDR regions are underlined.
FIG. 3: the SEC graphs of humanized antibodies.
FIG. 4: the ELISA results for humanized antibodies.
FIG. 5: the sensorgrams of humanized antibodies.
FIG. 6: the expression level of CD40 on A549 cells. 10 µg of CD40 mRNA was electroporated in A549 cells, and 24 h later, the expression level of CD40 was detected by flow cytometry and PE-anti-CD40 antibody.
FIG. 7: the expression levels of diabodies. 15 µg of the mRNA of diabody and 0.5 µg of 4D5.BBZ mRNA were electroporated in T cells, and 24 h later, the expression levels of the diabodies were detected by flow cytometry, CD40-Fc, and PE-anti-IgG-Fc antibody.
FIG. 8: the expression level of Her2-CAR. 15 µg of the mRNA of diabody and 0.5 µg of 4D5.BBZ mRNA were electroporated in T cells, and 24 h later, the expression level of Her2-CAR was detected by flow cytometry, Her2-Fc, and PE-anti-Fc antibody.
FIG. 9: the release level of IFN-gamma. After CAR-T cells and target cells were co-cultured for 24 h according to E:T = 1: 1, the level of IFN-gamma in the supernatant was detected by ELISA.
FIG. 10: the killing-promoting effect. CAR-T cells and target cells were co-cultured for 72 h according to E:T = 10:1, and the cell killing was observed by incucyte. The ordinate TGOII (GCU× µm²/well) is the abbreviation for "Total Green object integrated intensity (GCU × µm²/well)", and the same applies below.
FIG. 11: the expression level of CD40 on A549 cells. 10 µg of CD40 mRNA was electroporated in A549 cells, and 24 h later, the expression level of CD40 was detected by flow cytometry and PE-anti-CD40 antibody.
FIG. 12: the expression level of Meso on A549 cells. 10 µg of Meso mRNA was electroporated in A549 cells, and 24 h later, the expression level of Meso was detected by flow cytometry, Meso-biotin, and PE-streptavidin antibody.
FIG. 13: the expression levels of diabodies. 15 µg of the mRNA of diabody and 2 µg of M12 mRNA were electroporated in T cells, and 24 h later, the expression levels of the diabodies were detected by flow cytometry, CD40-Fc, and PE-anti-Fc antibody.
FIG. 14: the expression level of Meso-CAR. 15 µg of the mRNA of diabody and 2 µg of M12 mRNA were electroporated in T cells, and 24 h later, the expression level of Meso-CAR was detected by flow cytometry, Meso-Fc, and PE-anti-Fc antibody.
FIG. 15: the release levels of IL-2 and IFN-gamma. After CAR-T cells and target cells were co-cultured for 24 h according to E:T = 1: 1, the levels of IL-2 and IFN-gamma in the supernatant were detected by ELISA.
FIG. 16: the killing-promoting effect. CAR-T cells and target cells were co-cultured for 72 h according to E:T = 10:1, and the cell killing was observed by incucyte.
FIG. 17: the detection of gene overexpression levels of CD40, Her2-CAR and diabodies. (A) Expression level of CD40 on A549 cells. (B) Expression levels of Her2-CARs and diabodies.
FIG. 18: the ELISA detection of IL-2 and IFN-gamma release levels in the case where target cells are under the action of Her2-CAR-T (with/without co-electroporated BsAb).
FIG. 19: the killing-promoting effect of Her2-CAR-T (with/without co-electroporated BsAb) on target cells detected by an incucyte S3 live cell dynamic imaging and analysis system.
FIG. 20: the detection of gene overexpression levels of CD40, Meso-CAR and diabodies. (A) Expression levels of Meso and CD40 on A549 cells. (B) Expression levels of Meso-CARs and diabodies.
FIG. 21: the ELISA detection of IL-2 and IFN-gamma release levels in the case where target cells are under the action of Meso-CAR-T (with co-electroporated BsAb).
FIG. 22: the killing-promoting effect of Meso-CAR-T (with co-electroporated BsAb) on target cell diabodies detected by an incucyte S3 live cell dynamic imaging and analysis system.
FIG. 23: the detection of gene overexpression levels of CD40, Her2-CARs, and 9.3-40.52 optimized diabodies. (A) Expression level of CD40 on A549 cells. (B) Expression levels of Her2-CARs. (C) Expression levels of 9.3-40.52 optimized BsAbs.
FIG. 24: the ELISA detection of IFN-gamma release levels in the case where target cells are under the action of Her2-CAR-T (with/without co-electroporated 9.3-40.52 optimized BsAbs).
FIG. 25: the killing-promoting effect of Her2-CAR-T (with/without co-electroporated 9.3-40.52 optimized BsAbs) on target cells detected by an incucyte S3 live cell dynamic imaging and analysis system.
FIG. 26: the detection of gene overexpression levels of CD40, Meso-CARs and 9.3-40.52 optimized diabodies. (A) Expression levels of Meso and CD40 on A549 cells. (B) Expression levels of Meso-CARs and 9.3-40.52 optimized diabodies.
FIG. 27: the IFN-gamma and IL-2 release levels in the case where target cells are under the action of Meso-CAR-T (with/without co-electroporated 9.3-40.52 optimized BsAbs).
FIG. 28: the killing-promoting effect of Meso-CAR-T (with/without co-electroporated 9.3-40.52 optimized BsAbs) on target cells detected by an incucyte S3 live cell dynamic imaging and analysis system.
FIG. 29: the detection of gene overexpression levels of CD40, Her2-CARs, and 9.3-A40C optimized diabodies. (A) Expression level of CD40 on A549 cells. (B) Expression levels of Her2-CARs and 9.3-A40C optimized diabodies.
FIG. 30: the killing-promoting effect of Her2-CAR-T (with/without co-electroporated 9.3-A40C optimized BsAbs) on target cells detected by an incucyte S3 live cell dynamic imaging and analysis system.
FIG. 31: the detection of gene overexpression levels of CD40, Meso-CARs, and 9.3-A40C optimized diabodies. (A) Expression levels of Meso and CD40 on A549 cells. (B) Expression levels of Meso-CARs and 9.3-A40C optimized diabodies.
FIG. 32: the IFN-gamma release levels in the case where target cells are under the action of Meso-CAR-T (with/without co-electroporated 9.3-A40C optimized BsAbs).
FIG. 33: the killing-promoting effect of Meso-CAR-T (with/without co-electroporated 9.3-A40C optimized BsAbs) on diabodies of target cells detected by an incucyte S3 live cell dynamic imaging and analysis system.
FIG. 34: the affinity ELISA results of F2.103\40.52\A40C-CD40 antibodies.
FIG. 35: the sequences of affinity-matured antibodies. "·" indicates the same amino acid, and the CDR regions are underlined. (A) Results of VH region sequence alignment of affinity matured antibodies. (B) Results of VL region sequence alignment of affinity-matured antibodies.
FIG. 36: the results of phage ELISA.
FIG. 37-A: the expression level of CD40 on A549 cells.

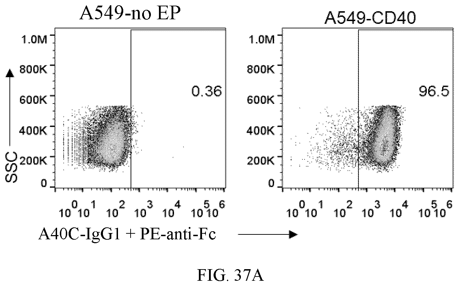

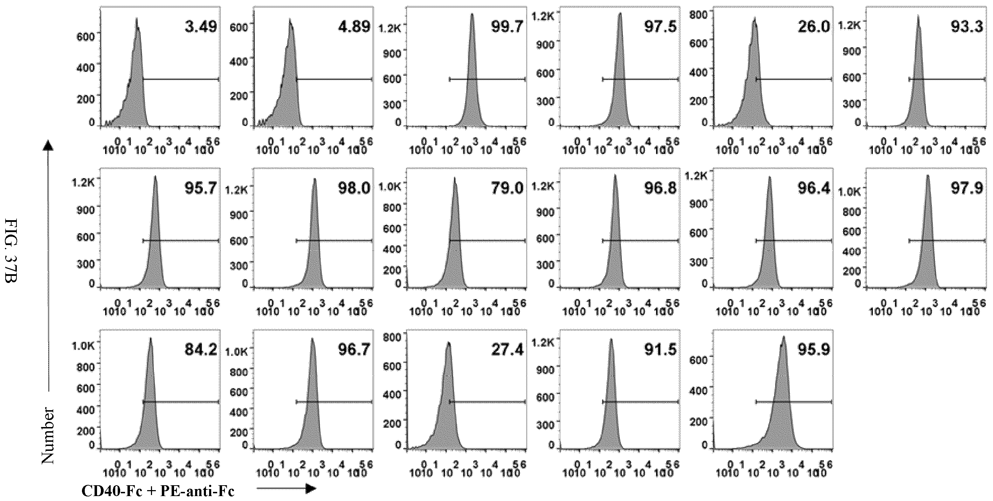

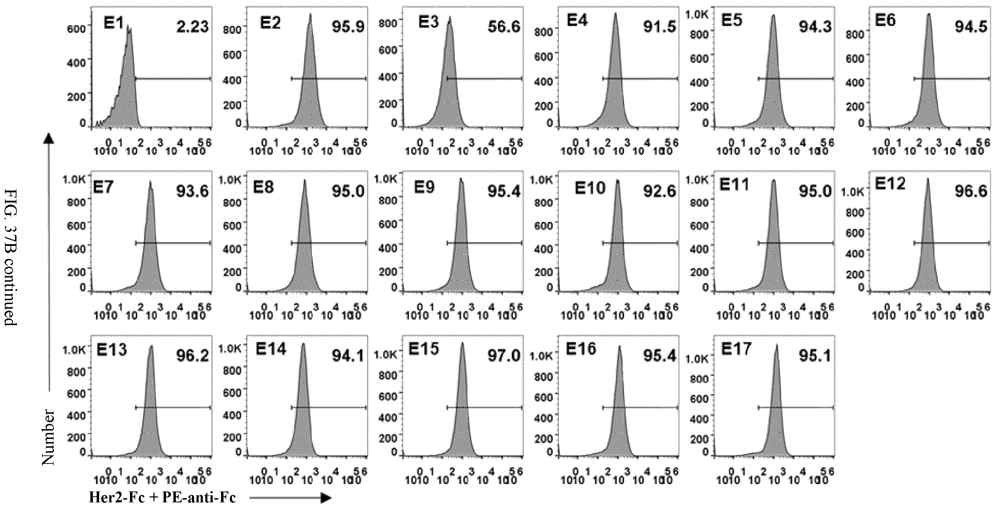

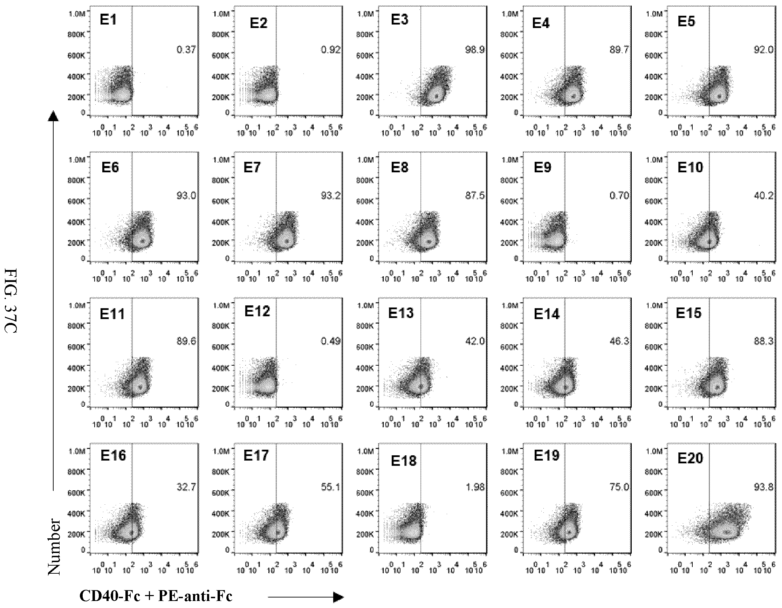

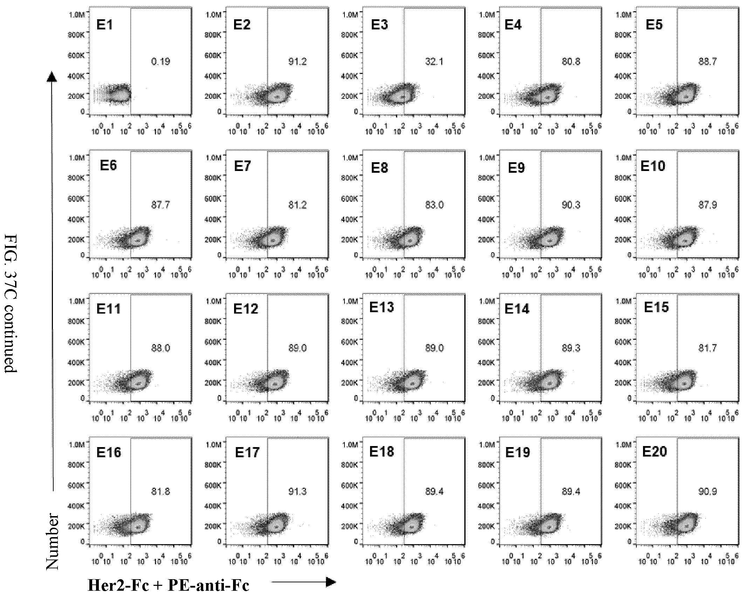

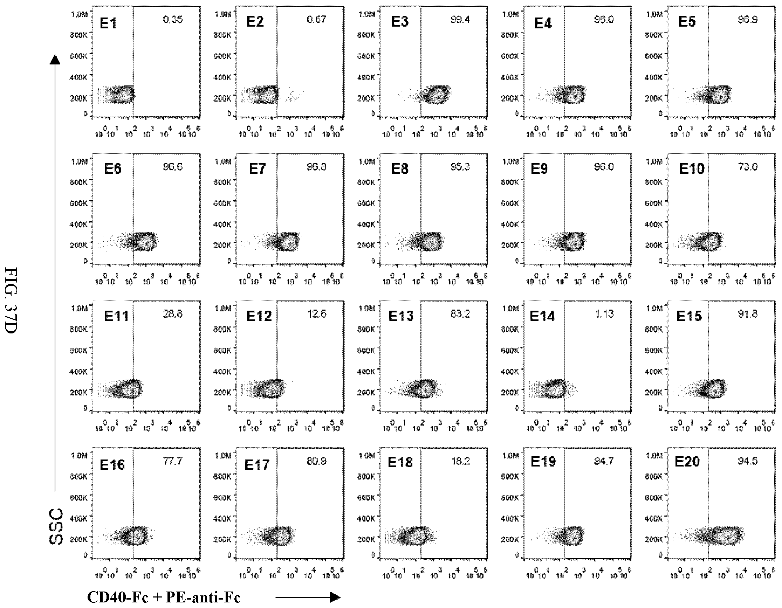

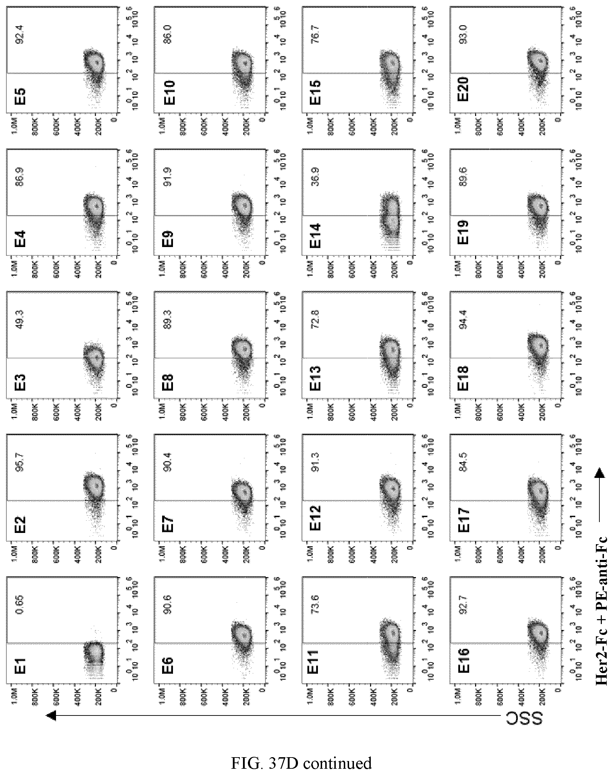

FIG. 37 (B-D): the expression levels of Her2-CARs and AM40.52-9.3h11 diabodies.

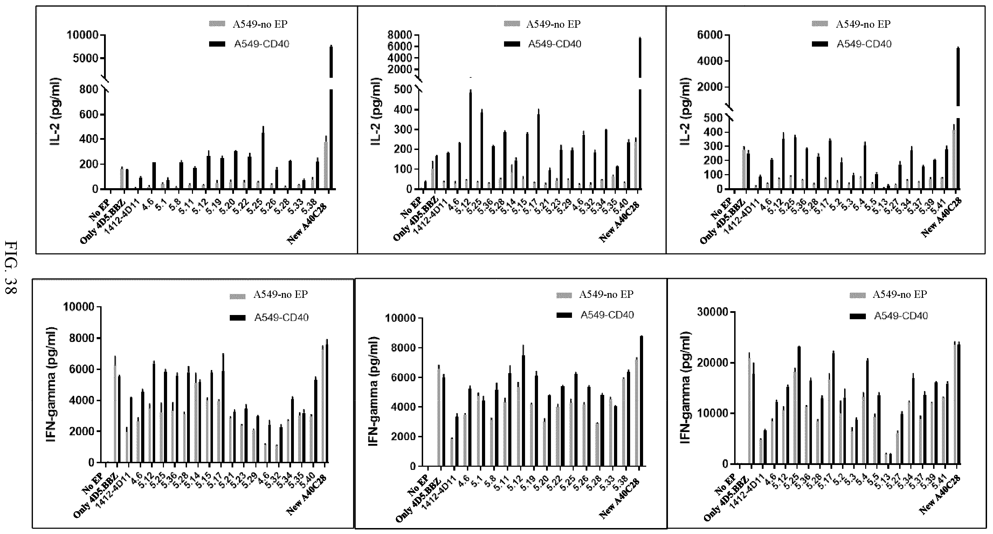

FIG. 38: the ELISA detection of IL-2 and IFN-gamma release levels in the case where target cells are under the action of Her2-CAR-T (with/without co-electroporated AM40.52-9.3h11 BsAbs).

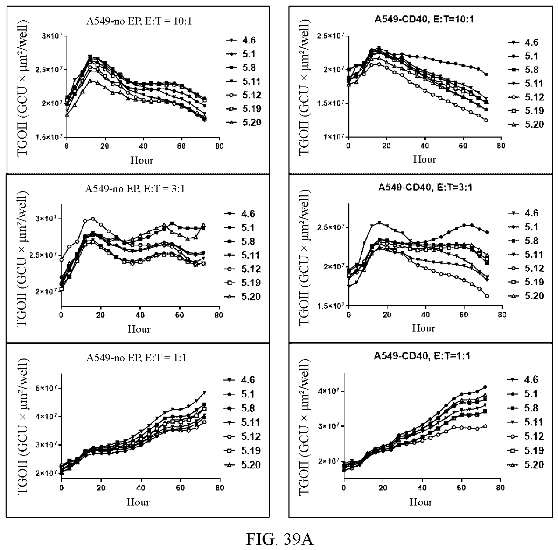

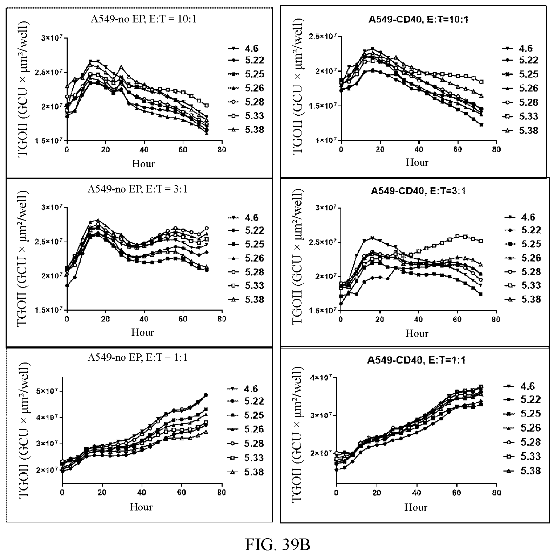

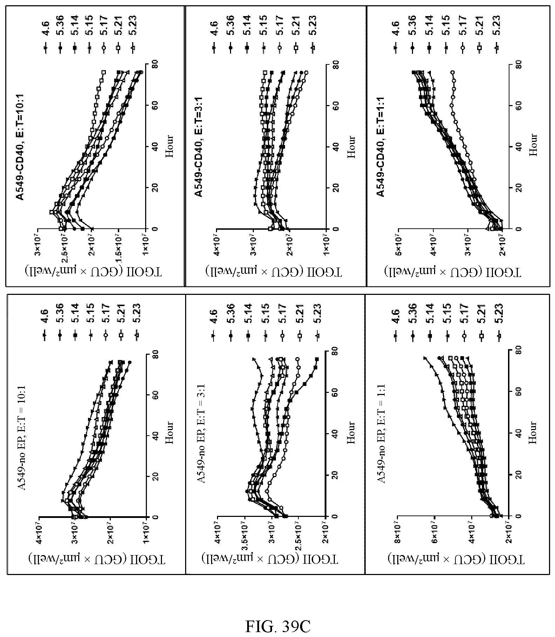

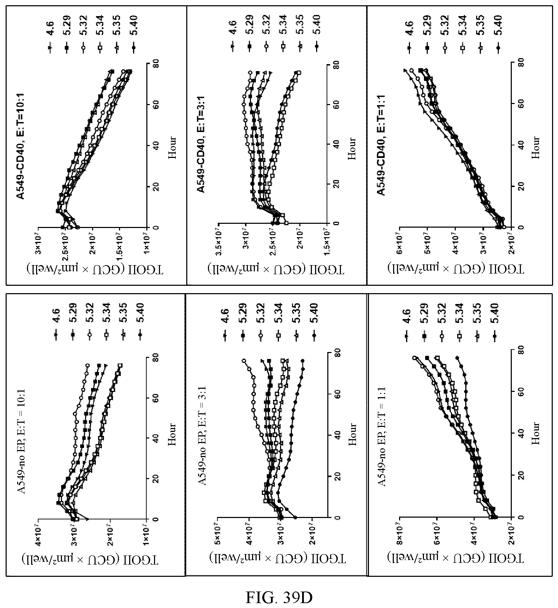

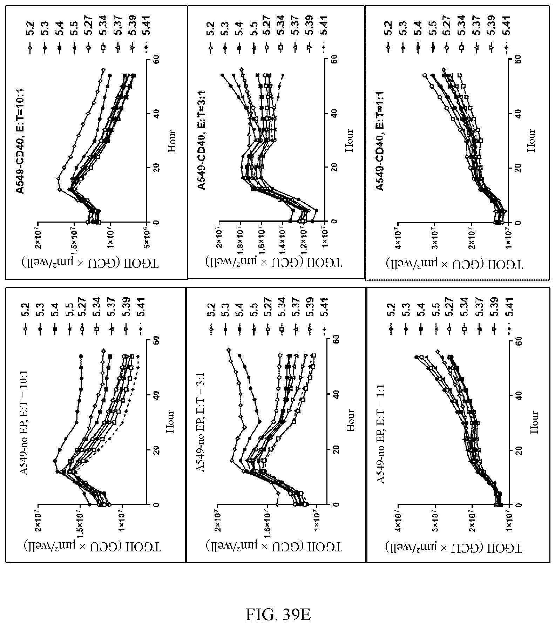

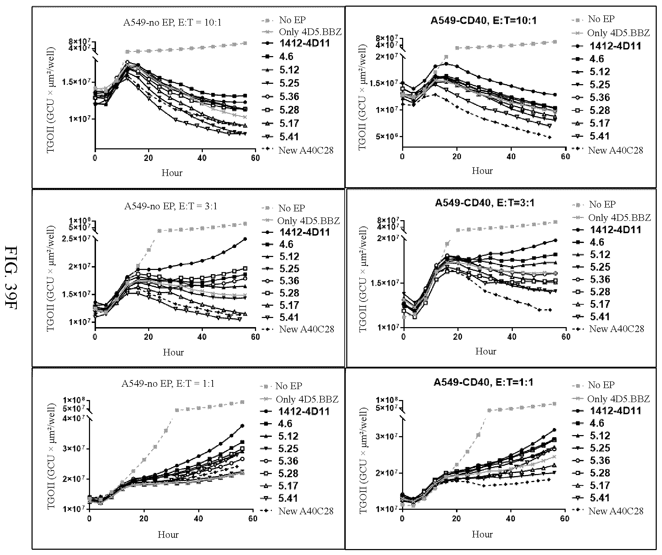

FIG. 39 (A-F): the killing-promoting effect of Her2-CAR-T (with/without co-electroporated AM40.52-9.3h11 BsAbs) on target cells detected by an incucyte S3 live cell dynamic imaging and analysis system.

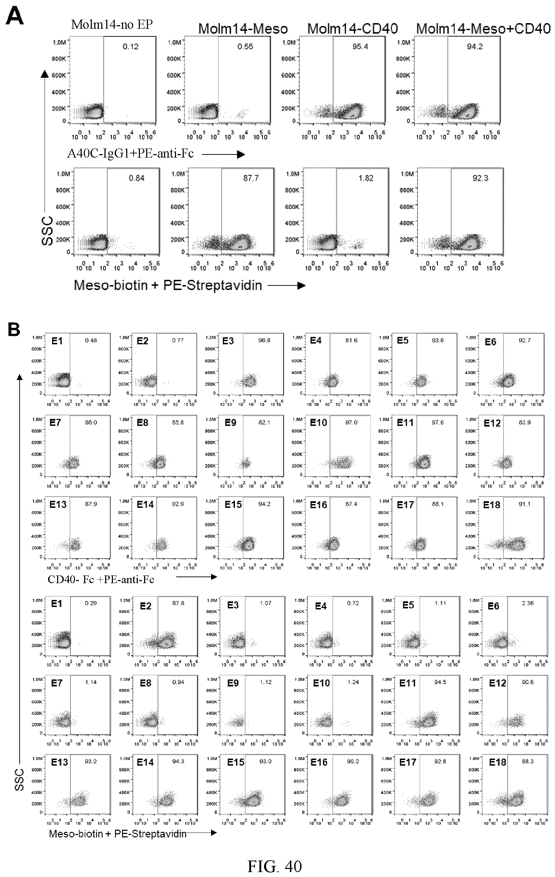

FIG. 40: the detection of gene overexpression levels. (A) Expression levels of Meso and CD40 on Molm14 cells. (B) Expression levels of Meso-CARs and AM40.52-9.3h11 diabodies.

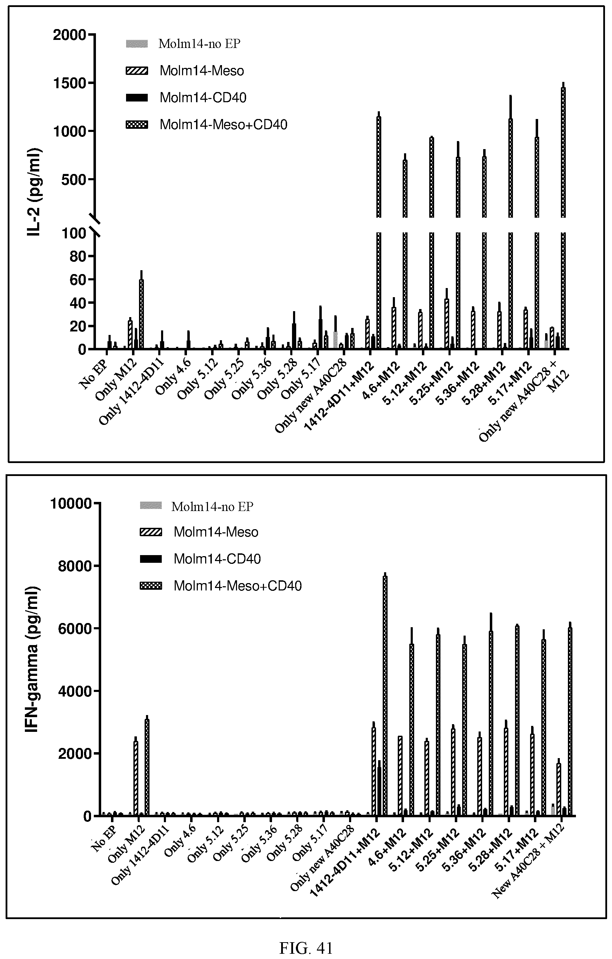

FIG. 41: the ELISA detection of IL-2 and IFN-gamma release levels in the case where target cells are under the action of Meso-CAR-T (with/without co-electroporated AM40.52-9.3h11 BsAbs).

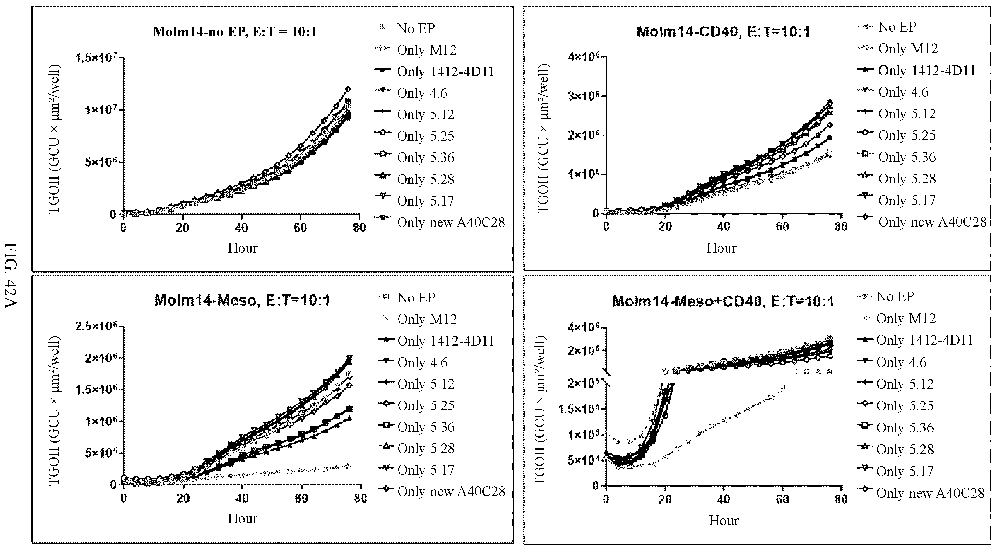

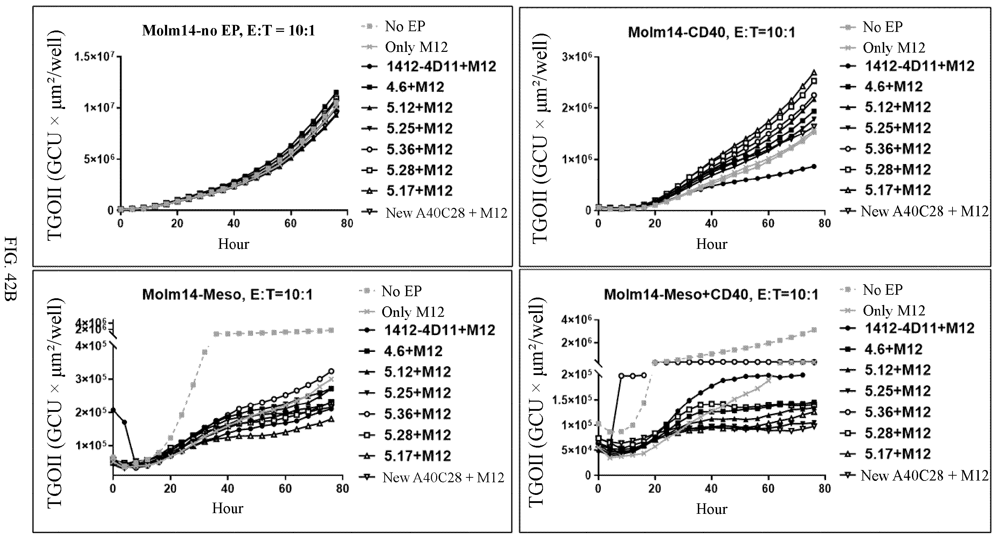

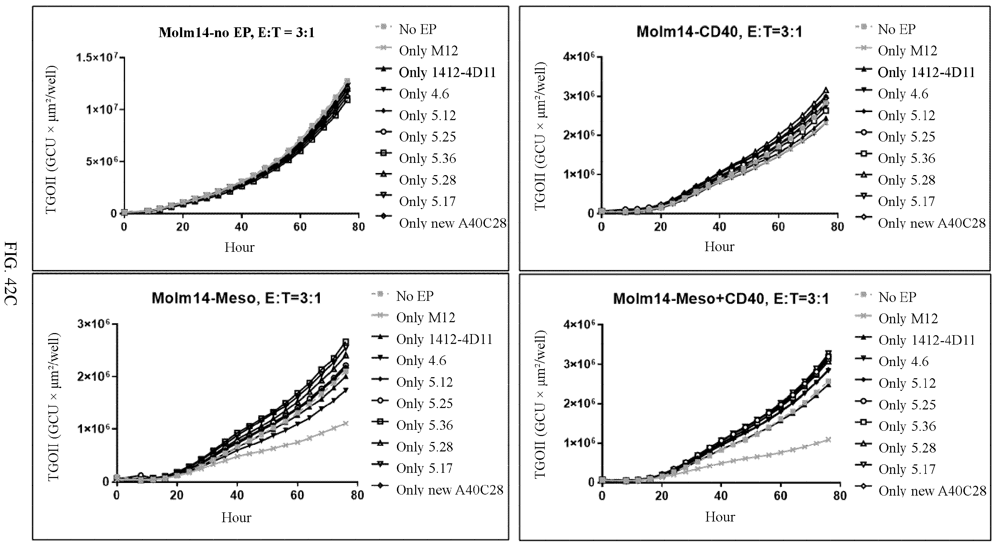

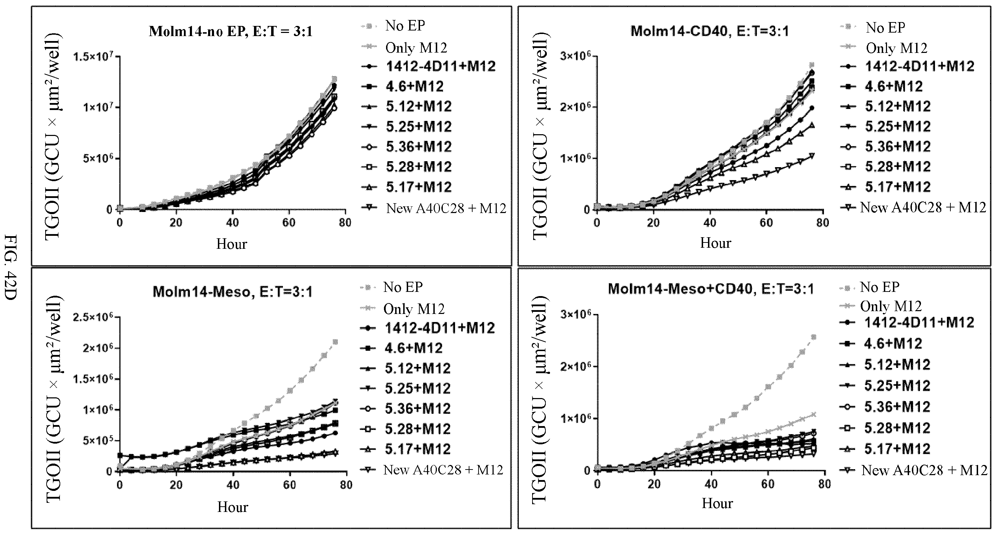

FIG. 42 (A-D): the killing-promoting effect of Meso-CAR-T (with/without co-electroporated AM40.52-9.3h11 BsAbs) on target cells detected by an incucyte S3 live cell dynamic imaging and analysis system.

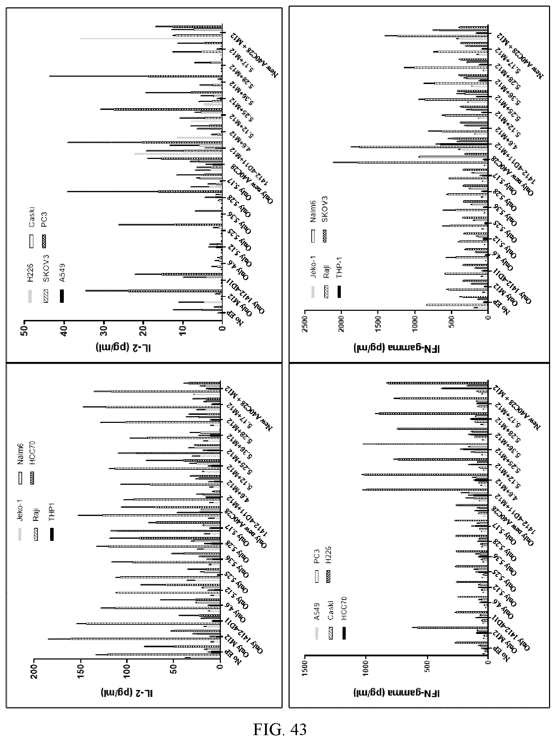

FIG. 43: the ELISA detection of IL-2 and IFN-gamma release levels in the case where target cells are under the action of Meso-CAR-T (with/without co-electroporated AM40.52-9.3h11 BsAbs).

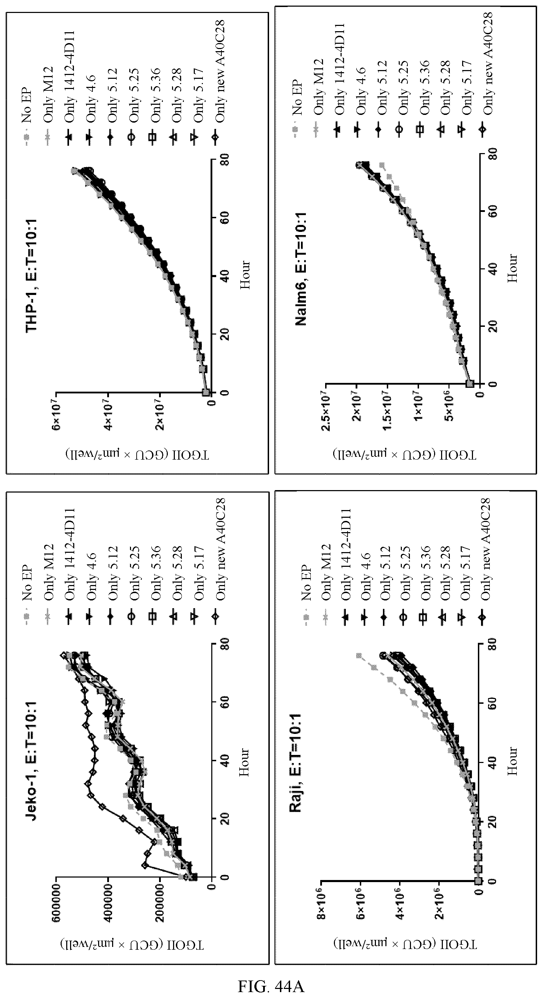

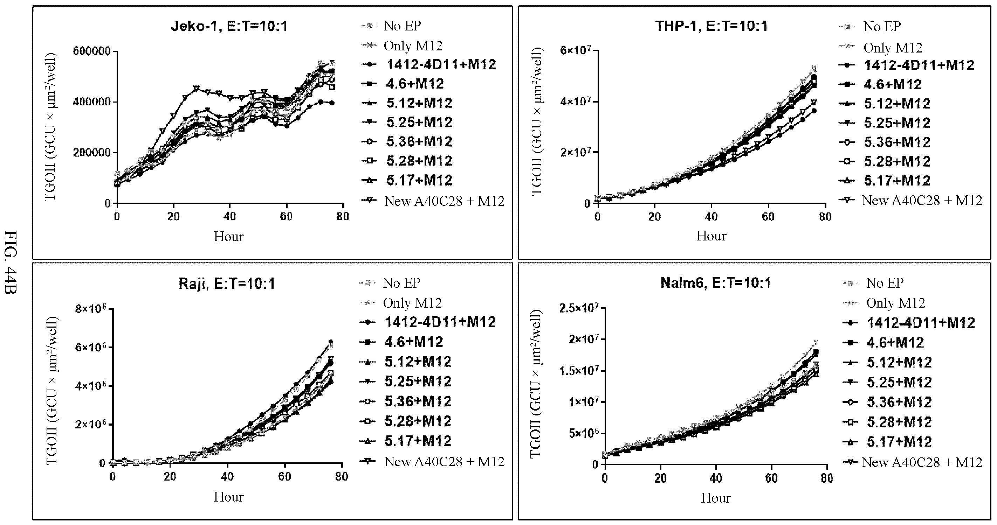

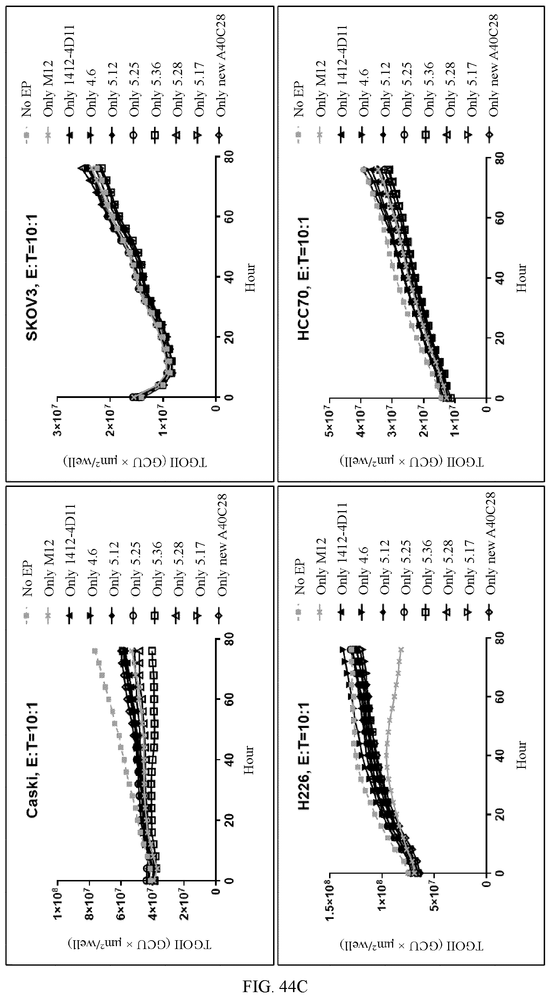

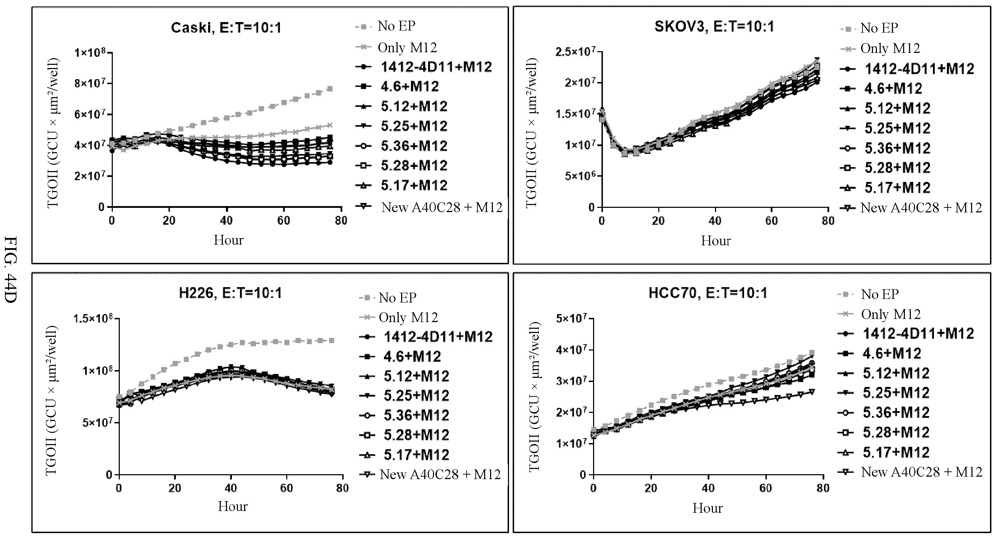

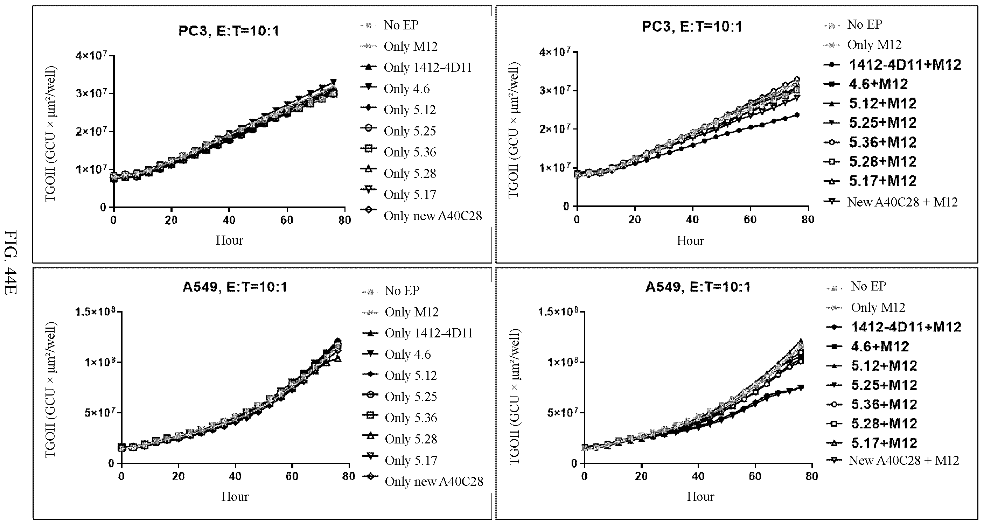

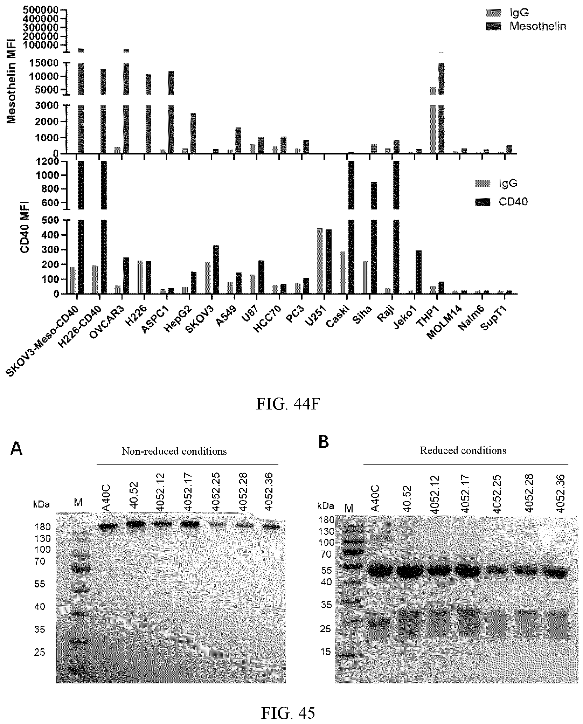

FIG. 44 (A-F): the specificity of diabodies detected by an incucyte S3 live cell dynamic imaging and analysis system and the expression abundance of Mesothelin and CD40 on the surface of different tumor cells (F).
FIG. 45: the SDS-PAGE profiles of affinity-matured antibodies under non-reduced (A) and reduced (B) conditions.

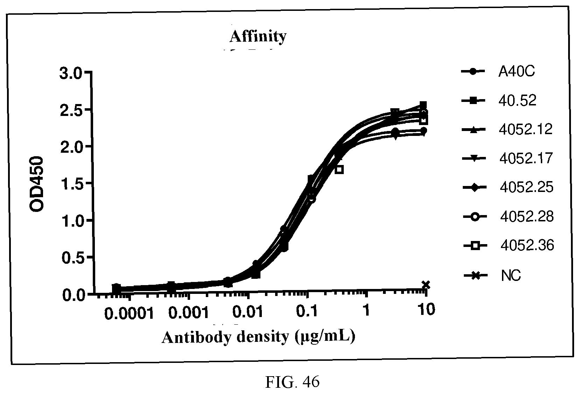

FIG. 46: the ELISA results of affinity-matured antibodies.

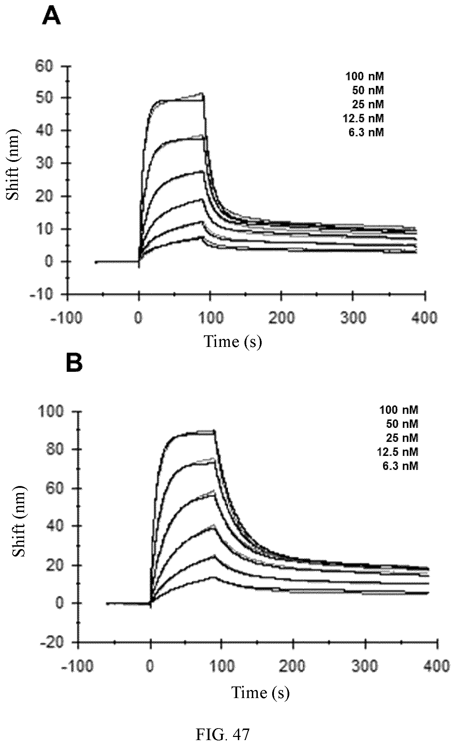

FIG. 47: the SPR fitting curves of the 40.52 antibody (A) and 4052.17 antibody (B).

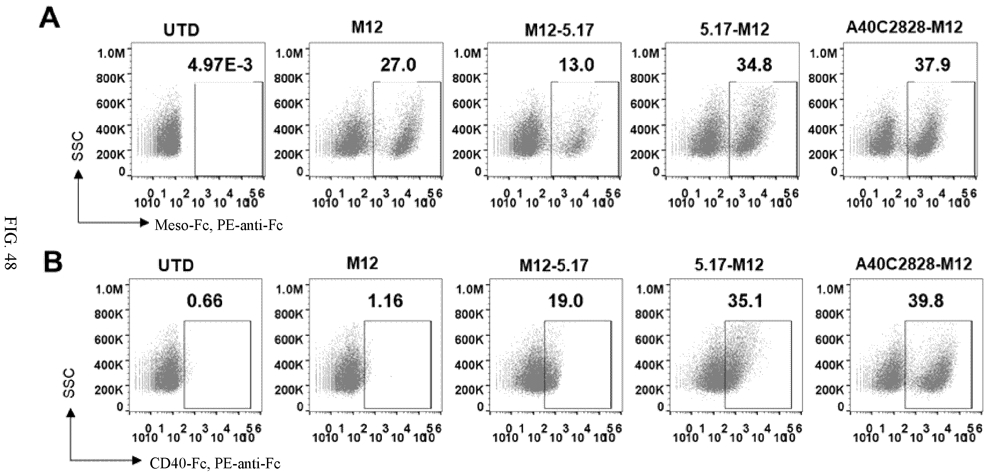

FIG. 48: the transduction efficiency of lentivirus in CAR-T cells.

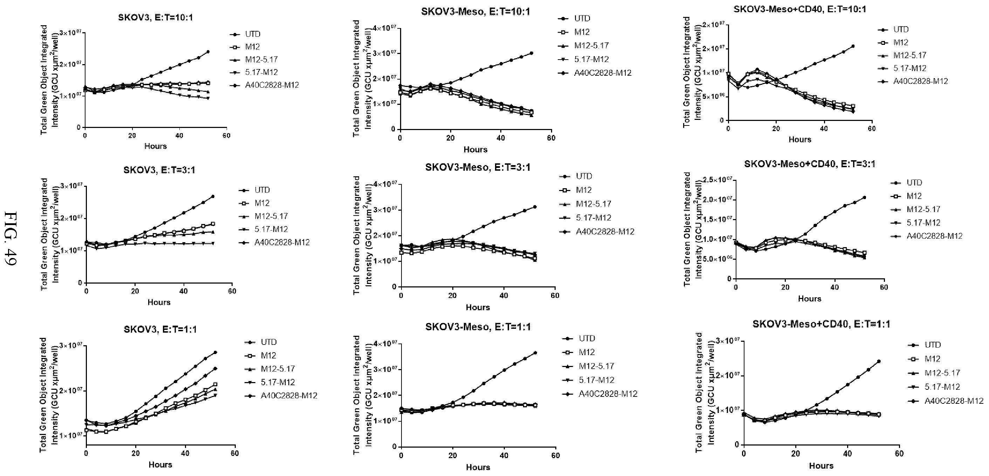

FIG. 49: the killing results of lentivirus-transduced CAR-T cells.

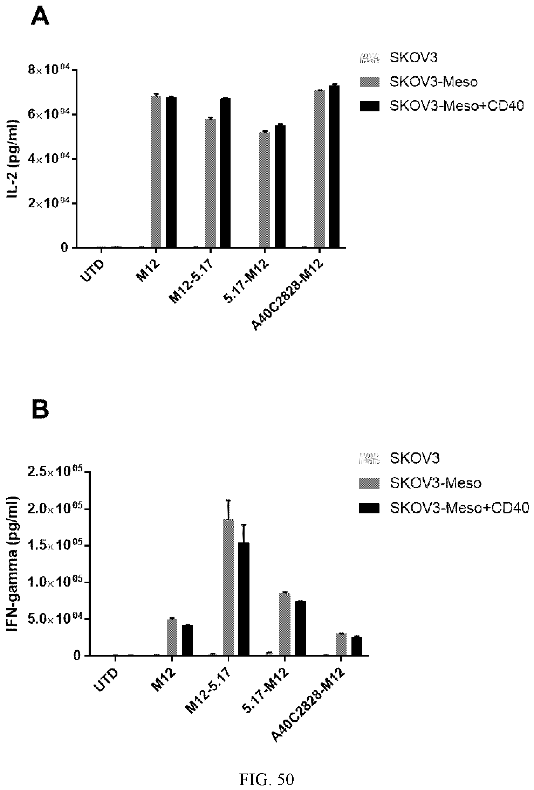

FIG. 50: the cytokines released by the lentivirus-transduced CAR-T cells.

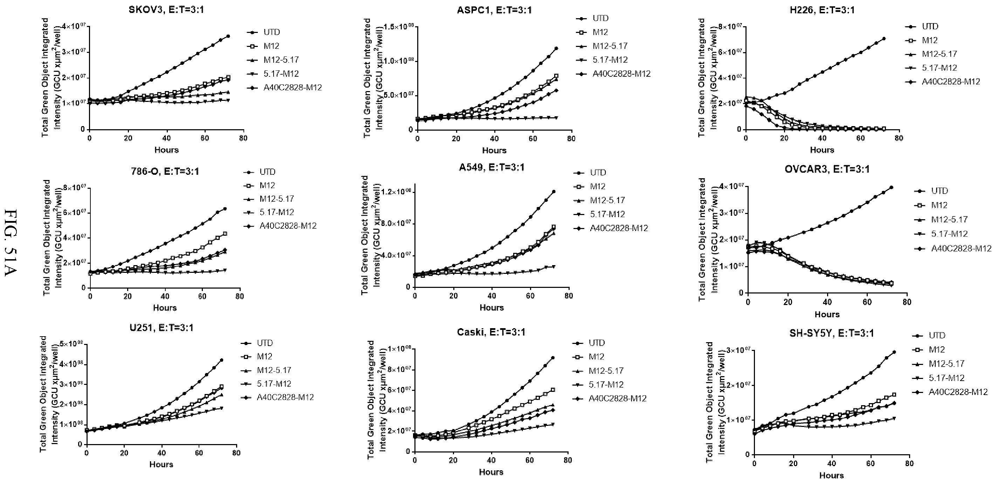

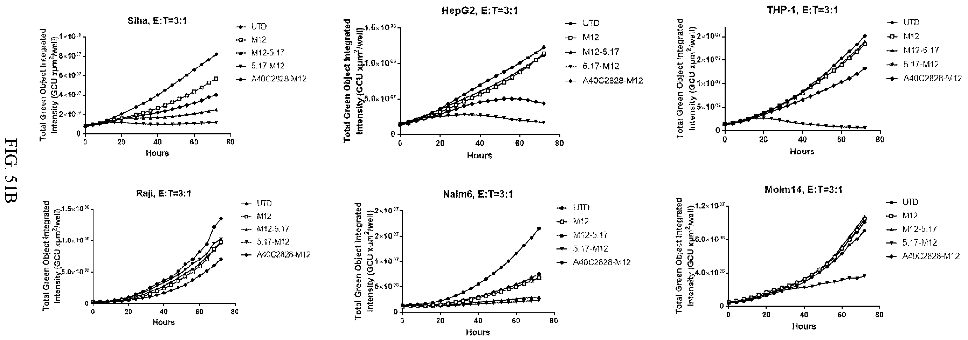

FIG. 51: the killing of different tumor cells by the lentivirus-transduced CAR-T cells.

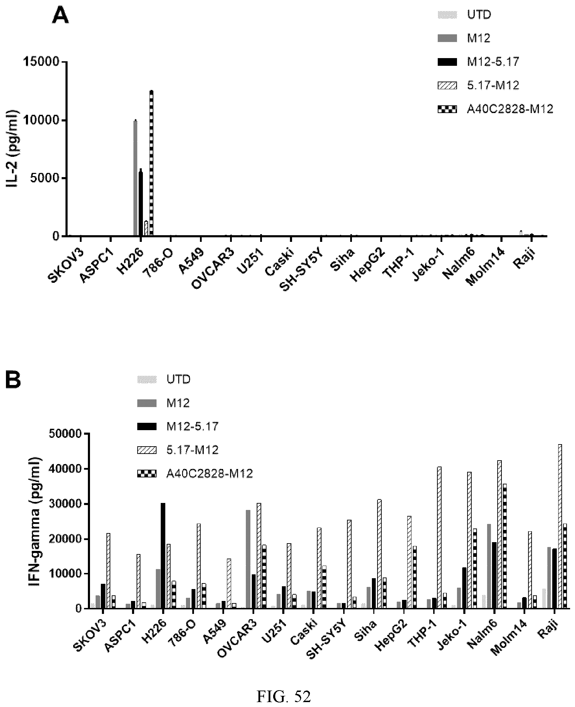

FIG. 52: the cytokines released by the lentivirus-transduced CAR-T cells for different tumor cells.

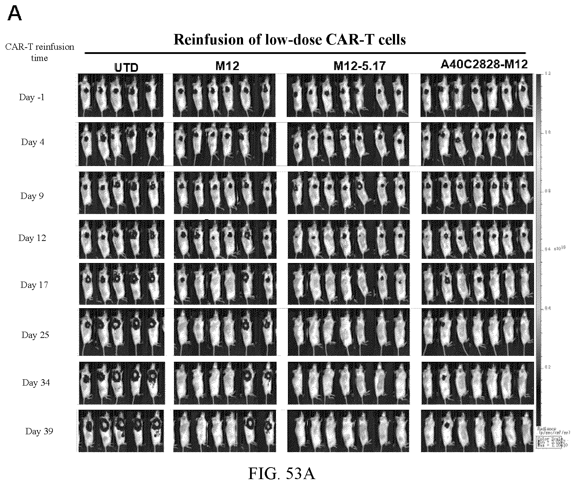

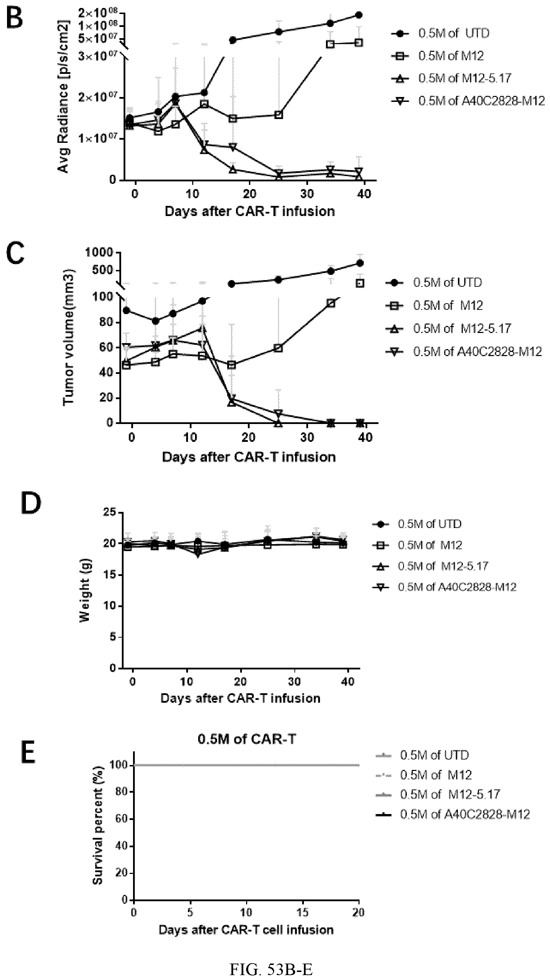

FIG. 53: the animal experiment results of low-dose CAR-T reinfusion. (A) Graphs of tumor quantification in mice, (B) Fluorescence values for tumors, (C) Mouse tumor size, (D) Mouse weight change, and (E) Mouse survival curve.

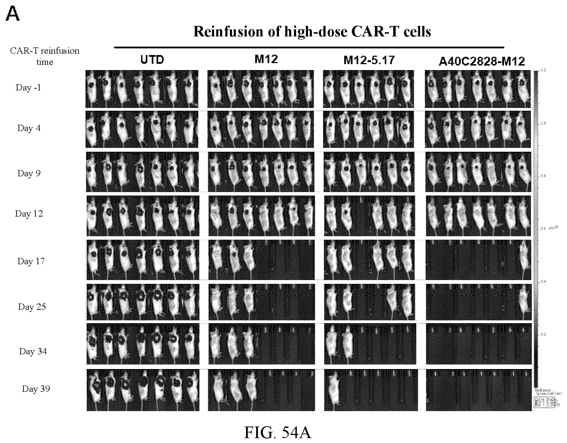

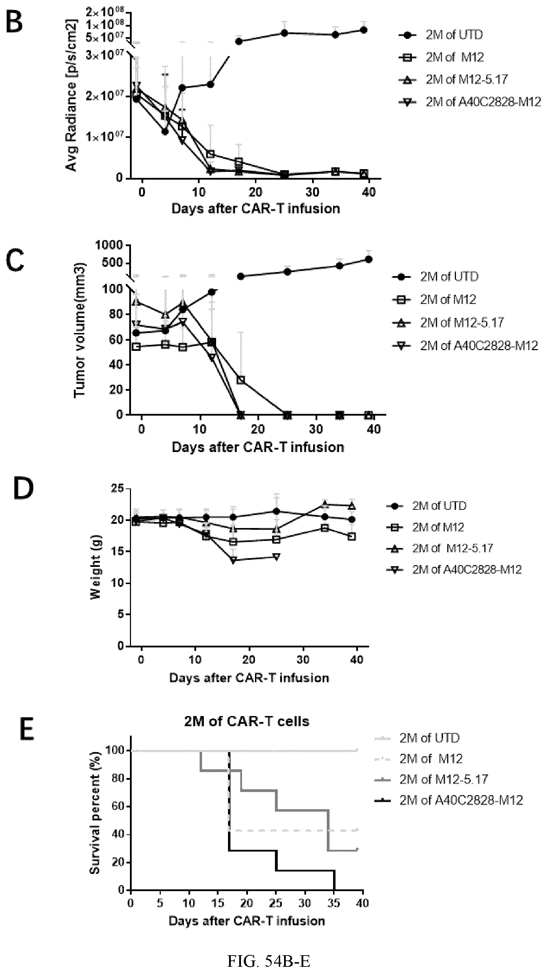

FIG. 54: the animal experiment results of high-dose CAR-T reinfusion. (A) Graphs of tumor quantification in mice, (B) Fluorescence values for tumors, (C) Mouse tumor size, (D) Mouse weight change, and (E) Mouse survival curve.

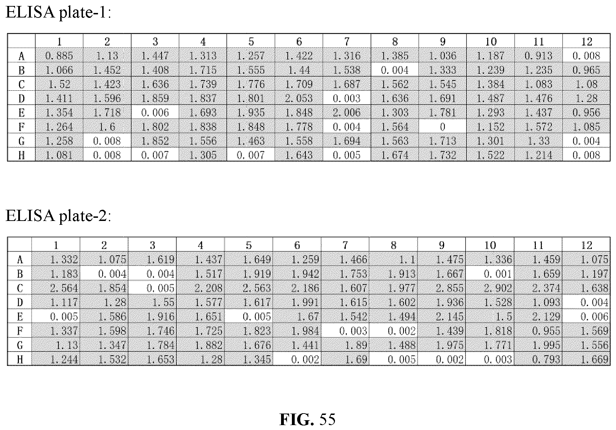

FIG 55 provides readings from two 96-well plates for anti-human mesothelin-Fc monoclonal phage ELISA.

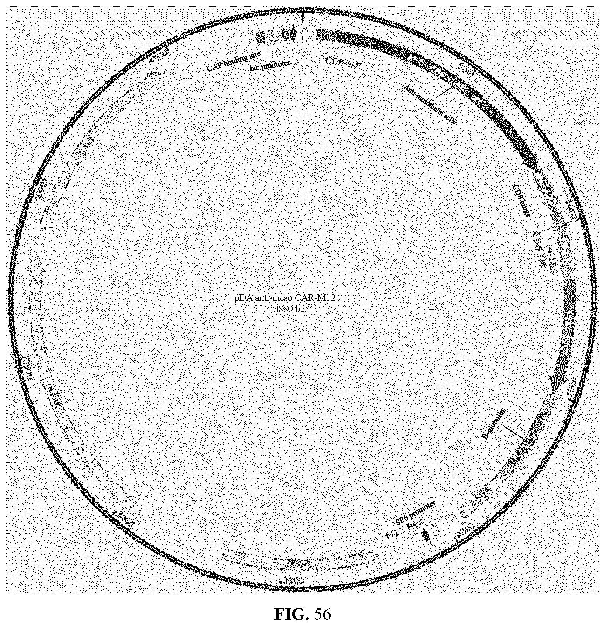

FIG. 56 provides a schematic diagram of a pDA-CAR vector for producing anti-mesothelin CAR mRNA.

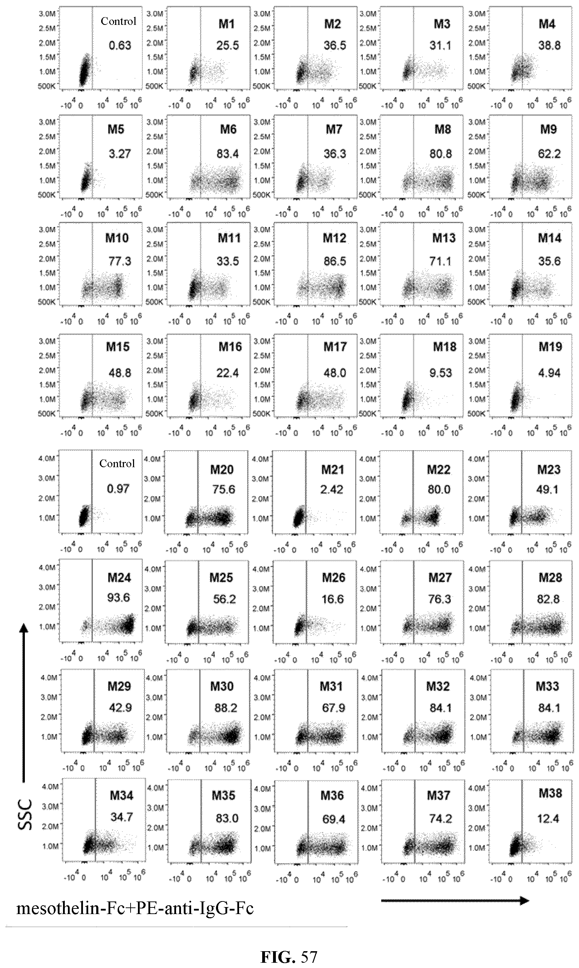

FIG. 57 provides FACS staining results showing the binding of anti-mesothelin scFv expressed in CART cells to mesothelin-Fc protein.

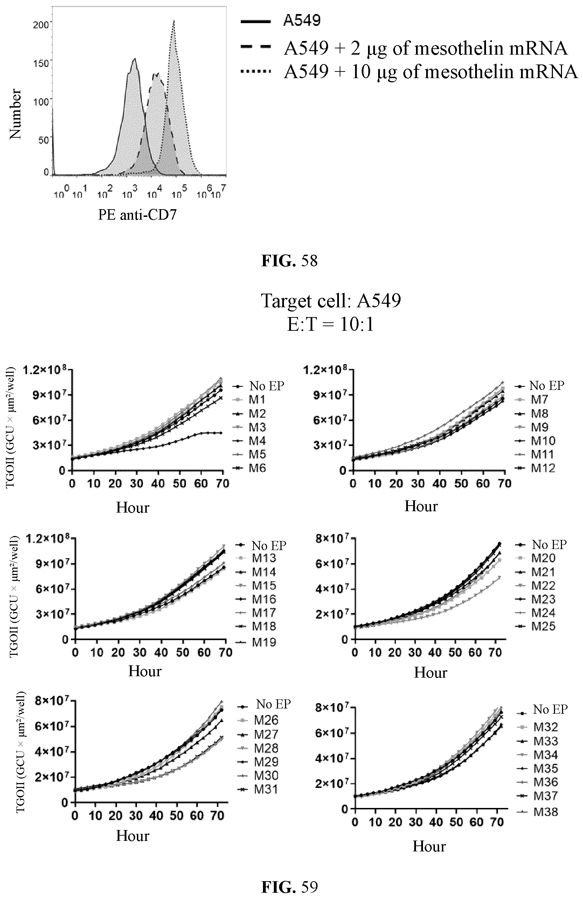

FIG. 58 provides the FACS staining of A549 cells with the anti-mesothelin antibody, where the A549 cells are electroporated with different amounts of mesothelin mRNA.
FIG. 59 provides the killing curves of different mRNA-based mesothelin CART cells for A549-GFP tumor cells at an E/T ratio of 10: 1.

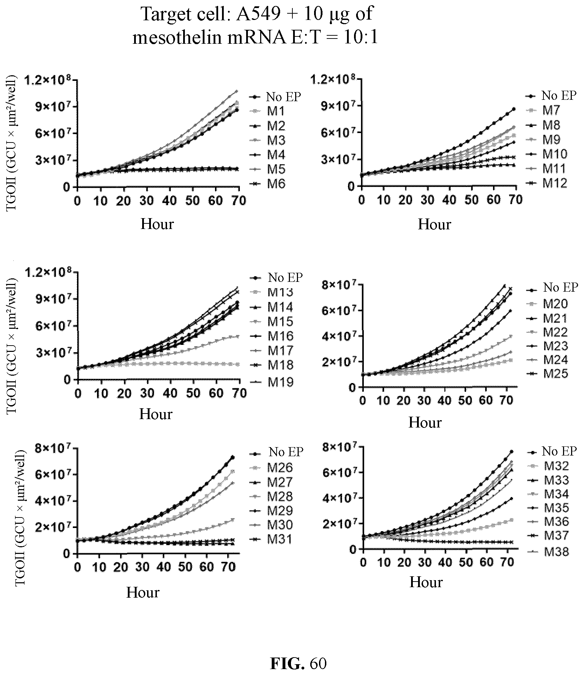

FIG. 60 provides the killing curves of different mRNA-based mesothelin CART cells for A549-GFP tumor cells at an E/T ratio of 10:1, where the A549-GFP tumor cells are electroporated with 10 µg of mesothelin mRNA.

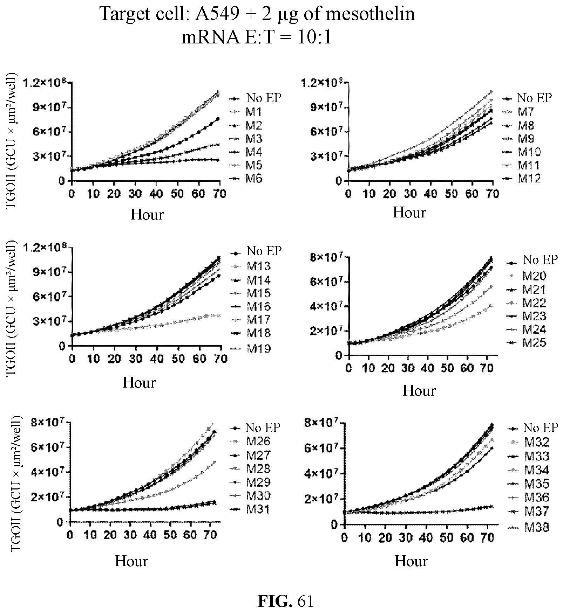

FIG. 61 provides the killing curves of different mRNA-based mesothelin CART cells for A549-GFP tumor cells at an E/T ratio of 10:1, where the A549-GFP tumor cells are electroporated with 2 µg of mesothelin mRNA.

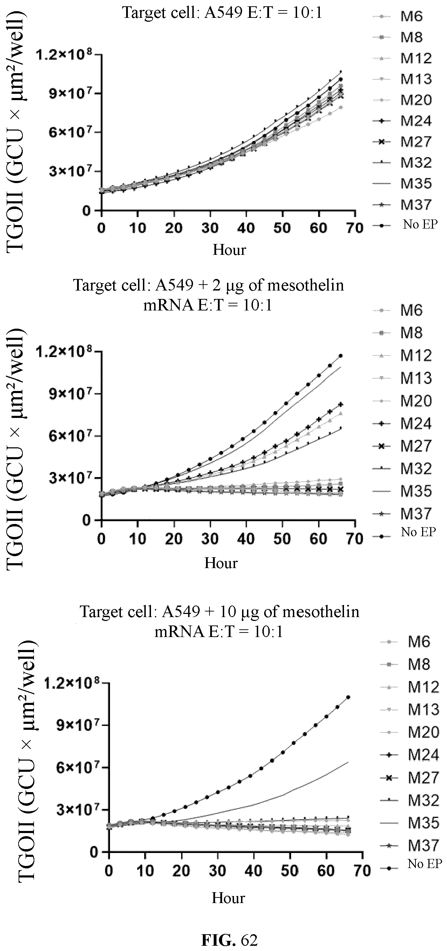

FIG. 62 provides the killing curves of different mRNA-based mesothelin CART cells for A549-GFP tumor cells at an E/T ratio of 10:1, where the A549-GFP tumor cells are electroporated with 0 µg, 2 µg, or 10 µg of mesothelin mRNA.

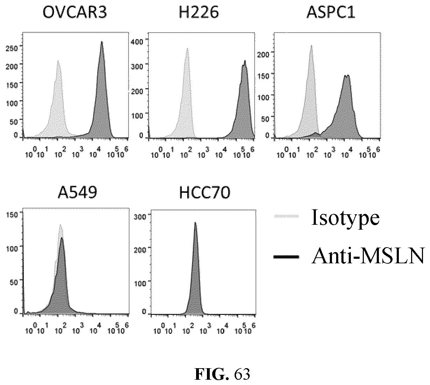

FIG. 63 provides the FACS staining of OVCAR3, H226, ASPC1, A549, and HCC70 with the isotype control and anti-mesothelin mAb.

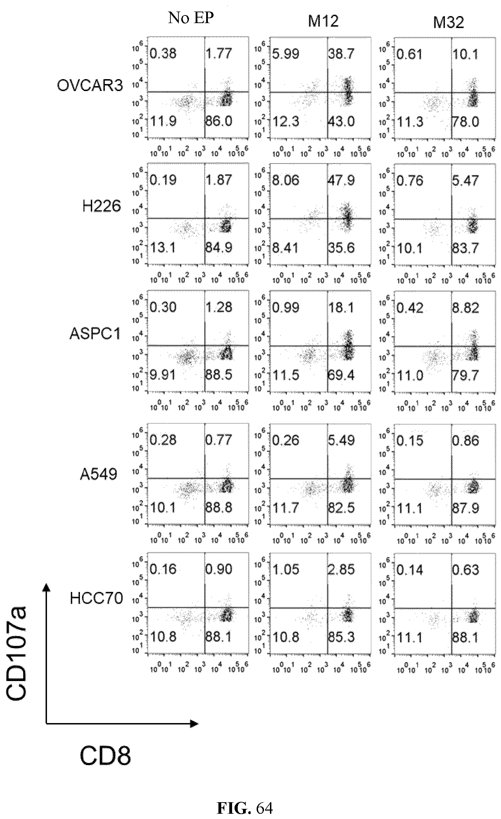

FIG. 64 provides the CD107a staining of anti-mesothelin M12 and M32 CART cells in the assay of coculturing with OVCAR3, H226, ASPC1, A549, and HCC70 tumor cell lines and killing.

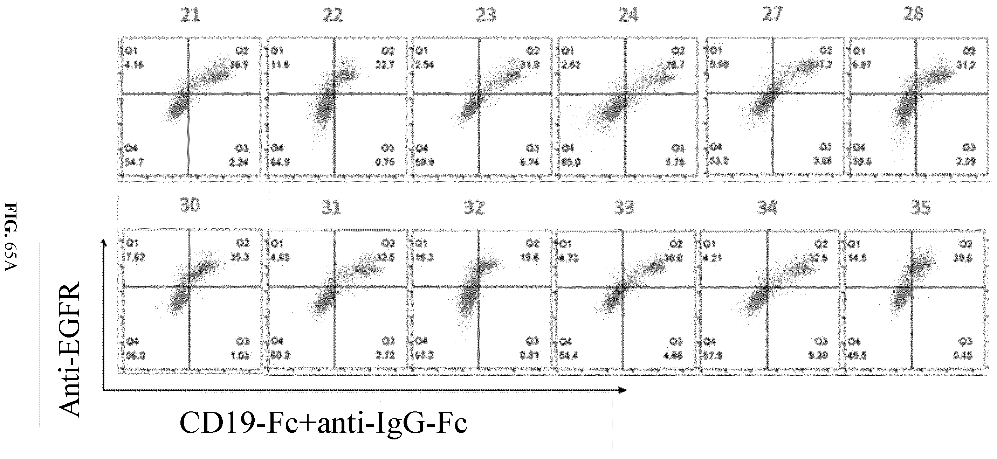

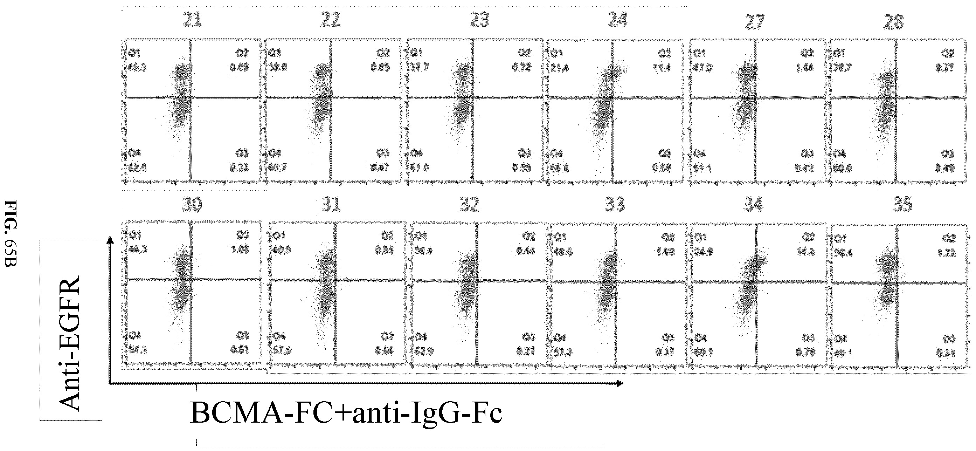

FIGs. 65A-65B provide the flow cytometry data for anti-BCMA CAR-T cells stained with CD19-Fc (FIG. 65A) or BCMA-Fc (FIG. 65B).

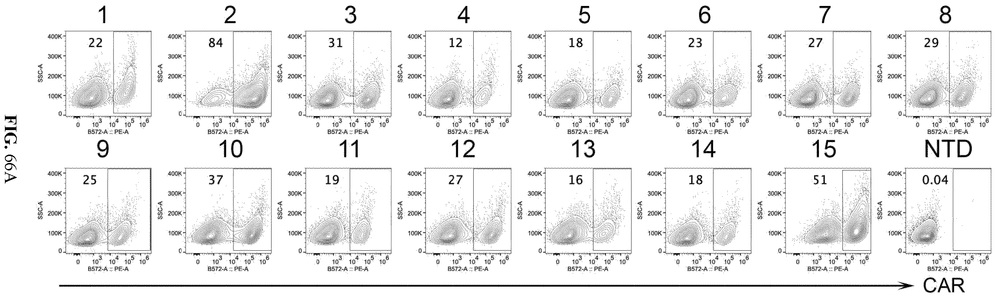

FIG. 66A provides the frequencies of CAR+ cells in T cells transduced with designated BCMA CARs.

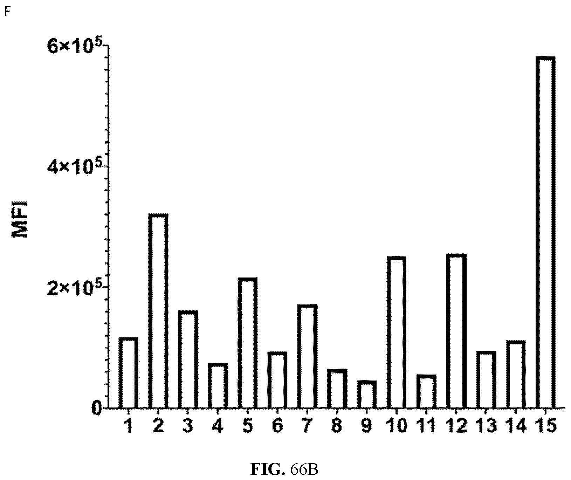

FIG. 66B provides the mean fluorescence intensity (MFI) of CAR expression in T cells transduced with designated BCMA CARs.

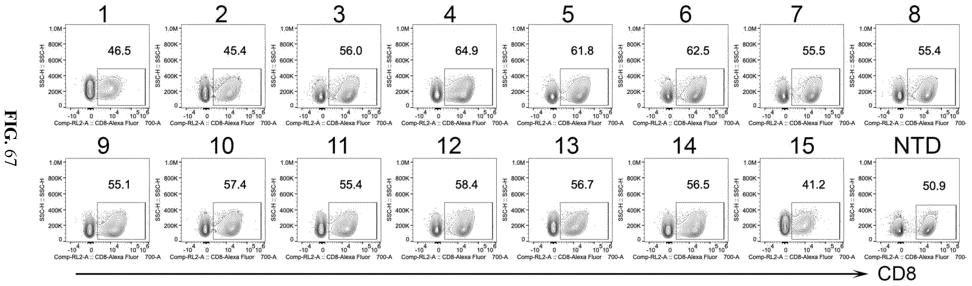

FIG. 67 provides the frequencies of CAR+CD8 cells in T cells transduced with designated BCMA CARs.

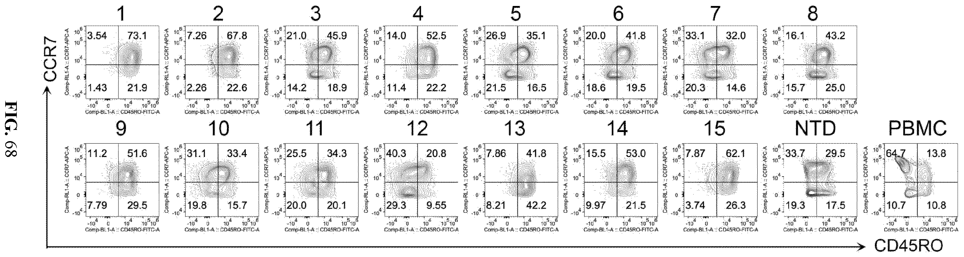

FIG. 68 provides phenotypes of designated CART cells characterized by CCR7 expression and CD45RO expression.

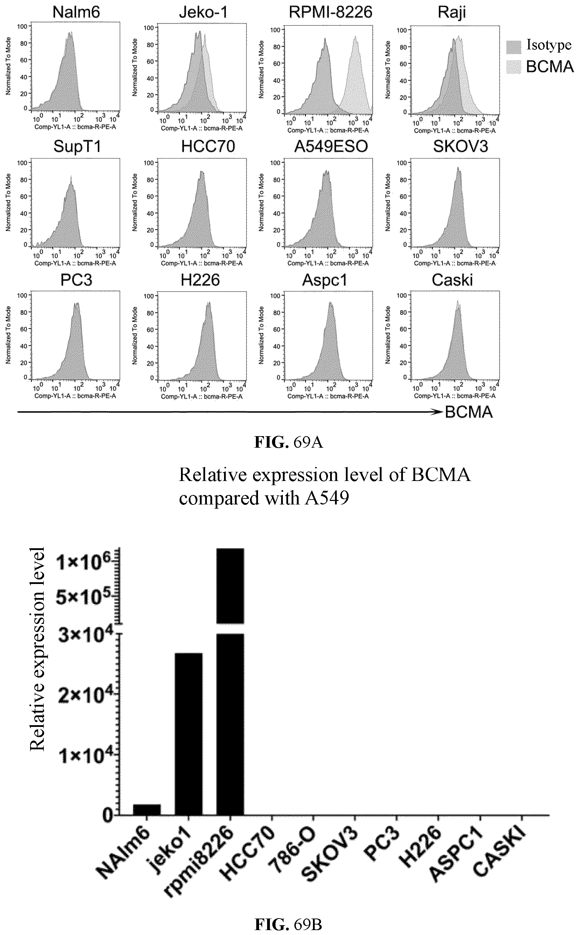

FIGs. 69A-69B provide expression of BCMA in tumor cell lines. FIG. 69A provides the FACS results. FIG. 69B provides the relative expression levels compared with A549 cells.

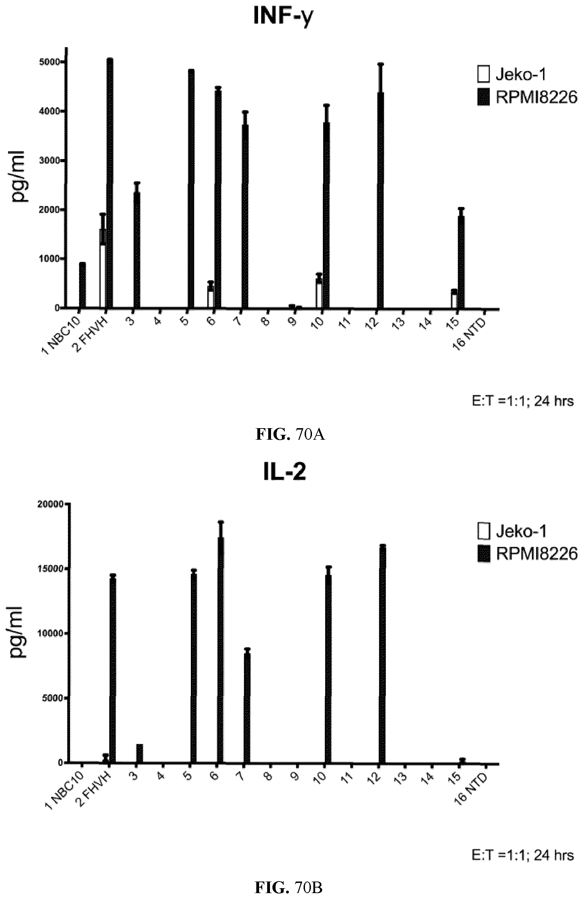

FIGs. 70A-70B provide ELISA results showing the production of INF-γ and IL-2 by designated CART cells. FIG. 70A shows the INF-γ production. FIG. 70B shows the IL-2 production.

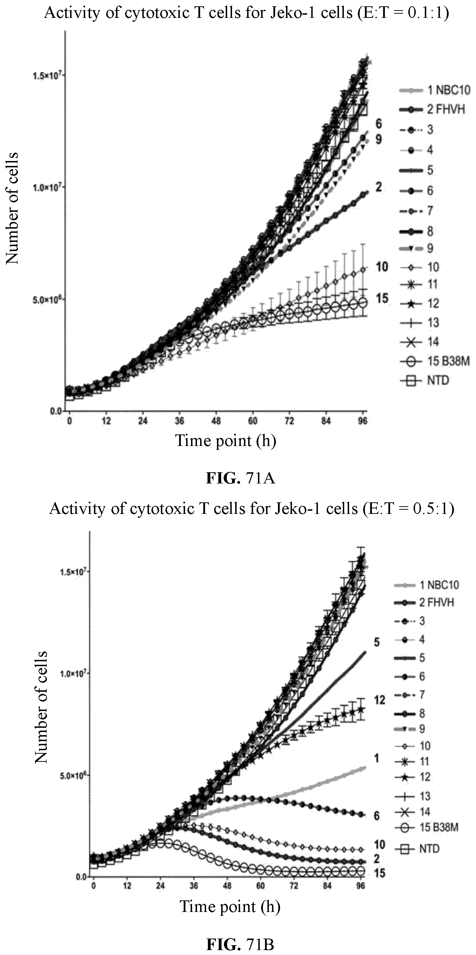

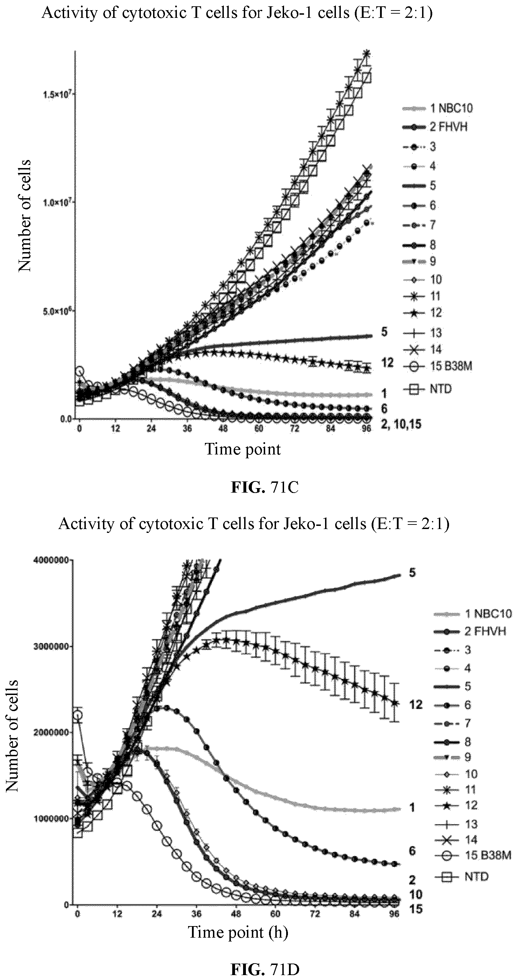

FIGs. 71A-71D provide the results of tumor killing assays, which show the cytolytic activities of designated CART cells against Jeko-1 cells at different ratios of E (T cell): T (tumor cell). FIG. 71A: E:T = 0.1:1; FIG. 71B: E:T = 0.5:1; FIG. 71C: E:T = 2:1; FIG. 71D: E:T = 2:1 (enlarged view).

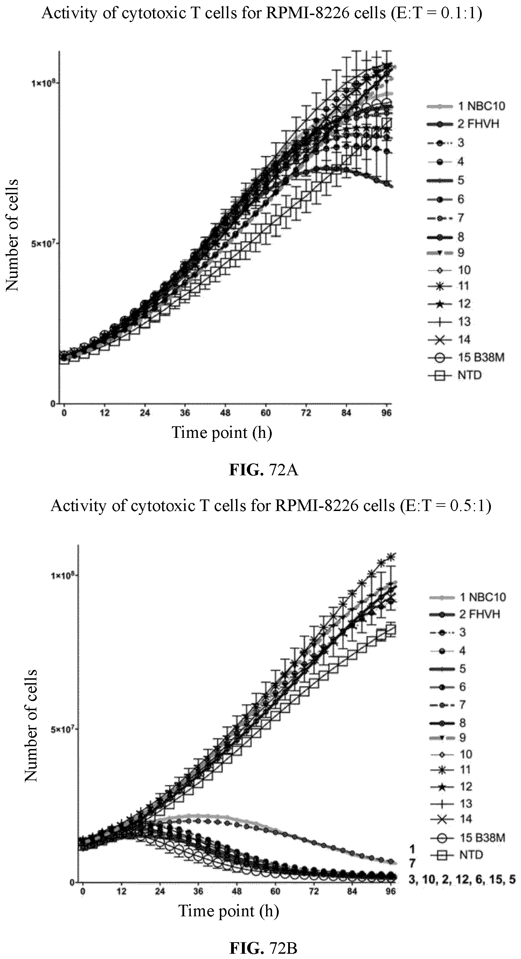

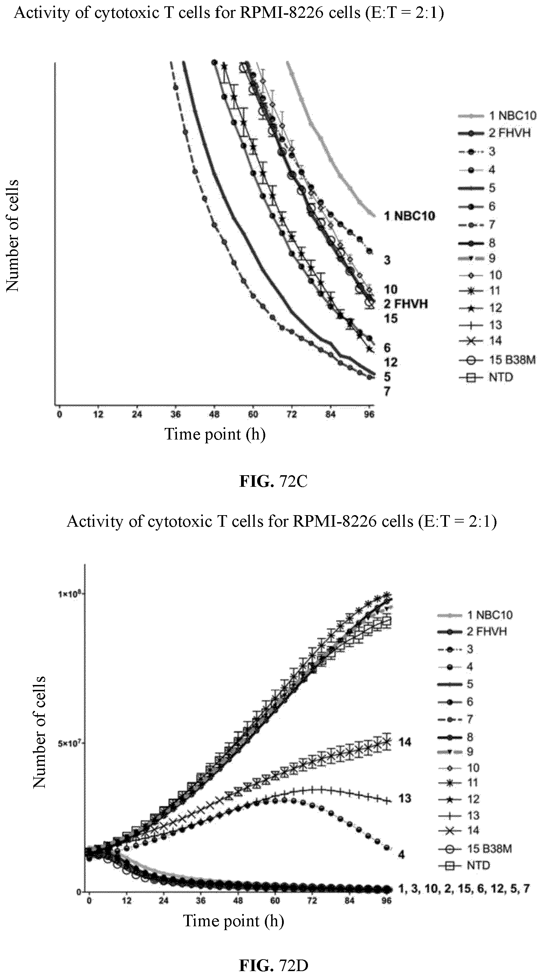

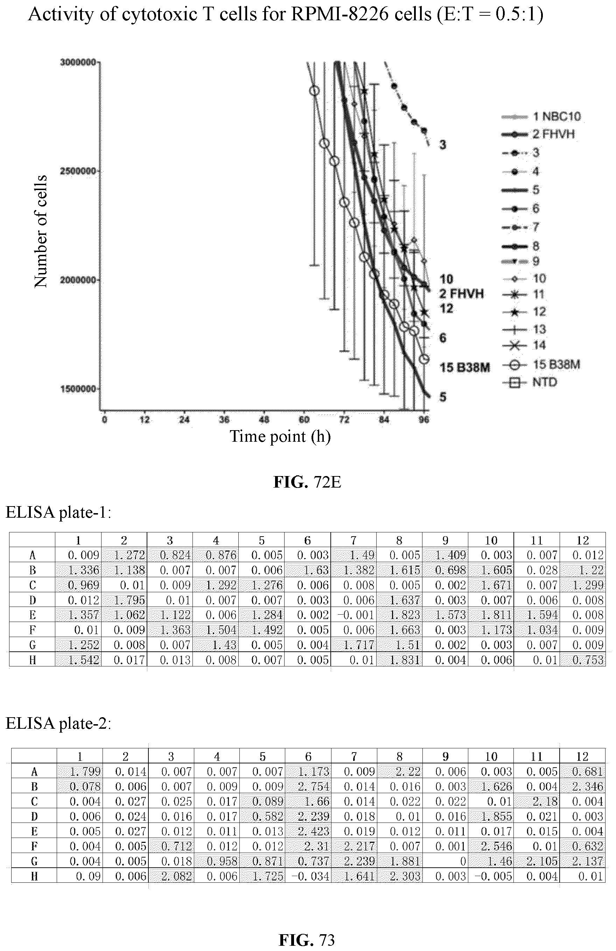

FIGs. 72A-72E provide the results of tumor killing assays, which show the cytolytic activities of designated CART cells against RPMI-8226 cells. FIG. 72A: E:T = 0.1:1; FIG. 72B: E:T = 0.5:1; FIG. 72C: E:T = 2:1 (enlarged view); FIG. 72D: E:T = 2:1; FIG. 72E: E:T = 0.5:1 (enlarged view).
FIG 73 provides readings from two 96-well plates for anti-human CD123-Fc monoclonal phage ELISA.

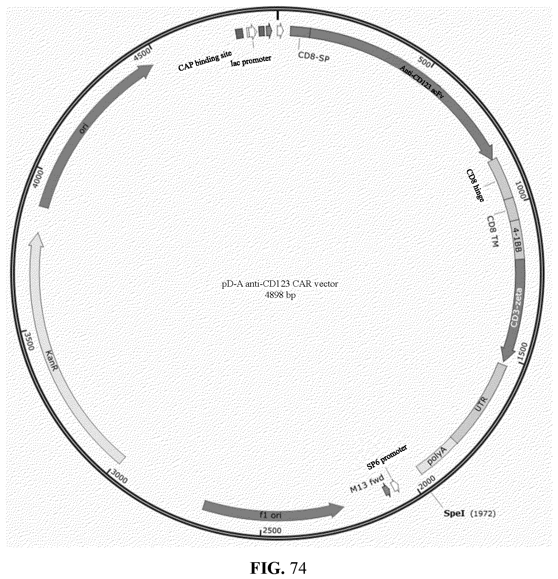

FIG. 74 provides a schematic diagram of a pDA-CAR vector for producing CAR mRNA.

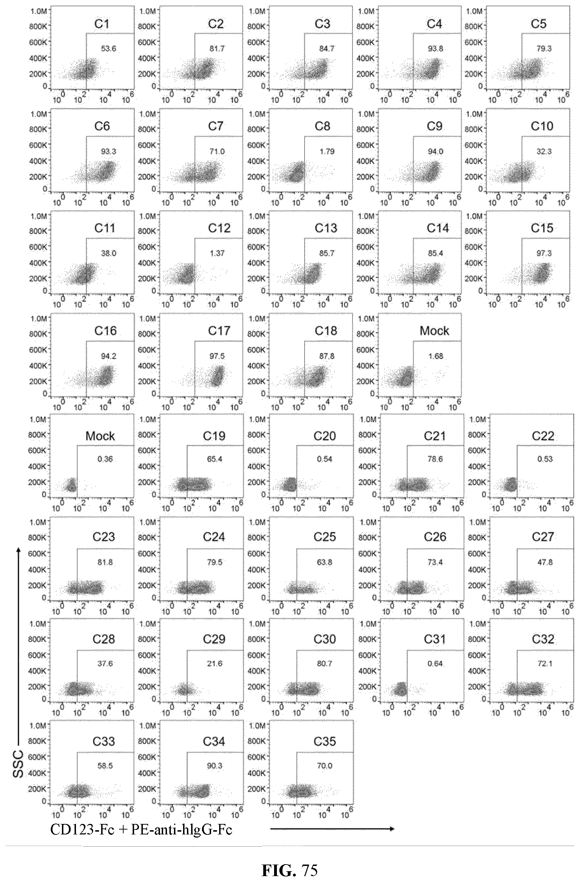

FIG. 75 provides the results of FACS staining, which show the binding of anti-CD123 scFv expressed in CART cells to CD123-Fc protein.

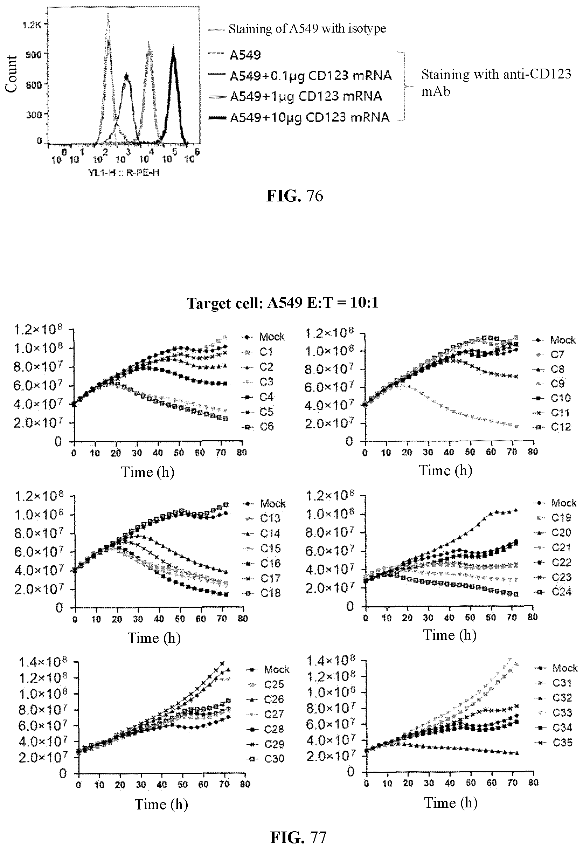

FIG. 76 provides the results of FACS staining of A549 cells electroporated with different amounts of CD123 mRNA with the isotype and anti-CD123 antibody.
FIG. 77 provides the killing curves of different mRNA-based anti-CD123 CART cells for A549-GFP tumor cells at an E/T ratio of 10:1.

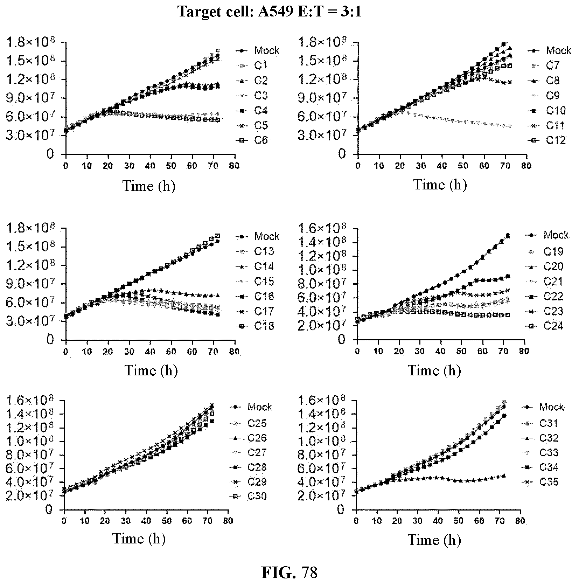

FIG. 78 provides the killing curves of different mRNA-based anti-CD123 CART cells for A549-GFP tumor cells at an E/T ratio of 3:1.

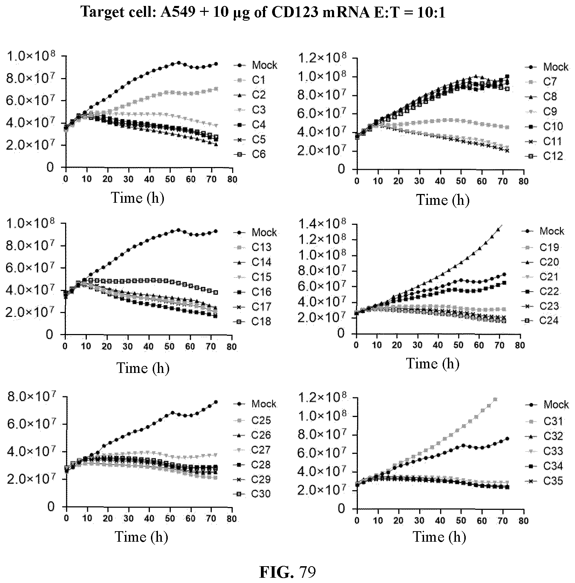

FIG. 79 provides the killing curves of different mRNA-based anti-CD123 CART cells for A549-GFP tumor cells electroporated with 10 µg of CD123 mRNA at an E/T ratio of 10:1.

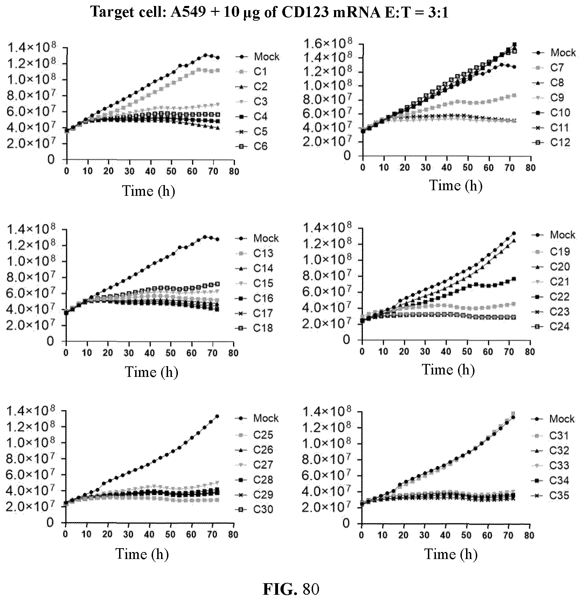

FIG. 80 provides the killing curves of different mRNA-based anti-CD123 CART cells for A549-GFP tumor cells electroporated with 10 µg of CD123 mRNA at an E/T ratio of 3:1.

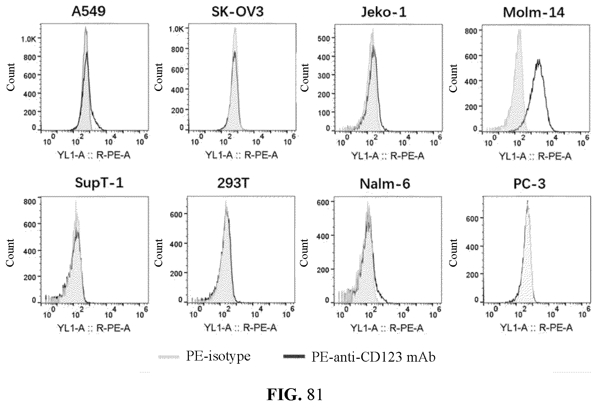

FIG. 81 shows the results of FACS staining of A549, SK-OV3, Jeko-1, Molm-14, SupT-1, 293T, Nalm-6, and PC-3 cells with the PE-isotype control and PE-anti-CD123 mAb.

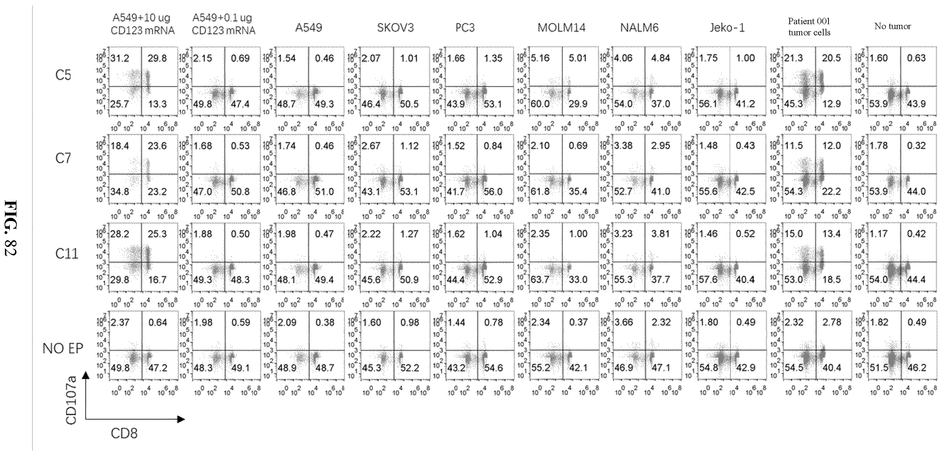

FIG. 82 shows the CD107a staining of anti-CD123-C5, anti-CD123-C7, and anti-CD123-C11 CART cells in the assay of coculturing with different tumor cells and killing.

### DETAILED DESCRIPTION

Unless otherwise indicated, the terms used in the present invention have the meanings commonly understood in the art and can be understood by referring to standard textbooks, reference books, and literature known to those skilled in the art. All publications mentioned herein are incorporated herein by reference in their entireties. It should be understood that the specific methods and materials described in the examples are for the purpose of describing particular embodiments only and are not intended to be limiting, and any methods and materials similar or equivalent to those described herein may be used in the practice or testing of the present invention. The explanation of the relevant theory or mechanism in the present invention is only for the understanding of the present invention and should not be construed as limiting the protected scheme of the present invention.

The terms "comprise," "comprising," or other terms forms having a meaning similar thereto such as "include" "contain" or "have", used herein should be construed as including elements that are listed, but not excluding the presence of other elements. These terms also include cases consisting only of the listed elements. The term "consist of" or "consisting of" refers to consisting only of the listed elements. The term "consist essentially of" or "consisting essentially of" refers to not excluding elements that do not significantly affect the solution involved.

Unless otherwise specifically stated, as used herein, the terms "a", "an", or "the" includes both singular and plural forms. The meaning of the term "at least one" or other similar expressions is equivalent to the meaning of "one or more".

The numerical value ranges described herein, such as temperature ranges, time ranges, composition or concentration ranges, or other numerical value ranges, include the end values within the ranges, all intermediate ranges and subranges (e.g., a range between certain intermediate value and certain end value), and all individual numerical values, particularly intermediate ranges and subranges with integer values as end values, and individual integer values. In addition, any intermediate ranges and subranges and all individual numerical values described in the numerical value ranges can be excluded from the numerical value ranges.

The "about" or "approximately" described herein means a range of ±10% of the stated value.

The "and/or" described herein should be construed as any one or a combination of the elements connected by the term.

As used herein, the terms "nucleic acid sequence" and "polynucleotide" are used interchangeably and refer to a sequence formed by linking bases, sugars, and a phosphate backbone. The nucleic acid sequence of the present invention may be a deoxyribonucleic acid sequence (DNA) or a ribonucleic acid sequence (RNA) and may comprise natural bases or unnatural bases. It may be single-stranded or double-stranded and may be a coding sequence or a non-coding sequence.

The terms "polypeptide" and "protein" are used interchangeably and refer to a polymer of amino acid residues. Such polymers may contain natural or unnatural amino acid residues and include, but are not limited to, peptides, oligopeptides, dimers, trimers, and multimers composed of amino acid residues. Full-length proteins and fragments thereof are encompassed by this definition. The term also includes polypeptides with post-expression modifications, such as glycosylation, sialylation, acetylation, phosphorylation, and similar modifications.

The term "isolated protein" or "isolated polypeptide" refers to a protein or polypeptide that: (1) is not associated with naturally associated components that accompany it in its native state; (2) is free of other proteins from the same species; (3) is expressed by a cell from a different species; or (4) does not occur in nature. A polypeptide that is chemically synthesized or synthesized in a cellular system different from the cell from which it naturally originates will be "isolated" from the components to which it naturally binds. A protein may also be rendered substantially free of the components to which it naturally binds by isolation, using protein purification techniques well known in the art. Proteins or polypeptides, such as antibodies or antigen-binding fragments thereof, referred to in the present invention are preferably isolated.

The term "isolated nucleic acid" refers to a nucleic acid molecule of genomic, cDNA, or synthetic origin, or a combination thereof, which is separated from other nucleic acid molecules present in the natural source of the nucleic acid. For example, with regard to genomic DNA, the term "isolated" includes nucleic acid molecules which are separated from the chromosome with which the genomic DNA is naturally associated.

Preferably, an "isolated" nucleic acid is free of sequences which naturally flank the nucleic acid (i.e., sequences located at the 5' and 3' ends of the nucleic acid of interest). The polynucleotides or nucleic acid sequences mentioned in the present invention are preferably isolated.

In the present invention, unless otherwise specified, the description of the nucleic acid sequence is usually from the 5' end to the 3' end, and the description of the amino acid sequence is usually from the N-terminus to the C-terminus.

The term "antigen" has the meaning commonly understood by those of ordinary skill in the art and includes polypeptides, polysaccharides, glycoproteins, polynucleotides, and the like. The term "antigenic epitope", which may also be called an "antigenic determinant", refers to a chemical functional group on the surface of an antigen that determines the specificity of the antigen. An antigenic epitope can be recognized and bound by an antigen binding site in an antibody.

The term "antibody" used herein is used in its broadest sense and includes immunoglobulins or other types of molecules that comprise one or more antigen-binding domains that specifically bind to an antigen, and it is a protein or polypeptide that exhibits binding specificity for a particular antigen. Specific examples of antibodies may include (e.g., classical four-chain antibody molecules), single-chain antibodies, single-domain antibodies, multispecific antibodies, and the like.

A complete antibody, which may also be referred to herein as a full-length antibody or a classical antibody molecule, is an immunoglobulin molecule composed of four polypeptide chains (e.g., IgG) or a multimer thereof (e.g., IgA or IgM). The four polypeptide chains comprise two identical heavy (H) chains and two identical light (L) chains, which are interconnected by disulfide bonds to form a tetramer. Each heavy chain consists of a heavy chain variable region ("HCVR" or "VH") and a heavy chain constant region (CH, including domains CH1, CH2, and CH3). Each light chain consists of a light chain variable region ("LCVR" or "VL") and a light chain constant region (CL). The constant regions of the heavy and light chains (CHs and CLs) are not directly involved in binding of the antibody to the antigen, but exhibit a variety of effector functions, such as being able to mediate binding of the antibody to host tissues or factors, including various cells of the immune system (e.g., effector cells) and the first component of the classical complement system (C1q). The variable regions of the heavy and light chains (VH and VL) form the antigen binding sites, and the VH and VL each have hypervariable regions called complementarity determining regions (CDRs), which have a relatively high degree of variation in amino acid composition and arrangement order and are key positions for the binding of an antibody to an antigen, with more conserved sequences called framework regions (FRs) interposed therebetween. Each of VH and VL consists of three CDRs and four FRs, arranged from the amino terminus to the carboxyl terminus in the following order: FR1, CDR1, FR2, CDR2, FR3, CDR3, FR4. Herein, the three heavy chain complementarity determining regions are referred to as HCDR1 (CDR-H1), HCDR2 (CDR-H2), and HCDR3 (CDR-H3), and the four heavy chain framework regions are referred to as HFR1 (FR-H1), HFR2 (FR-H2), HFR3 (FR-H3), and HFR4 (FR-H4); the three light chain complementarity determining regions are referred to as LCDR1 (CDR-L1), LCDR2 (CDR-L2), and LCDR3 (CDR-L3), and the four light chain framework regions are referred to as LFR1 (FR-L1), LFR2 (FR-L2), LFR3 (FR-L3), and LFR4 (FR-L4). The variable regions (VH and VL) of the heavy chain and the light chain form antigen binding sites.

The term "complementarity determining region" or "CDR" used herein refers to the amino acid residues in the variable regions of an antibody that are responsible for antigen binding. The precise boundaries of CDRs may be defined according to various numbering systems known in the art, e.g., according to the Kabat numbering system (Kabat et al., Sequences of Proteins of Immunological Interest, 5th Ed. Public Health Service, National Institutes of Health, Bethesda, Md., 1991), Chothia numbering system (Chothia & Lesk (1987) J. Mol. Biol. 196:901-917; Chothia et al., (1989) Nature 342:878-883), or IMGT numbering system (Lefranc et al., Dev. Comparat. Immunol. 27:55-77, 2003). For a given antibody, those skilled in the art will readily identify the CDRs defined by the numbering systems. In addition, the correspondence between different numbering systems is well known to those skilled in the art (see, e.g., Lefranc et al., Dev. Comparat. Immunol. 27:55-77, 2003). In the examples of the present invention and in the description of specific sequences, the CDRs are defined using the Kabat, Chothia, AbM, Contact, or IMGT numbering system, but it should be understood that CDRs of antibodies in the present invention may also be defined using other numbering systems.

The term "framework region" or "FR" used herein refers to those amino acid sequences in the variable regions of an antibody except the CDR sequences defined above.

Antibodies may be divided into five major distinct classes, namely IgA, IgD, IgE, IgG, and IgM, based on the amino acid sequence of the heavy chain constant region of the antibody. These antibody types may be further divided into subclasses according to the size of the hinge region, the position of interchain disulfide bonds, and the difference in molecular weight, e.g., IgGl, IgG2a, IgG2b, IgG3, and IgG4. Light chains may be divided into two classes, namely κ and λ, according to the amino acid composition and arrangement of the light chain constant region of the antibody. Antibodies of the present invention include antibodies of any of the aforementioned classes or subclasses.

The term "monoclonal antibody" used herein refers to a homogeneous antibody directed against only a particular epitope of an antigen. In contrast to common polyclonal antibody formulations that typically comprise different antibodies directed against different epitopes, each monoclonal antibody is directed against a single epitope on the antigen. Although monoclonal antibodies may be obtained by hybridoma culturing, in the present invention, the modifier "monoclonal" indicates the homogeneous property of the antibody and should not be construed as requiring production of the antibody by any particular method. The monoclonal antibodies of the present invention may be produced by recombinant DNA methods or obtained by other screening methods.

The term "antigen-binding fragment" used herein refers to one or more portions of a complete antibody that retain the ability to specifically bind to an epitope against which the antibody is directed, see, e.g., Fundamental Immunology, Ch.7 (Paul,W., ed., 2th Edition, Raven Press, N.Y. (1989)). Examples of the antigen-binding fragment include: (1) a Fab fragment: an antigen-binding fragment produced by digesting a complete antibody with papain and consisting of a complete light chain (comprising a VL domain and a CL domain) of a full-length antibody and a heavy chain variable region VH and a heavy chain constant region domain CH1 of the full-length antibody, without a hinge region; (2) a F(ab')₂ fragment: an antigen-binding fragment produced by digesting a complete antibody with pepsin and comprising complete light chains (comprising a VL domain and a CL domain) of two full-length antibodies and heavy chain variable regions VH, heavy chain constant region domains CH1 and hinge regions of the two full-length antibodies, which may be regarded as a fragment formed by pairing two Fab' fragments having a disulfide bond therebetween; (3) a Fab' fragment that may be formed by cleavage of the disulfide bond between the F(ab')₂ hinge regions by treatment with a reducing agent; (4) an Fd fragment composed of a VH domain and a CH1 domain; and (5) an Fv fragment composed of a VL domain and a VH domain of a single arm of an antibody. The term "antigen-binding fragment" generally comprises at least one or both of a heavy chain variable region and a light chain variable region of a complete antibody and may also comprise a portion or the entirety of a light chain constant region and/or a heavy chain constant region of the complete antibody, e.g., may comprise one or two or more of a CL domain, a CH1 domain, a CH2 domain, and a CH3 domain.

The term "Fc region" used herein refers to the C-terminal region of an immunoglobulin heavy chain, i.e., the fragment crystallizable (Fc) that constitutes the handle region of a Y-shaped classical antibody molecule, including the second and third constant domains of the heavy chain (CH2 and CH3 domains). The Fc region can be obtained by hydrolysis of a complete antibody molecule using papain. In some examples, the Fc region may comprise a hinge, a CH2, and a CH3. Dimerization between two Fc-containing polypeptides may be mediated when the Fc region comprises a hinge. The Fc fragment may be derived from IgG, IgM, IgD, IgE, or IgA. In some examples, the Fc region is derived from IgG1, IgG2, IgG3, or IgG4. The "Fc fragment" also includes variant Fc fragments derived from a native Fc fragment, modified but retaining its effector functions. A "variant Fc fragment" comprises an amino acid sequence having at least one amino acid substitution relative to the amino acid sequence of the native Fc fragment. In some examples, the variant Fc fragment has at least one amino acid substitution as compared to the parent Fc fragment (native Fc fragment), e.g., about 1 to about 10 amino acids are substituted, and preferably about 1 to about 5 amino acid substitutions are present. In some examples, the variant Fc fragment Fc region has at least about 80% sequence identity, at least about 90% sequence identity, at least about 95% sequence identity, at least about 96% sequence identity, at least about 97% sequence identity, at least about 98% sequence identity, or at least about 99% sequence identity to the parent Fc fragment. Effector functions of the "Fc fragment" may include binding to Fc receptors, Clq binding and complement dependent cytotoxicity (CDC), antibody-dependent cell-mediated cytotoxicity (ADCC), mediating phagocytosis, and the like.

The term "single-domain antibody (sdAb)" used herein refers to an antigen-binding polypeptide having a single variable domain and comprising three complementarity determining regions (CDRs). A single-domain antibody is capable of binding to an antigen alone without pairing with another polypeptide comprising a CDR. Common single-domain antibodies comprise a heavy chain but lack the light chain normally found in antibodies, such as camelidae sdAbs (see e.g., Hamers-Casterman et al., Nature 363:446-8 (1993); Greenberg et al., Nature 374:168-73 (1995); Hassanzadeh-Ghassabeh et al., Nanomedicine (Lond), 8:1013-26 (2013)). A single-domain antibody may be artificially engineered from a camelidae heavy chain antibody, which is called V_{H}H and has the following structure from the N-terminus to the C-terminus: FR1-CDR1-FR2-CDR2-FR3-CDR3-FR4. A single-domain antibody may also be considered as a particular "antigen-binding fragment" of a full-length antibody.

The term "single-chain antibody (scFv)" used herein refers to a single polypeptide chain comprising VL and VH domains, wherein the VL and VH are connected by a linker (see, e.g., Bird et al., Science 242:423-426 (1988); Huston et al., Proc. Natl. Acad. Sci. USA 85:5879-5883 (1988); and Pluckthun, The Pharmacology of Monoclonal Antibodies, 113, Roseburg & Moore, Springer-Verlag, New York, 269-315 (1994)). Such scFv molecules may have a general structure of NH₂-VL-linker-VH-COOH or NH₂-VH-linker-VL-COOH. An appropriate linker may consist of repeated GGGGS amino acid sequence or a variant thereof. For example, a linker having the amino acid sequence (GGGGS)₄ or variants thereof may be used (Holliger et al., (1993), Proc. Natl. Acad. Sci. USA 90:6444-6448). In some cases, a disulfide bond may also be present between the VH and VL of the scFv. As used herein, the term "di-scFv" refers to an antibody fragment formed by connecting two scFvs.

As known to those skilled in the art, antibodies may be prepared by a variety of techniques, such as the hybridoma technique (see, e.g., Kohler et al., Nature, 256:495, 1975), the recombinant DNA technique (see, e.g., U.S. Patent Application No. 4,816,567), or the phage antibody library technique (see, e.g., Clackson et al., Nature, 352:624-628, 1991, or Marks et al., J. Mol. Biol. 222:581-597, 1991).

As known to those skilled in the art, antibodies may be purified by well-known techniques, such as affinity chromatography using protein A or protein G. Subsequently or alternatively, the specific antigen (the target molecule recognized by the antibody) or an epitope thereof may be immobilized on the column and the immunospecific antibody is purified by the immunoaffinity chromatography. For purification of antibodies, reference may be made to e.g., D.Wilkinson The Scientist, published by The Scientist, Inc., Philadelphia PA (Vol.14, No.8 (Apr. 17, 2000), pp.25-28).

The term "chimeric antibody" used herein refers to an antibody in which a portion of the light chain or/and heavy chain is derived from one antibody (which may be derived from a particular species or belong to a particular antibody class or subclass) and another portion of the light chain or/and heavy chain is derived from another antibody (which may be derived from the same or a different species or belong to the same or a different antibody class or subclass), but which still retains binding activity for a target antigen (Morrison et al., Proc. Natl. Acad. Sci. USA, 81:6851-6855(1984)). The term "chimeric antibody" may refer to an antibody comprising heavy and light chain variable region sequences from one species and constant region sequences from another species, e.g., an antibody having human constant regions and murine heavy and light chain variable regions.

The term "human antibody" used herein refers to an antibody in which the entire sequence (e.g., variable regions and constant regions) of the antibody is derived from a human immunoglobulin. "Non-human antibody" refers to an antibody in which the entire sequence (e.g., variable regions and constant regions) of the antibody is derived from an immunoglobulin of a non-human species; for example, an antibody derived from a murine immunoglobulin may be referred to as a murine antibody.

The term "humanized antibody" used herein refers to a chimeric antibody obtained by engineering a non-human antibody, e.g., an antibody having variable regions and constant regions (if any) that are not derived from a human immunoglobulin, to allow it to have increased homology to a human antibody sequence. Generally, all or part of the CDR regions of a humanized antibody are derived from a non-human antibody (donor antibody), and all or part of the non-CDR regions (e.g., variable region FRs and/or constant regions) are derived from a human immunoglobulin (receptor antibody). The humanized antibody generally retains the desired properties of the donor antibody, including but not limited to, antigen specificity, affinity, reactivity, ability to increase the activity of immune cells, ability to enhance the immune response, and the like. The donor antibody may be a mouse, rat, rabbit, or non-human primate (e.g., cynomolgus monkey) antibody having a desired property (e.g., antigen specificity, affinity, reactivity, ability to increase the activity of immune cells, and/or ability to enhance the immune response). Humanized antibodies are particularly advantageous in that they can retain the desired properties of non-human donor antibodies (e.g., a murine antibody) and effectively reduce the immunogenicity of the non-human donor antibodies (e.g., a murine antibody) in human subjects as well. However, due to matching issues between the CDRs of a donor antibody and the FRs of an acceptor antibody, humanized antibodies are generally lower than non-human donor antibodies (e.g., a murine antibody) regarding the desired properties (e.g., antigen specificity, affinity, reactivity, ability to increase the activity of immune cells, and/or ability to enhance the immune response).

In the present invention, to allow a humanized antibody to retain the properties of a donor antibody (including, e.g., antigen specificity, affinity, reactivity, ability to increase the activity of immune cells, and/or ability to enhance the immune response) as much as possible, the framework regions (FRs) in the humanized antibody may comprise amino acid residues of both the human acceptor antibody and the corresponding non-human donor antibody; for example, the humanized antibody may comprise a back mutation. The term "back mutation" is a mutation introduced to an amino acid of a humanized antibody, and an amino acid resulting from the mutation corresponds to an amino acid in a parent antibody (e.g., a donor antibody, e.g., a murine antibody). Certain framework residues from the parent antibody may be retained during humanization of the antibody to substantially retain the binding properties of the parent antibody while minimizing potential immunogenicity of the resulting antibody. In one embodiment of the present invention, the parent antibody is a murine antibody. For example, back mutations change human framework residues to parent murine residues. Examples of framework residues that can be back-mutated include, but are not limited to, canonical residues, interface packing residues, unusual parent residues which are close to the binding site, residues in the "Vernier Zone" (which forms a platform on which the CDRs rest) (Foote&Winter, 1992, J. Mol. Biol. 224, 487-499), and residues close to CDRH3.

The term "affinity-matured" antibody used herein refers to an antibody that binds to the same antigen as a reference antibody but has a higher binding affinity for that antigen than the reference antibody. An affinity-matured antibody may be obtained by mutation of a reference antibody, in which case the reference antibody may also be referred to as the parent antibody. An affinity-matured antibody typically has one or more amino acid changes in one or more CDRs relative to a parent antibody, and these changes result in an increase in the affinity of the antibody for the target antigen as compared to the parent antibody without these changes. Exemplary affinity-matured antibodies will have nanomolar or even picomolar affinity for the target antigen. Methods for generating affinity-matured antibodies are known in the art. For example, Marks et al., BioTechnology, 10:779-783 (1992) described affinity maturation by VH and VL domain shuffling. Affinity maturation may also be performed by random mutagenesis of CDR and/or framework residues, see, e.g.: Barbas et al., Proc. Nat. Acad. Sci. USA, 91:3809-3813 (1994); Schier et al., Gene, 169:147-155 (1995); Yelton et al., J. Immunol., 155: 1994-2004 (1995); Jackson et al., J. Immunol., 154(7):3310-3319 (1995); Hawkins et al., J. Mol. Biol., 226:889-896 (1992). Affinity-matured variants may then be screened by phage display libraries and phage ELISA.

The term "multispecific antibody" used herein refers to an antibody capable of specifically binding to multiple antigenic epitopes, including bispecific antibodies, trispecific antibodies, or antibodies of greater multi specificity. The multiple epitopes may be different epitopes on the same antigen or different epitopes on different antigens. A multispecific antibody comprises a plurality of antigen-binding moieties. Each antigen-binding moiety targets one epitope and the epitopes targeted by the plurality of antigen-binding moieties are different from each other. Each antigen-binding moiety may be independently selected from a monoclonal antibody (e.g., IgG immunoglobulin), a Fab fragment, a Fab' fragment, a F(ab')₂ fragment, an Fv fragment, an Fd fragment, an scFv, a dsFv, a single-domain antibody, a chimeric antibody, a humanized antibody, an affinity-matured antibody, and the like. One antigen-binding moiety may be functionally linked (e.g., by chemical coupling, genetic fusion, noncovalent binding, or other means) to one or more additional antigen-binding moieties to form a multispecific antibody.

The term "target," "direct to," or "specifically bind to", with respect to an antibody or an antigen-binding fragment thereof, means that an antibody or an antigen-binding fragment thereof forms, together with an antigen, a complex relatively stable under physiological conditions and preferably does not exhibit significant binding to other undesired antigens. One molecule may target, be directed to, or specifically bind to no less than one molecule. For example, a bispecific antibody may have a higher binding affinity for two different antigens relative to other molecules. Specific binding may be characterized by the equilibrium dissociation constant KD for antigen-antibody binding (smaller KD indicates tighter binding) and also by the EC50 value for antibody-antigen binding. Methods for determining whether two molecules bind specifically are well known in the art and include, e.g., equilibrium dialysis, surface plasmon resonance, and the like.

The term "EC50 (concentration for 50% of maximum effect)" refers to the concentration that causes 50% of the maximum effect. When used in an enzyme-linked immunosorbent assay (ELISA) to indicate the binding capacity of an antibody molecule for a corresponding antigen, it may refer to the concentration of the antibody molecule at which half of the maximum detectable signal (e.g., colorimetric or fluorescent intensity) is produced. The lower the EC50 value, the greater the binding affinity of the antibody for the antigen.

The term "ka" refers to the association rate constant for a particular antibody-antigen interaction, which indicates the association rate of the antibody with its target antigen or the rate of complex formation between the antibody and antigen and is expressed in M⁻¹s⁻¹; the term "kd" refers to the dissociation rate constant for a particular antibody-antigen interaction, which indicates the dissociation rate of the antibody from its target antigen or the dissociation rate of the separation of the antigen-antibody complex into a free antibody and an antigen and is expressed in s⁻¹.

The term "KD" refers to the equilibrium dissociation constant for a particular antibody-antigen interaction, which is derived from the ratio of kd to ka (i.e., kd/ka) and is expressed in molar concentration (M). KD may be used to measure the affinity of the binding of an antibody to its binding partner (e.g., an antigen). A smaller KD indicates either tighter binding between the antibody and the antigen or higher affinity between the antibody and the antigen. For example, an antibody with an equilibrium dissociation constant in the nanomolar (nM) range binds more tightly to a particular antigen than an antibody with a dissociation constant in the micromolar (µM) range. The KD value of an antibody may be determined using methods well known in the art. One method for determining the ka, kd, and KD values of an antibody is to use surface plasmon resonance, typically using a biosensor system such as the Biacore^{™} system. Another method for determining the KD value of an antibody is biolayer interferometry (BLI).

The term "chimeric antigen receptor" or "CAR" refers to a fusion protein comprising an extracellular domain (i.e., binding domain) capable of binding to an antigen, a transmembrane domain, and an intracellular domain comprising one or more intracellular signaling domains derived from a signal transduction protein. These intracellular signaling domains are generally distinct from the polypeptide from which the extracellular domain is derived. The extracellular domain may be any protein molecule or a portion thereof and can specifically bind to a predetermined antigen. In some embodiments, the extracellular domain comprises an antibody or an antigen-binding fragment thereof. In some embodiments, the intracellular domain may be any known oligopeptide or polypeptide domain that functions to transmit a signal that causes activation or inhibition of an intracellular biological process, e.g., activation of an immune cell, such as a T cell or an NK cell. The intracellular domain typically comprises an immunoreceptor tyrosine activation motif (ITAM), such as the signaling domain from the CD3ζ molecule, which is responsible for activating immune effector cells and producing the killing effect. In addition, the chimeric antigen receptor may also comprise a signal peptide at the amino terminus responsible for the intracellular localization of the fusion protein, as well as a hinge region between the extracellular domain and the transmembrane domain. The intracellular signaling domain may also comprise a costimulatory domain from, e.g., 4-1BB or CD28 molecule.

The term "CART" or "CART cell" refers to a T cell capable of expressing or producing a CAR. A CART cell is typically obtained by engineering a T cell to allow it to comprise a nucleic acid molecule encoding a CAR. For a subject, CART cells may be obtained by the engineering of autologous T cells or allogeneic T cells.

The term "recombinant" used herein refers to the expression of an antibody or an antigen-binding fragment thereof of the present invention by recombinant DNA techniques (which include, e.g., DNA splicing and transgenic expression), e.g., the expression of an antibody or an antigen-binding fragment thereof using a recombinant expression vector expression system transfected into a non-human mammal (e.g., a mouse) or a cell (e.g., a CHO cell), wherein the antibody may be or is isolated from an antibody of a recombinant or combinatorial human antibody library.

The term "sequence identity" used herein refers to the percentage of nucleotides or amino acid residues that are identical at corresponding positions when two or more sequences are aligned in such a way that they match maximally, i.e., taking into account gaps and insertions. Alignment of sequences and calculation of percent sequence identity may be performed using suitable computer programs known in the art. Such programs include local alignment programs and global alignment programs, including but not limited to BLAST, ALIGN, ClustalW, EMBOSS Needle, and the like. One example of a local alignment program is BLAST (Basic Local Alignment Search Tool) (see, e.g., Altschul et al., (1990) J. Mol. 215; 403-410), which is available from the website http://www.ncbi.nlm.nih.gov/ of National Center for Biotechnology Information. Examples of global alignment programs (optimizing alignment over the full-length sequence) are EMBOSS Needle and EMBOSS Stretcher programs based on the Needleman-Wunsch algorithm (Needleman, Saul B.; and Wunsch, Christian D. (1970), "A general method applicable to the search for similarities in the amino acid sequence of two proteins", Journal of Molecular Biology 48 (3): 443-53), both of which are available from http://www.ebi.ac.uk/Tools/psa/.

The term "vector" used herein refers to any molecule, such as a nucleic acid, a plasmid, or a virus, capable of transporting a heterologous nucleic acid contained therein into a host cell (either in free form or integrated into the genome of the host cell). A vector may exist independently and autonomously replicate after being introduced into a host cell (e.g., a bacterial vector having a bacterial origin of replication and an episomal mammalian vector) or may be integrated into the genome of the host cell and replicated together with the host genome (e.g., a non-episomal mammalian vector). A vector used to express a target gene in a host cell is referred to as an "expression vector" or a "recombinant expression vector". The vector may comprise regulatory elements such as an origin of replication, expression regulation sequences (e.g., a promoter and/or an enhancer), and/or selectable marker genes (e.g., an antibiotic resistance gene and genes useful in colorimetric assays, such as β-galactose) operably linked to the target gene. To express the antibody or the antigen-binding fragment thereof of the present invention, expression vectors encoding the heavy and light chains may be transfected into host cells by standard techniques. The term "transfect" is intended to encompass a variety of techniques usually used for introducing an exogenous DNA into a prokaryotic or eukaryotic host cell, such as electroporation, calcium phosphate precipitation, DEAE-dextran transfection, or the like. Although it is theoretically possible to express the antibody of the present invention in prokaryotic or eukaryotic host cells, the antibody is expressed in eukaryotic cells, most preferably in mammalian host cells. This is because such eukaryotic cells, particularly mammalian cells, are more likely than prokaryotic cells to assemble and secrete a correctly folded and immunologically active antibody.

The term "host cell" used herein refers to a cell that may be or has been a recipient of a vector or an isolated polynucleotide. Host cells may be prokaryotic cells or eukaryotic cells. Exemplary eukaryotic cells include mammalian cells, such as primate or non-primate animal cells; fungal cells, such as yeast; plant cells; and insect cells. Nonlimiting exemplary mammalian cells include, but are not limited to, CHO cells, HEK-293 cells, BHK cells, or PER-C6 cells, as well as derivative cells thereof, such as 293-6E, CHO-DG44, CHO-K1, CHO-S, and CHO-DS cells, and may also be immune effector cells, such as T cells. Host cells include progeny of a single host cell, and the progeny may not necessarily be completely identical, e.g., in morphology or in genomic DNA complement, to the original parent cell due to natural, accidental, or deliberate mutation. A host cell may be an isolated cell or cell line and also includes cells transfected *in vivo* with the nucleic acid molecule or expression vector provided herein.

According to the present invention, the mouse 9.3 antibody is humanized by means of homology modeling and back mutation, so that the defect of reduced affinity is overcome, and the humanized 9.3 antibody has stronger functionality. The present invention solves the weak point of low affinity of the CD40 antibody.

### Humanized Anti-CD28 Antibody

The term "CD28" as used herein, which may also be referred to as cluster of differentiation 28 or Tp44, is an antigen expressed on the surface of T cells as a costimulatory receptor and may provide costimulatory signals required for T cell activation and survival. CD28 herein refers to any CD28 protein from any vertebrate source, including mammals such as primates (e.g., humans), non-human primates (e.g., rhesus monkeys), and rodents (e.g., mice and rats).

The present invention provides a humanized anti-CD28 antibody, wherein the humanized anti-CD28 antibody is obtained by performing humanization on the basis of a murine anti-CD28 antibody, and the murine anti-CD28 antibody may be an antibody 9.3. The humanized anti-CD28 antibody of the present invention may specifically recognize CD28, particularly human CD28, including soluble CD28 or CD28 expressed on the cell surface. Soluble CD28 includes native CD28 proteins as well as recombinant CD28 proteins or variants thereof, monomeric or dimeric CD28 constructs, CD28 lacking a transmembrane domain, and the like.

The humanized anti-CD28 antibody of the present invention has an EC₅₀ for binding to human CD28 of about 0.008 µg/mL to about 0.016 µg/mL, such as about 0.008 µg/mL to about 0.013 µg/mL, about 0.008 µg/mL to about 0.011 µg/mL, about 0.008 µg/mL to about 0.010 µg/mL, or about 0.008 µg/mL to about 0.009 µg/mL; further, about 8.00 ng/mL to about 15.50 ng/mL, such as about 8.27 ng/mL to about 15.09 ng/mL, about 8.27ng/mL to about 15.07 ng/mL, about 8.27 ng/mL to about 12.67 ng/mL, about 8.27 ng/mL to about 12.65 ng/mL, about 8.27 ng/mL to about 12.59 ng/mL, about 8.27 ng/mL to about 12.54 ng/mL, about 8.27 ng/mL to about 12.11 ng/mL, about 8.27 ng/mL to about 10.90 ng/mL, about 8.27 ng/mL to about 10.40 ng/mL, or about 8.27 ng/mL to about 9.14 ng/mL. In some embodiments, the humanized anti-CD28 antibody of the present invention has an EC₅₀ for binding to human CD28 of about 8.27 ng/mL, about 9.14 ng/mL, about 10.40 ng/mL, about 10.90 ng/mL, about 12.11 ng/mL, about 12.54 ng/mL, about 12.59 ng/mL, about 12.65 ng/mL, about 12.67 ng/mL, about 15.07 ng/mL, or about 15.09 ng/mL. The EC₅₀ is determined by ELISA, which may be determining the binding activity of the humanized antibody for human CD28 (e.g., human CD28 containing 6 histidines at the N-terminus), and the concentration of human CD28 used for the measurement of the binding activity may be, e.g., 2 µg/mL.

The humanized anti-CD28 antibody of the present invention has an equilibrium dissociation constant KD for binding to human CD28 of about 6.1 × 10⁻⁹ M to about 1.2 × 10⁻⁸ M, such as about 6.1 × 10⁻⁹ M to about 1.1 × 10⁻⁸ M, about 6.1 × 10⁻⁹ M to about 9.6 × 10⁻⁹ M, about 6.1 × 10⁻⁹ M to about 9.2 × 10⁻⁹ M, about 6.1 × 10⁻⁹ M to about 8.4 × 10⁻⁹ M, about 6.1 × 10⁻⁹ M to about 7.5 × 10⁻⁹ M, about 6.1 × 10⁻⁹ M to about 6.6 × 10⁻⁹ M, or about 6.1 × 10⁻⁹ M to about 6.2 × 10⁻⁹ M; further, about 6.13 × 10⁻⁹ M to about 1.15 × 10⁻⁸ M, about 6.13 × 10⁻⁹ M to about 1.12 × 10⁻⁸ M, about 6.13 × 10⁻⁹ M to about 1.09 × 10⁻⁸ M, about 6.13 × 10⁻⁹ M to about 1.08 × 10⁻⁸ M, about 6.13 × 10⁻⁹ M to about 9.59 × 10⁻⁹ M, about 6.13 × 10⁻⁹ M to about 9.13 × 10⁻⁹ M, about 6.13 × 10⁻⁹ M to about 8.36 × 10⁻⁹ M, about 6.13 × 10⁻⁹ M to about 7.44 × 10⁻⁹ M, about 6.13 × 10⁻⁹ M to about 6.59 × 10⁻⁹ M, or about 6.13 × 10⁻⁹ M to about 6.54 × 10⁻⁹ M. In some embodiments, the humanized anti-CD28 antibody of the present invention has an equilibrium dissociation constant KD for binding to human CD28 of about 6.13 × 10⁻⁹ M, about 6.54 × 10⁻⁹ M, about 6.59 × 10⁻⁹ M, about 7.44 × 10⁻⁹ M, about 8.36 × 10⁻⁹ M, about 9.13 × 10⁻⁹ M, about 9.59 × 10⁻⁹ M, about 1.08 × 10⁻⁸ M, about 1.09 × 10⁻⁸ M, about 1.12 × 10⁻⁸ M, or about 1.15 × 10⁻⁸ M. The equilibrium dissociation constant KD for binding is determined by biolayer interferometry (BLI).

The three heavy chain CDRs of the murine anti-CD28 antibody (donor antibody) may be linked to the four heavy chain FRs of the human antibody (acceptor antibody) for humanization of the antibody heavy chain variable region, and/or the three light chain CDRs of the murine anti-CD28 antibody may be linked to the four light chain FRs of the human antibody for humanization of the antibody light chain variable region. The humanized anti-CD28 antibody may comprise a humanized heavy chain variable region and/or a humanized light chain variable region. In some embodiments, the heavy chain variable region of the murine anti-CD28 antibody comprises an HCDR1 comprising SEQ ID NO: 1, an HCDR2 comprising SEQ ID NO: 2, and an HCDR3 comprising SEQ ID NO: 3. In some embodiments, the light chain variable region of the murine anti-CD28 antibody comprises an LCDR1 comprising SEQ ID NO: 9, an LCDR2 comprising SEQ ID NO: 10, and an LCDR3 comprising SEQ ID NO: 10. The acceptor antibody used in the humanization may be any human germline antibody sequence; a human germline antibody sequence having the highest sequence identity to the heavy chain variable region of the donor antibody may be selected as the acceptor antibody for humanization of the antibody heavy chain variable region, and a human germline antibody sequence having the highest sequence identity to the light chain variable region of the donor antibody may be selected as the acceptor antibody for humanization of the antibody light chain variable region. In some embodiments, the human germline IGHV4 sequence is used as the receptor antibody for humanization of the heavy chain variable region of the murine anti-CD28 antibody. In some embodiments, the human germline IGKV1 sequence is used as the receptor antibody for humanization of the light chain variable region of the murine anti-CD28 antibody. The humanized anti-CD28 antibody of the present invention may further comprise amino acid substitutions in the CDRs of the murine anti-CD28 antibody. For example, the humanized anti-CD28 antibody may comprise amino acid substitutions in the HCDR1, the HCDR2, and/or the HCDR3, and may also comprise amino acid substitutions in the LCDR1, the LCDR2, and/or the LCDR3. In some embodiments, the HCDR2 comprised in the heavy chain variable region of the humanized anti-CD28 antibody has amino acid substitutions at one or more of positions S12, A12, and M15 relative to SEQ ID NO: 2 (amino acid residue numbering is based on SEQ ID NO:2, with the first amino acid residue of SEQ ID NO: 2 as position 1). In some embodiments, the HCDR2 comprised in the heavy chain variable region of the humanized anti-CD28 antibody has an amino acid substitution at position M15 relative to SEQ ID NO: 2, and the amino acid substitution is preferably M15K. In some embodiments, the HCDR2 comprised in the heavy chain variable region of the humanized anti-CD28 antibody has amino acid substitutions at positions S12, A12, and M15 relative to SEQ ID NO: 2, and the amino acid substitutions are preferably S12P, A13S, and M15K. Thus, in some embodiments, the heavy chain variable region of the humanized anti-CD28 antibody comprises an HCDR1 comprising SEQ ID NO: 1, an HCDR2 comprising SEQ ID NO: 17 or 18, and an HCDR3 comprising SEQ ID NO: 3.

In some embodiments, the heavy chain variable region and the light chain variable region of the humanized anti-CD28 antibody comprise: (1) an HCDR1 comprising SEQ ID NO: 1, an HCDR2 comprising SEQ ID NO: 2, an HCDR3 comprising SEQ ID NO: 3, an LCDR1 comprising SEQ ID NO: 9, an LCDR2 comprising SEQ ID NO: 10, and an LCDR3 comprising SEQ ID NO: 10; (2) an HCDR1 comprising SEQ ID NO: 1, an HCDR2 comprising SEQ ID NO: 17, an HCDR3 comprising SEQ ID NO: 3, an LCDR1 comprising SEQ ID NO: 9, an LCDR2 comprising SEQ ID NO: 10, and an LCDR3 comprising SEQ ID NO: 10; or (3) an HCDR1 comprising SEQ ID NO: 1, an HCDR2 comprising SEQ ID NO: 18, an HCDR3 comprising SEQ ID NO: 3, an LCDR1 comprising SEQ ID NO: 9, an LCDR2 comprising SEQ ID NO: 10, and an LCDR3 comprising SEQ ID NO: 10.

The humanized anti-CD28 antibody of the present invention may also comprise a back mutation in the FR regions derived from human germline antibody sequences, that is, some of the amino acid residues in the FR regions are substituted with corresponding residues from the murine antibody to restore or improve the specificity and affinity of the antibody.

In some embodiments, the heavy chain variable region of the humanized anti-CD28 antibody of the present invention comprises an HFR1 comprising a sequence selected from SEQ ID NOs: 19-22, an HFR2 comprising a sequence selected from SEQ ID NOs: 23-26, an HFR3 comprising a sequence selected from SEQ ID NOs: 27-30, and an HFR4 comprising SEQ ID NO: 7. The light chain variable region of the humanized anti-CD28 antibody of the present invention comprises an LFR1 comprising a sequence selected from SEQ ID NOs: 36-37, an LFR2 comprising a sequence selected from SEQ ID NOs: 38-39, an LFR3 comprising a sequence selected from SEQ ID NOs: 40-42, and an LFR4 comprising SEQ ID NO: 15.

In some embodiments, the heavy chain variable region of the humanized anti-CD28 antibody of the present invention comprises: (1) an HFR1 comprising SEQ ID NO: 19, an HFR2 comprising SEQ ID NO: 23, an HFR3 comprising SEQ ID NO: 27, and an HFR4 comprising SEQ ID NO: 7; (2) an HFR1 comprising SEQ ID NO: 20, an HFR2 comprising SEQ ID NO: 24, an HFR3 comprising SEQ ID NO: 28, and an HFR4 comprising SEQ ID NO: 7; (3) an HFR1 comprising SEQ ID NO: 21, an HFR2 comprising SEQ ID NO: 25, an HFR3 comprising SEQ ID NO: 29, and an HFR4 comprising SEQ ID NO: 7; (4) an HFR1 comprising SEQ ID NO: 21, an HFR2 comprising SEQ ID NO: 26, an HFR3 comprising SEQ ID NO: 30, and an HFR4 comprising SEQ ID NO: 7; or (5) an HFR1 comprising SEQ ID NO: 22, an HFR2 comprising SEQ ID NO: 26, an HFR3 comprising SEQ ID NO: 30, and an HFR4 comprising SEQ ID NO: 7. In some embodiments, the light chain variable region of the humanized anti-CD28 antibody of the present invention comprises: (1) an LFR1 comprising SEQ ID NO: 36, an LFR2 comprising SEQ ID NO: 38, an LFR3 comprising SEQ ID NO: 40, and an LFR4 comprising SEQ ID NO: 15; (2) an LFR1 comprising SEQ ID NO: 37, an LFR2 comprising SEQ ID NO: 39, an LFR3 comprising SEQ ID NO: 41, and an LFR4 comprising SEQ ID NO: 15; or (3) an LFR1 comprising SEQ ID NO: 37, an LFR2 comprising SEQ ID NO: 39, an LFR3 comprising SEQ ID NO: 42, and an LFR4 comprising SEQ ID NO: 15.

In some embodiments, the heavy chain variable region and the light chain variable region of the humanized anti-CD28 antibody of the present invention comprise: (1) an HFR1 comprising SEQ ID NO: 19, an HFR2 comprising SEQ ID NO: 23, an HFR3 comprising SEQ ID NO: 27, an HFR4 comprising SEQ ID NO: 7, an LFR1 comprising SEQ ID NO: 36, an LFR2 comprising SEQ ID NO: 38, an LFR3 comprising SEQ ID NO: 40, and an LFR4 comprising SEQ ID NO: 15; (2) an HFR1 comprising SEQ ID NO: 19, an HFR2 comprising SEQ ID NO: 23, an HFR3 comprising SEQ ID NO: 27, an HFR4 comprising SEQ ID NO: 7, an LFR1 comprising SEQ ID NO: 37, an LFR2 comprising SEQ ID NO: 39, an LFR3 comprising SEQ ID NO: 41, and an LFR4 comprising SEQ ID NO: 15; (3) an HFR1 comprising SEQ ID NO: 20, an HFR2 comprising SEQ ID NO: 24, an HFR3 comprising SEQ ID NO: 28, an HFR4 comprising SEQ ID NO: 7, an LFR1 comprising SEQ ID NO: 37, an LFR2 comprising SEQ ID NO: 39, an LFR3 comprising SEQ ID NO: 41, and an LFR4 comprising SEQ ID NO: 15; (4) an HFR1 comprising SEQ ID NO: 20, an HFR2 comprising SEQ ID NO: 24, an HFR3 comprising SEQ ID NO: 28, an HFR4 comprising SEQ ID NO: 7, an LFR1 comprising SEQ ID NO: 37, an LFR2 comprising SEQ ID NO: 39, an LFR3 comprising SEQ ID NO: 42, and an LFR4 comprising SEQ ID NO: 15; (5) an HFR1 comprising SEQ ID NO: 21, an HFR2 comprising SEQ ID NO: 25, an HFR3 comprising SEQ ID NO: 29, an HFR4 comprising SEQ ID NO: 7, an LFR1 comprising SEQ ID NO: 36, an LFR2 comprising SEQ ID NO: 38, an LFR3 comprising SEQ ID NO: 40, and an LFR4 comprising SEQ ID NO: 15; (6) an HFR1 comprising SEQ ID NO: 21, an HFR2 comprising SEQ ID NO: 25, an HFR3 comprising SEQ ID NO: 29, an HFR4 comprising SEQ ID NO: 7, an LFR1 comprising SEQ ID NO: 37, an LFR2 comprising SEQ ID NO: 39, an LFR3 comprising SEQ ID NO: 41, and an LFR4 comprising SEQ ID NO: 15; (7) an HFR1 comprising SEQ ID NO: 21, an HFR2 comprising SEQ ID NO: 25, an HFR3 comprising SEQ ID NO: 29, an HFR4 comprising SEQ ID NO: 7, an LFR1 comprising SEQ ID NO: 37, an LFR2 comprising SEQ ID NO: 39, an LFR3 comprising SEQ ID NO: 42, and an LFR4 comprising SEQ ID NO: 15; (8) an HFR1 comprising SEQ ID NO: 21, an HFR2 comprising SEQ ID NO: 26, an HFR3 comprising SEQ ID NO: 30, an HFR4 comprising SEQ ID NO: 7, (2) an LFR1 comprising SEQ ID NO: 37, an LFR2 comprising SEQ ID NO: 39, an LFR3 comprising SEQ ID NO: 41, and an LFR4 comprising SEQ ID NO: 15; (9) an HFR1 comprising SEQ ID NO: 21, an HFR2 comprising SEQ ID NO: 26, an HFR3 comprising SEQ ID NO: 30, an HFR4 comprising SEQ ID NO: 7, an LFR1 comprising SEQ ID NO: 37, an LFR2 comprising SEQ ID NO: 39, an LFR3 comprising SEQ ID NO: 42, and an LFR4 comprising SEQ ID NO: 15; (10) an HFR1 comprising SEQ ID NO: 22, an HFR2 comprising SEQ ID NO: 26, an HFR3 comprising SEQ ID NO: 30, an HFR4 comprising SEQ ID NO: 7, an LFR1 comprising SEQ ID NO: 37, an LFR2 comprising SEQ ID NO: 39, an LFR3 comprising SEQ ID NO: 41, and an LFR4 comprising SEQ ID NO: 15; or (11) an HFR1 comprising SEQ ID NO: 22, an HFR2 comprising SEQ ID NO: 26, an HFR3 comprising SEQ ID NO: 30, an HFR4 comprising SEQ ID NO: 7, an LFR1 comprising SEQ ID NO: 37, an LFR2 comprising SEQ ID NO: 39, an LFR3 comprising SEQ ID NO: 42, and an LFR4 comprising SEQ ID NO: 15.

In some embodiments, the heavy chain variable region of the humanized anti-CD28 antibody of the present invention comprises a sequence selected from SEQ ID NOs: 31-35. In some embodiments, the light chain variable region of the humanized anti-CD28 antibody of the present invention comprises a sequence selected from SEQ ID NOs: 43-45.

In some embodiments, the heavy chain variable region and the light chain variable region of the humanized anti-CD28 antibody of the present invention are selected from the following combinations: (1) a heavy chain variable region comprising SEQ ID NO: 31 and a light chain variable region comprising SEQ ID NO: 43; (2) a heavy chain variable region comprising SEQ ID NO: 31 and a light chain variable region comprising SEQ ID NO: 44; (3) a heavy chain variable region comprising SEQ ID NO: 32 and a light chain variable region comprising SEQ ID NO: 44; (4) a heavy chain variable region comprising SEQ ID NO: 32 and a light chain variable region comprising SEQ ID NO: 45; (5) a heavy chain variable region comprising SEQ ID NO: 33 and a light chain variable region comprising SEQ ID NO: 43; (6) a heavy chain variable region comprising SEQ ID NO: 33 and a light chain variable region comprising SEQ ID NO: 44; (7) a heavy chain variable region comprising SEQ ID NO: 33 and a light chain variable region comprising SEQ ID NO: 45; (8) a heavy chain variable region comprising SEQ ID NO: 34 and a light chain variable region comprising SEQ ID NO: 44; (9) a heavy chain variable region comprising SEQ ID NO: 34 and a light chain variable region comprising SEQ ID NO: 45; (10) a heavy chain variable region comprising SEQ ID NO: 35 and a light chain variable region comprising SEQ ID NO: 44; and (11) a heavy chain variable region comprising SEQ ID NO: 35 and a light chain variable region comprising SEQ ID NO: 45.

The humanized anti-CD28 antibody of the present invention may be any antibody form comprising the humanized heavy chain variable region and/or the humanized light chain variable region described above, e.g., a full-length antibody (e.g., IgG), a single-chain antibody, or a single-domain antibody. In some embodiments, the humanized anti-CD28 antibody of the present invention comprises a constant region of an immunoglobulin (such as IgG, e.g., IgG1, IgG2, IgG3, or IgG4), e.g., a constant region of IgG1, IgG2, IgG3, or IgG4. In some embodiments, the constant region is a constant region of a human IgG, e.g., a constant region of human IgG1, human IgG2, human IgG3, or human IgG4. In some embodiments, the humanized heavy chain variable region described above is fused to an immunoglobulin heavy chain constant region (including CH1, CH2, and/or CH3). In some embodiments, the humanized light chain variable region described above is fused to an immunoglobulin light chain constant region (CL). In some embodiments, the immunoglobulin heavy chain constant region comprises SEQ ID NO: 81. In some embodiments, the immunoglobulin light chain constant region comprises SEQ ID NO: 82.

In some embodiments, the humanized anti-CD28 antibody of the present invention is a single-chain antibody (scFv), in which the humanized heavy chain variable region is fused to the humanized light chain variable region via a peptide linker. The humanized heavy chain variable region and the humanized light chain variable region may be linked in a different order; for example, the C-terminus of the humanized heavy chain variable region is fused to the N-terminus of the humanized light chain variable region via a peptide linker, or the N-terminus of the humanized heavy chain variable region is fused to the N-terminus of the humanized light chain variable region via a peptide linker. Peptide linkers for linking the humanized heavy chain variable region and the humanized light chain variable region are well known to those skilled in the art and may be, e.g., a flexible linker such as a glycine-serine polymer. In some embodiments, the peptide linker for linking the humanized heavy chain variable region and the humanized light chain variable region is GGGGSGGGGSGGGGS (SEQ ID NO: 80). In some embodiments, the humanized anti-CD28 antibody of the present invention may also be a single-domain antibody, e.g., a single-domain antibody that comprises a heavy chain variable region but does not comprise a light chain variable region. Methods for preparing single-domain antibodies are well known to those skilled in the art.

The present invention also relates to an antigen-binding fragment of the humanized anti-CD28 antibody, such as a Fab fragment, a F(ab')₂ fragment, or a Fab' fragment of a full-length antibody.

The humanized anti-CD28 antibody or the binding fragment thereof of the present invention may also be fused, as part of a fusion protein, to other functional polypeptides.

### Affinity-Matured CD40 Antibody

The term "CD40" used herein is a glycoprotein expressed on the cell surface, belongs to the tumor necrosis factor receptor (TNFR) superfamily, and plays a central role in the immune system. It is expressed on a variety of immune cells, such as B cells, dendritic cells, monocytes, and macrophages. CD40 herein refers to any CD40 protein from any vertebrate source, including mammals such as primates (e.g., humans), non-human primates (e.g., rhesus monkeys), and rodents (e.g., mice and rats).

The present invention provides an affinity-matured anti-CD40 antibody. The affinity-matured CD40 antibody has increased binding affinity for CD40 as identified by phage display library. Affinity maturation using phage display libraries has been described, e.g., in Lowman et al., Biochemistry 30(45):10832-10838 (1991); reference may also be made to Hawkins et al., J. Mol Biol. 254: 889-896 (1992) and the examples hereinafter. Without being strictly limited to the following description, this process may be described simply as follows: mutating one or more sites within a predetermined region of a parent antibody to generate possible amino acid substitutions at each site. The antibody mutants thus generated are displayed in a monovalent fashion from filamentous phage particles as fusions to the gene III product of M13 packaged within each particle. The phage expressing the various mutants may be cycled through rounds of binding selection (e.g., determination of the binding affinity of the mutants for an antigen by ELISA), followed by isolation and sequencing of those mutants which display high affinity. It will be appreciated that although the anti-CD40 antibody variants of the present invention are identified using the phage display technique, other techniques may also be used to identify anti-CD40 antibody variants with improved binding affinity, including affinity-matured anti-CD40 antibody variants.

In some embodiments, the parent antibody may be, e.g., an anti-CD40 antibody having the following heavy chain variable region and light chain variable region: a heavy chain variable region that comprises an HCDR1 comprising SEQ ID NO: 46, an HCDR2 comprising SEQ ID NO: 47 and an HCDR3 comprising SEQ ID NO: 48 and a light chain variable region that comprises an LCDR1 comprising SEQ ID NO: 54, an LCDR2 comprising SEQ ID NO: 55 and an LCDR3 comprising SEQ ID NO: 56. In some embodiments, the VH of the parent antibody comprises SEQ ID NO: 53, and/or the VL comprises SEQ ID NO: 61. In some embodiments, the parent antibody comprises a VH having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID NO: 53 and/or a VL having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID NO: 61. The parent antibody may be a full-length antibody, e.g., an immunoglobulin (such as IgG, e.g., IgG1, IgG2, IgG3, or IgG4), an scFv or a single-domain antibody, or an antigen-binding fragment thereof, such as a Fab fragment, a F(ab')₂ fragment, or a Fab' fragment.

The affinity-matured anti-CD40 antibody of the present invention has a higher binding affinity for CD40 (e.g., human CD40) compared with the parent antibody. In some embodiments, the affinity-matured anti-CD40 antibody of the present invention has a lower EC₅₀ value for binding to CD40 (e.g., human CD40) compared with the parent antibody. In some embodiments, the affinity-matured anti-CD40 antibody of the present invention binds to human CD40 with an EC₅₀ of less than about 0.04919 µg/mL. In some embodiments, the affinity-matured anti-CD40 antibody of the present invention binds to human CD40 with an EC₅₀ of less than about 3.44 × 10⁻⁷ M. The EC₅₀ is determined by ELISA, which may be determining the binding activity of the antibody for human CD40 (e.g., human CD40-His), and the concentration of human CD28 used for the determination of the binding activity may be, e.g., 2 µg/mL.

In some embodiments, the affinity-matured anti-CD40 antibody of the present invention comprises a VH and a VL and has one, two, or more amino acid substitutions in at least one CDR relative to the parent antibody. In some embodiments, the affinity-matured anti-CD40 antibody of the present invention comprises a VH and a VL and has one, two, or more amino acid substitutions in at least one of the HCDR1, HCDR2, HCDR3, LCDR1, LCDR2, and LCDR3 relative to the parent antibody. In some embodiments, the VH of the affinity-matured anti-CD40 antibody of the present invention has one, two, or more amino acid substitutions in at least one of the HCDR1, HCDR2, and HCDR3 relative to the parent antibody. In some embodiments, the VL of the affinity-matured anti-CD40 antibody of the present invention has one, two, or more amino acid substitutions in at least one of the LCDR1, LCDR2, and LCDR3 relative to the parent antibody. In some embodiments, the VH of the affinity-matured anti-CD40 antibody of the present invention comprises an HCDR1 comprising a sequence selected from SEQ ID NO: 46 and SEQ ID NO: 62, an HCDR2 comprising SEQ ID NO: 47, and an HCDR3 comprising SEQ ID NO: 48. In some embodiments, the VL of the affinity-matured anti-CD40 antibody of the present invention comprises an LCDR1 comprising SEQ ID NO: 54, an LCDR2 comprising a sequence selected from SEQ ID NOs: 55 and 64-66, and an LCDR3 comprising a sequence selected from SEQ ID NOs: 56 and 67-68.

In some embodiments, the VH of the affinity-matured anti-CD40 antibody of the present invention comprises: (1) an HCDR1 comprising SEQ ID NO: 46, an HCDR2 comprising SEQ ID NO: 47, and an HCDR3 comprising SEQ ID NO: 48; or (2) an HCDR1 comprising SEQ ID NO: 62, an HCDR2 comprising SEQ ID NO: 47, and an HCDR3 comprising SEQ ID NO: 48.

In some embodiments, the VL of the affinity-matured anti-CD40 antibody of the present invention comprises: (1) an LCDR1 comprising SEQ ID NO: 54, an LCDR2 comprising SEQ ID NO: 64, and an LCDR3 comprising SEQ ID NO: 56; (2) an LCDR1 comprising SEQ ID NO: 54, an LCDR2 comprising SEQ ID NO: 55, and an LCDR3 comprising SEQ ID NO: 67; (3) an LCDR1 comprising SEQ ID NO: 54, an LCDR2 comprising SEQ ID NO: 55, and an LCDR3 comprising SEQ ID NO: 68; (4) an LCDR1 comprising SEQ ID NO: 54, an LCDR2 comprising SEQ ID NO: 65, and an LCDR3 comprising SEQ ID NO: 56; (5) an LCDR1 comprising SEQ ID NO: 54, an LCDR2 comprising SEQ ID NO: 66, and an LCDR3 comprising SEQ ID NO: 56. In some embodiments, the VH and VL of the affinity-matured anti-CD40 antibody of the present invention comprises: (1) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 64, and an LCDR3 comprising SEQ ID NO: 56; (2) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 67; (3) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 68; (4) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 65, and an LCDR3 comprising SEQ ID NO: 56; (5) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 66, and an LCDR3 comprising SEQ ID NO: 56.

In some embodiments, the affinity-matured anti-CD40 antibody of the present invention does not comprise a VH that comprises an HCDR1 comprising SEQ ID NO: 46, an HCDR2 comprising SEQ ID NO: 47, and an HCDR3 comprising SEQ ID NO: 48. In some embodiments, the affinity-matured anti-CD40 antibody of the present invention does not comprise a VL that comprises an LCDR1 comprising SEQ ID NO: 54, an LCDR2 comprising SEQ ID NO: 55, and an LCDR3 comprising SEQ ID NO: 56.

In some embodiments, the HFR region and/or LFR region of the affinity-matured anti-CD40 antibody of the present invention are the same as those of the parent antibody. For example, the VH of the affinity-matured anti-CD40 antibody of the present invention may comprise an HFR1 comprising SEQ ID NO: 49, an HFR2 comprising SEQ ID NO: 50, an HFR3 comprising SEQ ID NO: 51, and an HFR4 comprising SEQ ID NO: 52. For example, the VL of the affinity-matured anti-CD40 antibody of the present invention may comprise an LFR1 comprising SEQ ID NO: 57, an LFR2 comprising SEQ ID NO: 58, an LFR3 comprising SEQ ID NO: 59, and an LFR4 comprising SEQ ID NO: 60.

In some embodiments, the affinity-matured anti-CD40 antibody of the present invention may have one or more amino acid substitutions in the FR region relative to the parent antibody. In some embodiments, the HFR1 of the affinity-matured anti-CD40 antibody of the present invention has an amino acid substitution at position E10 relative to SEQ ID NO: 49 (amino acid residue numbering is based on SEQ ID NO: 49, with the first amino acid residue of SEQ ID NO: 49 as position 1). In some embodiments, the HFR1 of the affinity-matured anti-CD40 antibody of the present invention has amino acid substitution E10Q relative to SEQ ID NO: 49. In some embodiments, the VH of the affinity-matured anti-CD40 antibody of the present invention comprises an HFR1 comprising SEQ ID NO: 63, an HFR2 comprising SEQ ID NO: 50, an HFR3 comprising SEQ ID NO: 51, and an HFR4 comprising SEQ ID NO: 52.

In some embodiments, the VH of the affinity-matured anti-CD40 antibody of the present invention comprises a sequence selected from SEQ ID NOs: 69-73 and/or the VL comprises a sequence selected from SEQ ID NOs: 74-78. In some embodiments, the affinity-matured anti-CD40 antibody of the present invention comprises a VH having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to a sequence selected from SEQ ID NOs: 69-73 and/or a VL having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to a sequence selected from SEQ ID NOs: 74-78.

The affinity-matured anti-CD40 antibody of the present invention is any antibody form comprising the heavy chain variable region and/or the light chain variable region described above, e.g., a full-length antibody (such as IgG, e.g., IgG1, IgG2, IgG3, or IgG4), a single-chain antibody, or a single-domain antibody. In some embodiments, the affinity-matured anti-CD40 antibody of the present invention comprises a constant region of an immunoglobulin (e.g., an IgG), such as a constant region of IgG1, IgG2, IgG3, or IgG4. In some embodiments, the constant region is a constant region of a human IgG, e.g., a constant region of human IgG1, human IgG2, human IgG3, or human IgG4. In some embodiments, the heavy chain variable region described above is fused to an immunoglobulin heavy chain constant region (including CH1, CH2, and/or CH3). In some embodiments, the light chain variable region described above is fused to an immunoglobulin light chain constant region (CL).

In some embodiments, the affinity-matured anti-CD40 antibody of the present invention is a single-chain antibody (scFv), in which the heavy chain variable region is fused to the light chain variable region via a peptide linker. The heavy chain variable region and the light chain variable region may be linked in a different order; for example, the C-terminus of the heavy chain variable region is fused to the N-terminus of the light chain variable region via a peptide linker, or the N-terminus of the heavy chain variable region is fused to the N-terminus of the light chain variable region via a peptide linker. Peptide linkers for linking the heavy chain variable region and the light chain variable region are well known to those skilled in the art and may be, e.g., a flexible linker such as a glycine-serine polymer. In some embodiments, the peptide linker for linking the heavy chain variable region and the light chain variable region is GGGGSGGGGSGGGGS (SEQ ID NO: 80).

In some embodiments, the affinity-matured anti-CD40 antibody of the present invention may also be a single-domain antibody, e.g., a single-domain antibody that comprises a heavy chain variable region but does not comprise a light chain variable region. Methods for preparing single-domain antibodies are well known to those skilled in the art.

The present invention also relates to an antigen-binding fragment of the affinity-matured anti-CD40 antibody, such as a Fab fragment, a F(ab')₂ fragment, or a Fab' fragment of a full-length antibody.

The affinity-matured anti-CD40 antibody or the binding fragment thereof of the present invention may also be fused, as part of a fusion protein, to other functional polypeptides.

### Multispecific Antibody

the present invention also provides a multispecific antibody, including but not limited to a bispecific antibody, a trispecific antibody, or a tetraspecific antibody. The multispecific antibody comprises at least two antigen-binding moieties: a first antigen-binding moiety and a second antigen-binding moiety. The first antigen-binding moiety and the second antigen-binding moiety specifically bind to different antigens or different epitopes of the same antigen. The first antigen-binding moiety and the second antigen-binding moiety may each independently be a full-length antibody, a single-chain antibody, a single-domain antibody, or an antigen-binding fragment thereof, such as a Fab fragment, a F(ab')₂ fragment, a Fab' fragment, or an Fv fragment of a full-length antibody. Herein, the "first" and "second" are for the purpose of distinction only and do not imply a particular order.

In some embodiments, the first antigen-binding moiety is an anti-CD28 antibody, e.g., the murine anti-CD28 antibody or the humanized anti-CD28 antibody described herein, and the second antigen-binding moiety is capable of specifically binding to a second antigen that is different from CD28. In some embodiments, the second antigen-binding moiety may specifically bind to a tumor antigen or is capable of stimulating an immune cell. In some embodiments, the second antigen-binding moiety is an anti-CD40 antibody, e.g., the parent anti-CD40 antibody or the affinity-matured anti-CD40 antibody described herein.

In some embodiments, the first antigen-binding moiety is an anti-CD40 antibody, e.g., the parent anti-CD40 antibody or the affinity-matured anti-CD40 antibody described herein, and the second antigen-binding moiety is capable of specifically binding to a second antigen that is different from CD40. In some embodiments, the second antigen-binding moiety may specifically bind to a tumor antigen or is capable of stimulating an immune cell. In some embodiments, the second antigen-binding moiety is an anti-CD28 antibody, e.g., the murine anti-CD28 antibody or the humanized anti-CD28 antibody described herein.

In some preferred embodiments, the first antigen-binding moiety may be any anti-CD28 antibody, e.g., the murine anti-CD28 antibody or the humanized anti-CD28 antibody described herein. The second antigen-binding moiety may be any anti-CD40 antibody, e.g., the parent anti-CD40 antibody or the affinity-matured anti-CD40 antibody described herein.

In some embodiments, the first antigen-binding moiety and the second antigen-binding moiety of the bispecific antibody are both an scFv and are linked via a peptide linker. In some embodiments, the first antigen-binding moiety is an anti-CD28 scFv and the second antigen-binding moiety is an anti-CD40 scFv. In some embodiments, the C-terminus of the first antigen-binding moiety is linked to the N-terminus of the second antigen-binding moiety via a peptide linker. In some embodiments, the N-terminus of the first antigen-binding moiety is linked to the C-terminus of the second antigen-binding moiety via a peptide linker.

In some embodiments, peptide linkers for linking the first antigen-binding moiety and the second antigen-binding moiety of the bispecific antibody are well known to those skilled in the art and may be, e.g., a flexible linker, such as a glycine-serine polymer. In some embodiments, the peptide linker for linking the first antigen-binding moiety and the second antigen-binding moiety of the bispecific antibody is GGGGS (SEQ ID NO: 79).

### Nucleic Acid and Vector

The present invention also provides a polynucleotide encoding the humanized anti-CD28 antibody or the antigen-binding fragment thereof, the affinity-matured anti-CD40 antibody or the antigen-binding fragment thereof, or the multispecific antibody comprising the anti-CD28 antibody or the antigen-binding fragment thereof and/or the anti-CD40 antibody or the antigen-binding fragment thereof (e.g., a bispecific antibody, which may be called CD28-CD40 diabody for short hereinafter) of the present invention. The polynucleotide may be a DNA or an RNA (e.g., an mRNA). The present invention also provides a vector comprising the polynucleotide described above, which may be used to express the antibody or the antigen-binding fragment thereof described above in a host cell. The vector may be an expression vector, such as a plasmid vector or a viral vector. The viral vector may be a retroviral vector, a lentiviral vector, an adenoviral vector or an adeno-associated viral vector.

In some embodiments, the vector may be a circular RNA of the polynucleotide described above, and the circular RNA comprises, in sequence, an internal ribosome entry site (IRES) element, the polynucleotide described above, and a polyA. The circular RNA may be used for expressing the antibody described above.

The IRES may be derived from a virus. The IRES sequence is an IRES derived from Coxsackie virus B3 (CVB3) or Coxsackie virus A (CVB 1/2), encephalomyocarditis virus (EMCV), Taura syndrome virus, tripartite virus, Theiler's encephalomyelitis virus, simian virus 40, *Solenopsis invicta* virus 1, *Rhopalosiphum padi* virus, reticuloendotheliosis virus, human poliovirus 1, *Plautia stali* intestine virus, Kashmir bee virus, human rhinovirus 2, *Homalodisca coagulata* virus-1, human immunodeficiency virus type 1, Himetobi P virus, hepatitis C virus, hepatitis A virus, hepatitis GB virus, foot and mouth disease virus, human enterovirus 71, equine rhinitis virus, *Ectropis obliqua* picorna-like virus, *Drosophila* C virus, Crucifer tobamo virus, cricket paralysis virus, bovine viral diarrhea virus 1, black queen cell virus, aphid lethal paralysis virus, avian encephalomyelitis virus, acute bee paralysis virus, hibiscus chlorotic ringspot virus, classical swine fever virus, human FGF2, human SFTPA1, human AMLURUNX1, *Drosophila* antennapedia, human AQP4, human AT 1R, human BAG-1, human BCL2, human BiP, human c-IAP1, human c-myc, human eIF4G, mouse NDST4L, human LEF1, mouse HIF1 alpha, human n.myc, mouse Gtx, human p27kip, human PDGF2/c-sis, human p53, human Pim-1, mouse Rbm3, *Drosophila* reaper, canine scamper, *Drosophila* Ubx, human UNR, mouse UtrA, human VEGF-A, human XIAP, Salivirus, Cosavirus, Parechovirus, *Drosophila* hairless, *S. cerevisiae* TFIID, *S. cerevisiae* YAP1, human c-src, human FGF-1, simian picomavirus, turnip crinkle virus, or an aptamer to eIF4G.

The circular RNA of the present invention may be prepared by the general strategies of RNA circularization, such as enzymatic methods using RNA or DNA ligases or ribozymatic methods using self-splicing introns. See Petkovic, S. & Muller, S., "RNA circularization strategies in vivo and in vitro", Nucleic Acids Research, 43(4): 2454-2465 (2015); Beadudry, D. & Perreault, J., "An efficient strategy for the synthesis of circular RNA molecules", Nucleic Acids Research, 23(15): 3064-3066 (1995); Micura, R., "Cyclic Oligoribonucleotides (RNA) by Solid-Phase Synthesis", Chemistry A European Journal, 5(7): 2077-2082 (1999).

In some embodiments, the present invention also provides a precursor RNA for preparing the circular RNA, wherein the circular RNA may be obtained by circularizing the precursor RNA, and the precursor RNA may also be used as a vector comprising the polynucleotide described above. The precursor RNA comprises a circularization element, an internal ribosome entry site (IRES) element, the polynucleotide described above, and a polyA. In some embodiments, the circularization element comprises a first intron sequence located at 5' position of the internal ribosome entry site (IRES) element and a second intron sequence located at 3' position of the polyA. In some embodiments, the first intron sequence and the second intron sequence are derived from Group I or Group II intron self-splicing sequences. In some embodiments, the first intron sequence comprises a 3' Group I intron segment containing 3' splice site dinucleotides and the second intron element comprises a 5' Group I intron segment containing 5' splice site dinucleotides. In some embodiments, the precursor RNA further comprises a 5' spacer sequence between the first intron sequence and the internal ribosome entry site (IRES) element and a 3' spacer sequence between the polyA and the second intron element. In some embodiments, the precursor RNA further comprises a 5' homology arm outside the first intron element and a 3' homology arm outside the second intron element.

In some embodiments, the vector comprising the polynucleotide encoding the antibody or the antigen-binding fragment described above may also be a vector for producing the precursor RNA described above, and the vector comprises a DNA template for the precursor RNA, which may be transcribed to obtain the precursor RNA. The transcription may be *in vitro* or in a cell.

The term "polyA" used herein is an abbreviation for polyadenylation and refers to a sequence that is consisting of consecutive adenine nucleotides and is at least 30 in length. The polyA sequence may be a ribonucleic acid sequence or a deoxyribonucleic acid sequence. The length of consecutive adenine nucleotides in a polyA sequence may be at least 30 nucleotides, e.g., at least 45 nucleotides, at least 50 nucleotides, at least 55 nucleotides, at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 105 nucleotides, at least 110 nucleotides, at least 115 nucleotides, at least 120 nucleotides, at least 125 nucleotides, at least 130 nucleotides, at least 135 nucleotides, at least 140 nucleotides, at least 145 nucleotides, at least 150 nucleotides, at least 155 nucleotides, at least 160 nucleotides, at least 165 nucleotides, at least 170 nucleotides, at least 175 nucleotides, at least 180 nucleotides, at least 185 nucleotides, at least 190 nucleotides, at least 195 nucleotides, at least 200 nucleotides, at least 205 nucleotides, at least 210 nucleotides, at least 215 nucleotides, at least 220 nucleotides, at least 225 nucleotides, at least 230 nucleotides, at least 235 nucleotides, or at least 240 nucleotides. The length of consecutive adenine nucleotides in the polyA sequence may be 30-240 nucleotides, e.g., 40-230 nucleotides, 45-220 nucleotides, 50-210 nucleotides, 60-200 nucleotides, 70-190 nucleotides, 80-180 nucleotides, 90-170 nucleotides, 100-160 nucleotides, 110-150 nucleotides, or 120-140 nucleotides. In some embodiments, the polyA sequence may consist of only consecutive adenine nucleotides. In some embodiments, the polyA is at least 45 nucleotides in length. In some embodiments, the polyA is at least 70 nucleotides in length.

The present invention also provides a method for producing the circular RNA described above, which comprises circularizing the precursor RNA described above to obtain the circular RNA. In some embodiments, the method further comprises transcribing the DNA template for the precursor RNA contained in the vector to obtain the precursor RNA. In some embodiments, the method further comprises purifying the circular RNA, e.g., by interaction between oligo dT and polyA using an oligo dT-based capture method.

### Use of Antibody

The humanized anti-CD28 antibody or the antigen-binding fragment thereof of the present invention can be used to induce T cell proliferation, activate T cells, and promote the killing effect of immune effector cells (e.g., T cells, such as CAR-T cells). The humanized anti-CD28 antibody, the affinity-matured anti-CD40 antibody or the antigen-binding fragment thereof of the present invention, together with an antibody that specifically binds to an antigen on the surface of a tumor cell, can form a bispecific antibody, e.g., BiTE (bispecific T-cell engager), or a multispecific antibody to enable targeted activation of T cells of oneself to kill tumor cells.

Accordingly, the present invention provides a method for inducing T cell proliferation using the humanized anti-CD28 antibody or the antigen-binding fragment thereof of the present invention or use of the humanized anti-CD28 antibody or the antigen-binding fragment thereof of the present invention in preparing a medicament for inducing T cell proliferation. The method comprises contacting the humanized anti-CD28 antibody or the antigen-binding fragment thereof of the present invention, the multispecific antibody comprising the humanized anti-CD28 antibody or the antigen-binding fragment thereof of the present invention, the polynucleotide encoding the humanized anti-CD28 antibody or the antigen-binding fragment thereof of the present invention, the vector comprising the polynucleotide, or the pharmaceutical composition comprising the antibody or the antigen-binding fragment thereof, the multispecific antibody, the polynucleotide, or the vector with a T cell, such that the humanized anti-CD28 antibody or the antigen-binding fragment thereof stimulates T cell activation and induces the proliferation thereof. The polynucleotide or vector may be introduced into the T cell and the humanized anti-CD28 antibody or the antigen-binding fragment thereof is thereby expressed in the T cell to stimulate T cell activation and induce the proliferation thereof. The polynucleotide may be a DNA or an RNA, e.g., an mRNA.

In some embodiments, the T cell may be a CAR-T cell. In some embodiments, the humanized anti-CD28 antibody or the antigen-binding fragment thereof of the present invention or the multispecific antibody comprising the humanized anti-CD28 antibody or the antigen-binding fragment thereof of the present invention can be administered to a subject in combination with a CAR-T cell to enhance the killing effect of the CAR-T cell on the target cell.

The affinity-matured anti-CD40 antibody of the present invention can be used to stimulate immune cells (e.g., dendritic cells, macrophages, NK cells, granulocytes, etc.) and thus can be used to stimulate the immune response, e.g., activating antigen presenting cells (APCs) such as dendritic cells (DCs), stimulating activation and/or proliferation of B cells, or inducing cytotoxicity of effector T cells, and can also be used to treat tumors. The humanized anti-CD28 antibody, the affinity-matured anti-CD40 antibody or the antigen-binding fragment thereof of the present invention, together with an antibody that specifically binds to an antigen on the surface of a tumor cell, can form a bispecific antibody or a multispecific antibody to activate cytotoxic T cells to kill tumor cells.

Accordingly, the present invention provides a method for enhancing an immune response in a subject using the affinity-matured anti-CD40 antibody or the antigen-binding fragment thereof of the present invention or use of the affinity-matured anti-CD40 antibody or the antigen-binding fragment thereof of the present invention in preparing a medicament for enhancing an immune response in a subject. The method comprises administering to the subject the affinity-matured anti-CD40 antibody or the antigen-binding fragment thereof of the present invention, the multispecific antibody comprising the affinity-matured anti-CD40 antibody or the antigen-binding fragment thereof of the present invention, the polynucleotide encoding the affinity-matured anti-CD40 antibody or the antigen-binding fragment thereof of the present invention or the vector comprising the polynucleotide, or the pharmaceutical composition comprising the antibody or the antigen-binding fragment thereof, the multispecific antibody, the polynucleotide, or the vector. The polynucleotide may be a DNA or an RNA, e.g., an mRNA.

The term "enhance an immune response" used herein refers to stimulating, eliciting, increasing, modifying, or enhancing any response of the immune system of a subject. The immune response may be a cellular response (i.e., a cell-mediated, e.g., cytotoxic T lymphocyte-mediated response) or a humoral response (i.e., an antibody-mediated response) and may be a primary or secondary immune response. Exemplary immune response enhancement includes inducing proliferation of T cells (e.g., CD4+ T cells or CD8+ T cells), stimulating activation of T cells (e.g., CD4+ T cells or CD8+ T cells), activating resting T lymphocytes, promoting activation and proliferation of B cells, activating dendritic cells, promoting proliferation and activation of NK cells, increasing killing effect of NK cells, promoting survival of human peripheral blood eosinophils, promoting release of macrophage colony-stimulating factor (GMCSF), increasing survival rate of T cells and/or B cells, improving antigen presentation by antigen presenting cells (e.g., dendritic cells), improving antigen clearance, increasing production of cytokines (e.g., interleukin-2 or IFN-gamma), and the like. Typically, the immune response of a subject to which the antibody of the present invention is administered is enhanced when compared with the immune response of a subject without administration of the antibody of the present invention.

An anti-CD28 antibody (e.g., a humanized anti-CD28 antibody of the present invention) or antigen-binding fragment thereof, together with an anti-CD40 antibody (e.g., the affinity-matured anti-CD40 antibody of the present invention) or an antigen-binding fragment thereof, can form a multispecific antibody, e.g., a bispecific antibody (which may be called CD28-CD40 diabody for short hereinafter). The anti-CD28 antibody or the antigen-binding fragment thereof can be used as the agonist of a CD28 molecule and can provide a costimulatory signal for T cell activation, and the anti-CD40 antibody or the antigen-binding fragment thereof is the agonist of a CD40 molecule and can stimulate various immune cells (such as dendritic cells, macrophages, NK cells, granulocytes, etc.) to activate and play a role; the CD28-CD40 diabody can play an important role in immune response, e.g., enhancing the immune response of a subject, improving the effect of cellular immunotherapy, and particularly promoting the killing effect of CAR-T cells.

The term "cellular immunotherapy" or "cellular immunization therapy" used herein refers to the treatment of a disease, such as a tumor, an autoimmune disease, or an infectious disease, using cells contained in the immune system that are capable of resisting diseases or capable of helping the immune system resist diseases. Cells that can be used for cellular immunotherapy include, but are not limited to, T cells, NK cells, dendritic cells, regulatory T cells, cytotoxic T Cells (CTLs), macrophages, and the like. In cellular immunotherapy, autoimmune cells can be separated from a subject's blood or tumor, and then these cells are cultured *in vitro* before being transfused into the subject to kill the tumor cells. In addition, the immune cells can be genetically engineered to express a tumor-specific receptor and then cultured and transfused back to the patient, e.g., CAR-T cells. Accordingly, the present invention provides a pharmaceutical combination of the CD28-CD40 diabody of the present invention and an immune cell, which can be used to kill a target cell in a subject. The CD28-CD40 diabody of the present invention can enhance the killing effect of the immune cells on a target cell. The present invention also provides a method for enhancing the killing effect of an immune cell on a target cell in a subject, which comprises administering to a subject receiving the immune cell the CD28-CD40 diabody of the present invention, the polynucleotide encoding the diabody, the vector comprising the polynucleotide, or the pharmaceutical composition comprising the diabody, the polynucleotide, or the vector.

In some embodiments, the polynucleotide encoding the CD28-CD40 diabody of the present invention or the vector comprising the polynucleotide may be introduced into an immune cell, so that the immune cell can express the diabody. The polynucleotide may be a DNA or an RNA, e.g., an mRNA. The vector may be a plasmid vector or a viral vector, such as a lentiviral vector, an adenoviral vector, an adeno-associated viral vector, a retroviral vector, and the like. The vector may also be a circular RNA described above, a precursor RNA, or a vector comprising a DNA template for the precursor RNA. The "introducing" may be understood as "transfecting". The polynucleotide or vector may be transfected into an immune cell by, e.g., electroporation, calcium phosphate precipitation, DEAE-dextran transfection, virus-like particle infection, and other known manners. Thus, in some embodiments, the immune cell capable of expressing the CD28-CD40 diabody of the present invention can be administered to a subject to kill target cells in the subject. The present invention also provides an immune cell capable of expressing the CD28-CD40 diabody of the present invention.

The term "immune cell" or "immune effector cell" refers to immune cells used in cellular immunotherapy and having a therapeutic effect, e.g., immune cells that can kill target cells in cellular immunotherapy, such as T cells, NK cells, dendritic cells, regulatory T cells, cytotoxic T Cells (CTLs), macrophages, and the like. The "target cell" refers to a target cell to which the cellular immunotherapy is directed. In some embodiments, the immune cell comprises a CAR or can express a CAR. In some embodiments, the immune cell is a CAR-T cell. The CAR-T cell comprises a binding domain which can specifically bind to the antigen on the surface of a target cell, and thus can specifically kill the target cell. The method of the present invention can enhance the killing effect of the immune cell on the target cell compared with administration of the immune cell alone. The killing effect can be determined, e.g., by a reduction in the number of target cells. In some embodiments, the target cell is a tumor cell.

The present invention also provides use of the CD28-CD40 diabody of the present invention, the polynucleotide encoding the diabody, and the vector comprising the polynucleotide in preparing a medicament for enhancing the killing effect of an immune cell on a target cell in a subject.

In some embodiments, the present invention also provides a genetically engineered immune effector cell capable of expressing the CD28-CD40 diabody of the present invention, and the immune effector cell may be a T cell, an NK cell, an NKT cell, a macrophage, a neutrophil, or a granulocyte. The immune effector cell may comprise a polynucleotide encoding the CD28-CD40 diabody of the present invention or a vector comprising the polynucleotide. In some embodiments, the genetically engineered immune effector cell further recombinantly expresses a chimeric antigen receptor (CAR), a T cell receptor (TCR), or a bispecific T cell engager (BiTE), wherein the CAR, TCR, or BiTE may be a CAR, a TCR, or a BiTE that binds to a tumor antigen or a viral antigen. The viral antigen may be selected from HPV, EBV, and HIV The tumor antigen may be selected from Her2, NY-ESO-1, CD19, CD20, CD22, PSMA, c-Met, GPC3, IL13ra2, EGFR, CD123, CD7, GD2, PSCA, EBV16-E7, H3.3, EGFRvIII, BCMA, and mesothelin. In some embodiments, the CAR, the TCR, or the BiTE binds to mesothelin (MESO), CD123, BCMA, HER2, IL13Ra2, B7H3, or CD19.

In some embodiments, the CAR, TCR, or BiTE may bind to TSHR, CD19; CD123; CD22; CD30; CD171; CS-1; C-type lectin-like molecule-1, CD33; epidermal growth factor receptor variant III (EGFRvIII); ganglioside G2 (GD2); ganglioside GD3; TNF receptor family members; B cell maturation antigen (BCMA); Tn antigen ((Tn Ag) or (GalNAca-Ser/Thr)); prostate-specific membrane antigen (PSMA); receptor tyrosine kinase-like orphan receptor 1 (ROR1); Fms-like tyrosine kinase 3 (FLT3); tumor-associated glycoprotein 72 (TAG72); CD38; CD44v6; carcinoembryonic antigen (CEA); epithelial cell adhesion molecule (EPCAM); B7H3 (CD276); KIT (CD117); interleukin-13 receptor subunit α-2; mesothelin; interleukin 11 receptor α (IL-1 1Ra); prostate stem cell antigen (PSCA); protease serine 21; vascular endothelial growth factor receptor 2 (VEGFR2); Lewis(Y) antigen; CD24; platelet-derived growth factor receptor β (PDGFR-beta); stage-specific embryonic antigen-4 (SSEA-4); CD20; folate receptor α; receptor tyrosine-protein kinase ERBB2 (Her2/neu); mucin 1, cell surface associated (MUC1); epidermal growth factor receptor (EGFR); neural cell adhesion molecule (NCAM); prostase; prostatic acid phosphatase (PAP); elongation factor 2 mutated (ELF2M); Ephrin B2; fibroblast activation protein α (FAP); insulin-like growth factor 1 receptor (IGF-I receptor); carbonic anhydrase IX (CAIX); proteasome (Prosome, Macropain) subunit, β Type, 9 (LMP2); glycoprotein 100 (gp100); oncogene polypeptide composed of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weight-melanoma-associated antigen (HMWMAA); o-acetyl-GD2 ganglioside (OAcGD2); folate receptor β; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); polysialic acid; placenta-specific 1 (PLAC1); hexasaccharide portion of globoH glycoceramide (GloboH); mammary gland differentiation antigen (NY-BR-1); uroplakin 2 (UPK2); hepatitis A virus cellular receptor 1 (HAVCR1); adrenoceptor β3 (ADRB3); pannexin 3 (PANX3); G protein-coupled receptor 20 (GPR20); lymphocyte antigen 6 complex, locus K9 (LY6K); olfactory receptor 51E2 (OR51E2); TCR gamma alternate reading frame protein (TARP); Wilms tumor protein (WTl); cancer/testis antigen 1 (NY-ESO-1); cancer/testis antigen 2 (LAGE-la); melanoma-associated antigen 1 (MAGE-A1); ETS translocation-variant gene 6, located on chromosome 12p (ETV6-AML); sperm protein 17 (SPA 17); X antigen family, member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1; melanoma antigen recognized by T cells 1; rat sarcoma (Ras) mutant; human telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-acetyl glucosaminyl-transferase V (NA17); paired box protein Pax-3 (PAX3); androgen receptor; Cyclin Bl; v-myc avian myelocytomatosis viral oncogene neuroblastoma derived homolog (MYCN); Ras homolog family member C (RhoC); tyrosinase-related protein 2 (TRP-2); cytochrome P450 1B 1 (CYP1B1); CCCTC-binding factor (zinc finger protein)-Like, squamous cell carcinoma antigen recognized by T cells 3 (SART3); paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); receptor for advanced glycation endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; leukocyte-associated immunoglobulin-like receptor 1 (LAIRl); Fc fragment of IgA receptor (FCAR or CD89); leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); or immunoglobulin λ-like polypeptide 1 (IGLL1).

In some embodiments, the genetically engineered immune effector cell comprises a first polynucleotide encoding the CD40-CD28 diabody of the present invention and a second polynucleotide encoding the CAR, TCR, or BiTE (CAR/TCR/BiTE). The first polynucleotide and the second polynucleotide may be separately expressed in separate expression cassettes or may be co-expressed in the same expression cassette. When co-expressed in the same expression cassette, the first polynucleotide and the second polynucleotide may have a linker between them. The linker may be a self-cleaving linker, e.g., 2A linkers, such as T2A, P2A, or F2A. The polynucleotide encoding the CD28-CD40 diabody of the present invention or a vector comprising the polynucleotide may be introduced into an immune cell, so that the immune cell can express the diabody. The first polynucleotide and the second polynucleotide may be DNA or RNA, e.g., mRNA. The first polynucleotide and the second polynucleotide may be introduced into the immune effector cell by any means known in the art, for example, they may be introduced into the immune effector cell by transfecting the immune effector cell with a vector or mRNA. The vector may be a plasmid vector or a viral vector, such as a lentiviral vector, an adenoviral vector, an adeno-associated viral vector, a retroviral vector, and the like. The vector may also be a circular RNA described above, a precursor RNA, or a vector comprising a DNA template for the precursor RNA.

In some embodiments, the CAR-T cell comprises a CAR with a HER2-binding molecule (such as an anti-HER2 antibody) as a binding domain and targets a cell expressing HER2. In some embodiments, the CAR-T cell comprises a CAR with a mesothelin-binding molecule (e.g., an anti-mesothelin antibody) as a binding domain and targets a cell expressing mesothelin. In some embodiments, the target cell of the CAR-T cell is a tumor cell expressing HER2 or mesothelin.

In some embodiments, the CAR may comprise a signal peptide, a hinge region, a transmembrane domain, and an intracellular signaling domain. The intracellular signaling domain may further comprise a costimulatory domain. In some embodiments, the signal peptide may include a CD8 signal peptide or a GM-CSF signal peptide. In some embodiments, the hinge region of the CAR may include a hinge region of CD28, CD8, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, or LIGHT, preferably a CD8 hinge region. In some embodiments, the transmembrane domain of the CAR may include a transmembrane domain of CD8, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, or SLAM, preferably a CD8 transmembrane (TM) domain. In some embodiments, the intracellular signaling domain of the CAR may include an intracellular signaling domain of CD3ζ, CD3δ, CD3γ, CD3ε, CD79a, CD79b, FceRIγ, FceRIβ, FcγRIIa, DAP10, or DAP-12, preferably a CD3ζ intracellular signaling domain. In some embodiments, the intracellular signaling domain of the CAR may further comprise a costimulatory domain, such as a costimulatory domain of CD28, 4-1BB (CD137), CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, or MyD88, preferably a costimulatory domain of 4-1BB.

In the present invention, the CAR having a "BBZ" structure refers to a CAR having a 4-1BB costimulatory molecule, generally comprising a CD8 hinge domain, a CD8 transmembrane (TM) domain, a 4-1BB costimulatory domain, and a CD3ζ domain. In some embodiments, the CAR used in the present invention has a BBZ structure.

In some embodiments, the CAR comprises an antibody or an antigen-binding fragment thereof as a binding domain. In some embodiments, the antibody or the antigen-binding fragment specifically binds to mesothelin (MESO), CD123, BCMA, HER2, IL13Ra2, or B7H3. In some embodiments, the antibody is an scFv. In some embodiments, the scFv comprises a heavy chain variable region (VH) fused to the N-terminus or C-terminus of a light chain variable region (VL). In some embodiments, there is a peptide linker between the VH and VL of the scFv. In some embodiments, the antibody that specifically binds to mesothelin, also referred to as an anti-mesothelin (or anti-MESO or anti-MSLN) antibody, as described in PCT/CN2021/112767 (incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 87, an LCDR2 set forth in SEQ ID NO: 102, an LCDR3 set forth in SEQ ID NO: 116, an HCDR1 set forth in SEQ ID NO: 131, an HCDR2 set forth in SEQ ID NO: 144, and an HCDR3 set forth in SEQ ID NO: 157;
(2) an LCDR1 set forth in SEQ ID NO: 88, an LCDR2 set forth in SEQ ID NO: 103, an LCDR3 set forth in SEQ ID NO: 117, an HCDR1 set forth in SEQ ID NO: 132, an HCDR2 set forth in SEQ ID NO: 145, and an HCDR3 set forth in SEQ ID NO: 158;
(3) an LCDR1 set forth in SEQ ID NO: 89, an LCDR2 set forth in SEQ ID NO: 104, an LCDR3 set forth in SEQ ID NO: 118, an HCDR1 set forth in SEQ ID NO: 133, an HCDR2 set forth in SEQ ID NO: 146, and an HCDR3 set forth in SEQ ID NO: 159;
(4) an LCDR1 set forth in SEQ ID NO: 90, an LCDR2 set forth in SEQ ID NO: 105, an LCDR3 set forth in SEQ ID NO: 119, an HCDR1 set forth in SEQ ID NO: 134, an HCDR2 set forth in SEQ ID NO: 147, and an HCDR3 set forth in SEQ ID NO: 160;
(5) an LCDR1 set forth in SEQ ID NO: 91, an LCDR2 set forth in SEQ ID NO: 106, an LCDR3 set forth in SEQ ID NO: 120, an HCDR1 set forth in SEQ ID NO: 135, an HCDR2 set forth in SEQ ID NO: 148, and an HCDR3 set forth in SEQ ID NO: 161;
(6) an LCDR1 set forth in SEQ ID NO: 92, an LCDR2 set forth in SEQ ID NO: 107, an LCDR3 set forth in SEQ ID NO: 121, an HCDR1 set forth in SEQ ID NO: 136, an HCDR2 set forth in SEQ ID NO: 149, and an HCDR3 set forth in SEQ ID NO: 162;
(7) an LCDR1 set forth in SEQ ID NO: 93, an LCDR2 set forth in SEQ ID NO: 108, an LCDR3 set forth in SEQ ID NO: 122, an HCDR1 set forth in SEQ ID NO: 137, an HCDR2 set forth in SEQ ID NO: 150, and an HCDR3 set forth in SEQ ID NO: 163;
(8) an LCDR1 set forth in SEQ ID NO: 94, an LCDR2 set forth in SEQ ID NO: 109, an LCDR3 set forth in SEQ ID NO: 123, an HCDR1 set forth in SEQ ID NO: 138, an HCDR2 set forth in SEQ ID NO: 151, and an HCDR3 set forth in SEQ ID NO: 164;
(9) an LCDR1 set forth in SEQ ID NO: 95, an LCDR2 set forth in SEQ ID NO: 110, an LCDR3 set forth in SEQ ID NO: 124, an HCDR1 set forth in SEQ ID NO: 139, an HCDR2 set forth in SEQ ID NO: 152, and an HCDR3 set forth in SEQ ID NO: 165;
(10) an LCDR1 set forth in SEQ ID NO: 96, an LCDR2 set forth in SEQ ID NO: 111, an LCDR3 set forth in SEQ ID NO: 125, an HCDR1 set forth in SEQ ID NO: 134, an HCDR2 set forth in SEQ ID NO: 147, and an HCDR3 set forth in SEQ ID NO: 166;
(11) an LCDR1 set forth in SEQ ID NO: 97, an LCDR2 set forth in SEQ ID NO: 112, an LCDR3 set forth in SEQ ID NO: 126, an HCDR1 set forth in SEQ ID NO: 140, an HCDR2 set forth in SEQ ID NO: 153, and an HCDR3 set forth in SEQ ID NO: 167;
(12) an LCDR1 set forth in SEQ ID NO: 98, an LCDR2 set forth in SEQ ID NO: 113, an LCDR3 set forth in SEQ ID NO: 127, an HCDR1 set forth in SEQ ID NO: 139, an HCDR2 set forth in SEQ ID NO: 152, and an HCDR3 set forth in SEQ ID NO: 168;
(13) an LCDR1 set forth in SEQ ID NO: 99, an LCDR2 set forth in SEQ ID NO: 114, an LCDR3 set forth in SEQ ID NO: 128, an HCDR1 set forth in SEQ ID NO: 141, an HCDR2 set forth in SEQ ID NO: 154, and an HCDR3 set forth in SEQ ID NO: 169;
(14) an LCDR1 set forth in SEQ ID NO: 100, an LCDR2 set forth in SEQ ID NO: 115, an LCDR3 set forth in SEQ ID NO: 129, an HCDR1 set forth in SEQ ID NO: 142, an HCDR2 set forth in SEQ ID NO: 155, and an HCDR3 set forth in SEQ ID NO: 170; and
(15) an LCDR1 set forth in SEQ ID NO: 101, an LCDR2 set forth in SEQ ID NO: 104, an LCDR3 set forth in SEQ ID NO: 130, an HCDR1 set forth in SEQ ID NO: 143, an HCDR2 set forth in SEQ ID NO: 156, and an HCDR3 set forth in SEQ ID NO: 171.

In some embodiments, the anti-MESO antibody comprises a light chain variable region and a heavy chain variable region selected from the group consisting of:
(1) a light chain variable region set forth in SEQ ID NO: 172 and a heavy chain variable region set forth in SEQ ID NO: 187;
(2) a light chain variable region set forth in SEQ ID NO: 173 and a heavy chain variable region set forth in SEQ ID NO: 188;
(3) a light chain variable region set forth in SEQ ID NO: 174 and a heavy chain variable region set forth in SEQ ID NO: 189;
(4) a light chain variable region set forth in SEQ ID NO: 175 and a heavy chain variable region set forth in SEQ ID NO: 190;
(5) a light chain variable region set forth in SEQ ID NO: 176 and a heavy chain variable region set forth in SEQ ID NO: 191;
(6) a light chain variable region set forth in SEQ ID NO: 177 and a heavy chain variable region set forth in SEQ ID NO: 192;
(7) a light chain variable region set forth in SEQ ID NO: 178 and a heavy chain variable region set forth in SEQ ID NO: 193;
(8) a light chain variable region set forth in SEQ ID NO: 179 and a heavy chain variable region set forth in SEQ ID NO: 194;
(9) a light chain variable region set forth in SEQ ID NO: 180 and a heavy chain variable region set forth in SEQ ID NO: 195;
(10) a light chain variable region set forth in SEQ ID NO: 181 and a heavy chain variable region set forth in SEQ ID NO: 196;
(11) a light chain variable region set forth in SEQ ID NO: 182 and a heavy chain variable region set forth in SEQ ID NO: 197;
(12) a light chain variable region set forth in SEQ ID NO: 183 and a heavy chain variable region set forth in SEQ ID NO: 198;
(13) a light chain variable region set forth in SEQ ID NO: 184 and a heavy chain variable region set forth in SEQ ID NO: 199;
(14) a light chain variable region set forth in SEQ ID NO: 185 and a heavy chain variable region set forth in SEQ ID NO: 200; and
(15) a light chain variable region set forth in SEQ ID NO: 186 and a heavy chain variable region set forth in SEQ ID NO: 201.

In some embodiments, the anti-MESO antibody is an anti-MESO scFv and may comprise an amino acid sequence selected from SEQ ID NOs: 202-216.

In some embodiments, the CAR comprises a binding domain that may specifically bind to mesothelin, which may be referred to as a CAR targeting mesothelin. In some embodiments, the CAR targeting mesothelin may be a CAR described in PCT/CN2021/112767 (incorporated herein by reference in its entirety). In some embodiments, the CAR targeting mesothelin may comprise any one of the anti-mesothelin antibodies described above. In some embodiments, the CAR targeting mesothelin may comprise an amino acid sequence selected from SEQ ID NOs: 217-231.

In some embodiments, the antibody that specifically binds to CD123, also referred to as an anti-CD123 antibody, is an antibody described in PCT/CN2021/112748 (incorporated herein by reference in its entirety), and comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 349, an LCDR2 set forth in SEQ ID NO: 379, an LCDR3 set forth in SEQ ID NO: 407, an HCDR1 set forth in SEQ ID NO: 437, an HCDR2 set forth in SEQ ID NO: 458, and an HCDR3 set forth in SEQ ID NO: 482;
(2) an LCDR1 set forth in SEQ ID NO: 363, an LCDR2 set forth in SEQ ID NO: 391, an LCDR3 set forth in SEQ ID NO: 421, an HCDR1 set forth in SEQ ID NO: 435, an HCDR2 set forth in SEQ ID NO: 468, and an HCDR3 set forth in SEQ ID NO: 495;
(3) an LCDR1 set forth in SEQ ID NO: 353, an LCDR2 set forth in SEQ ID NO: 383, an LCDR3 set forth in SEQ ID NO: 412, an HCDR1 set forth in SEQ ID NO: 441, an HCDR2 set forth in SEQ ID NO: 462, and an HCDR3 set forth in SEQ ID NO: 486;
(4) an LCDR1 set forth in SEQ ID NO: 364, an LCDR2 set forth in SEQ ID NO: 378, an LCDR3 set forth in SEQ ID NO: 422, an HCDR1 set forth in SEQ ID NO: 446, an HCDR2 set forth in SEQ ID NO: 469, and an HCDR3 set forth in SEQ ID NO: 496;
(5) an LCDR1 set forth in SEQ ID NO: 369, an LCDR2 set forth in SEQ ID NO: 396, an LCDR3 set forth in SEQ ID NO: 428, an HCDR1 set forth in SEQ ID NO: 445, an HCDR2 set forth in SEQ ID NO: 475, and an HCDR3 set forth in SEQ ID NO: 503;
(6) an LCDR1 set forth in SEQ ID NO: 370, an LCDR2 set forth in SEQ ID NO: 397, an LCDR3 set forth in SEQ ID NO: 429, an HCDR1 set forth in SEQ ID NO: 452, an HCDR2 set forth in SEQ ID NO: 474, and an HCDR3 set forth in SEQ ID NO: 504;
(7) an LCDR1 set forth in SEQ ID NO: 354, an LCDR2 set forth in SEQ ID NO: 384, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(8) an LCDR1 set forth in SEQ ID NO: 350, an LCDR2 set forth in SEQ ID NO: 380, an LCDR3 set forth in SEQ ID NO: 408, an HCDR1 set forth in SEQ ID NO: 435, an HCDR2 set forth in SEQ ID NO: 456, and an HCDR3 set forth in SEQ ID NO: 480;
(9) an LCDR1 set forth in SEQ ID NO: 371, an LCDR2 set forth in SEQ ID NO: 398, an LCDR3 set forth in SEQ ID NO: 430, an HCDR1 set forth in SEQ ID NO: 453, an HCDR2 set forth in SEQ ID NO: 476, and an HCDR3 set forth in SEQ ID NO: 505;
(10) an LCDR1 set forth in SEQ ID NO: 372, an LCDR2 set forth in SEQ ID NO: 399, an LCDR3 set forth in SEQ ID NO: 431, an HCDR1 set forth in SEQ ID NO: 441, an HCDR2 set forth in SEQ ID NO: 462, and an HCDR3 set forth in SEQ ID NO: 506;
(11) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(12) an LCDR1 set forth in SEQ ID NO: 363, an LCDR2 set forth in SEQ ID NO: 392, an LCDR3 set forth in SEQ ID NO: 423, an HCDR1 set forth in SEQ ID NO: 447, an HCDR2 set forth in SEQ ID NO: 470, and an HCDR3 set forth in SEQ ID NO: 497;
(13) an LCDR1 set forth in SEQ ID NO: 373, an LCDR2 set forth in SEQ ID NO: 400, an LCDR3 set forth in SEQ ID NO: 432, an HCDR1 set forth in SEQ ID NO: 451, an HCDR2 set forth in SEQ ID NO: 477, and an HCDR3 set forth in SEQ ID NO: 507;
(14) an LCDR1 set forth in SEQ ID NO: 355, an LCDR2 set forth in SEQ ID NO: 385, an LCDR3 set forth in SEQ ID NO: 414, an HCDR1 set forth in SEQ ID NO: 443, an HCDR2 set forth in SEQ ID NO: 464, and an HCDR3 set forth in SEQ ID NO: 488;
(15) an LCDR1 set forth in SEQ ID NO: 356, an LCDR2 set forth in SEQ ID NO: 386, an LCDR3 set forth in SEQ ID NO: 415, an HCDR1 set forth in SEQ ID NO: 444, an HCDR2 set forth in SEQ ID NO: 465, and an HCDR3 set forth in SEQ ID NO: 489;
(16) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 451, an HCDR2 set forth in SEQ ID NO: 474, and an HCDR3 set forth in SEQ ID NO: 502;
(17) an LCDR1 set forth in SEQ ID NO: 351, an LCDR2 set forth in SEQ ID NO: 381, an LCDR3 set forth in SEQ ID NO: 409, an HCDR1 set forth in SEQ ID NO: 438, an HCDR2 set forth in SEQ ID NO: 459, and an HCDR3 set forth in SEQ ID NO: 483;
(18) an LCDR1 set forth in SEQ ID NO: 347, an LCDR2 set forth in SEQ ID NO: 376, an LCDR3 set forth in SEQ ID NO: 410, an HCDR1 set forth in SEQ ID NO: 439, an HCDR2 set forth in SEQ ID NO: 460, and an HCDR3 set forth in SEQ ID NO: 484;
(19) an LCDR1 set forth in SEQ ID NO: 357, an LCDR2 set forth in SEQ ID NO: 387, an LCDR3 set forth in SEQ ID NO: 416, an HCDR1 set forth in SEQ ID NO: 437, an HCDR2 set forth in SEQ ID NO: 466, and an HCDR3 set forth in SEQ ID NO: 490;
(20) an LCDR1 set forth in SEQ ID NO: 347, an LCDR2 set forth in SEQ ID NO: 377, an LCDR3 set forth in SEQ ID NO: 405, an HCDR1 set forth in SEQ ID NO: 435, an HCDR2 set forth in SEQ ID NO: 456, and an HCDR3 set forth in SEQ ID NO: 480;
(21) an LCDR1 set forth in SEQ ID NO: 365, an LCDR2 set forth in SEQ ID NO: 393, an LCDR3 set forth in SEQ ID NO: 424, an HCDR1 set forth in SEQ ID NO: 448, an HCDR2 set forth in SEQ ID NO: 471, and an HCDR3 set forth in SEQ ID NO: 498;
(22) an LCDR1 set forth in SEQ ID NO: 374, an LCDR2 set forth in SEQ ID NO: 401, an LCDR3 set forth in SEQ ID NO: 433, an HCDR1 set forth in SEQ ID NO: 454, an HCDR2 set forth in SEQ ID NO: 478, and an HCDR3 set forth in SEQ ID NO: 508;
(23) an LCDR1 set forth in SEQ ID NO: 358, an LCDR2 set forth in SEQ ID NO: 385, an LCDR3 set forth in SEQ ID NO: 417, an HCDR1 set forth in SEQ ID NO: 442, an HCDR2 set forth in SEQ ID NO: 463, and an HCDR3 set forth in SEQ ID NO: 491;
(24) an LCDR1 set forth in SEQ ID NO: 359, an LCDR2 set forth in SEQ ID NO: 388, an LCDR3 set forth in SEQ ID NO: 418, an HCDR1 set forth in SEQ ID NO: 441, an HCDR2 set forth in SEQ ID NO: 462, and an HCDR3 set forth in SEQ ID NO: 492;
(25) an LCDR1 set forth in SEQ ID NO: 347, an LCDR2 set forth in SEQ ID NO: 376, an LCDR3 set forth in SEQ ID NO: 403, an HCDR1 set forth in SEQ ID NO: 435, an HCDR2 set forth in SEQ ID NO: 456, and an HCDR3 set forth in SEQ ID NO: 480;
(26) an LCDR1 set forth in SEQ ID NO: 366, an LCDR2 set forth in SEQ ID NO: 394, an LCDR3 set forth in SEQ ID NO: 425, an HCDR1 set forth in SEQ ID NO: 449, an HCDR2 set forth in SEQ ID NO: 472, and an HCDR3 set forth in SEQ ID NO: 499;
(27) an LCDR1 set forth in SEQ ID NO: 360, an LCDR2 set forth in SEQ ID NO: 389, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(28) an LCDR1 set forth in SEQ ID NO: 347, an LCDR2 set forth in SEQ ID NO: 376, an LCDR3 set forth in SEQ ID NO: 404, an HCDR1 set forth in SEQ ID NO: 435, an HCDR2 set forth in SEQ ID NO: 456, and an HCDR3 set forth in SEQ ID NO: 480;
(29) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(30) an LCDR1 set forth in SEQ ID NO: 361, an LCDR2 set forth in SEQ ID NO: 390, an LCDR3 set forth in SEQ ID NO: 419, an HCDR1 set forth in SEQ ID NO: 445, an HCDR2 set forth in SEQ ID NO: 467, and an HCDR3 set forth in SEQ ID NO: 493;
(31) an LCDR1 set forth in SEQ ID NO: 375, an LCDR2 set forth in SEQ ID NO: 402, an LCDR3 set forth in SEQ ID NO: 434, an HCDR1 set forth in SEQ ID NO: 455, an HCDR2 set forth in SEQ ID NO: 479, and an HCDR3 set forth in SEQ ID NO: 509;
(32) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 450, an HCDR2 set forth in SEQ ID NO: 473, and an HCDR3 set forth in SEQ ID NO: 5015;
(33) an LCDR1 set forth in SEQ ID NO: 362, an LCDR2 set forth in SEQ ID NO: 388, an LCDR3 set forth in SEQ ID NO: 420, an HCDR1 set forth in SEQ ID NO: 441, an HCDR2 set forth in SEQ ID NO: 462, and an HCDR3 set forth in SEQ ID NO: 494;
(34) an LCDR1 set forth in SEQ ID NO: 352, an LCDR2 set forth in SEQ ID NO: 382, an LCDR3 set forth in SEQ ID NO: 411, an HCDR1 set forth in SEQ ID NO: 440, an HCDR2 set forth in SEQ ID NO: 461, and an HCDR3 set forth in SEQ ID NO: 485; and
(35) an LCDR1 set forth in SEQ ID NO: 367, an LCDR2 set forth in SEQ ID NO: 391, an LCDR3 set forth in SEQ ID NO: 426, an HCDR1 set forth in SEQ ID NO: 435, an HCDR2 set forth in SEQ ID NO: 468, and an HCDR3 set forth in SEQ ID NO: 500.

In some embodiments, the anti-CD123 antibody comprises a light chain variable region and a heavy chain variable region selected from the group consisting of:
(1) a light chain variable region set forth in SEQ ID NO: 510 and a heavy chain variable region set forth in SEQ ID NO: 545;
(2) a light chain variable region set forth in SEQ ID NO: 511 and a heavy chain variable region set forth in SEQ ID NO: 546;
(3) a light chain variable region set forth in SEQ ID NO: 512 and a heavy chain variable region set forth in SEQ ID NO: 547;
(4) a light chain variable region set forth in SEQ ID NO: 513 and a heavy chain variable region set forth in SEQ ID NO: 548;
(5) a light chain variable region set forth in SEQ ID NO: 514 and a heavy chain variable region set forth in SEQ ID NO: 549;
(6) a light chain variable region set forth in SEQ ID NO: 515 and a heavy chain variable region set forth in SEQ ID NO: 550;
(7) a light chain variable region set forth in SEQ ID NO: 516 and a heavy chain variable region set forth in SEQ ID NO: 551;
(8) a light chain variable region set forth in SEQ ID NO: 517 and a heavy chain variable region set forth in SEQ ID NO: 552;
(9) a light chain variable region set forth in SEQ ID NO: 518 and a heavy chain variable region set forth in SEQ ID NO: 553;
(10) a light chain variable region set forth in SEQ ID NO: 519 and a heavy chain variable region set forth in SEQ ID NO: 554;
(11) a light chain variable region set forth in SEQ ID NO: 520 and a heavy chain variable region set forth in SEQ ID NO: 555;
(12) a light chain variable region set forth in SEQ ID NO: 521 and a heavy chain variable region set forth in SEQ ID NO: 556;
(13) a light chain variable region set forth in SEQ ID NO: 522 and a heavy chain variable region set forth in SEQ ID NO: 557;
(14) a light chain variable region set forth in SEQ ID NO: 523 and a heavy chain variable region set forth in SEQ ID NO: 558;
(15) a light chain variable region set forth in SEQ ID NO: 524 and a heavy chain variable region set forth in SEQ ID NO: 559;
(16) a light chain variable region set forth in SEQ ID NO: 525 and a heavy chain variable region set forth in SEQ ID NO: 560;
(17) a light chain variable region set forth in SEQ ID NO: 526 and a heavy chain variable region set forth in SEQ ID NO: 561;
(18) a light chain variable region set forth in SEQ ID NO: 527 and a heavy chain variable region set forth in SEQ ID NO: 562;
(19) a light chain variable region set forth in SEQ ID NO: 528 and a heavy chain variable region set forth in SEQ ID NO: 563;
(20) a light chain variable region set forth in SEQ ID NO: 529 and a heavy chain variable region set forth in SEQ ID NO: 564;
(21) a light chain variable region set forth in SEQ ID NO: 530 and a heavy chain variable region set forth in SEQ ID NO: 565;
(22) a light chain variable region set forth in SEQ ID NO: 531 and a heavy chain variable region set forth in SEQ ID NO: 566;
(23) a light chain variable region set forth in SEQ ID NO: 532 and a heavy chain variable region set forth in SEQ ID NO: 567;
(24) a light chain variable region set forth in SEQ ID NO: 533 and a heavy chain variable region set forth in SEQ ID NO: 568;
(25) a light chain variable region set forth in SEQ ID NO: 534 and a heavy chain variable region set forth in SEQ ID NO: 569;
(26) a light chain variable region set forth in SEQ ID NO: 535 and a heavy chain variable region set forth in SEQ ID NO: 570;
(27) a light chain variable region set forth in SEQ ID NO: 536 and a heavy chain variable region set forth in SEQ ID NO: 571;
(28) a light chain variable region set forth in SEQ ID NO: 537 and a heavy chain variable region set forth in SEQ ID NO: 572;
(29) a light chain variable region set forth in SEQ ID NO: 538 and a heavy chain variable region set forth in SEQ ID NO: 573;
(30) a light chain variable region set forth in SEQ ID NO: 539 and a heavy chain variable region set forth in SEQ ID NO: 574;
(31) a light chain variable region set forth in SEQ ID NO: 540 and a heavy chain variable region set forth in SEQ ID NO: 575;
(32) a light chain variable region set forth in SEQ ID NO: 541 and a heavy chain variable region set forth in SEQ ID NO: 576;
(33) a light chain variable region set forth in SEQ ID NO: 542 and a heavy chain variable region set forth in SEQ ID NO: 577;
(34) a light chain variable region set forth in SEQ ID NO: 543 and a heavy chain variable region set forth in SEQ ID NO: 578; and
(35) a light chain variable region set forth in SEQ ID NO: 544 and a heavy chain variable region set forth in SEQ ID NO: 579.

In some embodiments, the anti-CD123 antibody is an anti-CD123 scFv and may comprise any amino acid sequence selected from SEQ ID NOs: 583-617.

In some embodiments, the CAR comprises a binding domain that may specifically bind to CD123, which may be referred to as a CAR targeting CD123. In some embodiments, the CAR targeting CD123 may be a CAR described in PCT/CN2021/112748 (incorporated herein by reference in its entirety). In some embodiments, the CAR targeting CD123 may comprise any one of the anti-CD123 antibodies described above. In some embodiments, the CAR targeting CD123 may comprise an amino acid sequence selected from SEQ ID NOs: 580-582.

In some embodiments, the antibody that specifically binds to BCMA, also referred to as an anti-BCMA antibody, as described in PCT/CN2021/112798 (incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 232, an LCDR2 set forth in SEQ ID NO: 242, an LCDR3 set forth in SEQ ID NO: 253, an HCDR1 set forth in SEQ ID NO: 264, an HCDR2 set forth in SEQ ID NO: 275, and an HCDR3 set forth in SEQ ID NO: 287;
(2) an LCDR1 set forth in SEQ ID NO: 233, an LCDR2 set forth in SEQ ID NO: 243, an LCDR3 set forth in SEQ ID NO: 254, an HCDR1 set forth in SEQ ID NO: 265, an HCDR2 set forth in SEQ ID NO: 276, and an HCDR3 set forth in SEQ ID NO: 288;
(3) an LCDR1 set forth in SEQ ID NO: 234, an LCDR2 set forth in SEQ ID NO: 244, an LCDR3 set forth in SEQ ID NO: 255, an HCDR1 set forth in SEQ ID NO: 266, an HCDR2 set forth in SEQ ID NO: 277, and an HCDR3 set forth in SEQ ID NO: 289;
(4) an LCDR1 set forth in SEQ ID NO: 235, an LCDR2 set forth in SEQ ID NO: 245, an LCDR3 set forth in SEQ ID NO: 255, an HCDR1 set forth in SEQ ID NO: 267, an HCDR2 set forth in SEQ ID NO: 278, and an HCDR3 set forth in SEQ ID NO: 290;
(5) an LCDR1 set forth in SEQ ID NO: 236, an LCDR2 set forth in SEQ ID NO: 246, an LCDR3 set forth in SEQ ID NO: 256, an HCDR1 set forth in SEQ ID NO: 268, an HCDR2 set forth in SEQ ID NO: 279, and an HCDR3 set forth in SEQ ID NO: 291;
(6) an LCDR1 set forth in SEQ ID NO: 237, an LCDR2 set forth in SEQ ID NO: 247, an LCDR3 set forth in SEQ ID NO: 257, an HCDR1 set forth in SEQ ID NO: 269, an HCDR2 set forth in SEQ ID NO: 280, and an HCDR3 set forth in SEQ ID NO: 292;
(7) an LCDR1 set forth in SEQ ID NO: 238, an LCDR2 set forth in SEQ ID NO: 248, an LCDR3 set forth in SEQ ID NO: 258, an HCDR1 set forth in SEQ ID NO: 266, an HCDR2 set forth in SEQ ID NO: 281, and an HCDR3 set forth in SEQ ID NO: 293;
(8) an LCDR1 set forth in SEQ ID NO: 239, an LCDR2 set forth in SEQ ID NO: 249, an LCDR3 set forth in SEQ ID NO: 259, an HCDR1 set forth in SEQ ID NO: 270, an HCDR2 set forth in SEQ ID NO: 282, and an HCDR3 set forth in SEQ ID NO: 294;
(9) an LCDR1 set forth in SEQ ID NO: 233, an LCDR2 set forth in SEQ ID NO: 250, an LCDR3 set forth in SEQ ID NO: 260, an HCDR1 set forth in SEQ ID NO: 271, an HCDR2 set forth in SEQ ID NO: 283, and an HCDR3 set forth in SEQ ID NO: 295;
(10) an LCDR1 set forth in SEQ ID NO: 233, an LCDR2 set forth in SEQ ID NO: 251, an LCDR3 set forth in SEQ ID NO: 261, an HCDR1 set forth in SEQ ID NO: 272, an HCDR2 set forth in SEQ ID NO: 284, and an HCDR3 set forth in SEQ ID NO: 296;
(11) an LCDR1 set forth in SEQ ID NO: 240, an LCDR2 set forth in SEQ ID NO: 252, an LCDR3 set forth in SEQ ID NO: 262, an HCDR1 set forth in SEQ ID NO: 273, an HCDR2 set forth in SEQ ID NO: 285, and an HCDR3 set forth in SEQ ID NO: 297; and
(12) an LCDR1 set forth in SEQ ID NO: 241, an LCDR2 set forth in SEQ ID NO: 245, an LCDR3 set forth in SEQ ID NO: 263, an HCDR1 set forth in SEQ ID NO: 274, an HCDR2 set forth in SEQ ID NO: 286, and an HCDR3 set forth in SEQ ID NO: 298.

In some embodiments, the anti-BCMA antibody comprises a light chain variable region and a heavy chain variable region selected from the group consisting of:
(1) a light chain variable region set forth in SEQ ID NO: 299 and a heavy chain variable region set forth in SEQ ID NO: 311;
(2) a light chain variable region set forth in SEQ ID NO: 300 and a heavy chain variable region set forth in SEQ ID NO: 312;
(3) a light chain variable region set forth in SEQ ID NO: 301 and a heavy chain variable region set forth in SEQ ID NO: 313;
(4) a light chain variable region set forth in SEQ ID NO: 302 and a heavy chain variable region set forth in SEQ ID NO: 314;
(5) a light chain variable region set forth in SEQ ID NO: 303 and a heavy chain variable region set forth in SEQ ID NO: 315;
(6) a light chain variable region set forth in SEQ ID NO: 304 and a heavy chain variable region set forth in SEQ ID NO: 316;
(7) a light chain variable region set forth in SEQ ID NO: 305 and a heavy chain variable region set forth in SEQ ID NO: 317;
(8) a light chain variable region set forth in SEQ ID NO: 306 and a heavy chain variable region set forth in SEQ ID NO: 318;
(9) a light chain variable region set forth in SEQ ID NO: 307 and a heavy chain variable region set forth in SEQ ID NO: 319;
(10) a light chain variable region set forth in SEQ ID NO: 308 and a heavy chain variable region set forth in SEQ ID NO: 320;
(11) a light chain variable region set forth in SEQ ID NO: 309 and a heavy chain variable region set forth in SEQ ID NO: 321; and
(12) a light chain variable region set forth in SEQ ID NO: 310 and a heavy chain variable region set forth in SEQ ID NO: 322.

In some embodiments, the anti-BCMA antibody is an anti-BCMA scFv and may comprise an amino acid sequence selected from SEQ ID NOs: 323-334.

In some embodiments, the CAR comprises a binding domain that may specifically bind to BCMA, which may be referred to as a CAR targeting BCMA. In some embodiments, the CAR targeting BCMA may be a CAR described in PCT/CN2021/112798 (incorporated herein by reference in its entirety). In some embodiments, the CAR targeting BCMA may comprise any one of the anti-BCMA antibodies described above. In some embodiments, the CAR targeting BCMA may comprise an amino acid sequence selected from SEQ ID NOs: 335-346.

In some embodiments, the antibody that specifically binds to CD19, also referred to as an anti-CD19 antibody, as described in the Chinese patent application CN202210274255.1 (publication No. CN114349863A, which is incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 may be an LCDR1 set forth in SEQ ID NO: 628, an LCDR2 set forth in SEQ ID NO: 629, an LCDR3 set forth in SEQ ID NO: 630, an HCDR1 set forth in SEQ ID NO: 631, an HCDR2 set forth in SEQ ID NO: 632, and an HCDR3 set forth in SEQ ID NO: 633.

In some embodiments, the anti-CD19 antibody comprises a light chain variable region set forth in SEQ ID NO: 635 and a heavy chain variable region set forth in SEQ ID NO: 634. In some embodiments, the anti-CD19 antibody is an anti-CD19 scFv and may comprise an amino acid sequence set forth in SEQ ID NO: 636.

In some embodiments, the CAR comprises a binding domain that may specifically bind to CD19, which may be referred to as a CAR targeting CD19. In some embodiments, the CAR targeting CD19 may be a CAR described in the Chinese patent application CN202210274255.1 (publication No. CN114349863A, which is incorporated herein by reference in its entirety). In some embodiments, the CAR targeting CD19 may comprise any one of the anti-CD19 antibodies described above. In some embodiments, the CAR targeting CD19 may comprise an amino acid sequence set forth in SEQ ID NO: 637.

In some embodiments, the antibody that specifically binds to HER2, also referred to as an anti-HER2 antibody, as described in the Chinese patent application CN202210750853.1 (publication No. CN114805584A, which is incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 may be an LCDR1 set forth in SEQ ID NO: 618, an LCDR2 set forth in SEQ ID NO: 619, an LCDR3 set forth in SEQ ID NO: 620, an HCDR1 set forth in SEQ ID NO: 621, an HCDR2 set forth in SEQ ID NO: 622, and an HCDR3 set forth in SEQ ID NO: 623.

In some embodiments, the anti-HER2 antibody comprises a light chain variable region set forth in SEQ ID NO: 624 and a heavy chain variable region set forth in SEQ ID NO: 625. In some embodiments, the anti-HER2 antibody is an anti-HER2 scFv and may comprise an amino acid sequence set forth in SEQ ID NO: 626.

In some embodiments, the CAR comprises a binding domain that may specifically bind to HER2, which may be referred to as a CAR targeting HER2. In some embodiments, the CAR targeting HER2 may be a CAR described in the Chinese patent application CN202210750853.1 (publication No. CN114805584A, which is incorporated herein by reference in its entirety). In some embodiments, the CAR targeting HER2 may comprise any one of the anti-HER2 antibodies described above. In some embodiments, the CAR targeting HER2 may comprise an amino acid sequence set forth in SEQ ID NO: 627.

In some embodiments, the antibody that specifically binds to IL13Ra2, also referred to as an anti-IL13Ra2 antibody, as described in the Chinese patent application CN202210743595.4 (publication No. CN114805581A, which is incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 may be an LCDR1 set forth in SEQ ID NO: 638, an LCDR2 set forth in SEQ ID NO: 639, an LCDR3 set forth in SEQ ID NO: 640, an HCDR1 set forth in SEQ ID NO: 641, an HCDR2 set forth in SEQ ID NO: 642, and an HCDR3 set forth in SEQ ID NO: 642.

In some embodiments, the anti-IL13Ra2 antibody comprises a light chain variable region set forth in SEQ ID NO: 644 and a heavy chain variable region set forth in SEQ ID NO: 645. In some embodiments, the anti-IL13Ra2 antibody is an anti-IL13Ra2 scFv and may comprise an amino acid sequence set forth in SEQ ID NO: 646.

In some embodiments, the CAR comprises a binding domain that may specifically bind to IL13Ra2, which may be referred to as a CAR targeting IL13Ra2. In some embodiments, the CAR targeting IL13Ra2 may be a CAR described in the Chinese patent application CN202210743595.4 (publication No. CN114805581A, which is incorporated herein by reference in its entirety). In some embodiments, the CAR targeting IL13Ra2 may comprise any one of the anti-IL13Ra2 antibodies described above. In some embodiments, the CAR targeting IL13Ra2 may comprise an amino acid sequence set forth in SEQ ID NO: 647.

In some embodiments, the antibody that specifically binds to B7H3, also referred to as an anti-B7H3 antibody, as described in the Chinese patent application CN202210714289.8 (publication No. CN114773477A, which is incorporated herein by reference in its entirety), comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 may be an LCDR1 set forth in SEQ ID NO: 648, an LCDR2 set forth in SEQ ID NO: 649, an LCDR3 set forth in SEQ ID NO: 650, an HCDR1 set forth in SEQ ID NO: 651, an HCDR2 set forth in SEQ ID NO: 652, and an HCDR3 set forth in SEQ ID NO: 653.

In some embodiments, the anti-B7H3 antibody comprises a light chain variable region set forth in SEQ ID NO: 654 and a heavy chain variable region set forth in SEQ ID NO: 655. In some embodiments, the anti-B7H3 antibody is an anti-B7H3 scFv and may comprise an amino acid sequence set forth in SEQ ID NO: 656.

In some embodiments, the CAR comprises a binding domain that may specifically bind to B7H3, which may be referred to as a CAR targeting B7H3. In some embodiments, the CAR targeting B7H3 may be a CAR described in the Chinese patent application CN202210714289.8 (publication No. CN114773477A, which is incorporated herein by reference in its entirety). In some embodiments, the CAR targeting B7H3 may comprise any one of the anti-B7H3 antibodies described above. In some embodiments, the CAR targeting B7H3 may comprise an amino acid sequence set forth in SEQ ID NO: 657.

In some embodiments, the humanized anti-CD28 antibody and the affinity-matured anti-CD40 antibody or the antigen-binding fragment thereof, the multispecific antibody comprising them, the CD28-CD40 diabody, the polynucleotide encoding them, the vector comprising the polynucleotide, or the immune effector cell expressing the CD28-CD40 diabody of the present invention may be used as a medicament for treating a tumor, a cancer, a viral infection, an autoimmune disease, or the like, which may be formulated into a pharmaceutical composition. The humanized anti-CD28 antibody and the affinity-matured anti-CD40 antibody or the antigen-binding fragment thereof, the multispecific antibody comprising them, the CD28-CD40 diabody, the polynucleotide encoding them, or the vector comprising the polynucleotide may be used in combination with an immune effector cell. The immune effector cell may be selected from a CAR T cell, a TCR T cell, TIL, CIK, LAK, and MIL.

In some embodiments, the present invention provides a method for treating a disease, comprising administering to a subject in need of treatment the humanized anti-CD28 antibody and the affinity-matured anti-CD40 antibody or the antigen-binding fragment thereof, the multispecific antibody comprising them, the CD28-CD40 diabody, the polynucleotide encoding them, the vector comprising the polynucleotide, or the immune effector cell expressing the CD28-CD40 diabody. The disease may be a tumor, a cancer, a viral infection, or an autoimmune disease.

In some embodiments, the treatment method may comprise: (a) administering to a subject in need of treatment the humanized anti-CD28 antibody and the affinity-matured anti-CD40 antibody or the antigen-binding fragment thereof, the multispecific antibody comprising them, the CD28-CD40 diabody, the polynucleotide encoding them, or the vector comprising the polynucleotide, and (b) administering to the subject immune cell therapy. Steps (a) and (b) may be performed simultaneously or sequentially, wherein step (a) may be performed before or after step (b). The immune cell therapy in step (b) may include CAR T therapy, TCR T therapy, TIL therapy, CIK therapy, LAK therapy, and MIL therapy.

In some embodiments, the cancer is a solid tumor or a hematologic cancer (e.g., leukemia). In some embodiments, the cancer is acute myeloid leukemia (AML), B-type acute lymphocytic leukemia (B-ALL), T-type acute lymphocytic leukemia (T-ALL), B-cell precursor acute lymphocytic leukemia (BCP-ALL), or blastic placytoid dendritic cell neoplasm (BPDCN). In some embodiments, the disease (e.g., cancer) is characterized by disease cells expressing mesothelin, CD123, BCMA, HER2, CD19, IL13Ra2, and/or B7H3. In some embodiments, the cancer is a cancer expressing CD123. In some embodiments, the cancer is AML expressing CD123. In some embodiments, the cancer is mesothelioma. In some embodiments, the mesothelioma is pleural mesothelioma, peritoneal mesothelioma, or pericardial mesothelioma. In some embodiments, the cancer is pancreatic cancer. In some embodiments, the pancreatic cancer is pancreatic ductal carcinoma. In some embodiments, the cancer is ovarian cancer. In some embodiments, the cancer is epithelial ovarian cancer. In some embodiments, the cancer is lung cancer. In some embodiments, the cancer is non-Hodgkin's lymphoma, chronic lymphocytic leukemia, acute lymphocytic leukemia, human B-cell precursor leukemia, multiple myeloma, or malignant lymphoma. In some embodiments, the cancer is breast cancer, gastric cancer, ovarian cancer, cervical cancer, urothelial cancer, esophageal cancer, bladder cancer, colorectal cancer, endometrial cancer, renal cancer, lung cancer, pancreatic cancer, head and neck cancer, sarcoma, glioblastoma, prostate cancer, or thyroid cancer. In some embodiments, the cancer is glioma or head and neck cancer.

The terms "subject" and "patient" are used interchangeably herein. The term "subject" used herein refers to any organism to which the antibody or the antigen-binding fragment thereof of the present invention may be administered, e.g., for experimental, diagnostic, prophylactic, and/or therapeutic purposes. Typical subjects include animals (e.g., mammals such as mice, rats, rabbits, non-human primates (e.g., chimpanzees and other apes and monkey species), and humans). The subject may be a mammal, particularly a human, including a female or a male, and including a neonate, an infant, a juvenile, a youth, an adult, or an elderly adult, and further including various races and ethnicities. In some examples, a subject refers to an individual in need of diagnosis, treatment, or prevention of a disease or disorder, and the subject may have the disease or disorder, or be at risk of having the disease or disorder.

The term "treat", "treating", or "treatment" used herein refers to providing a beneficial or desired clinical outcome to a disease, such as eliminating the disease, alleviating symptoms, reducing the extent of the disease, stabilizing, ameliorating or relieving the state of the disease, or slowing the progression of the disease. The measurement of the treatment outcome may be based, for example, on the results of a physical examination, a pathological test, and/or a diagnostic test known in the art. The treatment may also refer to prolonging survival as compared to the expected survival of a subject if not receiving treatment. The treatment may also refer to reducing the incidence or morbidity of the disease, or the recurrence thereof, as compared to a disease that would occur in the absence of this measure. Clinically, such treatment may also be referred to as prophylaxis.

The terms "pharmaceutical composition" and "pharmaceutical formulation" of the present invention are used interchangeably. The pharmaceutical composition may comprise a pharmaceutically acceptable carrier. The term "pharmaceutically acceptable carrier" used herein refers to any carrier comprised in a pharmaceutical composition as an inactive ingredient, which enables the pharmaceutical composition to have a suitable appearance and properties for administration. The pharmaceutically acceptable carrier, such as a stabilizer, a diluent, an additive, an auxiliary agent, an excipient, and the like, has substantially no long-term or permanent adverse effects when administered to a subject. "Pharmaceutically acceptable carrier" should be a pharmaceutically inert material, which is substantially free of biological activity and constitutes an integral part of the formulation.

The pharmaceutical composition, the pharmaceutical combination, and the immune cell (including CAR-T cell) of the present invention may be formulated for various modes of administration according to known techniques. See, for example, Remington, The Science and Practice of Pharmacy (9th Ed. 1995). In the preparation of the pharmaceutical composition, an active agent is generally mixed with a pharmaceutically acceptable carrier and the like. Of course, the pharmaceutically acceptable carrier must be acceptable, i.e., it is compatible with any other ingredients in the formula and must not be deleterious to a subject. Pharmaceutically acceptable carriers may include, but are not limited to, buffers, excipients, stabilizers, preservatives, wetting agents, surfactants, emulsifiers, or combinations thereof. Examples of buffers include, but are not limited to, acetic acid, citric acid, histidine, boric acid, formic acid, succinic acid, phosphoric acid, carbonic acid, malic acid, aspartic acid, Tris buffer, HEPPSO, HEPES, neutral buffered saline, phosphate-buffered saline, and the like.

The pharmaceutical composition, the pharmaceutical combination, and the immune cell (including CAR-T cell) of the present invention may be administered in any mode suitable for diseases to be treated (or prevented) and subjects. In certain embodiments, the mode of administration may include, but is not limited to, parenteral or non-parenteral routes, including oral, sublingual, buccal, transdermal, rectal, vaginal, intradermal, intranasal routes, or parenteral routes, such as intravenous injection (i.v.), intraperitoneal, intradermal, subcutaneous, intramuscular, intracranial, intrathecal, intratumoral, transdermal, transmucosal, intra-articular, intrathecal, intrathecal, intrahepatic, intraneural, or intracranial injection or infusion. The pharmaceutical composition may be directly injected into tumors, lymph nodes, tissues, organs, or sites of infection.

Dosage forms suitable for oral administration include, but are not limited to, tablets, capsules, powders, pills, granules, suspensions, solutions or pre-concentrates of solutions, and emulsions or pre-concentrates of emulsions. Pharmaceutically acceptable carriers that may be used in oral dosage forms include water, ethylene glycol, oils, alcohols, flavoring agents, preservatives, colorants, and the like. Carriers such as starches, sugars, microcrystalline cellulose, diluents, fillers, lubricants, granulating agents, lubricants, binders, stabilizers, disintegrants, and the like may be used to prepare oral solid formulations such as powders, capsules, or tablets.

Dosage forms suitable for parenteral administration include, but are not limited to, sterile liquid formulations, such as isotonic aqueous solutions, emulsions, suspensions, dispersions, or viscous compositions, which may be buffered to reach a desired pH value. The parenteral dosage form may be ready-to-use, or may be a dry product ready to be dissolved or suspended in a pharmaceutically acceptable carrier. The parenteral dosage form may be a sterile formula, or may be sterilized before administration to a subject. Pharmaceutically acceptable carriers that may be used to provide parenteral dosage forms include, but are not limited to, water for injection; aqueous carriers, such as, but not limited to, sodium chloride injection, Ringer's injection, and glucose injection; water-soluble carriers, such as, but not limited to, ethanol, polyethylene glycol, and polypropylene glycol; non-aqueous carriers, such as, but not limited to, corn oil, cottonseed oil, peanut oil, sesame oil, ethyl oleate, isopropyl myristate, and benzyl benzoate; and solubilizers, such as cyclodextrin.

The pharmaceutical composition, the pharmaceutical combination, and the immune cell (including CAR-T cell) of the present invention are administered to a subject in a therapeutically effective amount. The terms "therapeutically effective amount" and "effective amount" used herein are used interchangeably, and refer to an amount that is effective, at dosages and for periods of time necessary, to achieve the desired therapeutic effect. The therapeutically effective amount may vary depending on various factors, such as the disease state, age, sex, and weight of the individual, and the ability of a treatment method or a combination of treatment methods to elicit a desired response in the individual. The effective amount may refer to an amount that causes a detectable change in a biological or chemical activity. The detectable change may be detected and/or further quantified by those skilled in the relevant art. In addition, the "effective amount" may specify an amount that maintains a desired physiological state, i.e., reduces or prevents a significant decline and/or promotes improvement in the disease condition.

The amount and frequency of administration will be determined by factors such as the condition of a subject (e.g., age, weight, sex, and response of the subject to the drug), as well as the type and severity of the subject's disease, although appropriate dosages may be determined by clinical trials.

The pharmaceutical composition, the pharmaceutical combination, and the immune cell (including CAR-T cell) of the present invention may be administered to a subject in a therapeutically effective amount of about 0.5 to about 250 mg/kg, e.g., about 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

In the present invention, an effective amount of immune cells (including CAR-T cells) may be, for example, 5 × 10⁶ cells, 1 × 10⁷ cells, 2 × 10⁷ cells, 5 × 10⁷ cells, 1 × 10⁸ cells, 2 × 10⁸ cells, 5 × 10⁸ cells, 1 × 10⁹ cells, 2 × 10⁹ cells, or 5 × 10⁹ cells administered in a single dose.

The pharmaceutical composition, the pharmaceutical combination, and the immune cell (including CAR-T cell) of the present invention may be administered once or twice a day; or once every 2, 3, 4, 5, 6, 7, 8, 9, or 10 days, once every 1, 2, 3, 4, 5, or 6 weeks, or once every 1, 2, 3, 4, 5, or 6 months or longer. The pharmaceutical composition may also be administered on a regimen of several times (e.g., 1, 2, 3, 4, or 5 times) a week or several times (e.g., 1, 2, 3, 4, 5, 6, 7, 8, 9, or 10 times) a month. For example, in a regimen of 5 times a week, the pharmaceutical composition may be administered once a day for 5 consecutive days, followed by a rest for two consecutive days.

The term "pharmaceutical composition" used herein refers to a combination of two or more drugs administered to a subject over a period of time (e.g., simultaneously, or several minutes to several hours apart, or even several days or several weeks apart). The two or more drugs may be formulated into the same pharmaceutical formulation, or may be formulated into different pharmaceutical formulations, respectively.

The pharmaceutical composition, the pharmaceutical combination, and the immune cell (including CAR-T cell) of the present invention may be used in combination with other drugs and treatment methods, such as other anti-tumor drugs, chemotherapy, or radiation therapy. As used herein, "used in combination" refers to the administration of two (or more) different drugs and/or therapies to a subject during treatment. The two or more drugs and/or therapies in the combination may be administered by different routes and regimens. The two or more drugs and/or treatment methods may be administered to the subject simultaneously or sequentially. In some embodiments, when administration of a second drug or therapy begins, administration of one drug or therapy is still ongoing, thus there is an overlap in the administration. Such a regimen may be referred to herein as "simultaneously". When administered simultaneously, the two or more drugs and/or therapies may be formulated together in a single dosage form, or separately in two or more separate dosage forms.

### Examples

In the following examples, CDRs of a heavy chain variable region and a light chain variable region of an antibody are defined by the Kabat nomenclature. Unless otherwise stated, the methods and materials of the examples described below are all conventional products commercially available.

### Example 1: Structural Simulation

Homology modeling, also referred to as "comparative modeling", predicts the structure of an unknown protein according to the sequence homology of known structures based on the principle that "if two proteins have sufficiently high sequence similarity, they are likely to have very similar three-dimensional structures".

If the homology between a target sequence and a template sequence reaches 30%, the accuracy of the prediction model can reach 80%; if the homology reaches 50%, the accuracy of the model can reach 95%; if the homology reaches more than 70%, the prediction model is considered to completely represent the true structure of the protein. However, there are special cases where the structures are different although the sequence homology is high.

Therefore, the structure of murine CD28 antibody 9.3 was predicted first by using the homology modeling. For some antibody structures with relatively low homology, *de novo* modeling was used to construct a model. The general experimental procedures were as follows:
1. Discovery Studio and Schrödinger Antibody Modeling software were used to model the murine CD28 antibody 9.3 by using the homology modeling, and 5-10 optimal structure analysis models were selected from the prediction models.
2. Loop regions of an antibody were first structurally predicted by the homology modeling, and if the homology of amino acid sequences between CDR regions of the 9.3 antibody and CDR regions of the template antibody was less than 50%, the structure model of the CDR regions was constructed by *de novo* modeling.
3. The BLAST program in the PDB protein structure database was used to search for 13 antibody crystal structure models with the highest homology to the amino acid sequence of the parent antibody 9.3 (the structure resolution was higher than 2.5 Å), and the optimal structure model was selected as compared to the automatically modeled model.

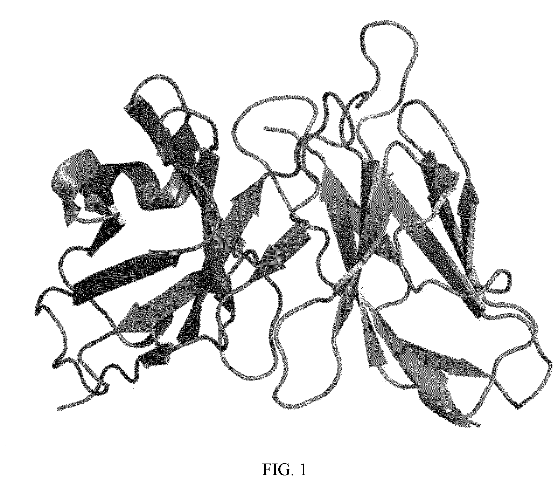

The optimal structure model is shown in FIG. 1. The homology of the sequences of light and heavy chains of the 9.3 antibody to the template sequence in the PDB structure database exceeded 90%, and the antibody structure was common, so that the reliability of the prediction model exceeded 95%. The charge distribution on the antibody surface was uniform, so that the possibility of forming an aggregate was low.

### Example 2: Humanization Design

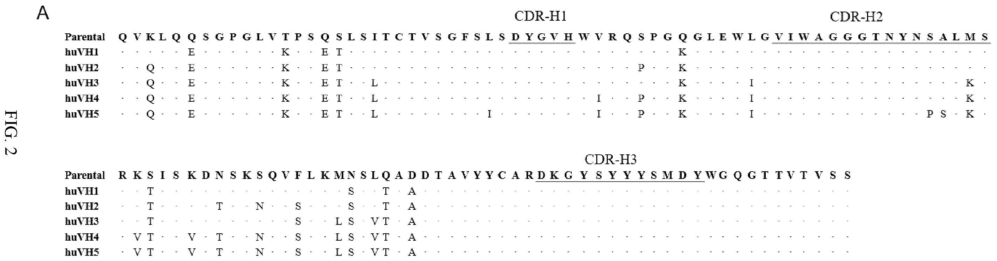

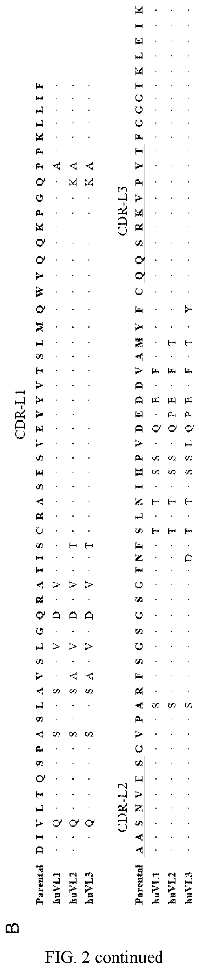

According to the predicted antibody structure model, humanized design was performed on the 9.3 antibody, and amino acids from mice were selected for point mutation and back mutation. The brief experimental procedures were as follows:
1. The sequence of the mouse 9.3 antibody was aligned with the human Germline sequence database by using the Ig-BLAST program of NCBI. The search results showed that the heavy chain variable region (VH) of the 9.3 antibody had the highest sequence homology to human Germline IGHV4, and contained 24 murine amino acid sites. The light chain variable region (VL) of the 9.3 antibody had the highest sequence homology to human Germline IGKV1, and also contained 24 murine amino acid sites.
2. For the heavy chain VH, IGHV4 with the highest homology was selected as a template for humanization design, and 5 sequences, VH1-VH5, were designed. For the light chain VL, IGKV1 was selected as the template for humanization design, and 3 sequences, VL1-VL3 (FIGs. 2A and B, wherein Parental was the VH and VL sequences of the 9.3 antibody, i.e., mVH and mVL sequences in Table 1), were designed.
3. Since IGHV4-KV1 is the most common heavy chain-light chain pairing mode, IGHV4-KV1 was selected as the corresponding light and heavy chain pairing mode.
4. Immunogenicity analysis: the VH was predicted to have low immunogenicity and a peptide fragment with immunogenicity of FLKMNSLQA. The VL was predicted to have low immunogenicity and a peptide fragment with immunogenicity of LLIFAASNV
5. Calculation of the humanization degree of the humanized antibody: the designed light and heavy chains of the humanized sequence were combined in pairs, and then the humanized sequence was aligned with the human Germline sequence to calculate the percent of the humanization degree of each part and the full-length antibody. According to the structure of the paired light and heavy chains, antibodies with sites that were easily oxidized and aminoated were eliminated, antibodies with protein modification sites such as glycosylation and phosphorylation were eliminated, and then 11 humanized antibodies were obtained. The results are summarized in Table 1, wherein all antibody samples were in the form of IgG1 and had identical heavy and light chain constant regions, "mVHmVL" was an antibody comprising the murine heavy chain variable region (mVH) and the murine light chain variable region (mVL) of the 9.3 antibody, "huVH1huVL1" indicated that the antibody comprised huVH1 and huVL1, and the names of other humanized antibodies had similar meanings. The specific sequences of all antibody samples are shown in the "Sequence" section appended to the examples.

**Table 1. Summary of humanization degree of antibodies**

| Sample name | Full length humanization degree | Back mutation (number of amino acids) |
|---|---|---|
| mVHmVL | N/A | N/A |
| huVH1huVL1 | 96.4% | 24 |
| huVH1huVL2 | 97.3% | 18 |
| huVH2huVL2 | 98.1% | 13 |
| huVH2huVL3 | 98.4% | 11 |
| huVH3huVL1 | 97.3% | 18 |
| huVH3huVL2 | 98.2% | 12 |
| huVH3huVL3 | 98.5% | 10 |
| huVH4huVL2 | 99.1% | 6 |
| huVH4huVL3 | 99.4% | 4 |
| huVH5huVL2 | 99.4% | 4 |
| huVH5huVL3 | 99.6% | 3 |

### Example 3: Identification of Molecular Weight and Purity of Humanized Antibodies by SDS-PAGE (SDS-Polyacrylamide Gel Electrophoresis)

The molecular weight and purity of the full-length humanized antibodies (IgG1) under non-reducing and reducing conditions were detected by SDS-PAGE electrophoresis, thereby facilitating the development of the later production process.
1. Sample preparation: a protein sample A280 was first determined by using a Nano-300 micro-spectrophotometer (Hangzhou Allsheng Instruments Co., Ltd.), and the protein concentration was calculated. Non-reducing sample: 1 µg of the protein was taken; 4×LDS loading buffer and 40 mM iodoacetamide were added; and the mixture was incubated at 75 °C for 10 min. Reducing sample: 2 µg of the protein was taken; 4×LDS loading buffer and 5 mM DTT (dithiothreitol) were added; and the mixture was incubated at 100 °C for 10 min. Electrophoresis was performed at 140 V for 50 min.
2. The gel was stained with Coomassie brilliant blue R250, decolorized, and then scanned with an EPSON V550 color scanner.
3. The purity of the reduced band or the purity of the sum of the reduced heavy and light chains was calculated by using the Image J software according to the peak area normalization method.

The experimental results (Table 2) show that the humanized antibodies all had a molecular weight of about 146 kDa and relatively high purity.

**Table 2. Summary of SDS-PAGE results of humanized antibodies**

| Sample name | Molecular weight (kDa) | Purity (%) |
|---|---|---|
| mVHmVL | 146.00 | >95.0 |
| huVH1huVL1 | 146.06 | >95.0 |
| huVH1huVL2 | 145.90 | >95.0 |
| huVH2huVL2 | 145.82 | >95.0 |
| huVH2huVL3 | 145.90 | >95.0 |
| huVH3huVL1 | 145.86 | >95.0 |
| huVH3huVL2 | 145.70 | >95.0 |
| huVH3huVL3 | 145.78 | >95.0 |
| huVH4huVL2 | 145.66 | >95.0 |
| huVH4huVL3 | 145.74 | >95.0 |
| huVH5huVL2 | 145.72 | >95.0 |
| huVH5huVL3 | 145.78 | >95.0 |

### Example 4: Analysis of Purity of Humanized Antibodies by SEC (Size exclusion Chromatography)

The ratio of monomers and polymers of the humanized antibody was measured under non-reducing conditions by the SEC method to identify the purity of the antibody.
1. Agilent HPLC 1100, XBridge BEH200Å, SEC 3.5 µm, 7.8 × 300 mm chromatographic column was selected.
2. 0.15 M PB with a pH of 7.4 was used as a mobile phase; the flow rate was set to be 0.8 mL/min; the loading volume of a sample was set to be 20 µL; the detection wavelength was set to be 280 nm; the detection bandwidth was set to be 16 nm; the reference wavelength was set to be 360 nm; the reference bandwidth was set to be 100 nm; the peak width (response time) was set to be > 0.1 min (2 s); the slit was set to be 4 nm; and the negative absorbance baseline was set to be 100 mAU.
3. The purity of the reference product Herceptin monomer was greater than 95%, and the resolution between the BSA monomer and the dimer was more than 2, which was considered as a stable baseline.

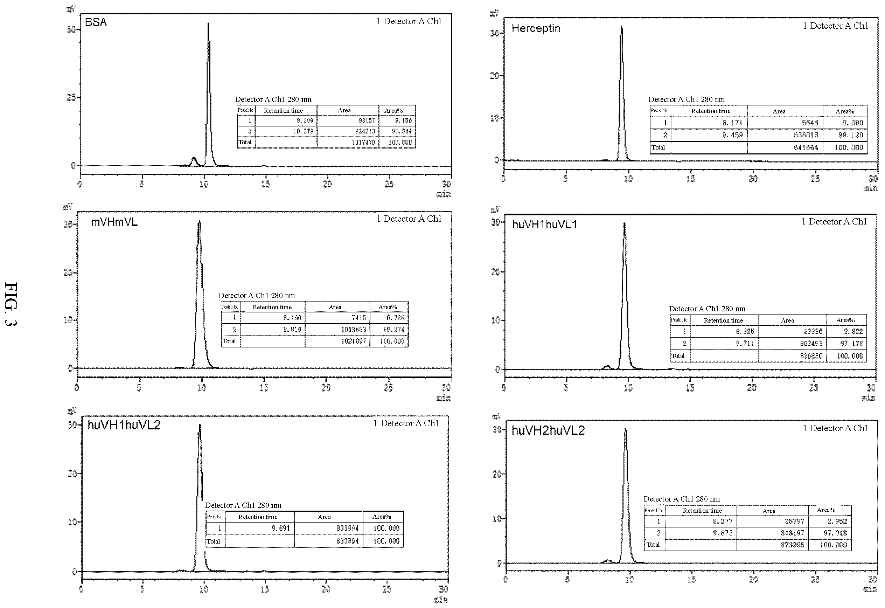

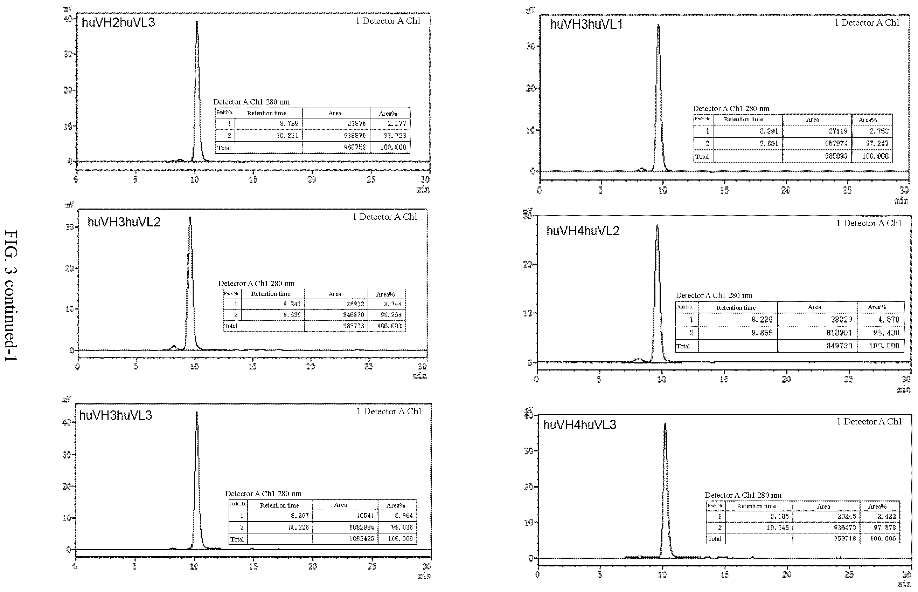

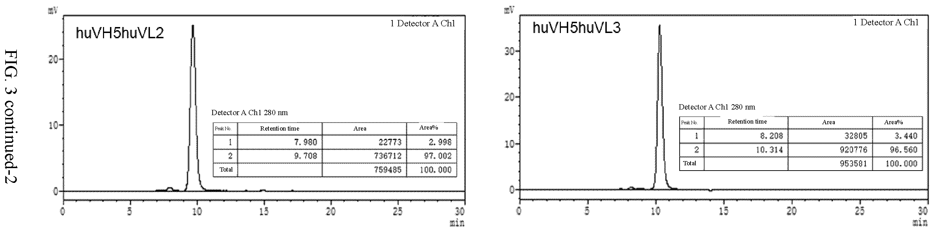

The experimental results (Table 3 and FIG. 3) show that the humanized antibodies all had relatively good purity, and the ratio of monomers in the total was high. There were no low-molecular-weight substances or antibody fragments except for a low ratio of polymers.

**Table 3. Summary of SEC results of humanized antibodies**

| Protein name | Monomer retention time (min) | High-molecular polymer (%) | Monomer (%) | Low-molecular-weight substance (%) |
|---|---|---|---|---|
| mVHmVL | 9.82 | 0.73 | 99.27 | / |
| huVH1huVL1 | 9.71 | 2.82 | 97.18 | / |
| huVH1huVL2 | 9.69 | / | 100.00 | / |
| huVH2huVL2 | 9.67 | 2.95 | 97.05 | / |
| huVH2huVL3 | 10.23 | 2.28 | 97.72 | / |
| huVH3huVL1 | 9.66 | 2.75 | 97.25 | / |
| huVH3huVL2 | 9.64 | 3.74 | 96.26 | / |
| huVH3huVL3 | 10.23 | 0.96 | 99.04 | / |
| huVH4huVL2 | 9.66 | 4.57 | 95.43 | / |
| huVH4huVL3 | 10.25 | 2.42 | 97.58 | / |
| huVH5huVL2 | 9.71 | 3.00 | 97.00 | / |
| huVH5huVL3 | 10.31 | 3.44 | 96.56 | / |

### Example 5: Detection of Binding Activities of Humanized Antibodies by ELISA (Enzyme Linked Immunosorbent Assay)

The binding activity of the humanized antibody to the antigen CD28 was detected by the ELISA method. The experimental procedures were as follows:
1. 30 µL of recombinant Human/Cynomolgus CD28 (N-6His) (Suzhou Novoprotein Scientific Inc., C406) at a concentration of 2 µg/mL was added to each well of an ELISA plate, and the plate was incubated at 4 °C overnight.
2. The next day, the ELISA plate was washed 3 times with PBST (containing 0.05% Tween-20).
3. 250 µL of 5% skim milk powder (dissolved in PBS) was added to each well of the ELISA plate, and the plate was incubated at room temperature for 2 h.
4. The plate was washed 3 times with PBST, antibodies with different dilution ratios were added to the ELISA plate at 30 µL/well, and the plate was incubated at room temperature for 60 min.
5. The plate was washed 3 times with PBST, 30 µL of HRP-anti-hFc antibody was added to each well, and the plate was incubated at room temperature for 60 min.
6. The plate was washed 3 times with PBST, 30 µL of TMB was added to each well, and after color development at room temperature, 30 µL of 2 M H₂SO₄ was added to each well to stop the reaction. The plate was read at 450 nm.

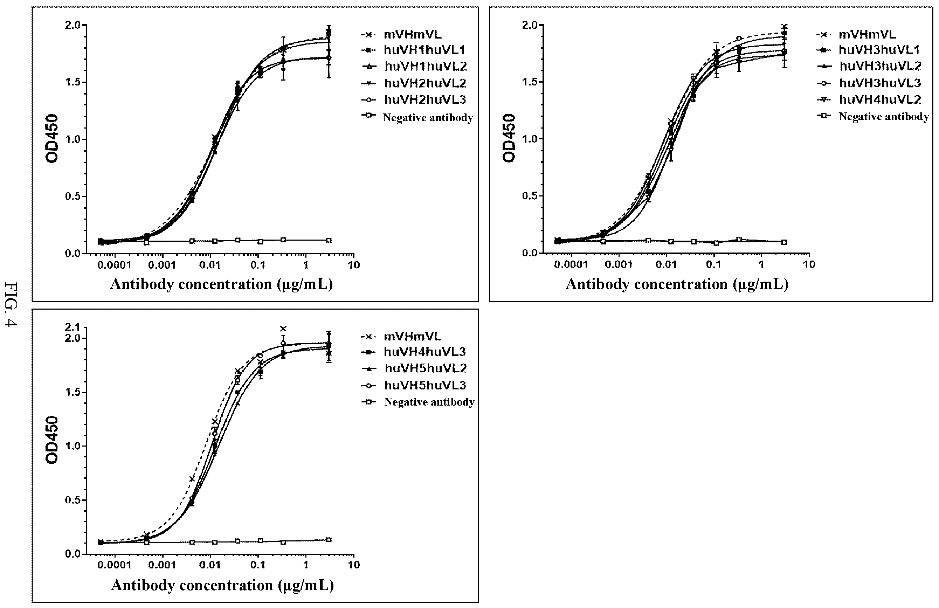

The experimental results (Table 4 and FIG. 4) show that the binding activities of only 4 humanized antibodies huVH2huVL3, huVH3huVL2, huVH3huVL3, and huVH5huVL3 were improved compared to the parent mVHmVL, and that the binding activities of other antibodies were all reduced to different degrees.

**Table 4. Summary of ELISA results of humanized antibodies**

| Protein name | **EC50 (µg/mL)** |
|---|---|
| mVHmVL | 0.01291 |
| huVH1huVL1 | 0.01507 |
| huVH1huVL2 | 0.01254 |
| huVH2huVL2 | 0.01259 |
| huVH2huVL3 | 0.01040 |
| huVH3huVL1 | 0.01211 |
| huVH3huVL2 | 0.00914 |
| huVH3huVL3 | 0.00827 |
| huVH4huVL2 | 0.01265 |
| huVH4huVL3 | 0.01267 |
| huVH5huVL2 | 0.01509 |
| huVH5huVL3 | 0.01090 |

### Example 6: Detection of Affinities of Humanized Antibodies by BLI

The affinity kinetics of the humanized antibodies were detected using biolayer interferometry (BLI). The experimental procedures were as follows:
1. GATOR instrument was run, and the Kinetics experimental mode was selected.
2. The analysis program was run according to Table 5

**Table 5. GATOR analysis program**

| **Experimental procedure** | **Run time (s)** | **Sample** |
|---|---|---|
| Baseline 1 | 60s | Buffer Q |
| Loading | 120 s | 30 nM antibody (the solvent was buffer Q) |
| Baseline 2 | 60s | Buffer Q |
| Binding | 120 s | The antigen was diluted in a 2-fold gradient from 300 nM to 37.5 nM with buffer Q |
| Dissociation | 180 s | Buffer Q |
| Regeneration | 25 s | Buffer Q |

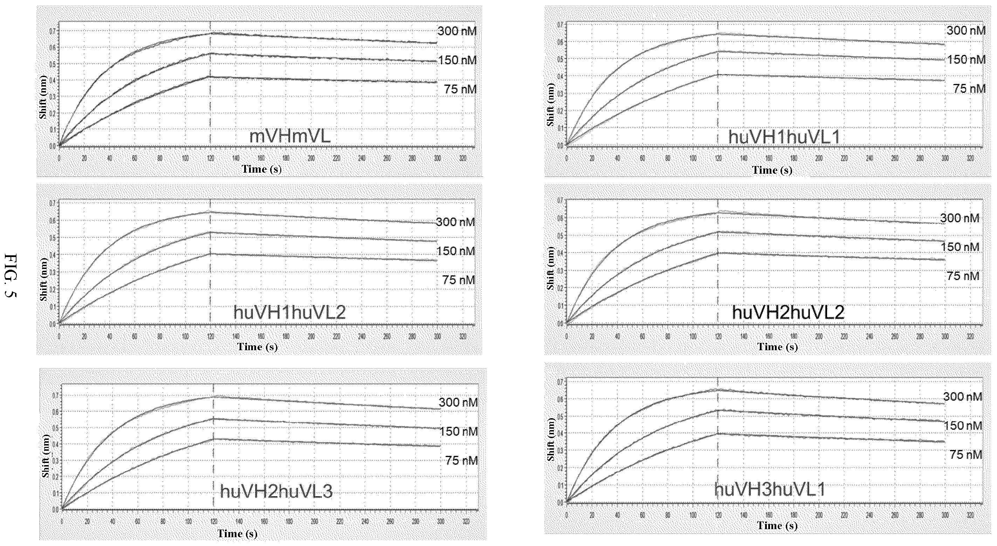

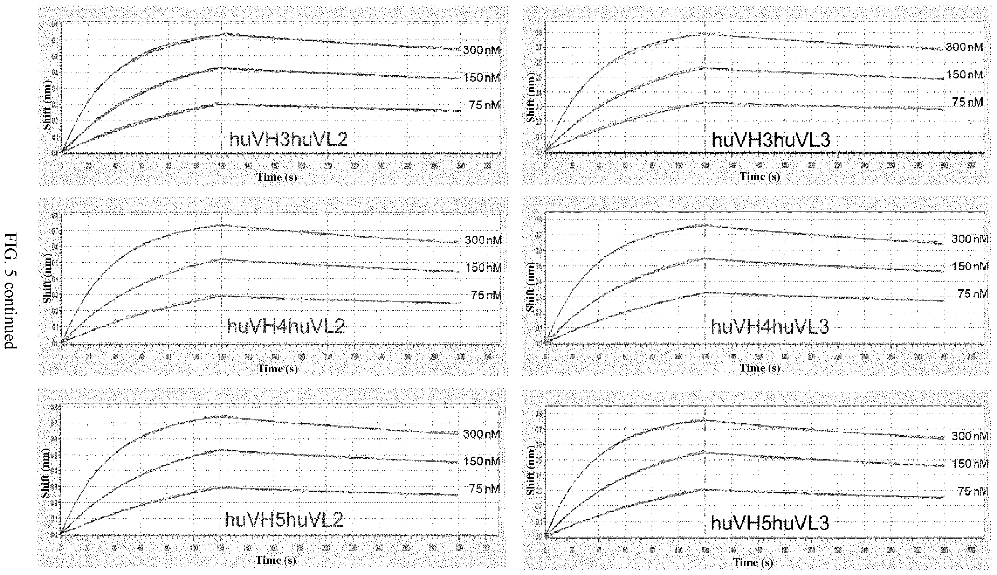

The experimental results are shown in Table 6 and FIG. 5. In the obtained humanized antibodies, huVH1VL1 had the lowest KD value and the highest affinity.

**Table 6. Summary of affinity kinetics assay results for humanized antibodies**

| **Loaded antibody** | **Detection antigen** | | | |
|---|---|---|---|---|
| | **KD (M)** | **ka (1/Ms)** | **kd (1/s)** | R² |
| mVHmVL | 5.51E-09 | 8.95E+04 | 4.93E-04 | 0.994 |
| huVH1huVL1 | 6.13E-09 | 8.66E+04 | 5.31E-04 | 0.994 |
| huVH1huVL2 | 6.54E-09 | 8.90E+04 | 5.82E-04 | 0.994 |
| huVH2huVL2 | 6.59E-09 | 8.87E+04 | 5.84E-04 | 0.994 |
| huVH2huVL3 | 7.44E-09 | 8.54E+04 | 6.35E-04 | 0.994 |
| huVH3huVL1 | 8.36E-09 | 8.69E+04 | 7.27E-04 | 0.994 |
| huVH3huVL2 | 9.13E-09 | 8.57E+04 | 7.82E-04 | 0.995 |
| huVH3huVL3 | 9.59E-09 | 8.53E+04 | 8.18E-04 | 0.995 |
| huVH4huVL2 | 1.09E-08 | 8.54E+04 | 9.32E-04 | 0.996 |
| huVH4huVL3 | 1.12E-08 | 8.66E+04 | 9.66E-04 | 0.996 |
| huVH5huVL2 | 1.08E-08 | 8.41E+04 | 9.09E-04 | 0.996 |
| huVH5huVL3 | 1.15E-08 | 8.78E+04 | 10.1E-04 | 0.996 |

### Example 7: Detection of Secondary Killing Effect

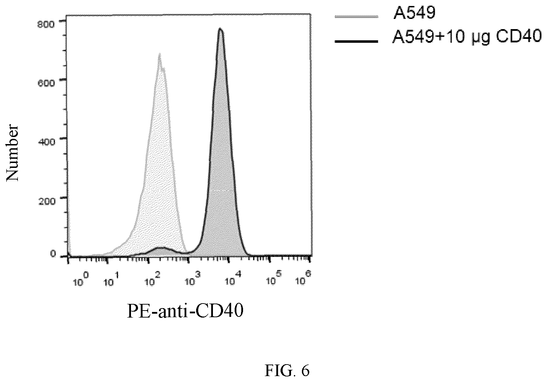

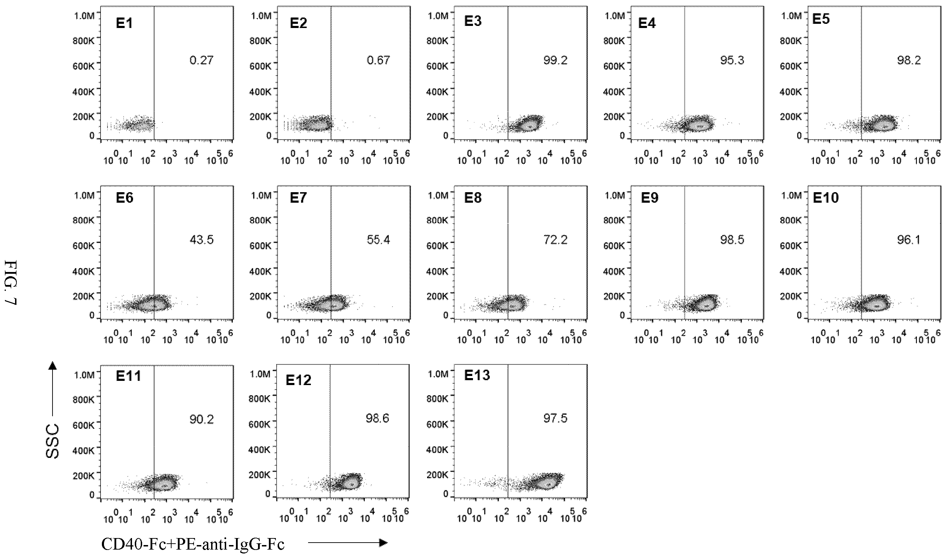

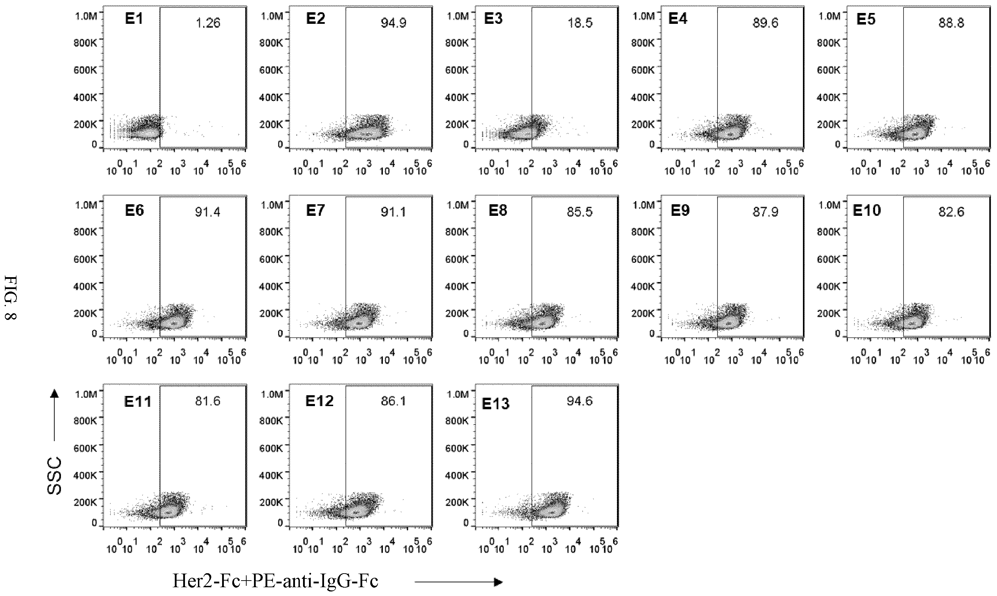

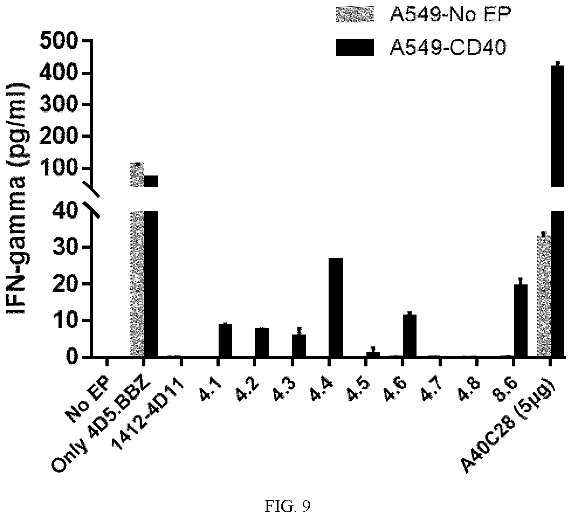

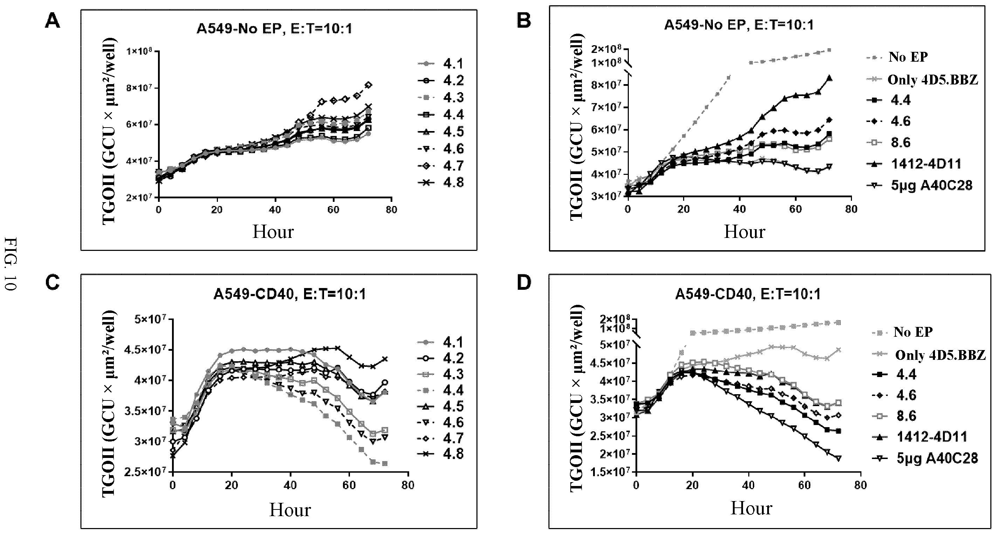

In order to detect the function of the CD28 humanized antibody at a cellular level, the humanized antibody huVH1huVL1 (9.3h11 for short) with the highest affinity was selected, the VH and VL of this humanized antibody were combined with a CD40 antibody 40.52 in the form of scFv in tandem to form a bispecific antibody (diabody or BsAb for short), and the two different scFvs were linked via GGGGS (G₄S for short). The VH and VL in each scFv were linked via three G₄S (GGGGSGGGGSGGGGS). Based on the relative positions of VH-VL and the relative position differences between the CD28 antibody and the CD40 antibody, there were 8 kinds of construction modes for diabodies (see Table 7), and the 8 kinds of diabodies were named 4.1-4.8, respectively.
1. The killing-promoting effects of the diabodies 4.1-4.8 were detected by Her2-CAR-T cells, and compared to the parent 8.6 (see Table 7 for the construction mode, also in the form of scFv in tandem) as well as 1412-4D11 diabody and A40C28 (LACO). The experimental procedures for the preparation of Her2-CAR-T cells and the killing-promoting effects of the diabodies were as follows:
(1) Preparation of target cells: 10 µg of CD40 mRNA (A549-CD40 for short) was electroporated into A549-CBG cells by using a BTX ECM 830 electroporator, or no mRNA was electroporated (A549-no EP for short, no EP in the present invention means that no mRNA was electroporated). The electroporation conditions were 300 V and 0.5 ms. After 24 hours, the expression level of CD40 was detected by flow cytometry and PE-anti-CD40 antibody. FIG. 6 shows that CD40 was expressed on the surface of A549 cells at a relatively high level.
(2) Preparation of CAR-T cells: 0.5 µg of Her2-CAR (4D5.BBZ) mRNA and 15 µg of BsAb mRNA or 5 µg of A40C28 (see Table 8) were co-electroporated into CD3⁺ T cells as Her2-CAR-T cells. The electroporation conditions were 500 V and 0.7 ms. Her2-CAR (4D5.BBZ) was a chimeric antigen receptor (CAR) and comprised an anti-HER2 scFV, a CD8 hinge domain, a CD8 transmembrane (TM) domain, a 4-1BB costimulatory domain, and CD3ζ, wherein the VH and VL in the anti-HER2 scFV were identical to the well-known anti-HER2 murine antibody 4D5. After 24 hours, the expression levels of the diabodies were detected by flow cytometry, CD40-Fc, and PE-anti-Fc antibody (FIG. 7); the expression level of Her2-CAR was detected by Her2-Fc and PE-anti-Fc antibody (FIG. 8). The experimental results show that the expression levels of the diabodies and Her2-CAR were all relatively high.
(3) Co-culture: Her2-CAR-T cells and target cells A549-no EP or A549-CD40 were co-cultured in a ratio of effector cell (E):target cell (T) = 10:1 for 72 h. The cell killing was observed by using an incucyte S3 live-cell dynamic imaging and analysis system, and killing curves were plotted. The experimental results show that among the diabodies 4.1-4.8, the diabodies 4.4 and 4.6 acted together with the Her2-CAR-T cells to reduce the fluorescence value of the target cell to the minimum, and the diabodies 4.4 and 4.6 had the strongest killing-promoting effects (FIGs. 10A and B). Meanwhile, the killing-promoting effects of 4.4 and 4.6 were stronger than those of the parent 8.6 and 1412-4D11, but still weaker than that of A40C28 (FIGs. 10B and D).
(4) Cytokine release: after the Her2-CAR-T cells and target cells A549-no EP or A549-CD40 were co-cultured according to E:T = 1:1 for 24 h, the supernatant was collected by centrifugation. The release level of IFN-gamma in the supernatant was detected by Cisbio bioassays' human IFN-γ assay. The experimental results (FIG. 9) show that among the diabodies 4.1-4.8, the diabodies 4.4 and 4.6 could promote the release of more IFN-gamma cytokine, and had release-promoting ability stronger than those of the diabodies 8.6 and 1412-4D11, but still weaker than that of A40C28.

**Table 7. Construction modes of diabodies**

| Diabody name | Construction method |
|---|---|
| 8.6 | 40.52(VH-VL)-9.3(VH-VL) |
| 4.1 | 9.3h11(VL-VH)-40.52(VL-VH) |
| 4.2 | 9.3h11(VL-VH)-40.52(VH-VL) |
| 4.3 | 9.3h11(VH-VL)-40.52(VL-VH) |
| 4.4 | 9.3h11(VH-VL)-40.52(VH-VL) |
| 4.5 | 40.52(VH-VL)-9.3h11(VL-VH) |
| 4.6 | 40.52(VH-VL)-9.3h11(VH-VL) |
| 4.7 | 40.52(VL-VH)-9.3h11(VH-VL) |
| 4.8 | 40.52(VL-VH)-9.3h11(VL-VH) |

**Table 8. mRNA co-electroporation in Her2-CAR-T cells**

| EP (electroporation sample No.) | BsAbs (15 µg) | CAR (0.5 µg) |
|---|---|---|
| E1 | / | / |
| E2 | / | 4D5.BBZ |
| E3 | 1412-4D11 | 4D5.BBZ |
| E4 | 4.1 | 4D5.BBZ |
| E5 | 4.2 | 4D5.BBZ |
| E6 | 4.3 | 4D5.BBZ |
| E7 | 4.4 | 4D5.BBZ |
| E8 | 4.5 | 4D5.BBZ |
| E9 | 4.6 | 4D5.BBZ |
| E10 | 4.7 | 4D5.BBZ |
| E11 | 4.8 | 4D5.BBZ |
| E12 | 8.6 | 4D5.BBZ |
| E13 | A40C28 (5 µg) | 4D5.BBZ |

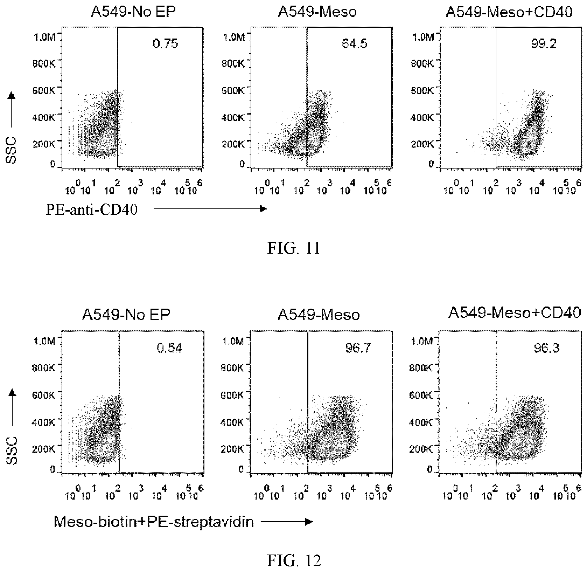

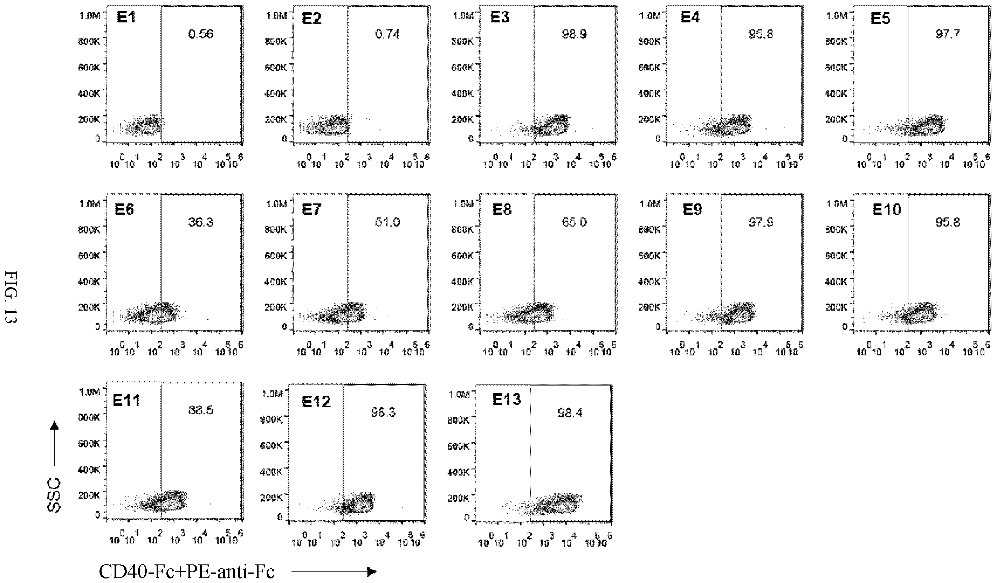

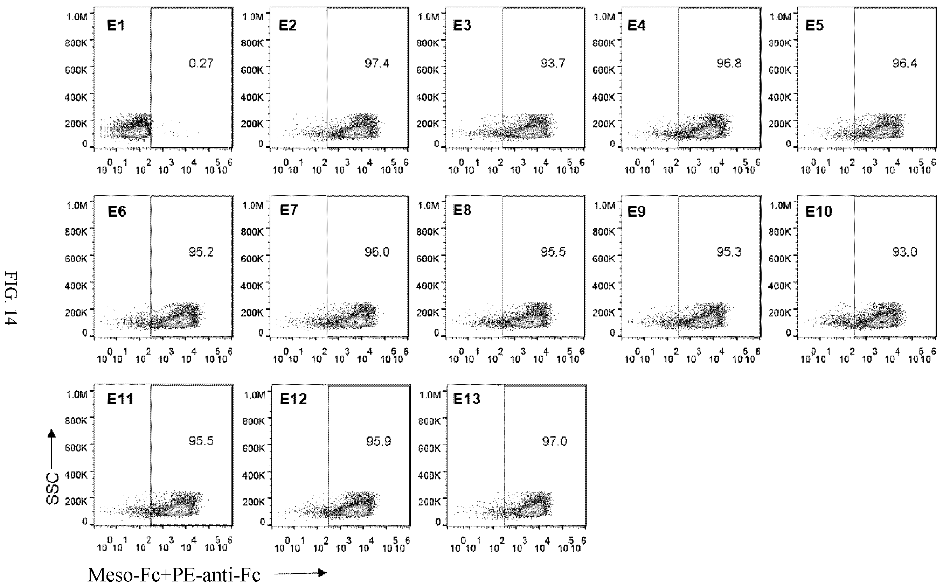

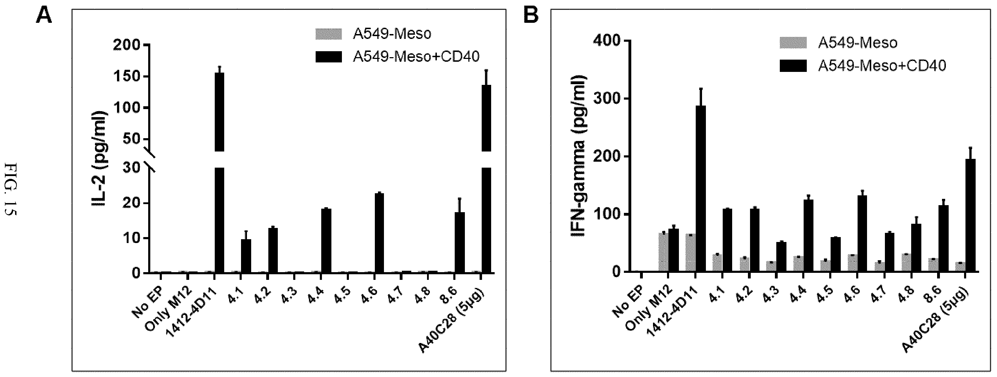

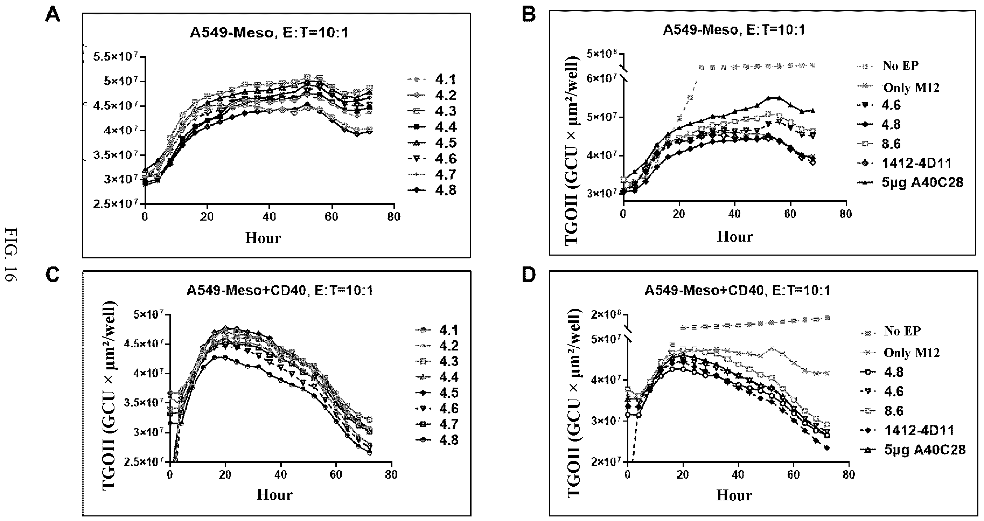

2. The killing-promoting effects of the diabodies 4.1-4.8 and the differences between these diabodies and the parent 8.6, the 1412-4D11 diabody, and A40C28 (LACO) were detected by mesothelin (Meso for short)-CAR-T cells. The experimental procedures for the preparation of Meso-CAR-T cells and the killing-promoting effects of the diabodies were as follows:
(1) Preparation of target cells: no mRNA was electroporated into A549-CBG cells (A549-no EP for short), 10 µg of Meso (mesothelin) mRNA was electroporated into A549-CBG cells (A549-Meso for short), or 10 µg of Meso and 10 µg of CD40 mRNA were electroporated into A549-CBG cells (A549-Meso + CD40 for short). The electroporation conditions were 300 V and 0.5 ms. After 24 hours, the expression level of CD40 was detected by flow cytometry and PE-anti-CD40 antibody. FIG. 11 shows that CD40 was expressed on the surface of A549 cells at a relatively high level. The expression level of Meso was detected by flow cytometry, Meso-biotin, and PE-streptavidin antibody. FIG. 12 shows that Meso was expressed on the surface of A549 cells at a relatively high level, and the high expression of Meso also caused the up-regulation of CD40 expression at the same time.
(2) Preparation of CAR-T cells: 2 µg of Meso-CAR (M12) mRNA and 15 µg of BsAb mRNA (see Table 9) were co-electroporated into CD3⁺ T cells as Meso-CAR-T cells. The electroporation conditions were 500 V and 0.7 ms. Meso-CAR (M12) was a chimeric antigen receptor (CAR) and comprised an anti-mesothelin scFV (M12), a CD8 hinge domain, a CD8 transmembrane (TM) domain, a 4-1BB costimulatory domain, and CD3ζ, the specific sequence of which was seen in the "Sequence Listing" section appended to the examples. After 24 hours, the expression levels of the diabodies were detected by flow cytometry, CD40-Fc, and PE-anti-Fc antibody (FIG. 13); the expression level of Meso-CAR was detected by Meso-Fc and PE-anti-Fc antibody (FIG. 14). The experimental results show that the expression levels of the diabodies and Meso-CAR were all relatively high.
(3) Co-culture: Meso-CAR-T cells and target cells A549-no EP, A549-Meso, or A549-Meso + CD40 were co-cultured according to E:T = 10:1 for 72 h. The cell killing was observed by using the incucyte S3 live-cell dynamic imaging and analysis system, and killing curves were plotted. The experimental results show that among the diabodies 4.1-4.8, the diabodies 4.6 and 4.8 acted together with the Meso-CAR-T cells to reduce the fluorescence value of the target cell to the minimum, and the diabodies 4.6 and 4.8 had the strongest killing-promoting effects (FIGs. 16A and B). Meanwhile, the killing-promoting effects of 4.6 and 4.8 were stronger than those of the parent 8.6 and A40C28, but weaker than that of 1412-4D11 (FIGs. 16C and D).
(4) Cytokine release: after the Meso-CAR-T cells and target cells A549-no EP, A549-Meso, or A549-Meso + CD40 were co-cultured according to E:T = 1:1 for 24 h, the supernatant was collected by centrifugation. The release levels of IL-2 and IFN-gamma in the supernatant were detected by Cisbio bioassays' human IL-2 assay and human IFN-γ assay. The experimental results show that among the diabodies 4.1-4.8, the diabodies 4.4 and 4.6 could promote the release of more IL-2 (FIG. 15A) and the release of more IFN-gamma (FIG. 15B), and had release-promoting ability stronger than that of the diabody 8.6, but still weaker than those of 1412-4D11 and A40C28.

**Table 9. mRNA co-electroporation in Meso-CAR-T cells**

| EP (electroporation sample No.) | BsAbs (15 µg) | CAR (2 µg) |
|---|---|---|
| E1 | / | / |
| E2 | / | M12 |
| E3 | 1412-4D11 | M12 |
| E4 | 4.1 | M12 |
| E5 | 4.2 | M12 |
| E6 | 4.3 | M12 |
| E7 | 4.4 | M12 |
| E8 | 4.5 | M12 |
| E9 | 4.6 | M12 |
| E10 | 4.7 | M12 |
| E11 | 4.8 | M12 |
| E12 | 8.6 | M12 |
| E13 | A40C28 (5 µg) | M12 |

### Example 8: Construction of Bispecific Antibodies

A bispecific antibody (diabody and BsAb for short) refers to a genetically engineered antibody that can specifically target 2 different epitopes on the same molecule or on different molecules simultaneously. The diabody herein consisted of scFv antibodies targeting CD28 and CD40, respectively, in tandem, that is, an scFv antibody targeting CD28 was formed by a VH and a VL of an antibody targeting CD28 (IgG1 or other forms), an scFv antibody targeting CD40 was formed by a VH and a VL of an antibody targeting CD40 (Fab or other forms), and the two scFv antibodies were linked in tandem. The two different scFvs were linked via GGGGS (G₄S for short). The VH and VL in each scFv were linked via three G₄S (GGGGSGGGGSGGGGS) (see Table 10). 1412 and 9.3 were CD28 antibodies, and 4D11, F2.103, F5.77, 40.37, 40.38, 40.45, 40.47, and 40.52 were all CD40 antibodies. 9.3h11 was the humanized antibody huVH1VL1 of 9.3, and 4052.12, 4052.17, 4052.25, 4052.28, and 4052.36 were affinity-matured antibodies of 40.52. A40C28 comprised an anti-CD40 scFv antibody, a transmembrane region, and an intracellular region of CD28 protein.

The CD40 antigen was specifically and highly expressed on the surface of some tumor cells, and the CD40 antibody at one terminus of the diabody could recognize and bind to CD40 on the surface of tumors; the CD28 antibody at the other terminus could bind to and activate the CD28 costimulatory signaling pathway on T cells, enhancing T cell functions. Therefore, the diabody could bind to CD40 on the tumor cells and the CD28 molecule on the T cells simultaneously, and the distance between the tumor cells and the T cells was shortened by bridging, thereby making it easier for the T cells to kill tumors.

The plasmid construction method of the bispecific antibody was as follows:
VH and VL fragments of the antibody were amplified by PCR, and subcloned into a pDA vector by a connection mode of Gibbson assembly homology arm recombination. The correctness of the plasmid was identified by Sanger sequencing.

**Table 10. Composition information of diabodies**

| **No.** | **Diabody name** | **Construction mode** | **Composition form** |
|---|---|---|---|
| 1 | 1412-4D11 | scFv-G₄S-scFv | 1412(VH-VL)-4D11(VH-VL) |
| 2 | 9.3-4D11 | scFv-G₄S-scFv | 9.3(VL-VH)-4D11(VH-VL) |
| 3 | 9.3-F2.103 | scFv-G₄S-scFv | 9.3(VL-VH)-F2.103(VH-VL) |
| 4 | 9.3-F5.77 | scFv-G₄S-scFv | 9.3(VL-VH)-F5.77(VH-VL) |
| 5 | 9.3-40.37 | scFv-G₄S-scFv | 9.3(VL-VH)-40.37(VL-VH) |
| 6 | 9.3-40.38 | scFv-G₄S-scFv | 9.3(VL-VH)-40.38(VL-VH) |
| 7 | 9.3-40.45 | scFv-G₄S-scFv | 9.3(VL-VH)-40.45(VL-VH) |
| 8 | 9.3-40.47 | scFv-G₄S-scFv | 9.3(VL-VH)-40.47(VL-VH) |
| 9 | 9.3-40.52 | scFv-G₄S-scFv | 9.3(VL-VH)-40.52(VL-VH) |
| 10 | 8.1 | scFv-G₄S-scFv | 9.3(VL-VH)-40.52(VL-VH) |
| 11 | 8.2 | scFv-G₄S-scFv | 9.3(VL-VH)-40.52(VH-VL) |
| 12 | 8.3 | scFv-G₄S-scFv | 9.3(VH-VL)-40.52(VL-VH) |
| 13 | 8.4 | scFv-G₄S-scFv | 9.3(VH-VL)-40.52(VH-VL) |
| 14 | 8.5 | scFv-G₄S-scFv | 40.52(VH-VL)-9.3(VL-VH) |
| 15 | 8.6 | scFv-G₄S-scFv | 40.52(VH-VL)-9.3(VH-VL) |
| 16 | 8.7 | scFv-G₄S-scFv | 40.52(VL-VH)-9.3(VH-VL) |
| 17 | 8.8 | scFv-G₄S-scFv | 40.52(VL-VH)-9.3(VL-VH) |
| 18 | 3.1 | scFv-G₄S-scFv | 9.3(VL-VH)-A40C(VH-VL) |
| 19 | 3.2 | scFv-G₄S-scFv | 9.3(VL-VH)-A40C(VL-VH) |
| 20 | 3.3 | scFv-G₄S-scFv | 9.3(VH-VL)-A40C(VH-VL) |
| 21 | 3.4 | scFv-G₄S-scFv | 9.3(VH-VL)-A40C(VL-VH) |
| 22 | 3.5 | scFv-G₄S-scFv | A40C(VH-VL)-9.3(VL-VH) |
| 23 | 3.6 | scFv-G₄S-scFv | A40C(VH-VL)-9.3(VH-VL) |
| 24 | 3.7 | scFv-G₄S-scFv | A40C(VL-VH)-9.3(VH-VL) |
| 25 | 3.8 | scFv-G₄S-scFv | A40C(VL-VH)-9.3(VL-VH) |
| 26 | 4.1 | scFv-G₄S-scFv | 9.3h11(VL-VH)-40.52(VL-VH) |
| 27 | 4.2 | scFv-G₄S-scFv | 9.3h11(VL-VH)-40.52(VH-VL) |
| 28 | 4.3 | scFv-G₄S-scFv | 9.3h11(VH-VL)-40.52(VL-VH) |
| 29 | 4.4 | scFv-G₄S-scFv | 9.3h11(VH-VL)-40.52(VH-VL) |
| 30 | 4.5 | scFv-G₄S-scFv | 40.52(VH-VL)-9.3h11(VL-VH) |
| 31 | 4.6 | scFv-G₄S-scFv | 40.52(VH-VL)-9.3h11(VH-VL) |
| 32 | 4.7 | scFv-G₄S-scFv | 40.52(VL-VH)-9.3h11(VH-VL) |
| 33 | 4.8 | scFv-G₄S-scFv | 40.52(VL-VH)-9.3h11(VL-VH) |
| 34 | 5.12 | scFv-G₄S-scFv | 4052.12(VH-VL)-9.3h11(VH-VL) |
| 35 | 5.17 | scFv-G₄S-scFv | 4052.17(VH-VL)-9.3h11(VH-VL) |
| 36 | 5.25 | scFv-G₄S-scFv | 4052.25(VH-VL)-9.3h11(VH-VL) |
| 37 | 5.28 | scFv-G₄S-scFv | 4052.28(VH-VL)-9.3h11(VH-VL) |
| 38 | 5.36 | scFv-G₄S-scFv | 4052.36(VH-VL)-9.3h11(VH-VL) |
| 39 | A40C28 | scFv-linker-TM & cytoplasmic domain | A40(VH-VL)-CD8 hinge - CD28 TM & cytoplasmic |

| | | | |
|---|---|---|---|
| Note: TM: a transmembrane region, cytoplasmic: an intracellular region | | | |

### Example 9: In Vitro Preparation of mRNA

mRNA was transcribed from a DNA template *in vitro* for subsequent detection of the functions of the diabodies at a cellular level.
(1) Plasmid linearization: the correct single clone was sequenced, and the plasmid was extracted by a mini plasmid extraction kit (Tiangen Biotech (Beijing) Co., Ltd., DP103). The plasmid was linearized by cleavage with SpeI endonuclease at 37 °C for 4 h or overnight. 2 µL of undigested and digested plasmids were taken out and subjected to electrophoresis on the 1% DNA nucleic acid gel to detect the digestion degree. The digested products were purified using a PCR product recovery kit (Qiagen, 28104).
*(2) In vitro* preparation of mRNA: mRNA was prepared *in vitro* by using an mMESSAGE mMACHINE^{™}T7 transcription kit (Invitrogen, AM1344). 1 µL of DNase I was added to this system to digest the template DNA at 37 °C for 30 min.
(3) mRNA was purified by using an RNeasy Kit (Qiagen).
(4) After the mRNA concentration was measured by using Nano-300, mRNA was cryopreserved at -80 °C.

### Example 10: Detection of Secondary Killing Effects of Diabodies

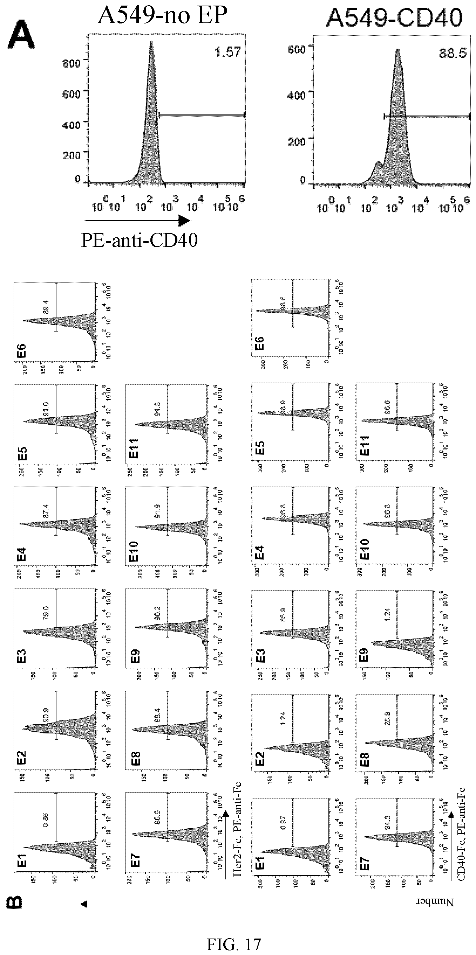

The killing-promoting effects of the diabodies were detected by co-culturing the diabodies, CAR-T cells, and target cells.
**1. The killing-promoting effects of the diabodies were detected by Her2-CAR-T cells.** The experimental procedures for the preparation of Her2-CAR-T cells and the killing-promoting effects of the diabodies were as follows:
   (1) Preparation of target cells: 10 µg of CD40 mRNA (A549-CD40 for short) was electroporated (EP) into A549-CBG cells by using a BTX ECM 830 electroporator, or no mRNA was electroporated (A549-no EP for short). The electroporation conditions were 300 V and 0.5 ms. After 24 hours, the expression level of CD40 was detected by flow cytometry and PE-anti-CD40 antibody. The detection results are shown in FIG. 17A.
   (2) Preparation of CAR-T cells: 0.5 µg of Her2-CAR (4D5.BBZ) mRNA, 15 µg of BsAb mRNA, or 10 µg of new A40C28 (i.e., A40C2828) mRNA was individually electroporated or co-electroporated into CD3⁺ T cells. The electroporation conditions were 500 V and 0.7 ms. Her2-CAR (4D5.BBZ) was identical to that of Example 7. After 24 hours, the expression levels of the diabodies and Her2-CAR were detected by flow cytometry, CD40-Fc, Her2-Fc, and PE-anti-Fc antibody. The detection results are shown in FIG. 17B, wherein A40C2828 was a fusion protein of an anti-CD40 antibody and a CD28 hinge region, the sequence of which can be found in SEQ ID NO: 76 of PCT/CN2022/126461 (incorporated herein by reference in its entirety). The sequence of A40C2828 in the present invention was numbered SEQ ID NO: 658. A40C2828 was also referred to as "new A40C28" in the drawing section of the present invention.
   (3) Co-culture: Her2-CAR-T cells and target cells A549-no EP or A549-CD40 were co-cultured in a ratio of effector cell (E):target cell (T) = 10:1 for 72 h. The cell killing was observed by using an incucyte S3 live-cell dynamic imaging and analysis system, and killing curves were plotted.
   (4) Cytokine release: after the Her2-CAR-T cells and target cells A549-no EP or A549-CD40 were co-cultured according to E:T = 1: 1 for 24 h, the supernatant was collected by centrifugation. The release level of IFN-gamma in the supernatant was detected by Cisbio bioassays' human IL-2 and IFN-γ assay kits.
**2. The effects of the diabodies were detected by Mesothelin (Meso for short)-CAR-T cells.** The experimental procedures for the preparation of Meso-CAR-T cells and the killing-promoting effects of the diabodies were as follows:
   (1) Preparation of target cells: no mRNA was electroporated into A549-CBG cells (A549-no EP for short), 10 µg of Meso (mesothelin) mRNA was electroporated into A549-CBG cells (A549-Meso for short), or 10 µg of Meso and 10 µg of CD40 mRNA were electroporated into A549-CBG cells (A549-Meso + CD40 for short). The electroporation conditions were 300 V and 0.5 ms. After 24 hours, the expression level of CD40 was detected by flow cytometry and PE-anti-CD40 antibody.
   (2) Preparation of CAR-T cells: 2 µg of Meso-CAR (M12) mRNA, 15 µg of BsAb mRNA, or 5 µg of A40C28 mRNA was electroporated into CD3⁺ T cells. The electroporation conditions were 500 V and 0.7 ms. Meso-CAR (M12) was identical to that of Example 7. After 24 hours, the expression levels of the diabodies and Meso-CAR were detected by flow cytometry, CD40-Fc and PE-anti-Fc, and Meso-Fc and PE-anti-Fc antibody.
   (3) Co-culture: Meso-CAR-T cells and target cells A549-no EP, A549-Meso, or A549-Meso + CD40 were co-cultured according to E:T = 10:1 for 72 h. The cell killing was observed by using the incucyte S3 live-cell dynamic imaging and analysis system, and killing curves were plotted.
   (4) Cytokine release: after the Meso-CAR-T cells and target cells A549-no EP, A549-Meso, or A549-Meso + CD40 were co-cultured according to E:T = 1:1 for 24 h, the supernatant was collected by centrifugation. The release levels of IL-2 and IFN-gamma in the supernatant were detected by Cisbio bioassays' human IL-2 assay and human IFN-γ assay.

### Analysis of results:

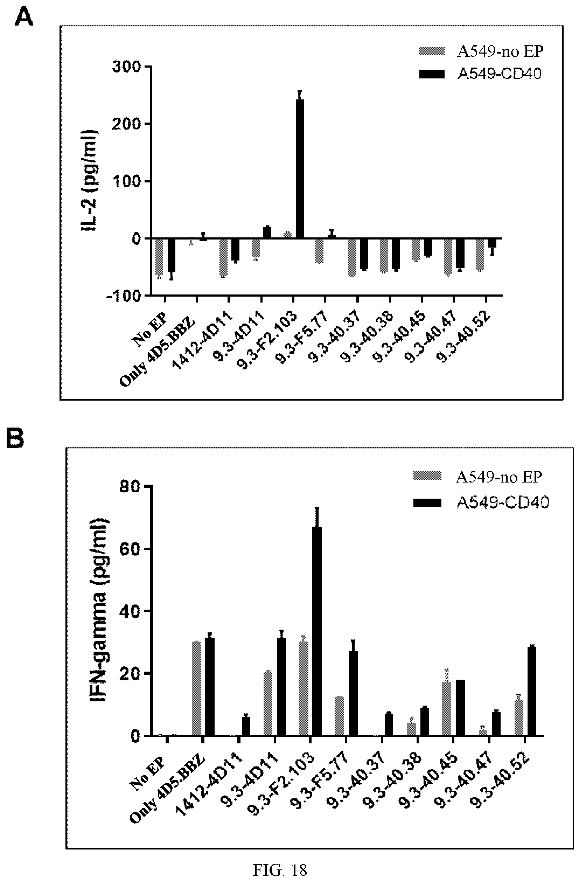

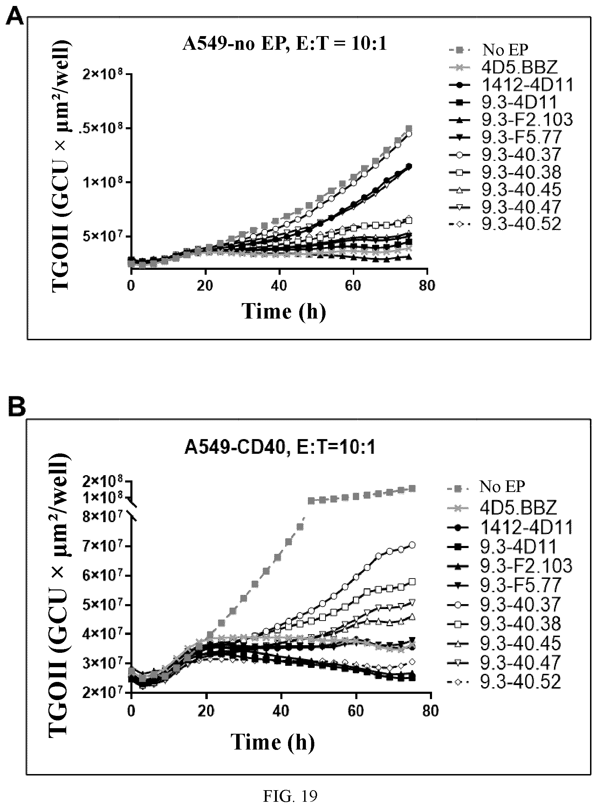

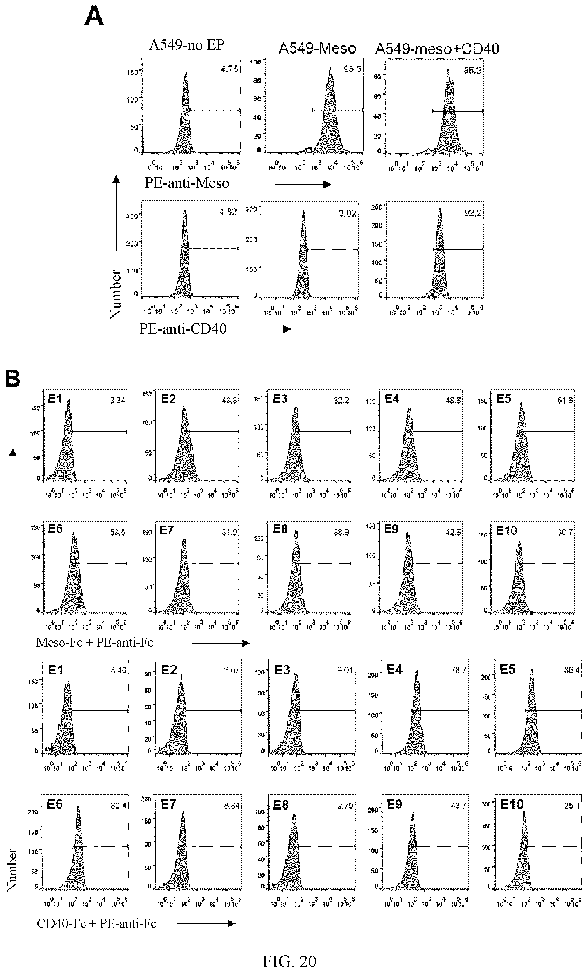

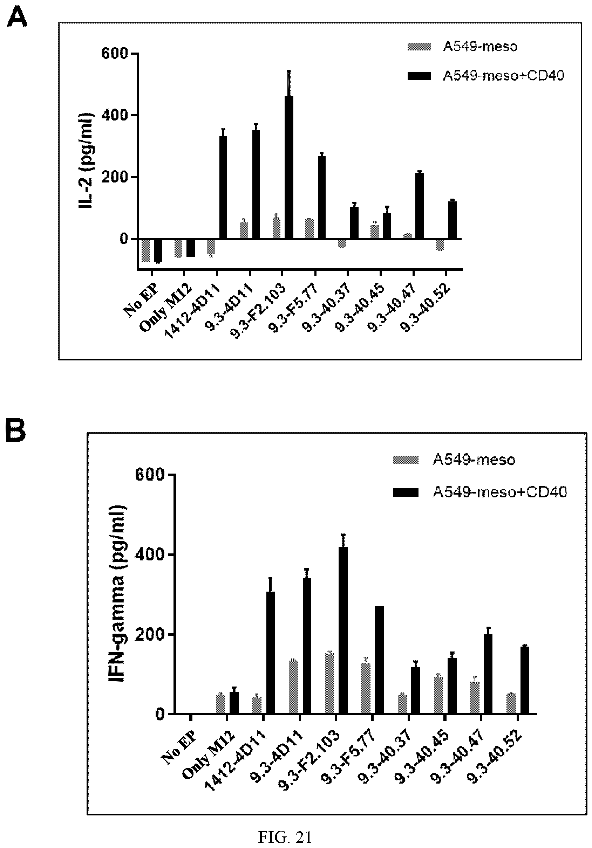

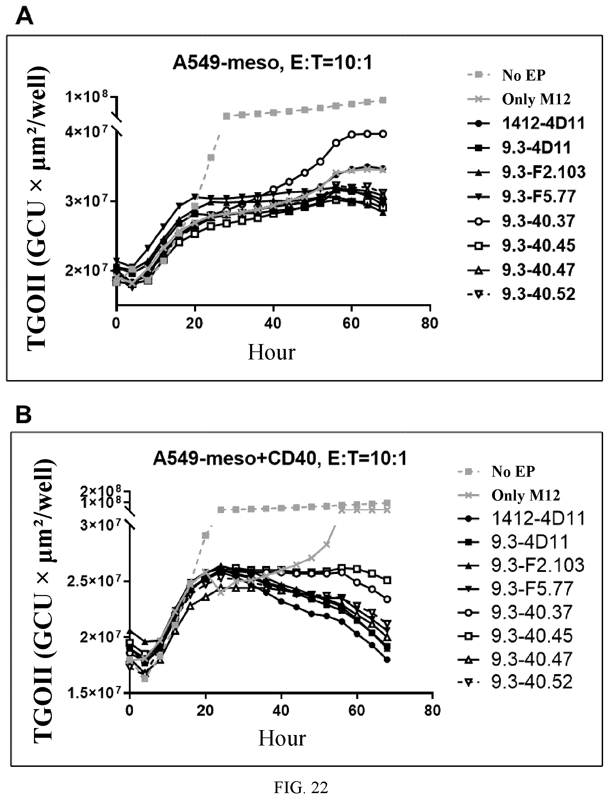

The CD28 antibody 9.3 and CD40 antibodies (4D11, F2.103, F5.77, 40.37, 40.38, 40.45, 40.47, and 40.52) constituted diabodies (see Table 10), and mRNA and CAR mRNA of these diabodies were co-electroporated into T cells. The diabody could be expressed by T cells and secreted out of the cells to form a diabody in a free state. When Her2-CAR-T cells secreting diabodies were co-cultured with target cells A549-no EP (or CD40), the diabodies 9.3-F2.103 and 9.3-40.52 could promote the release of more IL-2 and IFN-gamma cytokines as compared to Her2-CAR-T cells alone (FIG. 18), could reduce the fluorescence value of the target cells to be lower, and had stronger ability to improve the killing of the target cells (FIG. 19). After Meso-CAR-T cells secreting diabodies were co-cultured with target cells, the release of cytokines IL-2 and IFN-gamma and the killing of the target cells were similar to Her2-CAR-T cell co-culture (FIGs. 20, 21, and 22).

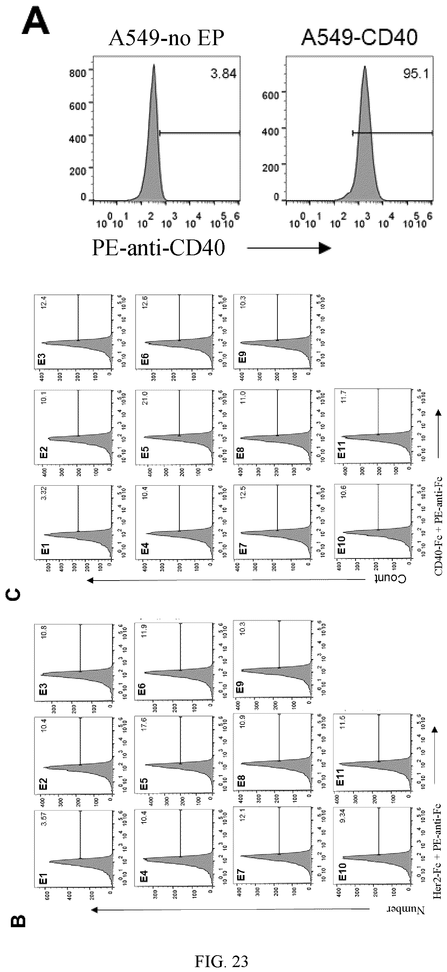

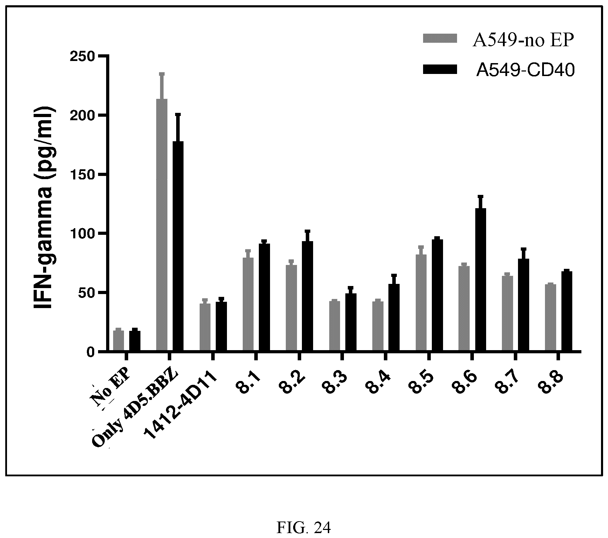

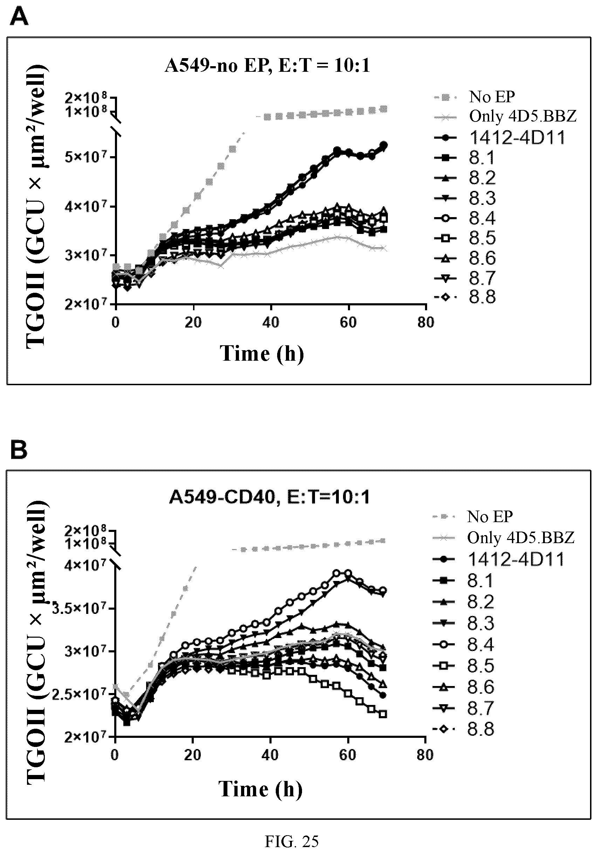

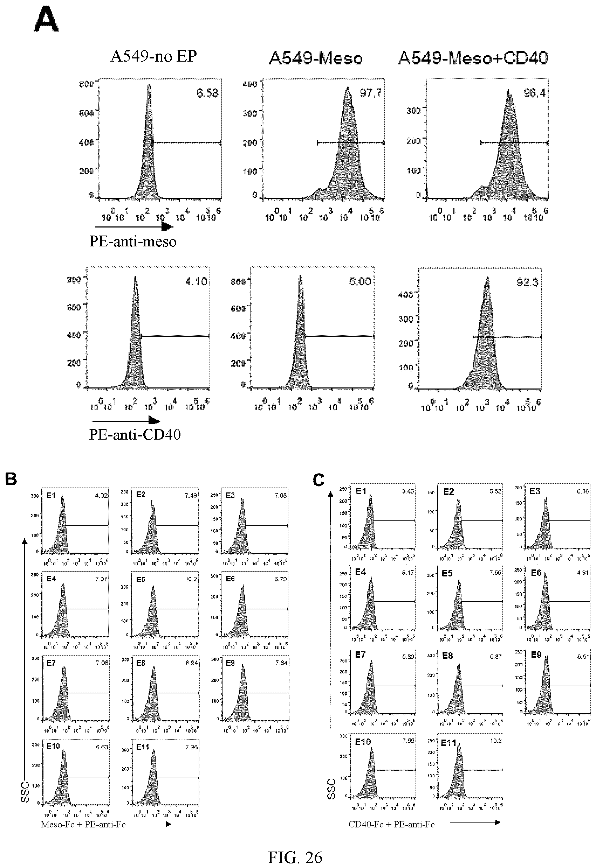

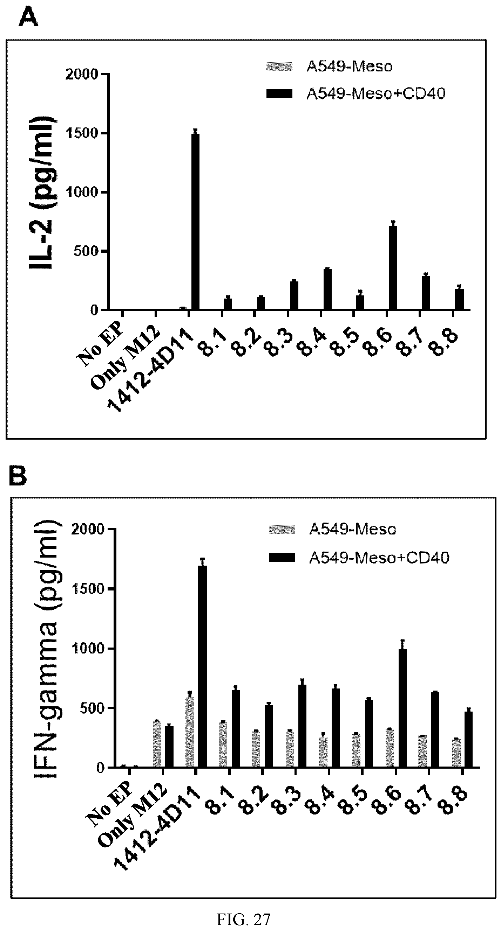

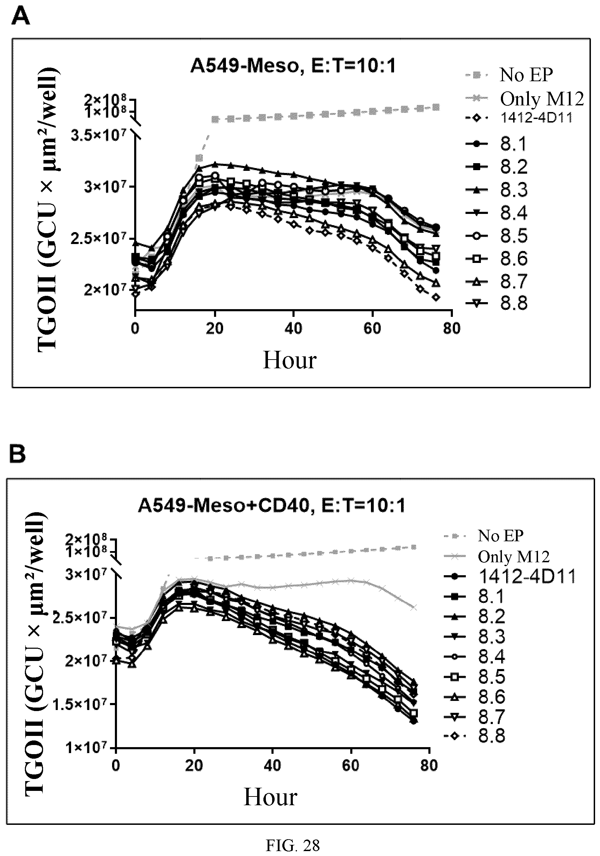

The diabody 9.3-40.52 was selected for subsequent optimization experiments. The relative positions of the VH and VL as well as 9.3 and 40.52 were recombined to obtain 8 different kinds of diabodies, which were named "8.1, 8.2 ... and 8.8" (see Table 10), to construct different cells, and the level of gene overexpression was detected (FIG. 23). After Her2-CAR-T cells secreting the diabodies 8.1-8.8 were co-cultured with target cells, the diabodies 8.5 and 8.6 could promote the release of more IFN-gamma (FIG. 24), and could enable the CAR-T cells to kill more target cells. However, the killing-promoting ability of these diabodies was still weaker than that of the diabody 1412-4D11 (FIG. 25). Similar experimental results were obtained after Meso-CAR-T cells secreting the diabodies 8.1-8.8 were co-cultured with target cells (FIGs. 26, 27, and 28).

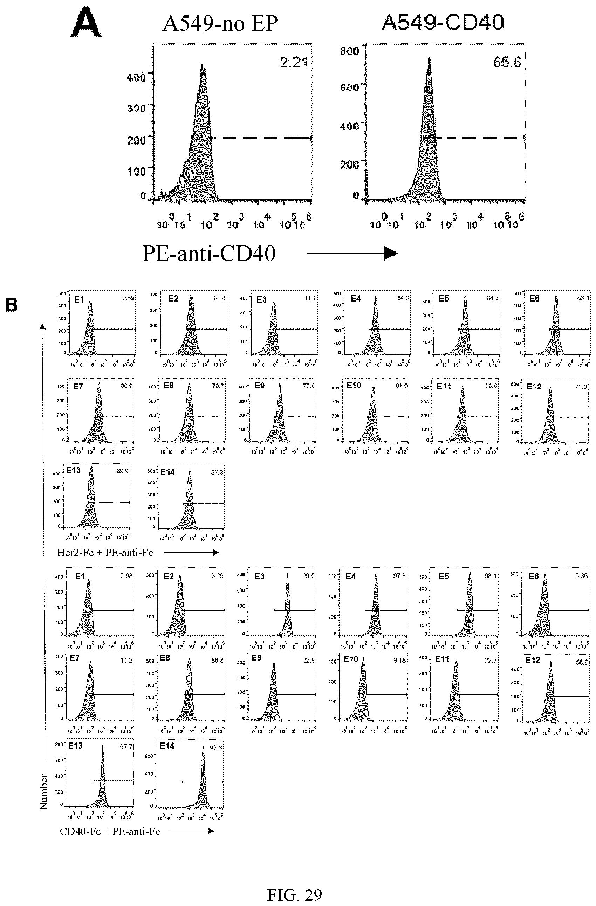

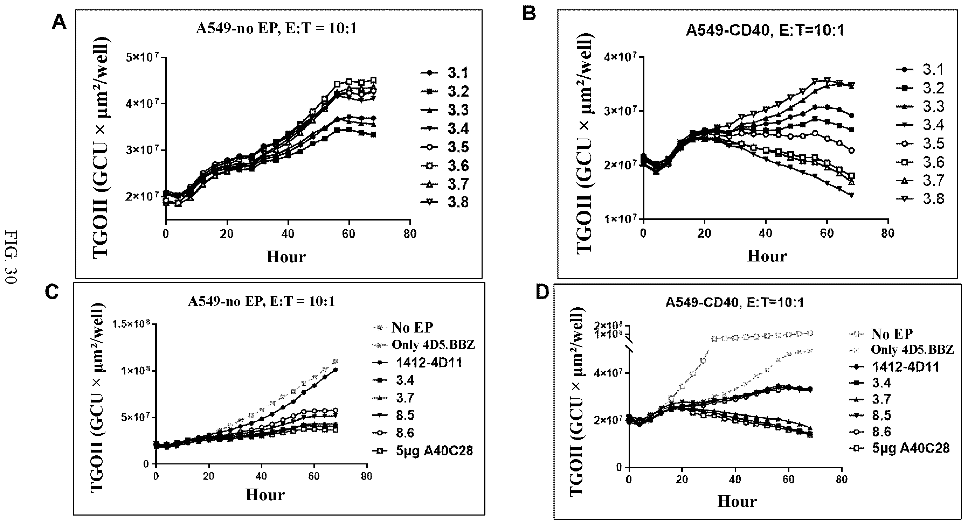

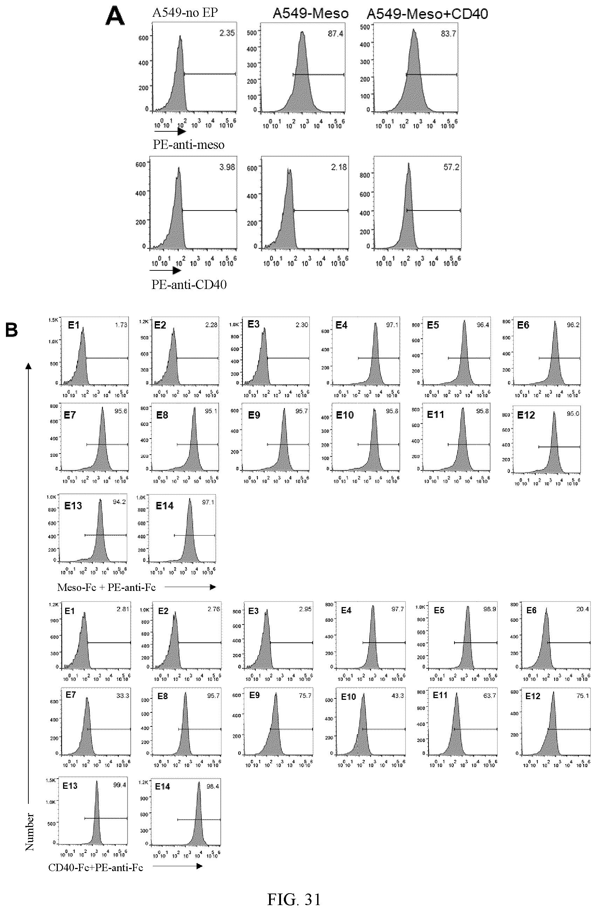

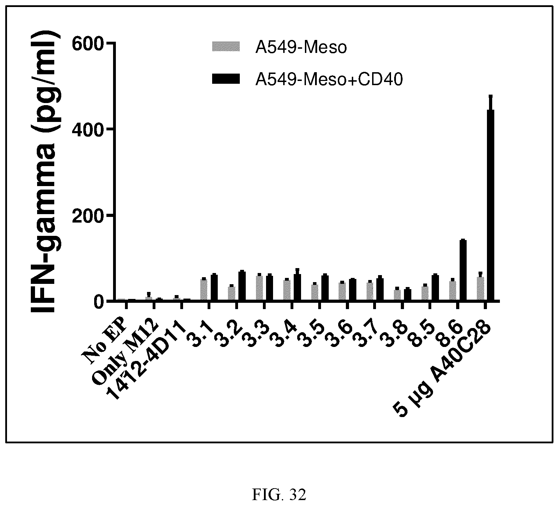

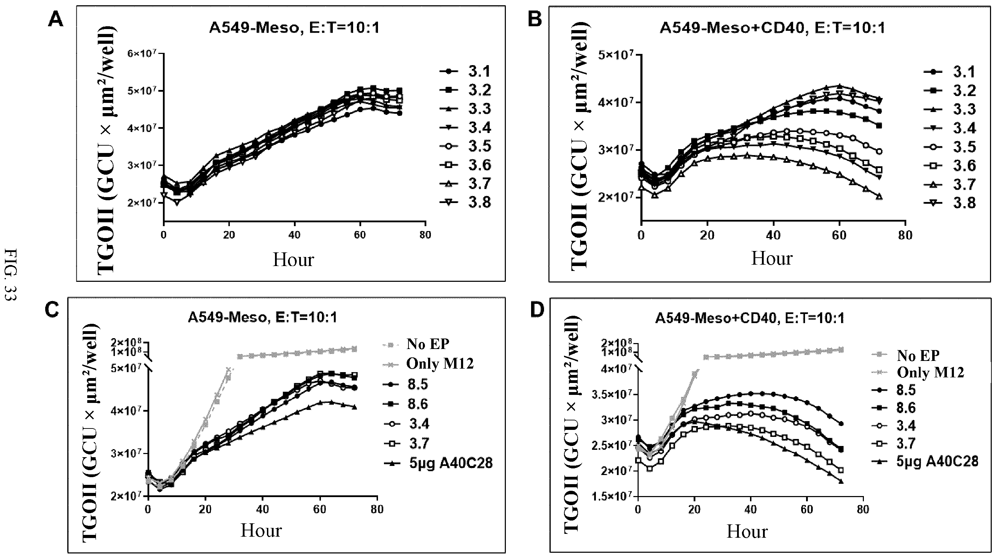

The CD40 antibody (A40C) in A40C28 was combined with the 9.3 antibody to form diabodies, which were named "3.1, 3.2 ... and 3.8" (see Table 10). After Her2-CAR-T cells secreting the diabodies 3.1-3.8 were co-cultured with target cells, the diabodies 3.4 and 3.7 could enable the CAR-T cells to kill more target cells, the killing-promoting ability of which was stronger than that of the diabody 8.5 and 8.6 (FIGs. 29 and 30). Similar experimental results were obtained after Meso-CAR-T cells secreting the diabodies 3.1-3.8 were co-cultured with target cells (FIGs. 31, 32, and 33).

### Example 11: Binding ELISA

The killing-promoting ability of the diabodies 3.4 and 3.7 was stronger than that of the diabodies 8.5 and 8.6, which may be caused by the different affinities of CD40 antibodies. Therefore, the affinity of the CD40 antibody (in the form of a human classical monoclonal antibody IgG1) was detected by *in vitro* binding ELISA. The brief experimental procedures were as follows:
(1) Construction of antibody expression plasmid: an antibody VH region was subcloned into a pEF vector containing a human CH1-Fc antibody fragment, and an antibody VL region was subcloned into a pEF vector containing a human CL antibody fragment.
(2) Antibody expression: 33 µg of an antibody heavy chain expression plasmid and 67 µg of an antibody light chain expression plasmid were co-transfected into 293F suspension cells by PEI. After 4 days of culture, the supernatant was collected by centrifugation.
(3) Antibody purification: the protein supernatant and protein A purification medium were incubated on ice for 2 h, and then the supernatant and the purification medium were separated by a gravity column. The purification medium was rinsed thoroughly with 100 mL of pre-cooled PBS to remove impure proteins, and finally, the target protein was eluted from the purification medium with 5-10 mL of 0.1 M Glycine-HCl (pH 2.7), and 1/10 volume of 1 M Tris-HCl (pH 9.0) was immediately added to neutralize the antibody. The buffer of the antibody was replaced with PBS through an ultrafiltration tube. After the protein concentration was measured with A280, the antibody was packaged and cryopreserved at -80 °C.
(4) Antigen coating: 50 µL of recombinant human CD40-His antigen protein (ACRO, CD0-H5228) at a concentration of 2 µg/mL was added to each well of an ELISA plate, and the plate was incubated at 4 °C overnight.
(5) The next day, the ELISA plate was rinsed 5 times with PBST (containing 0.05% Tween-20).
(6) Blocking: the plate was blocked with a blocking solution (5% skim milk powder) at room temperature for 1-2 h. The blocking solution was completely discarded.
(7) Primary antibody incubation: 50 µL of CD40 antibodies at various dilution concentrations were added to each well, and the plate was incubated at room temperature for 2 h. The plate was rinsed 5 times with PBST.
(8) Secondary antibody incubation: 50 µL of Goat Anti-Human IgG-Fc (HRP) antibody (Sino Biological, SSA001) was added to each well, and the plate was incubated at room temperature for 1 h. The plate was rinsed 5 times with PBST.
(9) Color development: 50 µL of TMB chromogenic solution (Invitrogen, 501129758) was added to each well, and after color development at room temperature, 50 µL of 2 M HCl was added to stop the reaction. The plate was read at 450 nm.

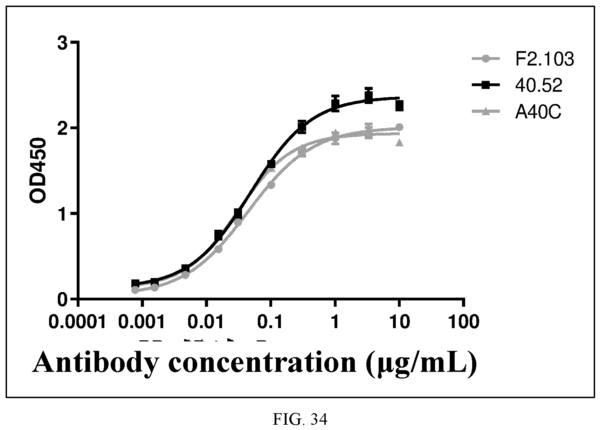

The experimental results (FIG. 34 and Table 11) show that the affinities, EC50 values, of the CD40 antibodies were in the following order: 40.52 < F2.103 < A40C. The 40.52 antibody had the lowest affinity for the CD40 antigen. Improving the affinity of 40.52 may improve the functions of the diabodies 8.5 and 8.6.

**Table 11. Summary of CD40 antibody affinities**

| Antibody name | EC50 (µg/mL) | Molecular weight (kDa) | EC50 (M) |
|---|---|---|---|
| F2.103 | 0.04482 | 146.9 | 3.05E-07 |
| 40.52 | 0.04919 | 142.9 | 3.44E-07 |
| A40C | 0.03127 | 144.6 | 2.16E-07 |

### Example 12: Construction of Mutant Library

In order to improve the affinity of the 40.52 antibody, this example constructed a mutant Fab antibody library by random point mutation of CDR regions of an Fab antibody of 40.52, and antibodies with high affinities were screened from the mutant library.
(1) The abYsis software was used to analyze the sequence of the 40.52 antibody, and the CDR regions were defined according to the Kabat, AbM, and Chothia antibody nomenclature.
(2) The random point mutation was performed on 2 adjacent amino acids in the CDR region by using a primer NNK mutation and PCR. The mutant fragment was subcloned into a phage vector pComb3XSS by digestion and linkage.
(3) The phage plasmid was electroporated into competent cells TG1 by a BIO-RAD Micropulser electroporator. 5 µL of the phage plasmid was added to 50 µL of TG1 electroporation competent cells, and the resulting mixture was gently pipetted and mixed well. The plasmid-competent cell mixture was transferred to electroporation cuvettes (Biorad, 1652089) spaced 0.1 cm apart, and electroporation was performed in the Ec1 mode (about 1800 V, 0.5 ms). The electroporation cuvettes were immediately inserted into ice and left to stand for 2 min after electroporation. The cells in the cuvette were resuspended in 1 mL of SOC (pre-heated at 37 °C) medium and transferred into a 15 mL centrifuge tube. 3 mL of SOC was supplemented into the centrifuge tube, and the cells were cultured with shaking at 37 °C and 275 rpm for 1 h.
(4) Some cells were diluted in gradient with SOC and applied on a 2× TY-AG plate (containing 100 µg/mL ampicillin and 2% glucose), and the remaining cells were completely applied on three to five 2× TY-AG plates with a diameter of 15 cm. The plates were cultured at 37 °C overnight.
(5) The next day, clones were counted, and library size was calculated. The cells on the large plate were scraped with 3 mL of 2× TY medium, and after OD600 was measured, the cells were packaged and cryopreserved at -80 °C.

### Example 13: Phage Screening

From the mutant library, the Fab antibody with the high affinity was panned by phage screening.
1. Preparation of phage library
   (1) Phage library amplification: about 500 µL of phage library liquid was added to 100 mL of 2× TY/AG medium (containing 100 µg/mL ampicillin and 2% glucose) according to OD600 = 0.1. The diluted phage library was cultured with shaking at 37 °C and 250 rpm for 2 h, enabling OD600 to reach 0.5-0.8.
   (2) Addition of helper phage: the helper phage M13KO7 was added to the phage library liquid according to MOI = 20: 1, and the resulting mixture was mixed well, left to stand at 37 °C for 30 min, and then cultured with shaking for 30 min.
   (3) Removing glucose: the infected liquid was centrifuged at 4000 rpm for 10 min, and the supernatant was completely discarded to eliminate the inhibitory effect of glucose.
   (4) Expression inducing: the cell precipitate was resuspended in 100 mL of 2× YT/AKI medium (containing 100 µg/mL ampicillin, 50 µg/mL kanamycin, and 100 µM IPTG). The resulting mixture was cultured with shaking at 25 °C and 250 rpm overnight in the dark.
   (5) Phage precipitation: after 16-20 hours, the phage liquid was collected and centrifuged at 4 °C and 4000 rpm for 10 min. 40 mL of the supernatant was transferred to a new 50 mL centrifuge tube, 10 mL of PEG/NaCl solution (20% PEG-8000, 2.5 M NaCl) was then added, and the resulting mixture was mixed well. The mixture was left to stand on ice for 1 h.
   (6) The precipitate was centrifuged at 4 °C and 4000 rpm for 30 min for sediment, and the supernatant was discarded.
   (7) Resuspending phage precipitate: the phage precipitate was resuspended in 2 mL of PBS to obtain the phage library.
2. Phage Screening
   (1) Antigen coating: the recombinant human CD40-Fc antigen protein was diluted to 1 µg/mL with PBS (1 µg/mL for the first round of screening, 0.5 µg/mL for the second round of screening, and 0.1 µg/mL for the third round of screening), and 300 µL of the diluted CD40-Fc was added to 6 wells of an ELISA plate. The plate was incubated at 4 °C overnight.
   (2) The next day, the ELISA plate was rinsed 5 times with PBST (containing 0.05% Tween-20).
   (3) Blocking: the plate was blocked with a blocking solution (2% skim milk powder + 1% BSA) at room temperature for 1-2 h. The blocking solution was completely discarded. When the ELISA plate was blocked, the phage was also blocked. 1/10 volume of the blocking solution was added to the phage solution, and the resulting mixture was incubated with rotation at room temperature for 1-2 h.
   (4) Phage incubation: the blocked phage was added evenly to antigen-containing wells at 300 µL/well, and the plate was cultured with shaking at room temperature for 2 h.
   (5) Rinsing: the phage was completely discarded, and the ELISA plate was washed 5 times with PBST.
   (6) Elution: 150 µL of an eluent (0.1 M glycine, pH 2.7, 0.5 M NaCl) was added to each well, the resulting mixture was gently pipetted on the bottom and wall of the well for 10 min, and the eluate was transferred to a 15 mL centrifuge tube. 1/10 volume of 1 M Tris-HCl (pH 9.0) was immediately added for neutralization.
   (7) Phage infection: 8 mL of TG1 (OD600 = 0.5) liquid was added to the centrifuge tube, and the tube was left to stand at 37 °C for 30 min and then cultured with shaking at 37 °C and 250 rpm for 30 min.
   (8) Applying plate: the phage liquid was centrifuged at 4000 rpm for 10 min, most of the supernatant was discarded, about 1 mL of the supernatant was left to resuspend the phage precipitate, and the phage liquid was applied evenly on a 2× TY-AG plate with a diameter of 15 cm. The plates were cultured at 37 °C overnight.
   (9) The next day, colonies were scraped from the plate using 3 mL of a 2× TY medium, 200 µL of the phage liquid was used for the next screening, and the remaining phage liquid was cryopreserved at -80 °C with an equal volume of 50% glycerol.

### Example 14: Phage ELISA

Clones with the specific high affinity were screened by phage ELISA. The specific experimental procedures were as follows:
1. Preparation of monoclonal phage
   (1) Preparation of single clone: an appropriate amount of the phage liquid obtained by the third round of screening was diluted with a 2× TY medium, applied on a 2× TY-AG plate, and cultured at 37 °C overnight.
   (2) Monoclonal colonies were picked and cultured in a deep-well plate containing 250 µL of 2× TY-AG medium (containing 100 µg/mL ampicillin and 2% glucose) with shaking at 37 °C and 250 rpm overnight.
   (3) Transferring: the next day, 10 µL of the overnight bacterial cultures were transferred to a deep-well plate containing 300 µL of 2× TY-AG medium and cultured with shaking at 37 °C and 250 rpm for 2 h.
   (4) Infection of helper phage: 20 µL of M13KO7 helper phage was added to each well according to MOI = 20: 1, and the resulting mixture was mixed gently, incubated at 37 °C for 30 min, and then cultured with shaking at 37 °C and 250 rpm for 30 min.
   (5) The phage liquid was centrifuged at 4000 rpm for 7 min, and the supernatant was completely discarded. The phage precipitate was resuspended in 300 µL of 2× TY-AKI medium (containing 100 µg/mL ampicillin, 50 µg/mL kanamycin, and 100 µM IPTG), and the resulting mixture was cultured with shaking at 25 °C and 250 rpm overnight.
   (6) The next day, the phage liquid was centrifuged at 4000 rpm for 10 min. The supernatant was used for phage ELISA.
2. Phage ELISA
   (1) Antigen coating: 50 µL of recombinant human CD40-His antigen protein (ACRO, CD0-H5228) at a concentration of 2 µg/mL was added to each well of an ELISA plate, and the plate was incubated at 4 °C overnight.
   (2) The next day, the ELISA plate was rinsed 5 times with PBST (containing 0.05% Tween-20).
   (3) Blocking: the plate was blocked with a blocking solution (2% skim milk powder + 1% BSA) at room temperature for 1-2 h. The blocking solution was completely discarded.
   (4) Phage incubation: 50 µL of monoclonal phage was added to each well, and the plate was incubated at room temperature for 2 h. The plate was rinsed 5 times with PBST.
   (5) Secondary antibody incubation: 50 µL of HRP-anti-M13 antibody (Sino Biological, 11973-MM05T-H) was added to each well, and the plate was incubated at room temperature for 1 h. The plate was rinsed 5 times with PBST.
   (6) Color development: 50 µL of TMB chromogenic solution (Invitrogen, 501129758) was added to each well, and after color development at room temperature, 50 µL of 2 M HCl was added to stop the reaction. The plate was read at 450 nm.

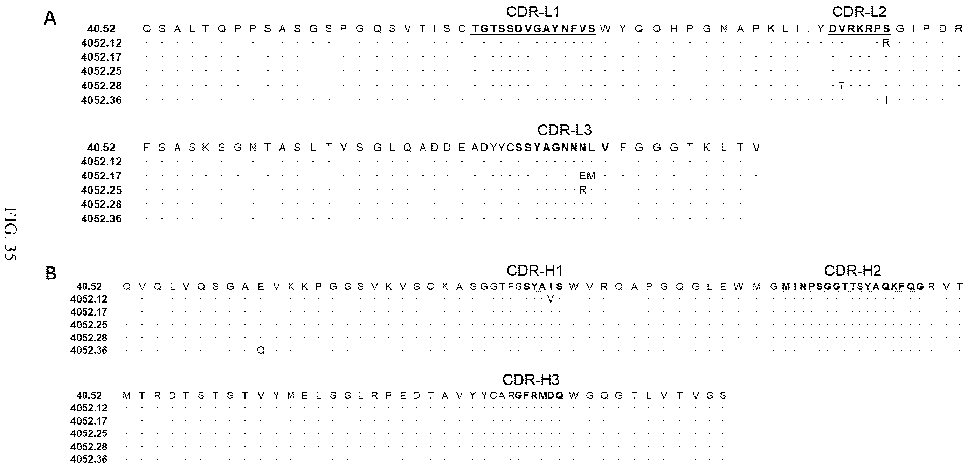

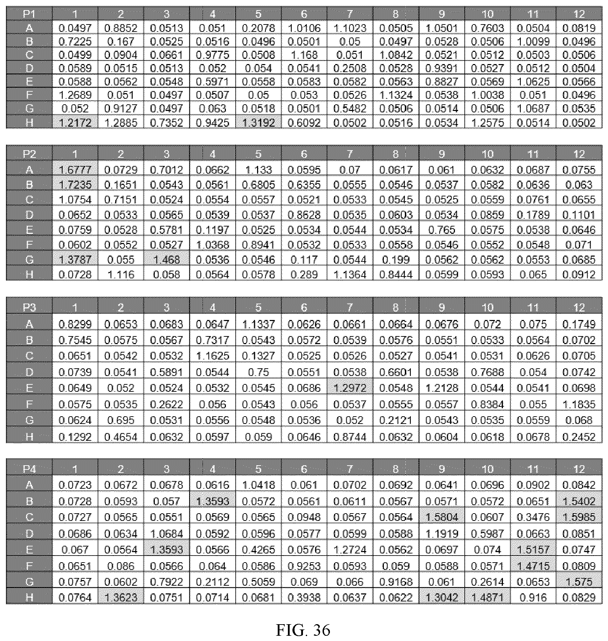

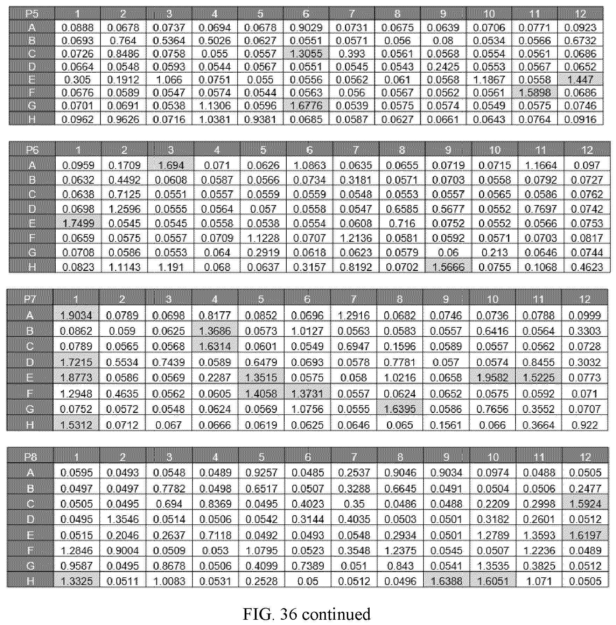

The experimental results are shown in FIG. 36. H1 wells in P1 and P8 plates were both parent controls, and a single clone with an OD450 value greater than that of the parent (grey wells in FIG. 36) was picked, colony PCR was performed, and DNA sequences were identified by Sanger sequencing to obtain a plurality of affinity-matured anti-CD40 Fab antibodies, wherein the sequences of antibodies 4052.12, 4052.17, 4052.25, 4052.28, and 4052.36 are shown in FIG. 35 and in the "Sequence" section below.

### Example 15: Plasmid Construction of Diabodies

The DNA sequence of the correctly sequenced positive single clone was subcloned into a pDA vector for *in vitro* preparation of mRNA.
(1) The structural form of a bispecific antibody (diabody for short) composed of VHs and VLs of the CD40 antibody parent 40.52 and the CD28 antibody (9.3h11) was as follows: CD40 antibody (VH)-(G₄S)₃ linker-CD40 antibody (VH)-(G₄S) linker-CD28 antibody (VH)-(G₄S)₃ linker-CD28 antibody (VL).
(2) Taking the positive monoclonal phage liquid as a template, the VH and VL fragments of the CD40 affinity-matured antibody were amplified by PCR and subcloned into the pDA vector containing the CD28 (VH-VL) fragment by a connection mode of Gibson assembly homology arm recombination. The correctness of the plasmid was identified by Sanger sequencing.
(3) Plasmid linearization: the correct single clone was sequenced, and the plasmid was extracted by a mini plasmid extraction kit (Tiangen Biotech (Beijing) Co., Ltd., DP103). The plasmid was linearized by cleavage with SpeI endonuclease at 37 °C for 4 h or overnight. 2 µL of undigested and digested plasmids were taken out and subjected to electrophoresis on the 1% DNA nucleic acid gel to detect the digestion degree. The digested products were purified using a PCR product recovery kit (Qiagen, 28104).
*(4) In vitro* preparation of mRNA: mRNA was prepared *in vitro* by using an mMESSAGE mMACHINE^{™}T7 transcription kit (Invitrogen, AM1344). 1 µL of DNase I was added to this system to digest the template DNA at 37 °C for 30 min.
(5) mRNA was purified by using an RNeasy Kit (Qiagen).
(6) After the mRNA concentration was measured by using Nano-300, mRNA was cryopreserved at -80 °C.

The mRNA prepared *in vitro* was used for a subsequent secondary killing effect experiment to detect the effects of the CD40 affinity-matured antibodies.

### Example 16: Detection of Secondary Killing Effect

A plurality of candidate CD40 affinity-matured antibodies were obtained by phage screening. VHs and VLs of the CD40 affinity-matured antibody and the CD28 antibody constituted a diabody (mature diabody for short), and then the diabody composed of the affinity-matured molecules was detected for the killing-promoting effect by co-culturing the diabody with Her2-CAR-T cells (or Meso-CAR-T cells) and target cells.
1. The killing-promoting effects of the mature diabodies were detected by Her2-CAR-T cells and compared to the parent diabody 4.6. The experimental procedures for the preparation of Her2-CAR-T cells and the killing-promoting effects of the diabodies were as follows:
   (1) Preparation of target cells: 10 µg of CD40 mRNA (A549-CD40 for short) was electroporated into A549-CBG cells by using a BTX ECM 830 electroporator, or no mRNA was electroporated (A549-no EP for short). The electroporation conditions were 300 V and 0.5 ms. After 24 hours, the expression level of CD40 was detected by flow cytometry and PE-anti-CD40 antibody. FIG. 37A shows that CD40 was expressed on the surface of A549 cells at a relatively high level.
   (2) Preparation of CAR-T cells: 1 µg of Her2-CAR (4D5.BBZ) mRNA, 10 µg of BsAb mRNA, or 10 µg of new A40C28 (i.e., A40C2828) mRNA was individually electroporated or co-electroporated into CD3⁺ T cells as Her2-CAR-T cells. The electroporation conditions were 500 V and 0.7 ms. Her2-CAR (4D5.BBZ) was identical to that of Example 7. After 24 hours, the expression levels of the diabodies and Her2-CAR were detected by flow cytometry, CD40-Fc, Her2-Fc, and PE-anti-Fc antibody (FIGs. 37B, C, and D). The experimental results show that the expression levels of the diabodies and Her2-CAR were all relatively high.
   (3) Co-culture: Her2-CAR-T cells and target cells A549-no EP or A549-CD40 were co-cultured in a ratio of effector cell (E):target cell (T) = 10:1, 3:1, or 1:1 for 72 h. The cell killing was observed by using the incucyte S3 live-cell dynamic imaging and analysis system, and killing curves were plotted. The experimental results (FIGs. 39A, B, C, D, and E) show that a plurality of mature diabodies had stronger killing-promoting effects than the parent diabody, and could reduce the fluorescence value of the target cells to be lower. Meanwhile, the killing-promoting effects of the diabody molecules 5.12, 5.17, 5.25, 5.28, and 5.36 were stronger than that of the parent diabody 4.6 and was also stronger than that of 1412-4D11, which were very close to the function of the new A40C28 (i.e., A40C2828) (FIG. 39F).
   (4) Cytokine release: after the Her2-CAR-T cells and target cells A549-no EP or A549-CD40 were co-cultured according to E:T = 1:1 for 24 h, the supernatant was collected by centrifugation. The release level of IFN-gamma in the supernatant was detected by Cisbio bioassays' human IL-2 and IFN-γ assay kits. The experimental results (FIG. 38) show that the diabody molecules 5.12, 5.17, 5.25, 5.28, and 5.36 could promote the release of more IL-2 and IFN-gamma cytokines, and had release-promoting ability greater than that of the parent diabodies 4.6 and 1412-4D11, but still weaker than that of the new A40C28 (i.e., A40C2828).
2. The killing-promoting effects of the mature diabodies were detected by Mesothelin (Meso for short)-CAR-T cells and compared to the parent diabody 4.6. The experimental procedures for the preparation of Meso-CAR-T cells and the killing-promoting effects of the diabodies were as follows:
   (1) Preparation of target cells: no mRNA was electroporated into Molm14-CBG cells (Molm 14-no EP for short), 10 µg of Meso (mesothelin) mRNA was electroporated into Molm14-CBG cells (Molm14-Meso for short), 10 µg of CD40 mRNA was electroporated into Molm14-CBG cells (Molm14-CD40 for short), or 10 µg of Meso and 10 µg of CD40 mRNA were co-electroporated into Molm14-CBG cells (Molm14-Meso + CD40 for short) simultaneously. The electroporation conditions were 400 V and 0.5 ms. After 24 hours, the expression levels of Meso and CD40 were detected by flow cytometry, Meso-biotin and PE-streptavidin antibody, A40C-IgG1 and PE-anti-Fc antibody. FIG. 40A shows that CD40 was expressed on the surface of A549 cells at a relatively high level. The expression level of Meso was detected by flow cytometry, Meso-biotin, and PE-streptavidin antibody. FIG. 40A shows that Meso and CD40 were expressed on the surface of Molm14 cells at a relatively high level, and the high expression of Meso also caused the up-regulation of CD40 expression at the same time.
   (2) Preparation of CAR-T cells: 1 µg of Meso-CAR (M12) mRNA, 10 µg of BsAb mRNA, or 10 µg of new A40C28 (i.e., A40C2828) mRNA (see Table 12) was individually electroporated or co-electroporated into CD3⁺ T cells as Meso-CAR-T cells. The electroporation conditions were 500 V and 0.7 ms. Meso-CAR (M12) was identical to that of Example 7. After 24 hours, the expression levels of the diabodies were detected by flow cytometry, CD40-Fc, and PE-anti-Fc antibody; the expression level of Meso-CAR was detected by Meso-Fc and PE-anti-Fc antibody. The experimental results (FIG. 40B) show that the expression levels of the diabodies and Meso-CAR were relatively high.
   (3) Co-culture: Meso-CAR-T cells and target cells Molm14-no EP, Molm14-Meso, Molm14-CD40, or Molm14-Meso + CD40 were co-cultured according to E:T = 10:1 or 3:1 for 72 h. The cell killing was observed by using the incucyte S3 live-cell dynamic imaging and analysis system, and killing curves were plotted. The experimental results (FIGs. 42A, B, C, and D) show that the diabodies 5.12, 5.17, 5.25, 5.28, and 5.36 had stronger killing-promoting effects compared to the parent diabody 4.6 and the diabody 1412-4D11, and could reduce the fluorescence value of the target cells to be lower after the combined action with Meso-CAR-T cells. The killing-promoting effect of the diabody 5.17 was very close to that of the new A40C28 (i.e., A40C2828) (FIGs. 42B and D).
   (4) Cytokine release: after the Meso-CAR-T cells and target cells Molm14-no EP, Molm 14-Meso, Molm 14-CD40, or Molm 14-Meso + CD40 were co-cultured according to E:T = 1:1 for 24 h, the supernatant was collected by centrifugation. The release levels of IL-2 and IFN-gamma in the supernatant were detected by Cisbio bioassays' human IL-2 assay and human IFN-γ assay. The experimental results (FIG. 41) show that the diabodies 5.12, 5.17, 5.25, 5.28, and 5.36 all had stronger release-promoting ability than that of the parent diabody 4.6, which was closer to the diabody 1412-4D11 and new A40C28 (i.e., A40C2828).

**Table 12. mRNA co-electroporation in Meso-CAR-T cells**

| **EP** | **10 µg BsAb** | **1 µg CAR** |
|---|---|---|
| E1 | / | / |
| E2 | / | M12 |
| E3 | Only 1412-4D11 | / |
| E4 | Only 4.6 | / |
| E5 | Only 5.12 | / |
| E6 | Only 5.25 | / |
| E7 | Only 5.36 | / |
| E8 | Only 5.28 | / |
| E9 | Only 5.17 | / |
| E10 | Only new A40C28 (i.e., A40C2828) | / |
| E11 | 1412-4D11 | M12 |
| E12 | 4.6 | M12 |
| E13 | 5.12 | M12 |
| E14 | 5.25 | M12 |
| E15 | 5.36 | M12 |
| E16 | 5.28 | M12 |
| E17 | 5.17 | M12 |
| E18 | New A40C28 (i.e., A40C2828) | M12 |

### Example 17: Specificity Detection of Bispecific Antibodies

The killing curve of target cells was observed by co-culturing Meso-CAR-T cells with different target cells, and the release of cytokines in the co-culture supernatant was detected.
(1) The preparation of the Meso-CAR-T cells was detailed in part 2(2) of Example 16.
(2) The Meso-CAR-T cells were co-cultured with different target cells (Jeko-1, Raji, THP1, Nolm6, HCC70, H226, SKOV3, Caski, PC3, and A549) according to E:T = 10:1. The cell killing was observed by using the incucyte S3 live-cell dynamic imaging and analysis system, and killing curves were plotted (FIGs. 44A-F). The experimental results (FIGs. 44A, B, C, D, and E) show that the specificities of the diabodies 5.12, 5.17, 5.25, 5.28, and 5.36 were better than those of the parent 4.6, the diabody 1412-4D11, and new A40C28 (i.e., A40C2828).
(3) Cytokine release: after Meso-CAR-T cells and different target cells (Jeko-1, Raji, THP1, Nolm6, HCC70, H226, SKOV3, Caski, PC3, and A549) were co-cultured according to E:T = 1:1 for 24 h, the supernatant was collected by centrifugation. The release levels of IL-2 and IFN-gamma in the supernatant were detected by Cisbio bioassays' human IL-2 assay and human IFN-γ assay. The experimental results (FIG. 43) also show that 5.12, 5.17, 5.25, 5.28, and 5.36 diabodies exhibited better specificity than the parent 4.6, diabody 1412-4D11, and new A40C28 (i.e., A40C2828).

### Example 18: Expression and Purification of Antibodies

Recombinant antibodies were expressed *in vitro* by 293F suspension cells and purified by protein A medium.

The experimental procedures were as follows: The V-region of the antibody was inserted into an expression vector containing the IgG1 framework. The expression plasmid was transiently transfected into the 293F suspension cells by PEI, and the supernatant was collected after 4 days of culturing. Protein A was added to the supernatant to capture the antibody, and the antibody was eluted with an eluent, thus obtaining a purified antibody. The yield of antibody was calculated by measuring A280, and the purity of the antibody was detected by SDS-PAGE electrophoresis. The results are shown in FIG. 45. The antibody had a single band and good purity under nonreduced conditions (FIG. 45-A); under reduced conditions (FIG. 45-B), the heavy chain was relatively intact and the light chain showed slight diffusion and degradation. The yield of different antibodies varied widely, and as shown in Table 13, the expression amount of the affinity-matured antibody 4052.17 was closest to that of the parent antibody 40.52.

**Table 13. Yield of antibodies**

| Antibody | Yield (mg/100 mL) |
|---|---|
| A40C | 0.371 |
| 40.52 | 0.73 |
| 4052.12 | 0.410 |
| 4052.17 | 0.715 |
| 4052.25 | 0.664 |
| 4052.28 | 0.478 |
| 4052.36 | 0.570 |

### Example 19: Detection of Binding Activities of Affinity-Matured Antibodies by ELISA (Enzyme Linked Immunosorbent Assay)

The binding activities of the affinity-matured antibodies for antigen CD40 were detected by ELISA. The experimental procedures were as follows:
1. 50 µL of recombinant CD40-His protein at a concentration of 2 µg/mL was added to each well of an ELISA plate, and the plate was incubated at 4 °C overnight.
2. The next day, the ELISA plate was washed 3 times with PBST (containing 0.05% Tween-20).
3. 250 µL of 5% skim milk powder (dissolved in PBS) was added to each well of the ELISA plate, and the plate was incubated at room temperature for 2 h.
4. The plate was washed 3 times with PBST, antibodies with different dilution ratios were added to the ELISA plate at 50 µL/well, and the plate was incubated at room temperature for 60 min.
5. The plate was washed 3 times with PBST, 50 µL of HRP-anti-hFc antibody was added to each well, and the plate was incubated at room temperature for 60 min.
6. The plate was washed 3 times with PBST, 50 µL of TMB was added to each well, and after color development at room temperature, 50 µL of 2 M HCl was added to each well to stop the reaction. The plate was read at 450 nm.

The experimental results (Table 14 and FIG. 46) show that compared with the parent 40.52, only one of the affinity-matured antibodies, the 4052.17, had higher binding activity, and the binding activities of other affinity-matured antibodies were all reduced to different degrees.

**Table 14. Summary of ELISA results of affinity-matured antibodies**

| Antibody | EC50 (µg/mL) |
|---|---|
| A40C | 0.0652±0.0021 |
| 40.52 | 0.0975±0.0043 |
| 4052.12 | 0.1242±0.0052 |
| 4052.17 | 0.0944±0.0131 |
| 4052.25 | 0.1107±0.0050 |
| 4052.28 | 0.1157±0.0015 |
| 4052.36 | 0.1338±0.0077 |

### Example 20: Detection of Affinities of Affinity-Matured Antibodies by SPR

The affinity kinetics of the affinity-matured antibodies were detected using surface plasmon resonance (SPR) technique.

The experimental procedures were as follows: The binding affinities between the antibodies and the CD40 antigen were detected by SPR technique using Biacore 2000 (Cytiva, Sweden). All experiments were performed at 25 °C. The machine was first equilibrated with 10 mM Glycine-HCl (pH 1.5) for 30 s, and then the antibody 40.52 or 4052.17 at a concentration of 2 µg/mL was captured using a protein A chip with PBST (pH 7.4, containing 0.05% tween-20) flowing at a rate of 10 µL/min. CD40 diluted in a 2-fold gradient (3.125 - 100 nM) was injected into the mobile phase. The association time was set at 90 s and the dissociation time at 300 s. The chip surface was regenerated by incubation with 10 mM Glycine-HCl (pH 1.5) for 30 s to remove the bound CD40 and antibody. The affinity of the antibody was simulated using a biphasic ligand model (heterogenerous ligand mode) in the build-in Biacore Evaluation software of the machine.

The experimental results (Table 15 and FIG. 47) show that both KD1 and KD2 of the affinity-matured antibody 4052.17 were increased relative to 40.52, indicating an increased affinity.

**Table 15. Summary of affinity kinetics assay results for affinity-matured antibodies**

| | | |
|---|---|---|
| Loaded antibody | 40.52 | 4052.17 |
| Detection antigen | CD40 | CD40 |
| ka1 (1/Ms) | 1.34E+06 | 1.49E+06 |
| kd1 (1/s) | 0.001154 | 0.001018 |
| KD1 (=kd1/ka1)(M) | 8.64E-10 | 6.86E-10 |
| ka2 (1/Ms) | 8.61E+05 | 7.48E+05 |
| kd2 (1/s) | 0.08849 | 0.03624 |
| KD2 (=kd2/ka2) (M) | 1.03E-07 | 4.84E-08 |
| Rmax1 (RU) | 13.5 | 25.31 |
| Rmax2 (RU) | 76.74 | 96.2 |
| Chi² (RU²) | 0.314 | 0.894 |
| Fitting model | Biphasic ligand model | Biphasic ligand model |

### Example 21: Preparation of Lentivirus-Transduced CAR-T Cells

The functional CAR-T cells were prepared by infecting T cells with a lentivirus containing a CAR structure. The diabody 5.17 consisted of the affinity-matured CD40 antibody 4052.17 and the humanized CD28 antibody 9.3h11. The 5.17 and mesothelin CAR (M12) were combined to form M12-5.17 and 5.17-M12.

The experimental procedures were as follows: A lentiviral plasmid containing a CAR structure (M12), a CAR and a diabody (M12-5.17 or 5.17-M12) or a CAR and LACO molecule (A40C2828-M12) and a helper plasmid were transiently transfected into 293T cells for virus packaging, the supernatant was collected, and the virus was concentrated after high speed centrifugation. The viral titer was determined, and human T cells that had been activated by CD3/CD28 Dynabeads (Thermo, #11161D) were infected at an MOI of 3. On day 5 after infection, beads were removed and CAR-T cells were continued to be cultured until day 13. The transduction efficiency of the CAR-T cells was detected, and the CAR-T cells were cryopreserved.

The transduction efficiency of the CAR-T cells was detected as follows: 5E6 CAR-T cells were plated into each well of a 96-well plate, and the plate was washed once with PBS. 50 µL of Meso-Fc or CD40-Fc protein at a final concentration of 2 µg/mL was added to each well, and the mixture was incubated for 30 min at 4 °C. The cells were washed 3 times with PBS, and 50 µL of PE-anti-Fc antibody at a final concentration of 1 µg/mL was added to each well. The mixture was incubated at 4 °C for 30 min. After being washed twice with PBS, the cells were detected by a flow cytometer.

The experimental results (FIG. 48) show that M12-5.17 had a relatively low transduction efficiency and that other CARs had a transduction efficiency of around 30%. The transduction efficiency of CAR was close to that of the LACO molecule A40C2828.

### Example 22: Detection of Secondary Killing Effect of Lentivirus-Transduced CAR-T cells

The lentivirus-transduced CAR-T cells obtained in Example 21 were co-cultured with target cells, the killing curves of the target cells were observed, and the release of cytokines in the co-culture supernatant was detected. At the same time, the functional difference comparison with M12 and A40C2828-M12 was performed.

Co-culturing: the lentivirus-transduced CAR-T cells and target cells SKOV3, SKOV3-Meso, or SKOV3-Meso+CD40 were co-cultured for 72 h according to E: T = 10:1, 3:1, or 1:1, respectively, and cell killing was observed with an incucyte S3 live cell dynamic imaging and analysis system and killing curves were plotted. The experimental results (FIG. 49) show that compared with M12-only situation and A40C2828-M12, M12-5.17 and 5.17-M12 had stronger killing effect and were able to further lower the fluorescence value of target cells.

Cytokine release: the lentivirus-transduced CAR-T cells and target cells SKOV3, SKOV3-Meso, or SKOV3-Meso+CD40 were co-cultured for 24 h according to E: T = 1:1, and the supernatant was collected by centrifugation. The release level of IFN-gamma in the supernatant was detected by Cisbio bioassays' human IL-2 and IFN-γ assay kits. The experimental results (FIG. 50) show that compared with M12-only situation and A40C2828-M12, M12-5.17 and 5.17-M12 failed to release more IL-2 but released more cytokine IFN-gamma.

### Example 23: Specificity Detection of Lentivirus-Transduced CAR-T cells

The lentivirus-transduced CAR-T cells obtained in Example 21 were co-cultured with different target cells, the killing curves of the target cells were observed, and the release of cytokines in the co-culture supernatant was detected.

The lentivirus-transduced CAR-T cells and different target cells (SKOV3, ASPC1, H226, 786-0, A549, OVCAR3, U251, Caski, SH-SYSY, Siha, HepG2, THP-1, Raji, Nolm6, and Molm14) were co-cultured according to E:T = 3:1, and cell killing was observed with an incucyte S3 live cell dynamic imaging and analysis system and killing curves were plotted. The experimental results (FIG. 51) show that the specificity of M12-5.17 was better than that of 5.17-M12.

(3) Cytokine release: the lentivirus-transduced CAR-T cells and different target cells (SKOV3, ASPC1, H226, 786-0, A549, OVCAR3, U251, Caski, SH-SY5Y, Siha, HepG2, THP-1, Raji, Nolm6, and Molm14) were co-cultured for 24 h according to E:T = 1:1, and the supernatant was collected by centrifugation. The release levels of IL-2 and IFN-gamma in the supernatant were detected by Cisbio bioassays' human IL-2 assay and human IFN-γ assay. The experimental results (FIG. 52) also show that the specificity of M12-5.17 was better than that of 5.17-M12.

### Example 24: In Vivo Efficacy of Lentivirus-Transduced CAR-T Cells

The lentivirus-transduced CAR-T cells obtained in Example 21 were reinfused to tumor-bearing NSG mice, tumor changes were observed, and the *in vivo* efficacy of the CAR-T cells was detected.

The experimental procedures were as follows:
(1) Tumor bearing: 5E6 SKOV3-Meso+CD40-CBG cells were inoculated subcutaneously into the upper dorsal part of NSG mice, and tumors were allowed to grow for 11 days.
(2) Reinfusion of CAR-T cells: Tumor-bearing NSG mice were reinfused with low (5E5 CAR-T cells per mouse) and high (3E6 CAR-T cells per mouse) doses of CAR-T cells. Every 7 days, the size of the tumor was measured by using a vernier caliper, and the mice were each weighed. The changes in the fluorescence values of the tumors were measured by using a small animal *in vivo* imaging system, and the deaths of the mice were recorded.

The experimental results (FIGs. 53 and 54) show that after the reinfusion of low-dose CAR-T, compared with the control group (UTD), the M12-only CAR group, and the A40C2828-M12 group, M12-5.17 CAR-T cells were able to reduce the mean fluorescence value of the tumor more rapidly (FIGs. 53-A and B), the tumor size decreased more rapidly (FIG. 53-C), and there was no significant change in mouse body weight (FIG. 53-D) and no mouse deaths (FIG. 53-E).

After the reinfusion of high-dose CAR-T, both the M12-5.17 and A40C2828-M12 groups showed rapid decrease in tumor size and mean fluorescence value compared with the control group (UTD) and the M12-only CAR group (FIG. 53-A, B and C); mice in the A40C2828-M12 group showed significant weight loss, and mice in the other groups didn't show significant change in body weight (FIG. 53-D); the A40C2828-M12 group showed the worst survival curve of mice, and the survival curve of the M12-5.17 group was better than that of the M12-only CAR group but was not as well as that of the control group (FIG. 53-E).

### The following examples 25-29 relate to antibodies, CARs and CAR-Ts targeting mesothelin (Meso)

### Example 25: Preparation of Anti-Mesothelin Antibodies

Anti-mesothelin antibodies were prepared using a fully human antibody phage display library according to the following procedures:
(1) Expression and purification of phage display library: The log-phase TG1 library culture was infected with freshly thawed M13K07 helper phage at a multiple infection rate of 20:1 (phage to cell ratio), followed by inducing overnight with IPTG; the phage library was purified by PEG/NaCl precipitation and then the phage titer was determined. Phages were stored at 4 °C and later subjected to scFv selection.
(2) Selection of mesothelin-specific scFv-phages: In the first round of selection, a Maxisorp plate was coated with 20 µg/mL mesothelin-6His protein dissolved in 1× PBS and incubated overnight at 4 °C. (In the subsequent rounds of selection, lower protein concentrations were used for more stringent selections, including 2 µg/mL in the second round of bioscreening and 0.5 µg/mL in the third round of bioscreening.) The plate was then washed three times with PBS and a blocking buffer (1× PBS containing 5% milk and 1% BSA) was added to each well. After incubation for 2 h at room temperature, the blocking buffer was discarded, the phage solution was added, and the plate was sealed with a preservative film and incubated for 2 h with gentle shaking. In the first round of selection, the plate was then washed 10 times with PBST. (In the subsequent rounds, the stringency of the washing was increased by adding more washing cycles: 20 washing cycles in the second round and 30 washing cycles in the third round). The antigen-bound scFv-phages were then eluted using 1 mL of acid elution buffer (pH 2.2), neutralized, and seeded into 15 mL of the log-phase TG1 culture (OD600 = 0.5). The mixture was left to stand at 37 °C for 30 min and then cultured for 30 min with shaking, seeded on a 2xYT-GA agar plate, and incubated overnight at 30 °C for subsequent selection.
(3) mpELISA screening: After three rounds of screening, 288 positive clones were selected for monoclonal phage ELISA (mpELISA) screening. Phage supernatant was generated from a single colony clone and tested for its binding to the mesothelin-Fc protein. The supernatant was incubated with a pre-sealed Maxisorp plate coated with 2 µg/mL mesothelin-6His protein. After three times of washing, the HRP-conjugated anti-M13 antibody was added at 100 µL/well. The HRP-conjugated anti-M13 antibody was diluted in a blocking buffer (1× PBS containing 5% milk and 1% BSA) at 1:5000. The mixture was incubated at room temperature for 60 min. After the plate was washed 5 times with PBST, a TMB substrate solution was added at 100 µL/well, and the mixture was incubated for 10-30 min until blue color appeared. The reaction was stopped by adding a stop solution (2N H2SO4) at 50 µL/well. The absorbance at 450 nm was read in a microplate reader. FIG. 55 shows the resulting absorbance readings from the mpELISA screening. As shown, positive colonies (absorbance at 450 nm ≥ 0.5) were identified to produce anti-mesothelin antibodies capable of binding to mesothelin-6His protein (FIG. 55).
(4) Cloning and sequence analysis: Positive clones were selected according to the ELISA results and used as PCR cloning templates for scFv sequences (the sequence of the forward primer is set forth in SEQ ID NO: 659: tgcagctggcacgacaggtttc, and the sequence of the reverse primer is set forth in SEQ ID NO: 660: cgtcagactgtagcacgtt). The PCR products were then sequenced by sanger sequencing (the sequence of the forward primer is set forth in SEQ ID NO: 661: aacaattgaattcaggagga, and the sequence of the reverse primer is set forth in SEQ ID NO: 662: cctcctaagaagcgtagtc). CDR regions of the scFvs were analyzed by the abysis website (http://abysis.org/), and for the numbering rules, the Kabat, Chothia, AbM, Contact, or IMGT system was used, see Table 16.

**Table 16. CDRs and their sequence numbers (SEQ ID NO)**

| **Antibody** | **VL CDR1** | **VL CDR2** | **VL CDR3** |
|---|---|---|---|
| **M6** | RASQSIGNSLA | DVSNRAT | QHRYSWPLT |
| | 87 | 102 | 116 |
| **M7** | RASQAISSALA | DASTLES | QQADSFPLT |
| | 88 | 103 | 117 |
| **M8** | RASQSISSSLN | AASSLQS | QQSYSTPLT |
| | 89 | 104 | 118 |
| **M10** | RASQSISSWLA | KASSLES | QQYYSYPLT |
| | 90 | 105 | 119 |
| **M12** | RASQGGGNYLA | GASKLQS | QQLNSYPVT |
| | 91 | 106 | 120 |
| **M13** | RASQGISNSLA | AASRLES | QQYYSTPFT |
| | 92 | 107 | 121 |
| **M15** | RASQGVNSALA | DASSLES | QQFSSYPLT |
| | 93 | 108 | 122 |
| **M20** | RASQGISSAVA | YASSLES | QQFNSYPLT |
| | 94 | 109 | 123 |
| **M22** | TGTRRDIGGYEYVS | SVNNRPS | SSYSSRDTLVL |
| | 95 | 110 | 124 |
| **M24** | SGSSSNIGSNYVY | MNNQRPS | AARDDSLSGYVT |
| | 96 | 111 | 125 |
| **M27** | RASQPIVASHLA | GASTRAA | QQYGISPF |
| | 97 | 112 | 126 |
| **M28** | SGTSSDVGGYNFV S | EVSKRPS | SSYAGRNNPYL |
| | 98 | 113 | 127 |
| **M31** | TGTSSDIGGYNSVS | GVSRRPS | SSYGGSNNLL |
| | 99 | 114 | 128 |
| **M32** | QASEDINNSLN | DASDLET | QQLNSYPLT |
| | 100 | 115 | 129 |
| **M37** | RASQGISNYLA | AASSLQS | QQYSSYPIT |
| | 101 | 104 | 130 |

| **Antibody** | **VH CDR1** | **VH CDR2** | **VH CDR3** |
|---|---|---|---|
| **M6** | DYYMS | YISSSGSTIYYA DSVKG | DGYRTHNWFDP |
| | 131 | 144 | 157 |
| **M7** | AYWIA | IIYPGDSRVIYS PYFQG | FGGPKFATNWFDI |
| | 132 | 145 | 158 |
| **M8** | GYAMY | AITTNGGSTNY ADSVKG | GAPGYRGYYMDV |
| | 133 | 146 | 159 |
| **M10** | SYAMS | AISGSGGSTYY ADSVKG | RGSSWYFDY |
| | 134 | 147 | 160 |
| **M12** | TYYIH | IINPSSGSTTYT QKFQG | GETLRGYFDY |
| | 135 | 148 | 161 |
| **M13** | ANTIN | RIIPSLNIRDYA QEFQG | DPGSTWSPNQFFPH |
| | 136 | 149 | 162 |
| **M15** | SYYMH | IINPSGGSTSYA QKFQG | AQRGGSVYFDY |
| | 137 | 150 | 163 |
| **M20** | DSWIA | IIFPGDSNPIYSP SFQG | HAAWGAGWFDP |
| | 138 | 151 | 164 |
| **M22** | SYAIS | GIIPIFGTANYA QKFQG | GRSGSYGLY |
| | 139 | 152 | 165 |
| **M24** | SYAMS | AISGSGGSTYY ADSVKG | GVATFDY |
| | 134 | 147 | 166 |
| **M27** | DYWIG | WITPNNGNTN YAPKFQG | RGRNS SGYLYYYSM DV |
| | 140 | 153 | 167 |
| **M28** | SYAIS | GIIPIFGTANYA QKFQG | DLGGYSYGHGLDY |
| | 139 | 152 | 168 |
| **M31** | SSWMA | NIKQDGSSQYY VDSVKG | DIWYSIDY |
| | 141 | 154 | 169 |
| **M32** | GYYLH | IINPSGGRTSMA QKFQG | ADNWNAGSMDV |
| | 142 | 155 | 170 |
| **M37** | PYYWT | YIHYSGRTNYN PSLES | VGDPGLFDY |
| | 143 | 156 | 171 |

### Example 26: Preparation of Mesothelin CARs

Vectors were constructed for the production of anti-mesothelin CAR mRNAs. The pDA vector was first digested with Xba1 and Sal1 enzymes and purified by the gel purification method. The scFv fragments obtained in Example 25 above and the CAR fragment (a CD8 hinge, a CD8 transmembrane domain, a 4-1BB costimulatory domain, and a CD3-zeta signaling domain) were amplified by PCR and purified by the gel purification method. The scFv fragments and the CAR fragment (from the hinge domain to the CD3-zeta domain) and the pDA vector were ligated by the Gibson assembly method and transformed into competent cells. Colonies with the correct construct were identified by Sanger sequencing and selected for further experiments. **FIG.** 56 provides a schematic **diagram** of a pDA-CAR vector for producing CAR mRNA.

The pDA-CAR plasmid was then linearized by digestion with Spe1. The linearized vector was purified using the PCR Cleanup kit and eluted with RNase-free water. The concentration of DNA was determined by nanodrop and checked by agarose DNAgel. *In vitro* transcription (IVT) was then carried out according to the experimental scheme of the manufacturer (Thermofisher, Cat. No.: AM13455). Briefly, a 20-µL system containing 1 µg of template DNA, NTP/ARCA buffer, T7 buffer, GTP, T7 enzyme, and RNase-free H₂O was added to a 0.2-mL PCR tube and incubated at 37 °C for 3 h. After 3 h, 2 µL of DNase was added for each reaction, and the mixture was incubated at 37 °C for 15 min. The final procedure was performed according to the manufacturer's recommendations. The IVT mRNA was purified using the RNasy kit (Qiagen), and the concentration of RNA was determined by nanodrop and checked by the PAGE gel.

### Example 27: Tumor Cell Lines and Human Primary Lymphocytes

Tumor cell lines, including A549-CBG (human lung cancer (carcinoma) cells), H226-CBG (human lung cancer (malignant epithelial tumor) cells), MOLM14-CBG (human leukemia cells), ASPC1-CBG (human pancreatic tumor cells), HCC70-CBG (human breast cancer cells), and OVCAR3-CBG (human ovarian cancer cells), were cultured in the RPMI-1640 medium containing 10% FCS. Primary lymphocytes from normal donors were stimulated with anti-CD3/CD28 immunomagnetic beads (Life Technologies) and cultured in the R10 medium (RPMI-1640 containing 10% FCS, penicillin-streptomycin (100×), HEPES (100×), sodium pyruvate (100×), Glutamax (100×), and NEAA (100×)). On day 10 after stimulation, T cells were cryopreserved at 1 × 10⁸ cells/vial in a solution consisting of 90% FCS and 10% DMSO.

### Example 28: Preparation and Characterization of Mesothelin CARTs

Mesothelin CAR mRNA obtained in Example 26 above was introduced into A549 tumor cells and T cells by electroporation, and the procedures are as follows: A549 tumor cells and T cells were collected and washed 3 times with the Opti-MEM medium. The cell pellet was resuspended in the Opti-MEM medium and the cell concentration was adjusted to 1 × 10e7 cells/mL. 10 µg of RNA was aliquoted into 1.5-mL EP tubes, 100 µL of T cells or A549 cells were added, followed by mixing well. 100 µL of cells mixed with RNA were added to the BTX electroporation cuvette and slight tapping was performed to avoid air bubbles. Electroporation was performed using a BTX instrument under the following parameters: for T cells: 500 V voltage, 0.7 ms; for A549 tumor cells: 300 V voltage, 0.5 ms. The cells were then transferred to a pre-warmed medium and cultured at 37 °C.

The binding of mesothelin CART cells to the mesothelin-Fc recombinant protein was determined by FACS staining. As shown in **FIG.** 57, anti-mesothelin scFv-M1, -M2, - M3, -M6, -M7, -M8, -M9, -M10, -M11, -M12, -M13, -M14, -M15, -M16, -M17, -M20, -M22, -M23, -M24, -M25, -M27, -M28, -M29, -M30, -M31, -M32, -M33, -M34, -M35, -M36, and -M37 bound to the mesothelin-Fc recombinant protein. T cells without CAR molecules served as the control ("Mock"). As shown in **FIG.** 58, the ectopic expression level of mesothelin was correlated with the amount of mesothelin mRNA introduced into A549 cells by electroporation.

The cytotoxicity of mesothelin CART cells against tumor cells was determined in an *in vitro* cytotoxicity assay. EGFP-expressing tumor cell lines or EGFP-A549 cells electroporated with different amounts of tumor antigen were seeded at 3000 cells/100 µL/well into a flat-bottom 96-well plate. CART cells were diluted to a proper concentration, seeded at 100 µL/well, and co-cultured with tumor cells according to different E/T ratios such as 10:1, 3:1, and 1:1. The co-culture plate was placed in an IncuCyte S3 instrument, and scanning parameters were set. After scanning for 3 days, the total green **object** integrated intensity (GCU × µm²/well) was analyzed, and the killing efficiency was calculated.

A549 cells expressed mesothelin at a low level. As shown in **FIG.** 59, CART cells expressing anti-mesothelin scFv-M4, -M22, -M28, and-M31 effectively blocked the growth of A549 cells, indicating that these scFv-based CART cells are quite cytotoxic to tumor cells. As shown in **FIG**. 60, CART cells expressing anti-mesothelin scFv-M4, -M6, -M7, -M8, -M9, -M10, -M11, -M12, -M13, -M15, -M20, -M22, -M23, -M24, - M27, -M28, -M31, -M32, -M35, -M37, and -M38 CAR showed effective killing effect on A549 tumor cells overexpressing mesothelin (electroporated with 10 µg of mesothelin mRNA). As shown in **FIG.** 61, CART cells expressing anti-mesothelin scFv-M4, -M6, -M13, -M20, -M27, -M31, and -M37 CAR maintained a strong killing effect on A549 tumor cells with less ectopic expression of mesothelin (electroporated with 2 µg of mesothelin mRNA). CART cells expressing anti-mesothelin scFv-M7, - M8, -M9, -M10, -M11, -M12, -M15, -M23, -M24, -M32, -M35, and -M38 selectively showed high cytotoxicity only for tumor cells with high expression of mesothelin, not for tumor cells with low expression of mesothelin, and thus exhibited excellent safety because mesothelin is also expressed in some normal tissues.

**FIG.** 62 shows killing curves of different mRNA-based anti-mesothelin CART cells, with A549-GFP tumor cells electroporated with 0 µg (upper **graph**), 2 µg (middle **graph**), or 10 µg (lower **graph**) of mesothelin mRNA as target cells (E/T ratio = 10:1). As shown, the anti-mesothelin scFv-M12, -M24 and -M32 CART cells had a mild killing effect on A549 tumor cells with low expression of mesothelin (2 µg group), but had a strong killing effect on A549 tumor cells with high expression of mesothelin (10 µg group). The results show that these CART cells will specifically target tumor cells with high expression of mesothelin and do not kill normal tissues with low expression of mesothelin.

**FIG.** 63 shows FACS staining of OVCAR3 (human ovarian cancer cells), H226 (human lung cancer (malignant epithelial tumor) cells), ASPC1 (human pancreatic tumor cells), A549 (human lung cancer (carcinoma) cells), and HCC70 (human breast cancer cells) with the isotype control and anti-mesothelin mAb. As shown, certain cancer cells, including OVCAR3, H226, and ASPC1, expressed mesothelin at a high level; A549 expressed mesothelin at a low level and HCC70 did not express mesothelin.

### Example 29: Specific Activation of CART Cells by Mesothelin-Expressing Cancer Cells

CD107a is an early activation marker for T cells. The activation of mesothelin CARTs by tumor cells expressing mesothelin was determined by CD107a staining, and the procedures are as follows: The PE-CD107a mAb was added to a 96-well plate at 20 µL/ well; the tumor cells were diluted to 2 × 10e6/mL and seeded to the 96-well round plate (100 µL/well); the CAR-T cells obtained in Example 28 above were diluted to 1×10e6/mL and seeded to the 96-well round plate (100 µL/well); the plate was centrifuged at 500 rpm for 5 min to allow the cells to adhere, and the cells were then cultured at 37 °C for 1 h; GolgiStop was subjected to 1500-fold dilution with a medium and added to each well (20 µL/well); the cells were cultured at 37 °C for another 2.5 h, stained with the anti-CD3-APC and anti-CD8-FITC antibody at 37 °C for 30 min, and then washed, followed by analyzing by flow cytometry.

**FIG.** 64 shows the CD107a staining of anti-mesothelin M12 and M32 CAR-T cells in the assay of coculturing with OVCAR3, H226, ASPC1, A549, and HCC70 and killing. These data show that anti-mesothelin M12 and M32 CART cells were specifically activated by OVCAR3, H226, and ASPC1 (tumor cells with high mesothelin expression levels), but not by A549 and HCC70 (tumor cell lines with low or no mesothelin expression).

### The following examples 30-33 relate to antibodies, CARs and CAR-Ts targeting BCMA

### Example 30: Preparation of Anti-BCMA Antibodies

Anti-BCMA antibodies were prepared using a fully human antibody phage display library according to the following procedures:
(1) Expression and purification of phage display library: The log-phase TG1 library culture was infected with freshly thawed M13K07 helper phage at a multiple infection rate of 20:1 (phage to cell ratio), followed by inducing overnight with IPTG; the phage library was purified by PEG/NaCl precipitation and then the phage titer was determined. Phages were stored at 4 °C and later subjected to scFv selection.
(2) Selection of BCMA-specific scFv-phages: In the first round of selection, a Maxisorp plate was coated with 20 µg/mL BCMA-6His protein dissolved in 1× PBS and incubated overnight at 4 °C. (In the subsequent rounds of selection, lower protein concentrations were used for more stringent selections, including 2 µg/mL in the second round of bioscreening and 0.5 µg/mL in the third round of bioscreening.) The plate was then washed three times with PBS and a blocking buffer (1× PBS containing 5% milk and 1% BSA) was added to each well. After incubation for 2 h at room temperature, the blocking buffer was discarded, the phage solution was added, and the plate was sealed with a preservative film and incubated for 2 h with gentle shaking. In the first round of selection, the plate was then washed 10 times with PBST. (In the subsequent rounds, the stringency of the washing was increased by adding more washing cycles: 20 washing cycles in the second round and 30 washing cycles in the third round). The antigen-bound scFv-phages were then eluted using 1 mL of acid elution buffer (pH 2.2), neutralized, and seeded into 15 mL of the log-phase TG1 culture (OD600 = 0.5). The mixture was left to stand at 37 °C for 30 min and then cultured for 30 min with shaking, seeded on a 2xYT-GA agar plate, and incubated overnight at 30 °C for subsequent selection.
(3) mpELISA screening: After three rounds of screening, 480 phage-infected colony clones were selected for monoclonal phage ELISA (mpELISA) screening. Phage supernatant was generated from a single colony clone and tested for its binding to the BCMA-Fc protein. The supernatant was incubated with a pre-sealed Maxisorp plate coated with 2 µg/mL BCMA-6His protein. After three times of washing, the HRP-conjugated anti-M13 antibody was added at 100 µL/well. The HRP-conjugated anti-M13 antibody was diluted in a blocking buffer (1× PBS containing 5% milk and 1% BSA) at 1:5000. The mixture was incubated at room temperature for 60 min. After the plate was washed 5 times with PBST, a TMB substrate solution was added at 100 µL/well, and the mixture was incubated for 10-30 min until blue color appeared. The reaction was stopped by adding a stop solution (2N H2SO4) at 50 µL/well.

The absorbance at 450 nm was read in a microplate reader. Table 17 below provides ELISA 96-well plate readings for three representative anti-human BCMA-Fc monoclonal phages. Clones with a gray highlight were positive clones. In the phage ELISA assay, a total of 36 positive clones were selected, and scFv fragments were amplified by PCR and sequenced.

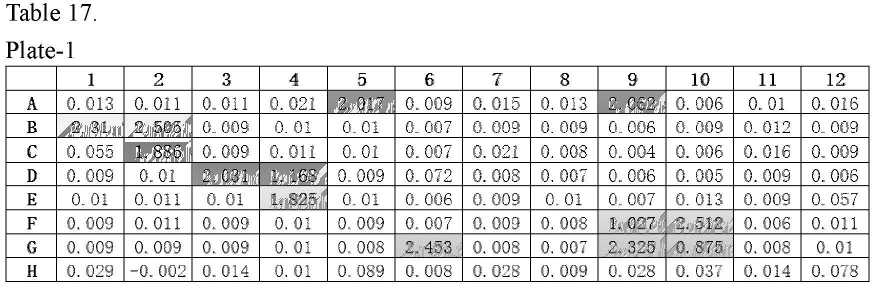

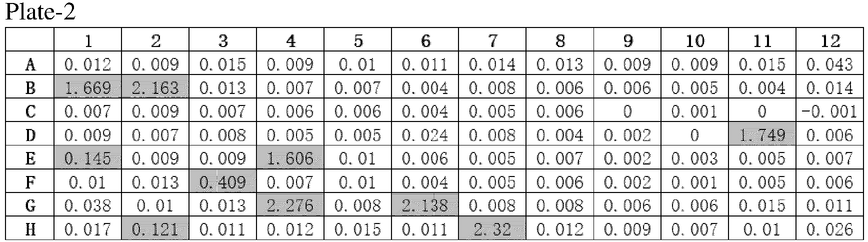

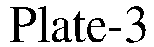

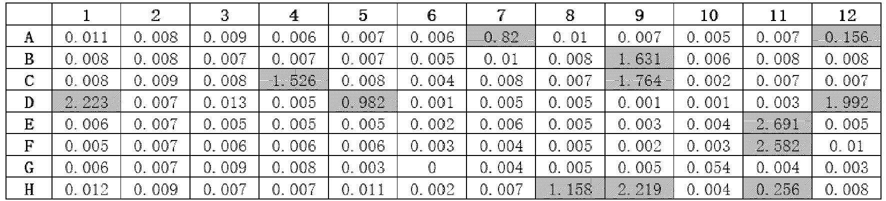

(4) Cloning and sequence analysis: Positive clones were selected according to the ELISA results and used as PCR cloning templates for scFv sequences (the sequence of the forward primer is set forth in SEQ ID NO: 659: tgcagctggcacgacaggtttc, and the sequence of the reverse primer is set forth in SEQ ID NO: 660: cgtcagactgtagcacgtt). The PCR products were then sequenced by sanger sequencing (the sequence of the forward primer is set forth in SEQ ID NO: 661: aacaattgaattcaggagga, and the sequence of the reverse primer is set forth in SEQ ID NO: 662: cctcctaagaagcgtagtc). CDR regions of the scFvs were analyzed by the abysis website (http://abysis.org/), and for the numbering rules, the Kabat, Chothia, AbM, Contact, or IMGT system was used, see Table 18.

**Table 18. CDRs and their sequence numbers (SEQ ID NO)**

| **Antibody** | **VL CDR1** | **VL CDR2** | **VL CDR3** |
|---|---|---|---|
| **BCMA21** | RASQSVSSNFLA | GASNRAT | QHYDGSPPMYT |
| | 232 | 242 | 253 |
| **BCMA22** | TGTSSDVGGYNYVS | EVTNRPS | ISYTSSSTLDYV |
| | 233 | 243 | 254 |
| **BCMA23** | SGSSPNIGGNSVN | TNNQRPS | AAWDDSLNGVV |
| | 234 | 244 | 255 |
| **BCMA24** | SGSSSNIGSYSVN | SNNQRPS | AAWDDSLNGVV |
| | 235 | 245 | 255 |
| **BCMA27** | TGSRSNVGSYNDVS | DVDKRPA | SSYGGTYSLFV |
| | 236 | 246 | 256 |
| **BCMA28** | SGSSSNIGQNAVN | YNDLVSS | ATWDDSLNGVV |
| | 237 | 247 | 257 |
| **BCMA30** | RASQGISSYLA | AASTLQS | QQLNSYPPWT |
| | 238 | 248 | 258 |
| **BCMA31** | TGTSSDVGTYNYVS | DVNQRPS | SSYGGSNNLV |
| | 239 | 249 | 259 |
| **BCMA32** | TGTSSDVGGYNYVS | DVSKRPS | SSYTSSSTVV |
| | 233 | 250 | 260 |
| **BCMA33** | TGTSSDVGGYNYVS | EVSKRPS | SSYAGSNNFVV |
| | 233 | 251 | 261 |
| **BCMA34** | TGNSNNVGNQGAA | RNNNRPS | SAWDSSLSARV |
| | 240 | 252 | 262 |
| **BCMA35** | SGSSSNIGSNTVN | SNNQRPS | AAWDDSLNGGV |
| | 241 | 245 | 263 |

| **Antibody** | **VH CDR1** | **VH CDR2** | **VH CDR3** |
|---|---|---|---|
| **BCMA21** | SYAMS | AISGSGGSTY YADSVKG | ITAMVTHNFYGM DV |
| | 264 | 275 | 287 |
| **BCMA22** | SSNWWS | EIYHSGSTNY NPSLKS | VGSGYSYGYGDR VLDY |
| | 265 | 276 | 288 |
| **BCMA23** | SYWIG | IIYPGDSDTRY SPSFQG | RGGALDY |
| | 266 | 277 | 289 |
| **BCMA24** | SYAIS | GIIPIFGTANY AQKFQG | GSFYSSVNV |
| | 267 | 278 | 290 |
| **BCMA27** | SYGMH | VISYDGSNKY YADSVKG | EEFYGDSSYGMD V |
| | 268 | 279 | 291 |
| **BCMA28** | SNSAAWN | RTYYRSKWY NDYAVSVKS | DYYYGMDV |
| | 269 | 280 | 292 |
| **BCMA30** | SYWIG | IIYPGDSDTRY SPPFQG | LDASGSQRGGMD V |
| | 266 | 281 | 293 |
| **BCMA31** | SYWMS | NIKPDGSDKY YVDSVKG | GATTYGS |
| | 270 | 282 | 294 |
| **BCMA32** | RYAIS | GIIPFFGTSDY AQKFQG | RVATTGTGFYYAM DV |
| | 271 | 283 | 295 |
| **BCMA33** | DYAMH | GISWNSGSIG YADSVKG | VGASAALGAFDI |
| | 272 | 284 | 296 |
| **BCMA34** | NPFVH | IINLSGGDTLY AQKFQG | DQAGFGDSAY |
| | 273 | 285 | 297 |
| **BCMA35** | SYDIS | WINAYNGNA NYAQKLQG | EYYYFWSNRAFY YGMDV |
| | 274 | 286 | 298 |

(5) Screening for functional anti-BCMA scFv in T cells: the anti-BCMA scFv was constructed into a bicistronic lentiviral CAR expression vector containing an IRES-truncated EGFR (tEGFR) expression cassette. The lentivirus was produced by transient transfection of 293T cells, followed by purification by ultracentrifugation and concentration. T cells were transduced with the CAR lentivirus to generate CAR-T cells, which were then cultured for 10 days. 10 days after lentivirus transduction, CAR-T cells were collected and stained with 5 µg/mL CD19-Fc protein (Ctrl Fc protein) or BCMA-Fc recombinant protein for 30 min at 4 °C. After washing, the CAR-T cells were stained with the anti-human IgG Fc and anti-EGFR mAb. The samples were analyzed using a flow cytometer. As shown in **FIGs.** 65A and 65B, T cells expressing CARs (containing the following anti-BCMA scFvs) showed binding to BCMA-Fc **(****FIG.** 65B) and were selected for further study: BCMA21, BCMA22, BCMA23, BCMA24, BCMA27, BCMA28, BCMA30, BCMA31, BCMA32, BCMA33, BCMA34, and BCMA35.

### Example 31: Preparation and Characterization of BCMA- CARTs

12 different anti-BCMA CARs were constructed using the anti-BCMA scFvs described above. Parallel testing was performed on 3 other CART products, including NBC10 (Novartis AG and University of Pennsylvania, BMCA10.BBz), FHVH33 (National Institutes of Health, US), and B38M (Legend Biotech). All CARs tested had a 41BBz costimulatory domain.

**Table 19. BCMA CARTs, CAR%, and expression levels**

| **CART** | **scFv** | **CAR %** | **MFI** |
|---|---|---|---|
| 1 | NBC10 | 22% | 1.2E+05 |
| 2 | FHVH33 | 84% | 3.2E+05 |
| 3 | BCMA21 | 31% | 1.6E+05 |
| 4 | BCMA22 | 12% | 7.5E+04 |
| 5 | BCMA23 | 18% | 2.2E+05 |
| 6 | BCMA24 | 23% | 9.4E+04 |
| 7 | BCMA27 | 27% | 1.7E+05 |
| 8 | BCMA28 | 29% | 6.5E+04 |
| 9 | BCMA30 | 25% | 4.6E+04 |
| 10 | BCMA31 | 37% | 2.5E+05 |
| 11 | BCMA32 | 19% | 5.6E+04 |
| 12 | BCMA33 | 27% | 2.6E+05 |
| 13 | BCMA34 | 16% | 9.5E+04 |
| 14 | BCMA35 | 18% | 1.1E+05 |
| 15 | B38M | 51% | 5.8E+05 |
| 16 | NTD | | |

T cells were transduced by lentiviral vectors to express different BCMA CARs. Table 19 above shows CART cells used in the studies disclosed herein, percentages of cells expressing CARs, and their respective expression levels. **FIGs.** 66A and 66B show the frequencies and expression levels ("MFI" is mean fluorescence intensity) of CAR+T cells, respectively. Of the 12 scFvs produced in the present invention, the expression level of BCMA31 (#10; SEQ ID NO: 342) and BCMA33 (#12; SEQ ID NO: 344) was higher than that of the other scFvs. **FIG.** 67 shows the comparable frequencies of CAR+CD8 cells in the CARTs tested. **FIG.** 68 shows the phenotype of CART cells. The frequency of naive T cell populations (CD45RO-; CCR7+) was higher in BCMA27 (#7), BCMA31 (#10), and BCMA33 (#12) T cells than in other samples, indicating that these T cells were less differentiated.

### Example 32: Expression of BCMA in Tumor Cells

As shown in **FIGs.** 69A and 69B, BCMA expression in different tumor cell lines was detected by FACS staining **(****FIG.** 69A) and RT-PCR (**FIG.** 69B). BCMA expression in Jeko-1 (low level), Raji (medium level) and RPMI-8226 cells (high level) was detected by FACS staining. Although BCMA expression in Nalm6 was not detected by FACS, RT-PCR analysis showed that BCMA was expressed in Nalm6, although at a low level.

### Example 33: Cytotoxicity of BCMA CARTs for Tumor Cells

The CART cells obtained in Example 31 above were co-cultured with Jeko-1 cells and RPMI-8226 tumor cells. The production of INF-γ and IL-2 was detected. As shown in **FIGs.** 70A (INF-γ) and 70B (IL-2), of the 12 CARTs produced by the present invention, BCMA23 (#5; SEQ ID NO: 337), BCMA24 (#6; SEQ ID NO: 338), BCMA27 (#7; SEQ ID NO: 339), BCMA31 (#10; SEQ ID NO: 342), and BCMA33 (#12; SEQ ID NO: 344) were able to produce more cytokines than other CART cells (including NBC10 and B38M CAR T cells).

The cytolytic activities of these CART cells against Jeko-1 (**FIGs.** 71A-71D) and RPMI-8226 cells (**FIGs.** 72A-72E) were also detected. BCMA23 (#5), BCMA24 (#6), BCMA31 (#10), and BCMA33 (#12) CART cells showed different degrees of cytotoxicity for Jeko-1 cells, where BCMA31 (#10) showed the highest cytotoxicity and was able to effectively eliminate Jeko-1. In addition, BCMA21 (#3; SEQ ID NO: 335), BCMA22 (#4; SEQ ID NO: 336), BCMA23 (#5; SEQ ID NO: 337), BCMA24 (#6; SEQ ID NO: 338), BCMA27 (#7; SEQ ID NO: 339), BCMA31 (#10; SEQ ID NO: 342), BCMA33 (#12; SEQ ID NO: 344), BCMA34 (#13; SEQ ID NO: 345), and BCMA35 (#14; SEQ ID NO: 346) showed different levels of cytotoxicity for RPMI-8226 cells, where BCMA21 (#3), BCMA23 (#5), BCMA24 (#6), BCMA27 (#7), BCMA31 (#10), and BCMA33 (#12) effectively eliminated RPMI-8226 cells.

### The following examples 34-37 relate to antibodies, CARs and CAR-Ts targeting CD123

### Example 34: Preparation of Human Anti-CD123 Antibodies

The following procedures were taken to prepare fully human anti-CD123 antibodies:
1) Expression and purification of phage display library: The log-phase TG1 library culture was infected with freshly thawed M13K07 helper phage at a multiple infection rate of 20:1 (phage to cell ratio), followed by inducing overnight with IPTG; the phage library was purified by PEG/NaCl precipitation and then the phage titer was determined.
2) Selection of CD123-specific scFv-phages: In the first round of selection, a Maxisorp plate was coated with 20 µg/mL CD123-6His protein and incubated overnight at 4 °C. The plate was then washed with PBS and blocked with 1× PBS containing 5% milk and 1% BSA, followed by incubation in the phage solution for 2 h and washing 10 times with PBST. The antigen-bound scFv phages were then eluted using an acid elution buffer (pH 2.2), neutralized, and seeded into 15 mL of the log-phase TG1 culture (OD600 = 0.5). The mixture was left to stand at 37 °C for 30 min and then cultured for 30 min with shaking, seeded on a 2xYT-GA agar plate, and incubated overnight at 30 °C for subsequent selection. In the subsequent rounds of selection, more stringent selection conditions were used, including decreasing the concentration of the coating protein (2 µg/mL for the second round and 0.5 µg/mL for the third round) and increasing the washing cycle (20 washing cycles for the second round and 30 washing cycles for the third round).
3) mpELISA screening. After three rounds of screening, 292 positive colonies were selected for monoclonal phage ELISA (mpELISA) screening. Phage supernatant generated from a single colony clone was incubated with a pre-blocked Maxisorp plate coated with 2 µg/mL CD123-6His protein. After three times of washing, the HRP-conjugated anti-M13 antibody was added at 100 µL/well. The HRP-conjugated anti-M13 antibody was diluted in a blocking buffer (5% milk +1% BSA, 1× PBS) at a ratio of 1:5000. The mixture was incubated at room temperature for 60 min. After the plate was washed 5 times with PBST, a TMB substrate solution was added at 100 µL/well, and the mixture was incubated for 10-30 min until blue color appeared. A stop solution (2 N H₂SO₄) was added at 50 µL/well to terminate the reaction. The absorbance at 450 nm was read in a microplate reader. FIG. 73 shows the resulting absorbance readings from the mpELISA screening. As shown, some positive colonies (absorbance at 450 nm ≥ 0.5) were identified to have produced anti-CD123 antibodies capable of binding to CD123-6His protein (FIG. 73).
(4) Cloning and sequence analysis: Positive clones were selected according to the ELISA results and used as PCR cloning templates for scFv sequences (the sequence of the forward primer is set forth in SEQ ID NO: 659: tgcagctggcacgacaggtttc, and the sequence of the reverse primer is set forth in SEQ ID NO: 660: cgtcagactgtagcacgtt). The PCR products were then sequenced by sanger sequencing (the sequence of the forward primer is set forth in SEQ ID NO: 661: aacaattgaattcaggagga, and the sequence of the reverse primer is set forth in SEQ ID NO: 662: cctcctaagaagcgtagtc). CDR regions of the scFvs were analyzed by the abysis website (http://abysis.org/), and for the numbering rules, the Kabat, Chothia, AbM, Contact, or IMGT system was used, see Table 20.

**Table 20. CDRs and their sequence numbers (SEQ ID NO)**

| **Antibody** | **VL CDR1** | **VL CDR2** | **VL CDR3** |
|---|---|---|---|
| **C1** | TGTSSDIGAYNYVS | DVSHRPS | SSDTNNNTLV |
| | 349 | 379 | 407 |
| **C2** | SGSSSNIGNNYVS | DNNKRPS | VTWDTSLSAGV |
| | 363 | 391 | 421 |
| **C3** | RASQGIGTYLA | AASNLQS | QQYKGYPLT |
| | 353 | 383 | 412 |
| **C4** | SGSSSNIGSNYVN | RNNQRPS | AAWDDSLSGHGV |
| | 364 | 378 | 422 |
| **C5** | TRSSGSIAGSYVQ | QDNQRPS | QSYDSNNQV |
| | 369 | 396 | 428 |
| **C6** | SGADLGDQYVS | EDDKRPS | QAWDGNAAI |
| | 370 | 397 | 429 |
| **C7** | RASQSVSSSYLA | GASSRAT | QQYGSSPLT |
| | 354 | 384 | 413 |
| **C8** | TGTSTDIGGYDFVS | DVSSRPS | SSYAGSGTWM |
| | 350 | 380 | 408 |
| **C9** | SGSNSNIENNYVY | QNNQRPS | SAWDDSLSAWV |
| | 371 | 398 | 430 |
| **C10** | GGNNIGSKSVH | DDSDRPS | QVWDSSSDHWV |
| | 372 | 399 | 431 |
| **C11** | SGSSSNIGSNYVY | RNNQRPS | AAWDDSLSGYV |
| | 348 | 378 | 406 |
| **C12** | SGSSSNIGNNYVS | DDNKRPS | SAWDDSLSGYV |
| | 363 | 392 | 423 |
| **C13** | RSSQGLVFSDGVTYL N | KVSNRDS | MQGTHWPHT |
| | 373 | 400 | 432 |
| **C14** | QASQDITNYLN | DASNLET | QQYDDLPLT |
| | 355 | 385 | 414 |
| **C15** | RASQSISSWLA | KTSALED | LHYGSYT |
| | 356 | 386 | 415 |
| **C16** | SGSSSNIEGNTVS | SNSYRAS | SAWDDSLNGPV |
| | 368 | 395 | 427 |
| **C17** | TGTSSDVGTYDYVS | DVTYRPS | QQYNNWPPRYT |
| | 351 | 381 | 409 |
| **C18** | TGTSSDVGGYNYVS | DVSNRPS | SSYTSSSTLFV |
| | 347 | 376 | 410 |
| **C19** | RASQDIRHDLG | ATSTLQS | LQDGDYPLT |
| | 357 | 387 | 416 |
| **C20** | TGTSSDVGGYNYVS | EVSERPS | SSYAGSNILV |
| | 347 | 377 | 405 |
| **C21** | SGSSSNVGGNPVY | GHNQRPP | AAWDDSLSGWV |
| | 365 | 393 | 424 |
| **C22** | GANNIETKSVH | YDSDRPS | QVWDSTSDHVV |
| | 374 | 401 | 433 |
| **C23** | QASQDISNYLN | DASNLET | QQYGNLPLT |
| | 358 | 385 | 417 |
| **C24** | RASQSISSYLN | AASSLQS | LQYKTYPYT |
| | 359 | 388 | 418 |
| **C25** | TGTSSDVGGYNYVS | DVSNRPS | SSYTSSSTWV |
| | 347 | 376 | 403 |
| **C26** | SGSRSNIGSNTVS | LNNQRPS | ATWDDDLNGV |
| | 366 | 394 | 425 |
| **C27** | RASQRVSATYLA | GGSRRAT | QQYGSSPLT |
| | 360 | 389 | 413 |
| **C28** | TGTSSDVGGYNYVS | DVSNRPS | SSYTSSSNWV |
| | 347 | 376 | 404 |
| **C29** | SGSSSNIGSNYVY | RNNQRPS | AAWDDSLSGYV |
| | 348 | 378 | 406 |
| **C30** | QASQNINNYLN | DASNLEA | QQNDNLPLT |
| | 361 | 390 | 419 |
| **C31** | SGGNSNIGDNSVS | NNNQRPL | SSWDDSLEGAQ |
| | 375 | 402 | 434 |
| **C32** | SGSSSNIEGNTVS | SNSYRAS | SAWDDSLNGPV |
| | 368 | 395 | 427 |
| **C33** | RASQDISSWIA | AASSLQS | QQGSTFPLA |
| | 362 | 388 | 420 |
| **C34** | TGTRSDVGAYNYVS | DVSKRPS | SSYAGSGKYV |
| | 352 | 382 | 411 |
| **C35** | SGSSPNIGNNYVS | DNNKRPS | GTWDSRLNIWV |
| | 367 | 391 | 426 |

| **Antibody** | **VH CDR1** | **VH CDR2** | **VH CDR3** |
|---|---|---|---|
| **C1** | SYGMH | VIWYDGSNKYY ADSVKG | DRDGFFLPMYYF DY |
| | 437 | 458 | 482 |
| **C2** | NAWMS | RIKSKTDGGTTD YAAPVKG | DPHGYYYGMDV |
| | 435 | 468 | 495 |
| **C3** | SYAIS | GIIPIFGTANYAQ KFQG | DSGWFGPRYYFD Y |
| | 441 | 462 | 486 |
| **C4** | RYYMN | AIDPSDGDTTYG QKFQG | DWELTNSYGLDV |
| | 446 | 469 | 496 |
| **C5** | DYGMS | GINWNGGRTGY ADSVKG | ARGSGSYFGYMD V |
| | 445 | 475 | 503 |
| **C6** | RYSMN | YISSSSSTIYYAD SVKG | DGYGGNSDYYG MDV |
| | 452 | 474 | 504 |
| **C7** | SYGIS | WISAYNGNTNY AQKLQG | DPYDDYGGNSRD EEDAFDI |
| | 442 | 463 | 487 |
| **C8** | NAWMS | RIKSKTDGGTTD SAAPVKG | YYFYSSGDMTDY |
| | 435 | 456 | 480 |
| **C9** | DYAMN | FIRSKASGGTTD YAASVKG | ALRGYSYGLFDY |
| | 453 | 476 | 505 |
| **C10** | SYAIS | GIIPIFGTANYAQ KFQG | EKWGYYGDYYY GMDV |
| | 441 | 462 | 506 |
| **C11** | DYAMS | FIRSKAYGGTTE YAASVKG | DIGYYGSGSYSPF DY |
| | 436 | 457 | 481 |
| **C12** | SYNIN | SISSSSSYRYYAD SVKG | DKTGGYGTAFDY |
| | 447 | 470 | 497 |
| **C13** | SYSMN | FISHDASKKFYA DSVKG | DRNWGFSDF |
| | 451 | 477 | 507 |
| **C14** | SSSHYWG | SIYYSGSTYYNP SLKS | RVWVWPTYGMD V |
| | 443 | 464 | 488 |
| **C15** | DYYIS | YISGSSTYTDFA DSVKG | DRRGRFDP |
| | 444 | 465 | 489 |
| **C16** | SYSMN | YISSSSSTIYYAD SVKG | DIYGDWDYYYYG MDV |
| | 451 | 474 | 502 |
| **C17** | SYYMH | LINPTNGRTTYA QKFQG | GSPPHIFDPDFDY |
| | 438 | 459 | 483 |
| **C18** | SGDYYWS | YIHSTGTTYYNP SLKS | GGVGTIFNY |
| | 439 | 460 | 484 |
| **C19** | SYGMH | VISYDGSNKYYA DSVKG | DPSLIQYYYYGM DV |
| | 437 | 466 | 490 |
| **C20** | NAWMS | RIKSKTDGGTTD SAAPVKG | YYFYSSGDMTDY |
| | 435 | 456 | 480 |
| **C21** | DYAMH | GISWNSGSIGYA DSVKG | GRYVDIVATINDA FDI |
| | 448 | 471 | 498 |
| **C22** | SYWIG | IINPSGGSTSYAQ KFQG | VVTPYYPLTN |
| | 454 | 478 | 508 |
| **C23** | SYGIS | WISAYNGNTNY AQKLQG | DRIAAAEVYYYY GMDV |
| | 442 | 463 | 491 |
| **C24** | SYAIS | GIIPIFGTANYAQ KFQG | DSPSGSTNYYYY YMDV |
| | 441 | 462 | 492 |
| **C25** | NAWMS | RIKSKTDGGTTD SAAPVKG | YYFYSSGDMTDY |
| | 435 | 456 | 480 |
| **C26** | NFEMN | YISATTSATSYAD SVKG | VRDGYNFGYYYY YMDV |
| | 449 | 472 | 499 |
| **C27** | SYGIS | WISAYNGNTNY AQKLQG | DPYDDYGGNSRD EEDAFDI |
| | 442 | 463 | 487 |
| **C28** | NAWMS | RIKSKTDGGTTD SAAPVKG | YYFYSSGDMTDY |
| | 435 | 456 | 480 |
| **C29** | DYAMS | FIRSKAYGGTTE YAASVKG | DIGYYGSGSYSPF DY |
| | 436 | 457 | 481 |
| **C30** | DYGMS | GINWNGGSTGY ADSVKG | DRAPDYGDYLQV GTTDY |
| | 445 | 467 | 493 |
| **C31** | DYYMS | YISSSGSTIYYAD SVKG | GHQYYDFWSGYS NWFDP |
| | 455 | 479 | 509 |
| **C32** | SYAMN | DISSSSSYIYYAD SVKG | DLYSGYDYGYYY GMDV |
| | 450 | 473 | 501 |
| **C33** | SYAIS | GIIPIFGTANYAQ KFQG | GLDNWNDVGWF DP |
| | 441 | 462 | 494 |
| **C34** | DTWMS | RIKSKTDGGTTN YAAPVKG | GDYPLFAY |
| | 440 | 461 | 485 |
| **C35** | NAWMS | RIKSKTDGGTTD YAAPVKG | EAAGFYYYYYG MDV |
| | 435 | 468 | 500 |

### Example 35: Preparation of CD123 CARs

Vectors were constructed for the production of mRNAs of anti-CD 123 CARs. First, the scFv sequences obtained in Example 34 above and the CAR fragment (from the hinge domain to the CD3-zeta domain) were amplified by PCR and cloned into the pDA vector (Xba1/Sal1). **FIG.** 74 provides a schematic **diagram** of a pDA-CAR vector for producing CAR mRNA. Then, the CD 123 CAR mRNA was prepared by *in vitro* transcription (IVT). The pDA-CAR plasmid was linearized by digestion with Spe1 and purified by the PCR Cleanup kit. After measuring the DNA concentration with nanodrop and checking by performing the agarose DNA gel method, IVT was performed according to the manufacturer's instructions (Thermofisher, Cat No: AM13455). The concentration of the RNA product was determined by nanodrop and checked by performing the PAGE gel method.

### Example 36: Preparation and Characterization of CD123 CARTs

The CD123 CAR mRNAs obtained in Example 35 above were each introduced into T cells by electroporation according to the following procedures: T cells were collected, washed with the Opti-MEM medium, and resuspended at 1 × 10e⁷/mL with the Opti-MEM medium; 10 µg of RNA and 100 µL of T cells were aliquoted, mixed well, and electroporated under the following parameters (BTX machine): 500 V, 0.7 ms; the cells were then transferred to a pre-warmed medium and cultured at 37 °C.

The binding of CD123 CART cells to the CD123-Fc recombinant protein was detected by FACS staining. As shown in **FIG.** 75, T cells expressing CARs with anti-CD123 scFv-C1, -C2, -C3, -C4, -C5, -C6, -C7, -C9, -C10, -C11, -C13, -C14, -C15, -C16, - C17, -C18, -C19, -C21, -C23, -C24, -C25, -C26, -C27, -C28, -C29, -C30, -C32, -C33, -C34, and -C35 were able to bind to the CD123-Fc recombinant protein. Empty vector (Mock) was a control T cell without CAR molecule.

A549 tumor cells were electroporated with different amounts of CD123 mRNA. The electroporation procedure was the same as described above, and only the parameters were changed, with the parameters 300 V, 0.5 ms used. CD123 expression in A549 tumor cells was detected by FACS staining of isotype or anti-CD123 antibodies and A549 cells electroporated with different amounts of CD123 mRNA. As shown in FIG. 76, A549 cells weakly expressed endogenous CD123, and the ectopic expression level of CD123 correlated with the amount of CD123 mRNA electroporated into A549 cells. The cytotoxicity of CD123 CART cells against tumor cells was detected in an *in vitro* cytotoxicity assay. Tumor cells expressing EGFP or EGFP-A549 cells electroporated with different amounts of tumor antigen were seeded at 3000 cells/100 µL/well into a flat-bottom 96-well plate; CART cells were diluted to a proper concentration and seeded to the 100 µL/well tumor cells according to different E/T ratios (e.g., 10:1, 3:1, or 1:1), and the co-culture plate was placed into the IncuCyte S3 machine and scanning parameters were set. After scanning for 3 days, the total green object integrated intensity (GCU × µm²/well) was analyzed to calculate the killing efficiency.

FIGs. 77 and 78 show killing curves of different mRNA-based anti-CD123 CART cells for A549-GFP tumor cells at an E/T ratio of 10:1 (FIG. 77) or 3:1 (FIG. 78). As shown, CART cells expressing anti-CD123 scFv-C2, -C3, -C4, -C6, -C9, -C11, -C13, -C14, - C15, -C16, -C17, -C19, -C21, -C23, -C24, and -C32 effectively blocked the growth of A549 cells and even eliminated A549 cells, although A549 cells expressed endogenous CD123 at a low level, indicating that these scFv-based CART cells have high cytotoxicity for tumor cells.

FIGs. 79 and 80 show killing curves of different mRNA-based anti-CD123 CART cells for A549-GFP tumor cells expressing exogenous CD123 (electroporated with 10 µg of CD123 mRNA) at an E/T ratio of 10:1 (FIG. 79) or 3:1 (FIG. 80). As shown in FIGs. 79 and 80, CART cells expressing anti-CD123 scFv-C1, -C2, -C3, -C4, -C5, -C6, -C7, -C9, -C11, -C13, -C14, -C15, -C16, -C17, -C18, -C19, -C21, -C23, -C24, -C25, -C26, -C27, -C28, -C29, -C30, -C32, -C33, -C34, and -C35 effectively blocked the growth of A549 tumor cells expressing CD123 and even reduced the number of A549 tumor cells expressing CD123, confirming their ability to kill tumor cells expressing CD123.

### Example 37: CD107a Staining of CART Cells

CD123 expression of different tumor cell lines was detected by FACS staining. FIG. 81 shows the results of FACS staining of A549, SK-OV3, Jeko-1, Molm-14, SupT-1, 293T, Nalm-6, and PC-3 cells with the PE-isotype control and PE-anti-CD123 mAb. As shown in FIG. 81, most of the tested tumor cell lines did not express CD123, and only the Molm-14 expressed a relatively high level of CD123.

CD107a is an early activation marker for T cells. The activation of CD123 CARTs by tumor cells expressing CD123 was detected by CD107a staining according to the following procedures: The PE-CD107a mAb was added to a 96-well plate at 20 µL/ well; the tumor cells were diluted to 2×10e⁶/mL and seeded to the 96-well round plate (100 µL/well); the CART cells obtained in Example 36 above were diluted to 1 × 10e⁶/mL and seeded to the 96-well round plate (100 µL/well); the plate was centrifuged at 500 rpm for 5 min to allow the cells to adhere well, and the cells were then cultured at 37 °C for 1 h; Golgi stop was subjected to 1500-fold dilution with a medium and added to each well (20 µL/well); the cells were cultured at 37 °C for another 2.5 h, stained with the anti-CD3-APC and anti-CD8-FITC antibody at 37 °C for 30 min, and then washed, followed by analyzing by a flow cytometer.

In the studies herein, the activation of CARTs (expressing anti-CD123-C5, anti-CD123-C7, and anti-CD123-C11) by tumor cells expressing CD123 was detected by CD107a staining. The cells tested included A549 electroporated with 10 µg, 0.1 µg, and 0 µg of CD123 mRNA, SK-OV3, PC-3, cord blood-derived CD34 hematopoietic stem cells (CD34+ cord), bone marrow-derived hematopoietic stem cells (CD34+ M), Molm-14, Nalm6, Jeko-1 tumor cell line, and freshly isolated patient AML tumor cells (CD123+). As shown in **FIG.** 82, CART cells expressing anti-CD123-C5, -C7, and - C11 were specifically activated by tumor cells with relatively high CD123 expression, in particular CD 123+AMI, tumor cells, but not by tumor cell lines with low CD123 expression. These results show that tumor cells expressing CD123 can activate CD123 CARTs.

The sequence numbers (SEQ ID NOs) corresponding to the sequences of antibody VHs and VLs, scFvs and CARs involved in Examples 25-37 above are shown in Table 21 below.

**Table 21.**

| Sequence NO | Sequence | Sequence NO | Sequence | Sequence NO | Sequence |
|---|---|---|---|---|---|
| 172 | M6 VL | 192 | M13 VH | 212 | M27 scFv |
| 173 | M7 VL | 193 | M15 VH | 213 | M28 scFv |
| 174 | M8 VL | 194 | M20 VH | 214 | M31 scFv |
| 175 | M10 VL | 195 | M22 VH | 215 | M32 scFv |
| 176 | M12 VL | 196 | M24 VH | 216 | M37 scFv |
| 177 | M13 VL | 197 | M27 VH | 217 | M6 CAR |
| 178 | M15 VL | 198 | M28 VH | 218 | M7 CAR |
| 179 | M20 VL | 199 | M31 VH | 219 | M8 CAR |
| 180 | M22 VL | 200 | M32 VH | 220 | M10 CAR |
| 181 | M24 VL | 201 | M37 VH | 221 | M12 CAR |
| 182 | M27 VL | 202 | M6 scFv | 222 | M13 CAR |
| 183 | M28 VL | 203 | M7 scFv | 223 | M15 CAR |
| 184 | M31 VL | 204 | M8 scFv | 224 | M20 CAR |
| 185 | M32 VL | 205 | M10 scFv | 225 | M22 CAR |
| 186 | M37 VL | 206 | M12 scFv | 226 | M24 CAR |
| 187 | M6 VH | 207 | M13 scFv | 227 | M27 CAR |
| 188 | M7 VH | 208 | M15 scFv | 228 | M28 CAR |
| 189 | M8 VH | 209 | M20 scFv | 229 | M31 CAR |
| 190 | M10 VH | 210 | M22 scFv | 230 | M32 CAR |
| 191 | M12 VH | 211 | M24 scFv | 231 | M37 CAR |
| 299 | BCMA21 VL | 315 | BCMA27 VH | 331 | BCMA32 scFv |
| 300 | BCMA22 VL | 316 | BCMA28 VH | 332 | BCMA33 scFv |
| 301 | BCMA23 VL | 317 | BCMA30 VH | 333 | BCMA34 scFv |
| 302 | BCMA24 VL | 318 | BCMA31 VH | 334 | BCMA35 scFv |
| 303 | BCMA27 VL | 319 | BCMA32 VH | 335 | BCMA21 CAR |
| 304 | BCMA28 VL | 320 | BCMA33 VH | 336 | BCMA22 CAR |
| 305 | BCMA30 VL | 321 | BCMA34 VH | 337 | BCMA23 CAR |
| 306 | BCMA31 VL | 322 | BCMA35 VH | 338 | BCMA24 CAR |
| 307 | BCMA32 VL | 323 | BCMA21 scFv | 339 | BCMA27 CAR |
| 308 | BCMA33 VL | 324 | BCMA22 scFv | 340 | BCMA28 CAR |
| 309 | BCMA34 VL | 325 | BCMA23 scFv | 341 | BCMA30 CAR |
| 310 | BCMA35 VL | 326 | BCMA24 scFv | 342 | BCMA31 CAR |
| 311 | BCMA21 VH | 327 | BCMA27 scFv | 343 | BCMA32 CAR |
| 312 | BCMA22 VH | 328 | BCMA28 scFv | 344 | BCMA33 CAR |
| 313 | BCMA23 VH | 329 | BCMA30 scFv | 345 | BCMA34 CAR |
| 314 | BCMA24 VH | 330 | BCMA31 scFv | 346 | BCMA35 CAR |
| 510 | C1 VL | 546 | C2 VH | 585 | C3 scFv |
| 511 | C2 VL | 547 | C3 VH | 586 | C4 scFv |
| 512 | C3 VL | 548 | C4 VH | 587 | C5 scFv |
| 513 | C4 VL | 549 | C5 VH | 588 | C6 scFv |
| 514 | C5 VL | 550 | C6 VH | 589 | C7 scFv |
| 515 | C6 VL | 551 | C7 VH | 590 | C8 scFv |
| 516 | C7 VL | 552 | C8 VH | 591 | C9 scFv |
| 517 | C8 VL | 553 | C9 VH | 592 | C10 scFv |
| 518 | C9 VL | 554 | C10 VH | 593 | C11 scFv |
| 519 | C10 VL | 555 | C11 VH | 594 | C12 scFv |
| 520 | C11 VL | 556 | C12 VH | 595 | C13 scFv |
| 521 | C12 VL | 557 | C13 VH | 596 | C14 scFv |
| 522 | C13 VL | 558 | C14 VH | 597 | C15 scFv |
| 523 | C14 VL | 559 | C15 VH | 598 | C16 scFv |
| 524 | C15 VL | 560 | C16 VH | 599 | C17 scFv |
| 525 | C16 VL | 561 | C17 VH | 600 | C18 scFv |
| 526 | C17 VL | 562 | C18 VH | 601 | C19 scFv |
| 527 | C18 VL | 563 | C19 VH | 602 | C20 scFv |
| 528 | C19 VL | 564 | C20 VH | 603 | C21 scFv |
| 529 | C20 VL | 565 | C21 VH | 604 | C22 scFv |
| 530 | C21 VL | 566 | C22 VH | 605 | C23 scFv |
| 531 | C22 VL | 567 | C23 VH | 606 | C24 scFv |
| 532 | C23 VL | 568 | C24 VH | 607 | C25 scFv |
| 533 | C24 VL | 569 | C25 VH | 608 | C26 scFv |
| 534 | C25 VL | 570 | C26 VH | 609 | C27 scFv |
| 535 | C26 VL | 571 | C27 VH | 610 | C28 scFv |
| 536 | C27 VL | 572 | C28 VH | 611 | C29 scFv |
| 537 | C28 VL | 573 | C29 VH | 612 | C30 scFv |
| 538 | C29 VL | 574 | C30 VH | 613 | C31 scFv |
| 539 | C30 VL | 575 | C31 VH | 614 | C32 scFv |
| 540 | C31 VL | 576 | C32 VH | 615 | C33 scFv |
| 541 | C32 VL | 577 | C33 VH | 616 | C34 scFv |
| 542 | C33 VL | 578 | C34 VH | 617 | C35 scFv |
| 543 | C34 VL | 579 | C35 VH | 580 | C5 CAR |
| 544 | C35 VL | 583 | C1 scFv | 581 | C7 CAR |
| 545 | C1 VH | 584 | C2 scFv | 582 | C11 CAR |

### Literature

1. Hillion, S., et al., The Innate Part of the Adaptive Immune System. Clin Rev Allergy Immunol, 2020. 58(2): p. 151-154.
2. Bajorath, J., R.J. Peach, and P.S. Linsley, Immunoglobulin fold characteristics of B7-1 (CD80) and B7-2 (CD86). Protein Sci, 1994. 3(11): p. 2148-50.
3. Tacke, M., et al., CD28-mediated induction of proliferation in resting T cells in vitro and in vivo without engagement of the T cell receptor: evidence for functionally distinct forms of CD28. Eur J Immunol, 1997. 27(1): p. 239-47.
4. Hansen, J.A., P.J. Martin, and R.C. Nowinski, Monoclonal antibodies identifying a novel T-Cell antigen and Ia antigens of human lymphocytes. Immunogenetics, 1980. 10(1): p. 247-260.
5. Tan, P., et al., "Superhumanized" antibodies: reduction of immunogenic potential by complementarity-determining region grafting with human germline sequences: application to an anti-CD28. J Immunol, 2002. 169(2): p. 1119-25.
6. Edner, N.M., et al., Targeting co-stimulatory molecules in autoimmune disease. Nat Rev Drug Discov, 2020. 19(12): p. 860-883.
7. Joyce, J.A. and D.T. Fearon, T cell exclusion, immune privilege, and the tumor microenvironment. Science, 2015. 348(6230): p. 74-80.
8. Terrazzano, G., et al., Interaction between natural killer and dendritic cells: the role of CD40, CD80 and major histocompatibility complex class i molecules in cytotoxicity induction and interferon-gamma production. Scand J Immunol, 2004. 59(4): p. 356-62.

### Sequences:

Heavy chain variable region of anti-CD28 antibody 9.3 (mVH)
HCDR1: DYGVH (SEQ ID NO: 1)
HCDR2: VIWAGGGTNYNSALMS (SEQ ID NO: 2)
HCDR3: DKGYSYYYSMDY (SEQ ID NO: 3)
HFR1: QVKLQQSGPGLVTPSQSLSITCTVSGFSLS (SEQ ID NO: 4)
HFR2: WVRQSPGQGLEWLG (SEQ ID NO: 5)
HFR3: RKSISKDNSKSQVFLKMNSLQADDTAVYYCAR (SEQ ID NO: 6)
HFR4: WGQGTTVTVSS (SEQ ID NO: 7)
VH: 
QVKLQQSGPGLVTPSQSLSITCTVSGFSLSDYGVHWVRQSPGQGLEWLGVIW
AGGGTNYNSALMSRKSISKDNSKSQVFLKMNSLQADDTAVYYCARDKGYSY
YYSMDYWGQGTTVTVSS (SEQ ID NO: 8)
Light chain variable region of anti-CD28 antibody 9.3 (mVL)
LCDR1: RASESVEYYVTSLMQ (SEQ ID NO: 9)
LCDR2: AASNVES (SEQ ID NO: 10)
LCDR3: QQSRKVPYT (SEQ ID NO: 11)
LFR1: DIVLTQSPASLAVSLGQRATISC (SEQ ID NO: 12)
LFR2: WYQQKPGQPPKLLIF (SEQ ID NO: 13)
LFR3: GVPARFSGSGSGTNFSLNIHPVDEDDVAMYFC (SEQ ID NO: 14)
LFR4: FGGGTKLEIK (SEQ ID NO: 15)
VL: 
DIVLTQSPASLAVSLGQRATISCRASESVEYYVTSLMQWYQQKPGQPPKLLIFA
ASNVESGVPARFSGSGSGTNFSLNIHPVDEDDVAMYFCQQSRKVPYT
 FGGGTKLEIK (SEQ ID NO: 16)
Heavy chain variable region 1 of humanized anti-CD28 antibody (huVH1)
HCDR1: DYGVH (SEQ ID NO: 1)
HCDR2: VIWAGGGTNYNSALMS (SEQ ID NO: 2)
HCDR3: DKGYSYYYSMDY (SEQ ID NO: 3)
HFR1: QVKLQESGPGLVKPSETLSITCTVSGFSLS (SEQ ID NO: 19)
HFR2: WVRQSPGKGLEWLG (SEQ ID NO: 23)
HFR3: RKTISKDNSKSQVFLKMSSLTAADTAVYYCAR (SEQ ID NO: 27)
HFR4: WGQGTTVTVSS (SEQ ID NO: 7)
VH: 
QVKLQESGPGLVKPSETLSITCTVSGFSLSDYGVHWVRQSPGKGLEWLGVIW
AGGGTNYNSALMSRKTISKDNSKSQVFLKMSSLTAADTAVYYCARDKGYSY
YYSMDYWGQGTTVTVSS (SEQ ID NO: 31)
Heavy chain variable region 2 of humanized anti-CD28 antibody (huVH2)
HCDR1: DYGVH (SEQ ID NO: 1)
HCDR2: VIWAGGGTNYNSALMS (SEQ ID NO: 2)
HCDR3: DKGYSYYYSMDY (SEQ ID NO: 3)
HFR1: QVQLQESGPGLUKPSETLSITCTVSGFSLS (SEQ ID NO: 20)
HFR2: WVRQPPGKGLEWLG (SEQ ID NO: 24)
HFR3: RKTISKDTSKNQVSLKMSSLTAADTAVYYCAR (SEQ ID NO: 28)
HFR4: WGQGTTVTVSS (SEQ ID NO: 7)
VH: 
QVQLQESGPGLVKPSETLSITCTVSGFSLSDYGVHWVRQPPGKGLEWLGVIW
AGGGTNYNSALMSRKTISKDTSKNQVSLKMSSLTAADTAVYYCARDKGYSY
YYSMDYWGQGTTVTVSS (SEQ ID NO: 32)
Heavy chain variable region 3 of humanized anti-CD28 antibody (huVH3)
HCDR1: DYGVH (SEQ ID NO: 1)
HCDR2: VIWAGGGTNYNSALKS (SEQ ID NO: 17)
HCDR3: DKGYSYYYSMDY (SEQ ID NO: 3)
HFR1: QVQLQESGPGLUKPSETLSLTCTVSGFSLS (SEQ ID NO: 21)
HFR2: WVRQSPGKGLEWIG (SEQ ID NO: 25)
HFR3: RKTISKDNSKSQVSLKLSSVTAADTAVYYCAR (SEQ ID NO: 29)
HFR4: WGQGTTVTVSS (SEQ ID NO: 7)
VH: 
QVQLQESGPGLVKPSETLSLTCTVSGFSLSDYGVHWVRQSPGKGLEWIGVIW
AGGGTNYNSALKSRKTISKDNSKSQVSLKLSSVTAADTAVYYCARDKGYSYY
YSMDYWGQGTTVTVSS (SEQ ID NO: 33)
Heavy chain variable region 4 of humanized anti-CD28 antibody (huVH4)
HCDR1: DYGVH (SEQ ID NO: 1)
HCDR2: VIWAGGGTNYNSALKS (SEQ ID NO: 17)
HCDR3: DKGYSYYYSMDY (SEQ ID NO: 3)
HFR1: QVQLQESGPGLVKPSETLSLTCTVSGFSLS (SEQ ID NO: 21)
HFR2: WIRQPPGKGLEWIG (SEQ ID NO: 26)
HFR3: RVTISVDTSKNQVSLKLSSVTAADTAVYYCAR (SEQ ID NO: 30)
HFR4: WGQGTTVTVSS (SEQ ID NO: 7)
VH: 
QVQLQESGPGLVKPSETLSLTCTVSGFSLSDYGVHWIRQPPGKGLEWIGVIWA
GGGTNYNSALKSRVTISVDTSKNQVSLKLSSVTAADTAVYYCARDKGYSYYY
SMDYWGQGTTVTVSS (SEQ ID NO: 34)
Heavy chain variable region 5 of humanized anti-CD28 antibody (huVH5)
HCDR1: DYGVH (SEQ ID NO: 1)
HCDR2: VIWAGGGTNYNPSLKS (SEQ ID NO: 18)
HCDR3: DKGYSYYYSMDY (SEQ ID NO: 3)
HFR1: QVQLQESGPGLVKPSETLSLTCTVSGFSIS (SEQ ID NO: 22)
HFR2: WIRQPPGKGLEWIG (SEQ ID NO: 26)
HFR3: RVTISVDTSKNQVSLKLSSVTAADTAVYYCAR (SEQ ID NO: 30)
HFR4: WGQGTTVTVSS (SEQ ID NO: 7)
VH: 
QVQLQESGPGLVKPSETLSLTCTVSGFSISDYGVHWIRQPPGKGLEWIGVIWA
GGGTNYNPSLKSRVTISVDTSKNQVSLKLSSVTAADTAVYYCARDKGYSYYY
SMDYWGQGTTVTVSS (SEQ ID NO: 35)
Light chain variable region 1 of humanized anti-CD28 antibody (huVL1)
LCDR1: RASESVEYYVTSLMQ (SEQ ID NO: 9)
LCDR2: AASNVES (SEQ ID NO: 10)
LCDR3: QQSRKVPYT (SEQ ID NO: 11)
LFR1: DIQLTQSPSSLSVSVGDRVTISC (SEQ ID NO: 36)
LFR2: WYQQKPGQAPKLLIF (SEQ ID NO: 38)
LFR3: GVPSRFSGSGSGTNFTLTISSVQEEDFAMYFC (SEQ ID NO: 40)
LFR4: FGGGTKLEIK (SEQ ID NO: 15)
VL: 
DIQLTQSPSSLSVSVGDRVTISCRASESVEYYVTSLMQWYQQKPGQAPKLLIF
AASNVESGVPSRFSGSGSGTNFTLTISSVQEEDFAMYFCQQSRKVPYTFGGGT
KLEIK (SEQ ID NO: 43)
Light chain variable region 2 of humanized anti-CD28 antibody (huVL2)
LCDR1: RASESVEYYVTSLMQ (SEQ ID NO: 9)
LCDR2: AASNVES (SEQ ID NO: 10)
LCDR3: QQSRKVPYT (SEQ ID NO: 11)
LFR1: DIQLTQSPSSLSASVGDRVTITC (SEQ ID NO: 37)
LFR2: WYQQKPGKAPKLLIF (SEQ ID NO: 39)
LFR3: GVPSRFSGSGSGTNFTLTISSVQPEDFATYFC (SEQ ID NO: 41)
LFR4: FGGGTKLEIK (SEQ ID NO: 15)
VL: 
DIQLTQSPSSLSASVGDRVTITCRASESVEYYVTSLMQWYQQKPGKAPKLLIF
AASNVESGVPSRFSGSGSGTNFTLTISSVQPEDFATYFCQQSRKVPYTFGGGTK
LEIK (SEQ ID NO: 44)
Light chain variable region 3 of humanized anti-CD28 antibody (huVL3)
LCDR1: RASESVEYYVTSLMQ (SEQ ID NO: 9)
LCDR2: AASNVES (SEQ ID NO: 10)
LCDR3: QQSRKVPYT (SEQ ID NO: 11)
LFR1: DIQLTQSPSSLSASVGDRVTITC (SEQ ID NO: 37)
LFR2: WYQQKPGKAPKLLIF (SEQ ID NO: 39)
LFR3: GVPSRFSGSGSGTDFTLTISSLQPEDFATYYC (SEQ ID NO: 42)
LFR4: FGGGTKLEIK (SEQ ID NO: 15)
VL: 
DIQLTQSPSSLSASVGDRVTITCRASESVEYYVTSLMQWYQQKPGKAPKLLIF
AASNVESGVPSRFSGSGSGTDFTLTISSLQPEDFATYYCQQSRKVPYTFGGGTK
LEIK (SEQ ID NO: 45)
Heavy chain constant region CH of humanized anti-CD28 IgG1 antibody: 






 (SEQ ID NO: 81)
Heavy chain constant region CL of humanized anti-CD28 IgG1 antibody: 


 (SEQ ID NO: 82)
Heavy chain variable region of anti-CD40 antibody 40.52
HCDR1: SYAIS (SEQ ID NO: 46)
HCDR2: MINPSGGTTSYAQKFQG (SEQ ID NO: 47)
HCDR3: GFRMDQ (SEQ ID NO: 48)
HFR1: QVQLVQSGAEVKKPGSSVKVSCKASGGTFS (SEQ ID NO: 49)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAEVKKPGSSVKVSCKASGGTFSSYAISWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 53)
Light chain variable region of anti-CD40 antibody 40.52
LCDR1: TGTSSDVGAYNFVS (SEQ ID NO: 54)
LCDR2: DVRKRPS (SEQ ID NO: 55)
LCDR3: SSYAGNNNLV (SEQ ID NO: 56)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDV
RKRPSGIPDRFSASKSGNTASLTVSGLQADDEADYYCSSYAGNNNLVFGGGT
KLTVL (SEQ ID NO: 61)
Heavy chain constant region of Fab antibody of 40.52 (CH1): 
ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKA (SEQ ID NO:
83)
Light chain constant region of Fab antibody of 40.52 (CL): 
GQPKANPTVTLFPPSSEELQANKATLVCLISDFYPGAVTVAWKADGSPVKAGV
ETTKPSKQSNNKYAASSYLSLTPEQWKSHRSYSCQVTHEGSTVEKTVAPTECS
(SEQ ID NO: 84)
Heavy chain variable region of affinity-matured anti-CD40 antibody 4052.12
HCDR1: SYAVS (SEQ ID NO: 62)
HCDR2: MINPSGGTTSYAQKFQG (SEQ ID NO: 47)
HCDR3: GFRMDQ (SEQ ID NO: 48)
HFR1: QVQLVQSGAEVKKPGSSVKVSCKASGGTFS (SEQ ID NO: 49)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAEVKKPGSSVKVSCKASGGTFSSYAVSWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 69)
Light chain variable region of affinity-matured anti-CD40 antibody 4052.12
LCDR1: TGTSSDVGAYNFVS (SEQ ID NO: 54)
LCDR2: DVRKRPR (SEQ ID NO: 64)
LCDR3: SSYAGNNNLV (SEQ ID NO: 56)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDV
RKRPRGIPDRFSASKSGNTASLTVSGLQADDEADYYCSSYAGNNNLVFGGGT
KLTVL (SEQ ID NO: 74)
Heavy chain variable region of affinity-matured anti-CD40 antibody 4052.17
HCDR1: SYAIS (SEQ ID NO: 46)
HCDR2: MINPSGGTTSYAQKFQG (SEQ ID NO: 47)
HCDR3: GFRMDQ (SEQ ID NO: 48)
HFR1: QVQLVQSGAEVKKPGSSVKVSCKASGGTFS (SEQ ID NO: 49)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAEVKKPGSSVKVSCKASGGTFSSYAISWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 70)
Light chain variable region of affinity-matured anti-CD40 antibody 4052.17
LCDR1: TGTSSDVGAYNFVS (SEQ ID NO: 54)
LCDR2: DVRKRPS (SEQ ID NO: 55)
LCDR3: SSYAGNNEMV (SEQ ID NO: 67)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDV
RKRPSGIPDRFSASKSGNTASLTVSGLQADDEADYYCSSYAGNNEMVFGGGT
KLTVL (SEQ ID NO: 75)
Heavy chain variable region of affinity-matured anti-CD40 antibody 4052.25
HCDR1: SYAIS (SEQ ID NO: 46)
HCDR2: MINPSGGTTSYAQKFQG (SEQ ID NO: 47)
HCDR3: GFRMDQ (SEQ ID NO: 48)
HFR1: QVQLVQSGAEVKKPGSSVKVSCKASGGTFS (SEQ ID NO: 49)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAEVKKPGSSVKVSCKASGGTFSSYAISWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 71)
Light chain variable region of affinity-matured anti-CD40 antibody 4052.25
LCDR1: TGTSSDVGAYNFVS (SEQ ID NO: 54)
LCDR2: DVRKRPS (SEQ ID NO: 55)
LCDR3: SSYAGNNRLV (SEQ ID NO: 68)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDV
RKRPSGIPDRFSASKSGNTASLTVSGLQADDEADYYCSSYAGNNRLVFGGGT
KLTVL (SEQ ID NO: 76)
Heavy chain variable region of affinity-matured anti-CD40 antibody 4052.28
HCDR1: SYAIS (SEQ ID NO: 46)
HCDR2: MINPSGGTTSYAQKFQG (SEQ ID NO: 47)
HCDR3: GFRMDQ (SEQ ID NO: 48)
HFR1: QVQLVQSGAEVKKPGSSVKVSCKASGGTFS (SEQ ID NO: 49)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAEVKKPGSSVKVSCKASGGTFSSYAISWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 72)
Light chain variable region of affinity-matured anti-CD40 antibody 4052.28
LCDR1: TGTSSDVGAYNFVS (SEQ ID NO: 54)
LCDR2: DTRKRPS (SEQ ID NO: 65)
LCDR3: SSYAGNNNLV (SEQ ID NO: 56)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDT
RKRPSGIPDRFSASKSGNTASLTVSGLQADDEADYYCSSYAGNNNLVFGGGT
KLTVL (SEQ ID NO: 77)
Heavy chain variable region of affinity-matured anti-CD40 antibody 4052.36
HCDR1: SYAIS (SEQ ID NO: 46)
HCDR2: MINPSGGTTSYAQKFQG (SEQ ID NO: 47)
HCDR3: GFRMDQ (SEQ ID NO: 48)
HFR1: QVQLVQSGAQVKKPGSSVKVSCKASGGTFS (SEQ ID NO: 63)
HFR2: WVRQAPGQGLEWMG (SEQ ID NO: 50)
HFR3: RVTMTRDTSTSTVYMELSSLRPEDTAVYYCAR (SEQ ID NO: 51)
HFR4: WGQGTLVTVSS (SEQ ID NO: 52)
VH: 
QVQLVQSGAQVKKPGSSVKVSCKASGGTFSSYAISWVRQAPGQGLEWMGMI
NPSGGTTSYAQKFQGRVTMTRDTSTSTVYMELSSLRPEDTAVYYCARGFRMD
QWGQGTLVTVSS (SEQ ID NO: 73)
Light chain variable region of affinity-matured anti-CD40 antibody 4052.36
LCDR1: TGTSSDVGAYNFVS (SEQ ID NO: 54)
LCDR2: DVRKRPI (SEQ ID NO: 66)
LCDR3: SSYAGNNNLV (SEQ ID NO: 56)
LFR1: QSALTQPPSASGSPGQSVTISC (SEQ ID NO: 57)
LFR2: WYQQHPGNAPKLIIY (SEQ ID NO: 58)
LFR3: GIPDRFSASKSGNTASLTVSGLQADDEADYYC (SEQ ID NO: 59)
LFR4: FGGGTKLTVL (SEQ ID NO: 60)
VL: 
QSALTQPPSASGSPGQSVTISCTGTSSDVGAYNFVSWYQQHPGNAPKLIIYDV
RKRPIGIPDRFSASKSGNTASLTVSGLQADDEADYYCSSYAGNNNLVFGGGTK
LTVL (SEQ ID NO: 78)
Linker:
GGGGS (SEQ ID NO: 79)
GGGGSGGGGSGGGGS (SEQ ID NO: 80)
Anti-mesothelin scFv (M12): 
AIRLTQSPSLLSASVGDRVTVTCRASQGGGNYLAWYQQKPGKAPKLLIYGAS
KLQSGVPSRFSGSGSGTEFTLTISSLQPEDFATYYCQQLNSYPVTFGQGTKVDI
KGGGGSGGGGSGGGGSEVQLVESGAEVKKPGASVKVSCKASGYTFTTYYIH
WVRQAPGQGLEWMGIINPSSGSTTYTQKFQGRVTMTRDTSTSTVYIELSGLR
SEDTAVYYCARGETLRGYFDYWGQGTLVTVSS (SEQ ID NO: 85)
 Note: the underlined parts are sequentially LCDR1, LCDR2, LCDR3, HCDR1, HCDR2, and HCDR3
Meso-CAR (M12):

AIRLTQSPSLLSASVGDRVTVTCRASQGGGNYLAWYQQKPGKAPKLLIYGAS
KLQSGVPSRFSGSGSGTEFTLTISSLQPEDFATYYCQQLNSYPVTFGQGTKVDI
KGGGGSGGGGSGGGGSEVQLVESGAEVKKPGASVKVSCKASGYTFTTYYIH
WVRQAPGQGLEWMGIINPSSGSTTYTQKFQGRVTMTRDTSTSTVYIELSGLR
SEDTAVYYCARGETLRGYFDYWGQGTLVTVSSTTTPAPRPPTPAPTIASQPLSL
RPEACRPAAGGAVHTRGLDFACDIYIWAPLAGTCGVLLLSLVITLYCKRGRKK
LLYIFKQPFMRPVQTTQEEDGCSCRFPEEEEGGCELRVKFSRSADAPAYKQGQ
NQLYNELNLGRREEYDVLDKRRGRDPEMGGKPRRKNPQEGLYNELQKDKM
AEAYSEIGMKGERRRGKGHDGLYQGLSTATKDTYDALHMQALPPR (SEQ ID
NO: 86)

## Claims

1. A humanized anti-CD28 antibody or an antigen-binding fragment thereof, wherein the humanized anti-CD28 antibody comprises a heavy chain variable region comprising an HCDR1, an HCDR2 and an HCDR3 and a light chain variable region comprising an LCDR1, an LCDR2 and an LCDR3, wherein,
the HCDR1 comprises SEQ ID NO: 1,
the HCDR2 comprises a sequence selected from SEQ ID NOs: 2, 17 and 18,
the HCDR3 comprises SEQ ID NO: 3,
the LCDR1 comprises SEQ ID NO: 9,
the LCDR2 comprises SEQ ID NO: 10, and
the LCDR3 comprises SEQ ID NO: 11;
the humanized anti-CD28 antibody has an EC50 of 0.008 µg/mL to 0.016 µg/mL for binding to human CD28 as determined by ELISA; or the humanized anti-CD28 antibody has an equilibrium dissociation constant KD of 6.1 × 10⁻⁹ M to 1.2 × 10⁻⁸ M for binding to human CD28 as determined by biolayer interferometry (BLI).

2. The humanized anti-CD28 antibody or the antigen-binding fragment thereof according to claim 1, wherein the heavy chain variable region of the humanized anti-CD28 antibody comprises an HFR1, an HFR2, an HFR3, and an HFR4, and the light chain variable region of the humanized anti-CD28 antibody comprises an LFR1, an LFR2, an LFR3, and an LFR4, wherein,
the HFR1 comprises a sequence selected from SEQ ID NOs: 19-22,
the HFR2 comprises a sequence selected from SEQ ID NOs: 23-26,
the HFR3 comprises a sequence selected from SEQ ID NOs: 27-30,
the HFR4 comprises the sequence set forth in SEQ ID NO: 7,
the LFR1 comprises a sequence selected from SEQ ID NOs: 36-37,
the LFR2 comprises a sequence selected from SEQ ID NOs: 38-39,
the LFR3 comprises a sequence selected from SEQ ID NOs: 40-42, and
the LFR4 comprises the sequence set forth in SEQ ID NO: 15.

3. The humanized anti-CD28 antibody or the antigen-binding fragment thereof according to claim 1 or 2, wherein the HFR1, the HFR2, the HFR3, and the HFR4 comprised in the heavy chain variable region of the humanized anti-CD28 antibody are selected from the group consisting of:
(1) an HFR1 comprising the sequence set forth in SEQ ID NO: 19, an HFR2 comprising the sequence set forth in SEQ ID NO: 23, an HFR3 comprising the sequence set forth in SEQ ID NO: 27, and an HFR4 comprising the sequence set forth in SEQ ID NO: 7;
(2) an HFR1 comprising the sequence set forth in SEQ ID NO: 20, an HFR2 comprising the sequence set forth in SEQ ID NO: 24, an HFR3 comprising the sequence set forth in SEQ ID NO: 28, and an HFR4 comprising the sequence set forth in SEQ ID NO: 7;
(3) an HFR1 comprising the sequence set forth in SEQ ID NO: 21, an HFR2 comprising the sequence set forth in SEQ ID NO: 25, an HFR3 comprising the sequence set forth in SEQ ID NO: 29, and an HFR4 comprising the sequence set forth in SEQ ID NO: 7;
(4) an HFR1 comprising the sequence set forth in SEQ ID NO: 21, an HFR2 comprising the sequence set forth in SEQ ID NO: 26, an HFR3 comprising the sequence set forth in SEQ ID NO: 30, and an HFR4 comprising the sequence set forth in SEQ ID NO: 7; and
(5) an HFR1 comprising the sequence set forth in SEQ ID NO: 22, an HFR2 comprising the sequence set forth in SEQ ID NO: 26, an HFR3 comprising the sequence set forth in SEQ ID NO: 30, and an HFR4 comprising the sequence set forth in SEQ ID NO: 7.

4. The humanized anti-CD28 antibody or the antigen-binding fragment thereof according to any one of claims 1-3, wherein the LFR1, the LFR2, the LFR3, and the LFR4 comprised in the light chain variable region of the humanized anti-CD28 antibody are selected from the group consisting of:
(1) an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(2) an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15; and
(3) an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15.

5. The humanized anti-CD28 antibody or the antigen-binding fragment thereof according to any one of claims 1-4, wherein the HFR1, the HFR2, the HFR3 and the HFR4 comprised in the heavy chain variable region and the LFR1, the LFR2, the LFR3 and the LFR4 comprised in the light chain variable region of the humanized anti-CD28 antibody are selected from the group consisting of:
(1) an HFR1 comprising the sequence set forth in SEQ ID NO: 19, an HFR2 comprising the sequence set forth in SEQ ID NO: 23, an HFR3 comprising the sequence set forth in SEQ ID NO: 27, an HFR4 comprising the sequence set forth in SEQ ID NO: 7, an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(2) an HFR1 comprising the sequence set forth in SEQ ID NO: 19, an HFR2 comprising the sequence set forth in SEQ ID NO: 23, an HFR3 comprising the sequence set forth in SEQ ID NO: 27, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(3) an HFR1 comprising the sequence set forth in SEQ ID NO: 20, an HFR2 comprising the sequence set forth in SEQ ID NO: 24, an HFR3 comprising the sequence set forth in SEQ ID NO: 28, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(4) an HFR1 comprising the sequence set forth in SEQ ID NO: 20, an HFR2 comprising the sequence set forth in SEQ ID NO: 24, an HFR3 comprising the sequence set forth in SEQ ID NO: 28, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(5) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 36, an LFR2 comprising the sequence set forth in SEQ ID NO: 38, an LFR3 comprising the sequence set forth in SEQ ID NO: 40, and an LFR4 comprising the sequence set forth in SEQ ID NO: 15;
(6) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(7) an HFR1 comprising the sequence set forth in SEQ ID NO: 21, an HFR2 comprising the sequence set forth in SEQ ID NO: 25, an HFR3 comprising the sequence set forth in SEQ ID NO: 29, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(8) an HFR1 comprising the sequence set forth in SEQ ID NO: 21, an HFR2 comprising the sequence set forth in SEQ ID NO: 26, an HFR3 comprising the sequence set forth in SEQ ID NO: 30, an HFR4 comprising the sequence set forth in SEQ ID NO: 7, (2) an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15;
(9) an HFR1 comprising the sequence set forth in SEQ ID NO: 21, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15;
(10) an HFR1 comprising the sequence set forth in SEQ ID NO: 22, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 41, and an LFR4 comprising the sequence set forth in SEQ ID NO: 15; and
(11) an HFR1 comprising the sequence set forth in SEQ ID NO: 22, an HFR2 comprising the sequence set forth in SEQ ID NO: 26, an HFR3 comprising the sequence set forth in SEQ ID NO: 30, an HFR4 comprising the sequence set forth in SEQ ID NO: 7, an LFR1 comprising the sequence set forth in SEQ ID NO: 37, an LFR2 comprising the sequence set forth in SEQ ID NO: 39, an LFR3 comprising the sequence set forth in SEQ ID NO: 42, and an LFR4 comprising the sequence set forth in SEQ ID NO: 15.

6. The humanized anti-CD28 antibody or the antigen-binding fragment thereof according to any one of claims 1-5, wherein the heavy chain variable region of the humanized anti-CD28 antibody comprises a sequence selected from SEQ ID NOs: 31-35.

7. The humanized anti-CD28 antibody or the antigen-binding fragment thereof according to any one of claims 1-6, wherein the light chain variable region of the humanized anti-CD28 antibody comprises a sequence selected from SEQ ID NOs: 43-45.

8. The humanized anti-CD28 antibody or the antigen-binding fragment thereof according to any one of claims 1-7, wherein the heavy chain variable region and the light chain variable region of the humanized anti-CD28 antibody are selected from the group consisting of:
(1) a heavy chain variable region comprising the sequence set forth in SEQ ID NO: 31 and a light chain variable region comprising the sequence set forth in SEQ ID NO: 43;
(2) a heavy chain variable region comprising the sequence set forth in SEQ ID NO: 31 and a light chain variable region comprising the sequence set forth in SEQ ID NO: 44;
(3) a heavy chain variable region comprising the sequence set forth in SEQ ID NO: 32 and a light chain variable region comprising the sequence set forth in SEQ ID NO: 44;
(4) a heavy chain variable region comprising the sequence set forth in SEQ ID NO: 32 and a light chain variable region comprising the sequence set forth in SEQ ID NO: 45;
(5) a heavy chain variable region comprising the sequence set forth in SEQ ID NO: 33 and a light chain variable region comprising the sequence set forth in SEQ ID NO: 43;
(6) a heavy chain variable region comprising the sequence set forth in SEQ ID NO: 33 and a light chain variable region comprising the sequence set forth in SEQ ID NO: 44;
(7) a heavy chain variable region comprising the sequence set forth in SEQ ID NO: 33 and a light chain variable region comprising the sequence set forth in SEQ ID NO: 45;
(8) a heavy chain variable region comprising the sequence set forth in SEQ ID NO: 34 and a light chain variable region comprising the sequence set forth in SEQ ID NO: 44;
(9) a heavy chain variable region comprising the sequence set forth in SEQ ID NO: 34 and a light chain variable region comprising the sequence set forth in SEQ ID NO: 45;
(10) a heavy chain variable region comprising the sequence set forth in SEQ ID NO: 35 and a light chain variable region comprising the sequence set forth in SEQ ID NO: 44; and
(11) a heavy chain variable region comprising the sequence set forth in SEQ ID NO: 35 and a light chain variable region comprising the sequence set forth in SEQ ID NO: 45.

9. The bispecific antibody according to any one of claims 1-8, wherein the humanized anti-CD28 antibody is an scFv.

10. The humanized anti-CD28 antibody or the antigen-binding fragment thereof according to claim 9, wherein the humanized anti-CD28 scFv comprises a heavy chain variable region and a light chain variable region linked via a peptide linker.

11. The humanized anti-CD28 antibody or the antigen-binding fragment thereof according to claim 10, wherein the sequence of the peptide linker in the humanized anti-CD28 scFv comprises SEQ ID NO: 80.

12. The humanized anti-CD28 antibody or the antigen-binding fragment thereof according to any one of claims 9-11, wherein the heavy chain variable region in the humanized anti-CD28 scFv is located at the N-terminus or C-terminus of the light chain variable region.

13. A multispecific antibody, comprising at least a first antigen-binding moiety and a second antigen-binding moiety, wherein the first antigen-binding moiety comprises the humanized anti-CD28 antibody or the antigen-binding fragment thereof according to any one of claims 1-12, and the second antigen-binding moiety and the first antigen-binding moiety bind to different antigens.

14. The multispecific antibody according to claim 13, wherein the second antigen-binding moiety specifically binds to a tumor antigen or is capable of stimulating an immune cell.

15. The multispecific antibody according to any one of claims 13-14, wherein the first antigen-binding moiety is an scFv and the second antigen-binding moiety is an scFv.

16. The multispecific antibody according to any one of claims 13-15, wherein the first antigen-binding moiety and the second antigen-binding moiety are linked via a peptide linker.

17. The multispecific antibody according to any one of claims 13-16, wherein the peptide linker comprises the sequence of SEQ ID NO: 79.

18. The multispecific antibody according to any one of claims 13-17, wherein the first antigen-binding moiety is located at the N-terminus or C-terminus of the second antigen-binding moiety.

19. The multispecific antibody according to any one of claims 13-18, wherein the second antigen-binding moiety is an anti-CD40 antibody.

20. The multispecific antibody according to any one of claims 13-19, wherein the multispecific antibody is a bispecific antibody comprising the first antigen-binding moiety and the second antigen-binding moiety.

21. The multispecific antibody according to claim 20, wherein the second antigen-binding moiety is an anti-CD40 antibody or an antigen-binding fragment thereof, wherein the anti-CD40 antibody has an EC50 of less than 0.04919 µg/mL for binding to human CD40 as determined by ELISA, or the anti-CD40 antibody has an EC50 of less than 3.44 × 10⁻⁷ M for binding to human CD40 as determined by ELISA.

22. The multispecific antibody according to any one of claims 13-21, wherein the anti-CD40 antibody is obtained by affinity maturation of a parent antibody and exhibits greater binding affinity for CD40 than the parent antibody; the parent antibody has a heavy chain variable region and a light chain variable region, wherein the heavy chain variable region comprises an HCDR1 comprising SEQ ID NO: 46, an HCDR2 comprising SEQ ID NO: 47, and an HCDR3 comprising SEQ ID NO: 48, and the light chain variable region comprises an LCDR1 comprising SEQ ID NO: 54, an LCDR2 comprising SEQ ID NO: 55, and an LCDR3 comprising SEQ ID NO: 56.

23. The multispecific antibody according to any one of claims 13-22, wherein a heavy chain variable region of the anti-CD40 antibody comprises an HCDR1 comprising a sequence selected from SEQ ID NO: 46 and SEQ ID NO: 62, an HCDR2 comprising SEQ ID NO: 47, and an HCDR3 comprising SEQ ID NO: 48.

24. The multispecific antibody according to any one of claims 13-23, wherein the heavy chain variable region of the anti-CD40 antibody comprises:
(1) an HCDR1 comprising SEQ ID NO: 46, an HCDR2 comprising SEQ ID NO: 47, and an HCDR3 comprising SEQ ID NO: 48; or
(2) an HCDR1 comprising SEQ ID NO: 62, an HCDR2 comprising SEQ ID NO: 47, and an HCDR3 comprising SEQ ID NO: 48.

25. The multispecific antibody according to any one of claims 13-24, wherein a light chain variable region of the anti-CD40 antibody comprises: an LCDR1 comprising SEQ ID NO: 54, an LCDR2 comprising a sequence selected from SEQ ID NOs: 55 and 64-66, and an LCDR3 comprising a sequence selected from SEQ ID NOs: 56 and 67-68.

26. The multispecific antibody according to any one of claims 13-25, wherein the light chain variable region of the anti-CD40 antibody comprises:
(1) an LCDR1 comprising SEQ ID NO: 54, an LCDR2 comprising SEQ ID NO: 64, and an LCDR3 comprising SEQ ID NO: 56;
(2) an LCDR1 comprising SEQ ID NO: 54, an LCDR2 comprising SEQ ID NO: 55, and an LCDR3 comprising SEQ ID NO: 67;
(3) an LCDR1 comprising SEQ ID NO: 54, an LCDR2 comprising SEQ ID NO: 55, and an LCDR3 comprising SEQ ID NO: 68;
(4) an LCDR1 comprising SEQ ID NO: 54, an LCDR2 comprising SEQ ID NO: 65, and an LCDR3 comprising SEQ ID NO: 56;
(5) an LCDR1 comprising SEQ ID NO: 54, an LCDR2 comprising SEQ ID NO: 66, and an LCDR3 comprising SEQ ID NO: 56.

27. The multispecific antibody according to any one of claims 13-26, wherein the heavy chain variable region and the light chain variable region of the anti-CD40 antibody comprise:
(1) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 64, and an LCDR3 comprising SEQ ID NO: 56;
(2) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 67;
(3) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 55, and an LCDR3 comprising SEQ ID NO: 68;
(4) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 65, and an LCDR3 comprising SEQ ID NO: 56;
(5) an HCDR1 comprising SEQ ID NO: 62, an HCDR2 comprising SEQ ID NO: 47, an HCDR3 comprising SEQ ID NO: 48, an LCDR1 comprising SEQ ID NO: 54, an LCDR2 comprising SEQ ID NO: 66, and an LCDR3 comprising SEQ ID NO: 56.

28. The multispecific antibody according to any one of claims 13-27, wherein the heavy chain variable region of the anti-CD40 antibody comprises:
(1) an HFR1 comprising SEQ ID NO: 49, an HFR2 comprising SEQ ID NO: 50, an HFR3 comprising SEQ ID NO: 51, and an HFR4 comprising SEQ ID NO: 52;
(2) an HFR1 comprising SEQ ID NO: 63, an HFR2 comprising SEQ ID NO: 50, an HFR3 comprising SEQ ID NO: 51, and an HFR4 comprising SEQ ID NO: 52.

29. The multispecific antibody according to any one of claims 13-28, wherein the light chain variable region of the anti-CD40 antibody comprises an LFR1 comprising SEQ ID NO: 57, an LFR2 comprising SEQ ID NO: 58, an LFR3 comprising SEQ ID NO: 59, and an LFR4 comprising SEQ ID NO: 60.

30. The multispecific antibody according to any one of claims 13-29, wherein the heavy chain variable region of the anti-CD40 antibody has at least 70% sequence identity to a sequence selected from SEQ ID NOs: 69-73.

31. The multispecific antibody according to claim 30, wherein the heavy chain variable region of the anti-CD40 antibody comprises a sequence selected from SEQ ID NOs: 69-73.

32. The multispecific antibody according to any one of claims 13-31, wherein the light chain variable region of the anti-CD40 antibody has at least 70% sequence identity to a sequence selected from SEQ ID NOs: 74-78.

33. The multispecific antibody according to claim 32, wherein the light chain variable region of the anti-CD40 antibody comprises a sequence selected from SEQ ID NOs: 74-78.

34. The multispecific antibody according to any one of claims 13-33, wherein the anti-CD40 antibody is an scFv.

35. The multispecific antibody according to claim 34, wherein the anti-CD40 scFv comprises a heavy chain variable region and a light chain variable region linked via a peptide linker.

36. The multispecific antibody according to claim 35, wherein the sequence of the peptide linker in the anti-CD40 scFv comprises SEQ ID NO: 80.

37. The multispecific antibody according to any one of claims 34-36, wherein the heavy chain variable region in the anti-CD40 scFv is located at the N-terminus or C-terminus of the light chain variable region.

38. A polynucleotide encoding the humanized anti-CD28 antibody or the antigen-binding fragment thereof according to any one of claims 1-12 or the multispecific antibody according to any one of claims 13-37.

39. A vector comprising the polynucleotide according to claim 38.

40. The vector according to claim 39, wherein the vector is a plasmid vector, a viral vector, a circular RNA comprising the polynucleotide, a precursor RNA from which the circular RNA can be obtained by circularization, or a vector comprising a DNA template for the precursor RNA, wherein the circular RNA comprises, in sequence, an internal ribosome entry site (IRES) element, the polynucleotide, and a poly A.

41. The vector according to claim 40, wherein the polyA is at least 45 nucleotides in length; preferably, the polyA is at least 70 nucleotides in length.

42. A host cell comprising the polynucleotide according to claim 38 or the vector according to any one of claims 39-41.

43. A pharmaceutical composition, comprising the humanized anti-CD28 antibody or the antigen-binding fragment thereof according to any one of claims 1-12, the multispecific antibody according to any one of claims 13-37, the polynucleotide according to claim 38 or the vector according to any one of claims 39-41 and a pharmaceutically acceptable carrier.

44. A pharmaceutical combination, comprising a combination of: a) the humanized anti-CD28 antibody or the antigen-binding fragment thereof according to any one of claims 1-12, the multispecific antibody according to any one of claims 13-37, the polynucleotide according to claim 38, or the vector according to any one of claims 39-41; and b) an immune cell.

45. An immune cell expressing the humanized anti-CD28 antibody or the antigen-binding fragment thereof according to any one of claims 1-12 or the multispecific antibody according to any one of claims 13-37.

46. The pharmaceutical combination according to claim 44 or the immune cell according to claim 45, wherein the immune cell is a T cell, an NK cell, an NKT cell, a macrophage, a neutrophil, or a granulocyte.

47. The pharmaceutical combination or the immune cell according to any one of claims 44-46, wherein the immune cell recombinantly expresses a chimeric antigen receptor (CAR), a T cell receptor (TCR), or a bispecific T cell engager (BiTE), wherein the CAR, the TCR, or the BiTE binds to a tumor antigen or a viral antigen.

48. The pharmaceutical combination or the immune cell according to any one of claims 44-47, wherein the immune cell is a CAR-T cell.

49. The pharmaceutical combination or the immune cell according to claim 47 or 48, wherein the CAR targets mesothelin, CD123, BCMA, HER2, II,13Ra2, CD19, or B7H3.

50. The pharmaceutical combination or the immune cell according to claim 49, wherein the CAR targeting mesothelin comprises an anti-mesothelin antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 87, an LCDR2 set forth in SEQ ID NO: 102, an LCDR3 set forth in SEQ ID NO: 116, an HCDR1 set forth in SEQ ID NO: 131, an HCDR2 set forth in SEQ ID NO: 144, and an HCDR3 set forth in SEQ ID NO: 157;
(2) an LCDR1 set forth in SEQ ID NO: 88, an LCDR2 set forth in SEQ ID NO: 103, an LCDR3 set forth in SEQ ID NO: 117, an HCDR1 set forth in SEQ ID NO: 132, an HCDR2 set forth in SEQ ID NO: 145, and an HCDR3 set forth in SEQ ID NO: 158;
(3) an LCDR1 set forth in SEQ ID NO: 89, an LCDR2 set forth in SEQ ID NO: 104, an LCDR3 set forth in SEQ ID NO: 118, an HCDR1 set forth in SEQ ID NO: 133, an HCDR2 set forth in SEQ ID NO: 146, and an HCDR3 set forth in SEQ ID NO: 159;
(4) an LCDR1 set forth in SEQ ID NO: 90, an LCDR2 set forth in SEQ ID NO: 105, an LCDR3 set forth in SEQ ID NO: 119, an HCDR1 set forth in SEQ ID NO: 134, an HCDR2 set forth in SEQ ID NO: 147, and an HCDR3 set forth in SEQ ID NO: 160;
(5) an LCDR1 set forth in SEQ ID NO: 91, an LCDR2 set forth in SEQ ID NO: 106, an LCDR3 set forth in SEQ ID NO: 120, an HCDR1 set forth in SEQ ID NO: 135, an HCDR2 set forth in SEQ ID NO: 148, and an HCDR3 set forth in SEQ ID NO: 161;
(6) an LCDR1 set forth in SEQ ID NO: 92, an LCDR2 set forth in SEQ ID NO: 107, an LCDR3 set forth in SEQ ID NO: 121, an HCDR1 set forth in SEQ ID NO: 136, an HCDR2 set forth in SEQ ID NO: 149, and an HCDR3 set forth in SEQ ID NO: 162;
(7) an LCDR1 set forth in SEQ ID NO: 93, an LCDR2 set forth in SEQ ID NO: 108, an LCDR3 set forth in SEQ ID NO: 122, an HCDR1 set forth in SEQ ID NO: 137, an HCDR2 set forth in SEQ ID NO: 150, and an HCDR3 set forth in SEQ ID NO: 163;
(8) an LCDR1 set forth in SEQ ID NO: 94, an LCDR2 set forth in SEQ ID NO: 109, an LCDR3 set forth in SEQ ID NO: 123, an HCDR1 set forth in SEQ ID NO: 138, an HCDR2 set forth in SEQ ID NO: 151, and an HCDR3 set forth in SEQ ID NO: 164;
(9) an LCDR1 set forth in SEQ ID NO: 95, an LCDR2 set forth in SEQ ID NO: 110, an LCDR3 set forth in SEQ ID NO: 124, an HCDR1 set forth in SEQ ID NO: 139, an HCDR2 set forth in SEQ ID NO: 152, and an HCDR3 set forth in SEQ ID NO: 165;
(10) an LCDR1 set forth in SEQ ID NO: 96, an LCDR2 set forth in SEQ ID NO: 111, an LCDR3 set forth in SEQ ID NO: 125, an HCDR1 set forth in SEQ ID NO: 134, an HCDR2 set forth in SEQ ID NO: 147, and an HCDR3 set forth in SEQ ID NO: 166;
(11) an LCDR1 set forth in SEQ ID NO: 97, an LCDR2 set forth in SEQ ID NO: 112, an LCDR3 set forth in SEQ ID NO: 126, an HCDR1 set forth in SEQ ID NO: 140, an HCDR2 set forth in SEQ ID NO: 153, and an HCDR3 set forth in SEQ ID NO: 167;
(12) an LCDR1 set forth in SEQ ID NO: 98, an LCDR2 set forth in SEQ ID NO: 113, an LCDR3 set forth in SEQ ID NO: 127, an HCDR1 set forth in SEQ ID NO: 139, an HCDR2 set forth in SEQ ID NO: 152, and an HCDR3 set forth in SEQ ID NO: 168;
(13) an LCDR1 set forth in SEQ ID NO: 99, an LCDR2 set forth in SEQ ID NO: 114, an LCDR3 set forth in SEQ ID NO: 128, an HCDR1 set forth in SEQ ID NO: 141, an HCDR2 set forth in SEQ ID NO: 154, and an HCDR3 set forth in SEQ ID NO: 169;
(14) an LCDR1 set forth in SEQ ID NO: 100, an LCDR2 set forth in SEQ ID NO: 115, an LCDR3 set forth in SEQ ID NO: 129, an HCDR1 set forth in SEQ ID NO: 142, an HCDR2 set forth in SEQ ID NO: 155, and an HCDR3 set forth in SEQ ID NO: 170; and
(15) an LCDR1 set forth in SEQ ID NO: 101, an LCDR2 set forth in SEQ ID NO: 104, an LCDR3 set forth in SEQ ID NO: 130, an HCDR1 set forth in SEQ ID NO: 143, an HCDR2 set forth in SEQ ID NO: 156, and an HCDR3 set forth in SEQ ID NO: 171.

51. The pharmaceutical combination or the immune cell according to claim 50, wherein the light chain variable region and the heavy chain variable region are selected from the group consisting of:
(1) a light chain variable region set forth in SEQ ID NO: 172 and a heavy chain variable region set forth in SEQ ID NO: 187;
(2) a light chain variable region set forth in SEQ ID NO: 173 and a heavy chain variable region set forth in SEQ ID NO: 188;
(3) a light chain variable region set forth in SEQ ID NO: 174 and a heavy chain variable region set forth in SEQ ID NO: 189;
(4) a light chain variable region set forth in SEQ ID NO: 175 and a heavy chain variable region set forth in SEQ ID NO: 190;
(5) a light chain variable region set forth in SEQ ID NO: 176 and a heavy chain variable region set forth in SEQ ID NO: 191;
(6) a light chain variable region set forth in SEQ ID NO: 177 and a heavy chain variable region set forth in SEQ ID NO: 192;
(7) a light chain variable region set forth in SEQ ID NO: 178 and a heavy chain variable region set forth in SEQ ID NO: 193;
(8) a light chain variable region set forth in SEQ ID NO: 179 and a heavy chain variable region set forth in SEQ ID NO: 194;
(9) a light chain variable region set forth in SEQ ID NO: 180 and a heavy chain variable region set forth in SEQ ID NO: 195;
(10) a light chain variable region set forth in SEQ ID NO: 181 and a heavy chain variable region set forth in SEQ ID NO: 196;
(11) a light chain variable region set forth in SEQ ID NO: 182 and a heavy chain variable region set forth in SEQ ID NO: 197;
(12) a light chain variable region set forth in SEQ ID NO: 183 and a heavy chain variable region set forth in SEQ ID NO: 198;
(13) a light chain variable region set forth in SEQ ID NO: 184 and a heavy chain variable region set forth in SEQ ID NO: 199;
(14) a light chain variable region set forth in SEQ ID NO: 185 and a heavy chain variable region set forth in SEQ ID NO: 200; and
(15) a light chain variable region set forth in SEQ ID NO: 186 and a heavy chain variable region set forth in SEQ ID NO: 201.

52. The pharmaceutical combination or the immune cell according to claim 50 or 51, wherein the anti-mesothelin antibody is an scFv comprising an amino acid sequence selected from SEQ ID NOs: 202-216.

53. The pharmaceutical combination or the immune cell according to any one of claims 50-52, wherein the CAR targeting mesothelin comprises an amino acid sequence selected from SEQ ID NOs: 217-231.

54. The pharmaceutical combination or the immune cell according to claim 49, wherein the CAR targeting CD123 comprises an anti-CD123 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 349, an LCDR2 set forth in SEQ ID NO: 379, an LCDR3 set forth in SEQ ID NO: 407, an HCDR1 set forth in SEQ ID NO: 437, an HCDR2 set forth in SEQ ID NO: 458, and an HCDR3 set forth in SEQ ID NO: 482;
(2) an LCDR1 set forth in SEQ ID NO: 363, an LCDR2 set forth in SEQ ID NO: 391, an LCDR3 set forth in SEQ ID NO: 421, an HCDR1 set forth in SEQ ID NO: 435, an HCDR2 set forth in SEQ ID NO: 468, and an HCDR3 set forth in SEQ ID NO: 495;
(3) an LCDR1 set forth in SEQ ID NO: 353, an LCDR2 set forth in SEQ ID NO: 383, an LCDR3 set forth in SEQ ID NO: 412, an HCDR1 set forth in SEQ ID NO: 441, an HCDR2 set forth in SEQ ID NO: 462, and an HCDR3 set forth in SEQ ID NO: 486;
(4) an LCDR1 set forth in SEQ ID NO: 364, an LCDR2 set forth in SEQ ID NO: 378, an LCDR3 set forth in SEQ ID NO: 422, an HCDR1 set forth in SEQ ID NO: 446, an HCDR2 set forth in SEQ ID NO: 469, and an HCDR3 set forth in SEQ ID NO: 496;
(5) an LCDR1 set forth in SEQ ID NO: 369, an LCDR2 set forth in SEQ ID NO: 396, an LCDR3 set forth in SEQ ID NO: 428, an HCDR1 set forth in SEQ ID NO: 445, an HCDR2 set forth in SEQ ID NO: 475, and an HCDR3 set forth in SEQ ID NO: 503;
(6) an LCDR1 set forth in SEQ ID NO: 370, an LCDR2 set forth in SEQ ID NO: 397, an LCDR3 set forth in SEQ ID NO: 429, an HCDR1 set forth in SEQ ID NO: 452, an HCDR2 set forth in SEQ ID NO: 474, and an HCDR3 set forth in SEQ ID NO: 504;
(7) an LCDR1 set forth in SEQ ID NO: 354, an LCDR2 set forth in SEQ ID NO: 384, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(8) an LCDR1 set forth in SEQ ID NO: 350, an LCDR2 set forth in SEQ ID NO: 380, an LCDR3 set forth in SEQ ID NO: 408, an HCDR1 set forth in SEQ ID NO: 435, an HCDR2 set forth in SEQ ID NO: 456, and an HCDR3 set forth in SEQ ID NO: 480;
(9) an LCDR1 set forth in SEQ ID NO: 371, an LCDR2 set forth in SEQ ID NO: 398, an LCDR3 set forth in SEQ ID NO: 430, an HCDR1 set forth in SEQ ID NO: 453, an HCDR2 set forth in SEQ ID NO: 476, and an HCDR3 set forth in SEQ ID NO: 505;
(10) an LCDR1 set forth in SEQ ID NO: 372, an LCDR2 set forth in SEQ ID NO: 399, an LCDR3 set forth in SEQ ID NO: 431, an HCDR1 set forth in SEQ ID NO: 441, an HCDR2 set forth in SEQ ID NO: 462, and an HCDR3 set forth in SEQ ID NO: 506;
(11) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(12) an LCDR1 set forth in SEQ ID NO: 363, an LCDR2 set forth in SEQ ID NO: 392, an LCDR3 set forth in SEQ ID NO: 423, an HCDR1 set forth in SEQ ID NO: 447, an HCDR2 set forth in SEQ ID NO: 470, and an HCDR3 set forth in SEQ ID NO: 497;
(13) an LCDR1 set forth in SEQ ID NO: 373, an LCDR2 set forth in SEQ ID NO: 400, an LCDR3 set forth in SEQ ID NO: 432, an HCDR1 set forth in SEQ ID NO: 451, an HCDR2 set forth in SEQ ID NO: 477, and an HCDR3 set forth in SEQ ID NO: 507;
(14) an LCDR1 set forth in SEQ ID NO: 355, an LCDR2 set forth in SEQ ID NO: 385, an LCDR3 set forth in SEQ ID NO: 414, an HCDR1 set forth in SEQ ID NO: 443, an HCDR2 set forth in SEQ ID NO: 464, and an HCDR3 set forth in SEQ ID NO: 488;
(15) an LCDR1 set forth in SEQ ID NO: 356, an LCDR2 set forth in SEQ ID NO: 386, an LCDR3 set forth in SEQ ID NO: 415, an HCDR1 set forth in SEQ ID NO: 444, an HCDR2 set forth in SEQ ID NO: 465, and an HCDR3 set forth in SEQ ID NO: 489;
(16) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 451, an HCDR2 set forth in SEQ ID NO: 474, and an HCDR3 set forth in SEQ ID NO: 502;
(17) an LCDR1 set forth in SEQ ID NO: 351, an LCDR2 set forth in SEQ ID NO: 381, an LCDR3 set forth in SEQ ID NO: 409, an HCDR1 set forth in SEQ ID NO: 438, an HCDR2 set forth in SEQ ID NO: 459, and an HCDR3 set forth in SEQ ID NO: 483;
(18) an LCDR1 set forth in SEQ ID NO: 347, an LCDR2 set forth in SEQ ID NO: 376, an LCDR3 set forth in SEQ ID NO: 410, an HCDR1 set forth in SEQ ID NO: 439, an HCDR2 set forth in SEQ ID NO: 460, and an HCDR3 set forth in SEQ ID NO: 484;
(19) an LCDR1 set forth in SEQ ID NO: 357, an LCDR2 set forth in SEQ ID NO: 387, an LCDR3 set forth in SEQ ID NO: 416, an HCDR1 set forth in SEQ ID NO: 437, an HCDR2 set forth in SEQ ID NO: 466, and an HCDR3 set forth in SEQ ID NO: 490;
(20) an LCDR1 set forth in SEQ ID NO: 347, an LCDR2 set forth in SEQ ID NO: 377, an LCDR3 set forth in SEQ ID NO: 405, an HCDR1 set forth in SEQ ID NO: 435, an HCDR2 set forth in SEQ ID NO: 456, and an HCDR3 set forth in SEQ ID NO: 480;
(21) an LCDR1 set forth in SEQ ID NO: 365, an LCDR2 set forth in SEQ ID NO: 393, an LCDR3 set forth in SEQ ID NO: 424, an HCDR1 set forth in SEQ ID NO: 448, an HCDR2 set forth in SEQ ID NO: 471, and an HCDR3 set forth in SEQ ID NO: 498;
(22) an LCDR1 set forth in SEQ ID NO: 374, an LCDR2 set forth in SEQ ID NO: 401, an LCDR3 set forth in SEQ ID NO: 433, an HCDR1 set forth in SEQ ID NO: 454, an HCDR2 set forth in SEQ ID NO: 478, and an HCDR3 set forth in SEQ ID NO: 508;
(23) an LCDR1 set forth in SEQ ID NO: 358, an LCDR2 set forth in SEQ ID NO: 385, an LCDR3 set forth in SEQ ID NO: 417, an HCDR1 set forth in SEQ ID NO: 442, an HCDR2 set forth in SEQ ID NO: 463, and an HCDR3 set forth in SEQ ID NO: 491;
(24) an LCDR1 set forth in SEQ ID NO: 359, an LCDR2 set forth in SEQ ID NO: 388, an LCDR3 set forth in SEQ ID NO: 418, an HCDR1 set forth in SEQ ID NO: 441, an HCDR2 set forth in SEQ ID NO: 462, and an HCDR3 set forth in SEQ ID NO: 492;
(25) an LCDR1 set forth in SEQ ID NO: 347, an LCDR2 set forth in SEQ ID NO: 376, an LCDR3 set forth in SEQ ID NO: 403, an HCDR1 set forth in SEQ ID NO: 435, an HCDR2 set forth in SEQ ID NO: 456, and an HCDR3 set forth in SEQ ID NO: 480;
(26) an LCDR1 set forth in SEQ ID NO: 366, an LCDR2 set forth in SEQ ID NO: 394, an LCDR3 set forth in SEQ ID NO: 425, an HCDR1 set forth in SEQ ID NO: 449, an HCDR2 set forth in SEQ ID NO: 472, and an HCDR3 set forth in SEQ ID NO: 499;
(27) an LCDR1 set forth in SEQ ID NO: 360, an LCDR2 set forth in SEQ ID NO: 389, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(28) an LCDR1 set forth in SEQ ID NO: 347, an LCDR2 set forth in SEQ ID NO: 376, an LCDR3 set forth in SEQ ID NO: 404, an HCDR1 set forth in SEQ ID NO: 435, an HCDR2 set forth in SEQ ID NO: 456, and an HCDR3 set forth in SEQ ID NO: 480;
(29) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(30) an LCDR1 set forth in SEQ ID NO: 361, an LCDR2 set forth in SEQ ID NO: 390, an LCDR3 set forth in SEQ ID NO: 419, an HCDR1 set forth in SEQ ID NO: 445, an HCDR2 set forth in SEQ ID NO: 467, and an HCDR3 set forth in SEQ ID NO: 493;
(31) an LCDR1 set forth in SEQ ID NO: 375, an LCDR2 set forth in SEQ ID NO: 402, an LCDR3 set forth in SEQ ID NO: 434, an HCDR1 set forth in SEQ ID NO: 455, an HCDR2 set forth in SEQ ID NO: 479, and an HCDR3 set forth in SEQ ID NO: 509;
(32) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 450, an HCDR2 set forth in SEQ ID NO: 473, and an HCDR3 set forth in SEQ ID NO: 5015;
(33) an LCDR1 set forth in SEQ ID NO: 362, an LCDR2 set forth in SEQ ID NO: 388, an LCDR3 set forth in SEQ ID NO: 420, an HCDR1 set forth in SEQ ID NO: 441, an HCDR2 set forth in SEQ ID NO: 462, and an HCDR3 set forth in SEQ ID NO: 494;
(34) an LCDR1 set forth in SEQ ID NO: 352, an LCDR2 set forth in SEQ ID NO: 382, an LCDR3 set forth in SEQ ID NO: 411, an HCDR1 set forth in SEQ ID NO: 440, an HCDR2 set forth in SEQ ID NO: 461, and an HCDR3 set forth in SEQ ID NO: 485; and
(35) an LCDR1 set forth in SEQ ID NO: 367, an LCDR2 set forth in SEQ ID NO: 391, an LCDR3 set forth in SEQ ID NO: 426, an HCDR1 set forth in SEQ ID NO: 435, an HCDR2 set forth in SEQ ID NO: 468, and an HCDR3 set forth in SEQ ID NO: 500.

55. The pharmaceutical combination or the immune cell according to claim 54, wherein the light chain variable region and the heavy chain variable region are selected from the group consisting of:
(1) a light chain variable region set forth in SEQ ID NO: 510 and a heavy chain variable region set forth in SEQ ID NO: 545;
(2) a light chain variable region set forth in SEQ ID NO: 511 and a heavy chain variable region set forth in SEQ ID NO: 546;
(3) a light chain variable region set forth in SEQ ID NO: 512 and a heavy chain variable region set forth in SEQ ID NO: 547;
(4) a light chain variable region set forth in SEQ ID NO: 513 and a heavy chain variable region set forth in SEQ ID NO: 548;
(5) a light chain variable region set forth in SEQ ID NO: 514 and a heavy chain variable region set forth in SEQ ID NO: 549;
(6) a light chain variable region set forth in SEQ ID NO: 515 and a heavy chain variable region set forth in SEQ ID NO: 550;
(7) a light chain variable region set forth in SEQ ID NO: 516 and a heavy chain variable region set forth in SEQ ID NO: 551;
(8) a light chain variable region set forth in SEQ ID NO: 517 and a heavy chain variable region set forth in SEQ ID NO: 552;
(9) a light chain variable region set forth in SEQ ID NO: 518 and a heavy chain variable region set forth in SEQ ID NO: 553;
(10) a light chain variable region set forth in SEQ ID NO: 519 and a heavy chain variable region set forth in SEQ ID NO: 554;
(11) a light chain variable region set forth in SEQ ID NO: 520 and a heavy chain variable region set forth in SEQ ID NO: 555;
(12) a light chain variable region set forth in SEQ ID NO: 521 and a heavy chain variable region set forth in SEQ ID NO: 556;
(13) a light chain variable region set forth in SEQ ID NO: 522 and a heavy chain variable region set forth in SEQ ID NO: 557;
(14) a light chain variable region set forth in SEQ ID NO: 523 and a heavy chain variable region set forth in SEQ ID NO: 558;
(15) a light chain variable region set forth in SEQ ID NO: 524 and a heavy chain variable region set forth in SEQ ID NO: 559;
(16) a light chain variable region set forth in SEQ ID NO: 525 and a heavy chain variable region set forth in SEQ ID NO: 560;
(17) a light chain variable region set forth in SEQ ID NO: 526 and a heavy chain variable region set forth in SEQ ID NO: 561;
(18) a light chain variable region set forth in SEQ ID NO: 527 and a heavy chain variable region set forth in SEQ ID NO: 562;
(19) a light chain variable region set forth in SEQ ID NO: 528 and a heavy chain variable region set forth in SEQ ID NO: 563;
(20) a light chain variable region set forth in SEQ ID NO: 529 and a heavy chain variable region set forth in SEQ ID NO: 564;
(21) a light chain variable region set forth in SEQ ID NO: 530 and a heavy chain variable region set forth in SEQ ID NO: 565;
(22) a light chain variable region set forth in SEQ ID NO: 531 and a heavy chain variable region set forth in SEQ ID NO: 566;
(23) a light chain variable region set forth in SEQ ID NO: 532 and a heavy chain variable region set forth in SEQ ID NO: 567;
(24) a light chain variable region set forth in SEQ ID NO: 533 and a heavy chain variable region set forth in SEQ ID NO: 568;
(25) a light chain variable region set forth in SEQ ID NO: 534 and a heavy chain variable region set forth in SEQ ID NO: 569;
(26) a light chain variable region set forth in SEQ ID NO: 535 and a heavy chain variable region set forth in SEQ ID NO: 570;
(27) a light chain variable region set forth in SEQ ID NO: 536 and a heavy chain variable region set forth in SEQ ID NO: 571;
(28) a light chain variable region set forth in SEQ ID NO: 537 and a heavy chain variable region set forth in SEQ ID NO: 572;
(29) a light chain variable region set forth in SEQ ID NO: 538 and a heavy chain variable region set forth in SEQ ID NO: 573;
(30) a light chain variable region set forth in SEQ ID NO: 539 and a heavy chain variable region set forth in SEQ ID NO: 574;
(31) a light chain variable region set forth in SEQ ID NO: 540 and a heavy chain variable region set forth in SEQ ID NO: 575;
(32) a light chain variable region set forth in SEQ ID NO: 541 and a heavy chain variable region set forth in SEQ ID NO: 576;
(33) a light chain variable region set forth in SEQ ID NO: 542 and a heavy chain variable region set forth in SEQ ID NO: 577;
(34) a light chain variable region set forth in SEQ ID NO: 543 and a heavy chain variable region set forth in SEQ ID NO: 578; and
(35) a light chain variable region set forth in SEQ ID NO: 544 and a heavy chain variable region set forth in SEQ ID NO: 579.

56. The pharmaceutical combination or the immune cell according to claim 54 or 55, wherein the CD123 antibody is an scFv comprising an amino acid sequence selected from SEQ ID NOs: 583-617.

57. The pharmaceutical combination or the immune cell according to any one of claims 54-56, wherein the CAR targeting CD123 comprises an amino acid sequence selected from SEQ ID NOs: 580-582.

58. The pharmaceutical combination or the immune cell according to claim 49, wherein the CAR targeting BCMA comprises an anti-BCMA antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 232, an LCDR2 set forth in SEQ ID NO: 242, an LCDR3 set forth in SEQ ID NO: 253, an HCDR1 set forth in SEQ ID NO: 264, an HCDR2 set forth in SEQ ID NO: 275, and an HCDR3 set forth in SEQ ID NO: 287;
(2) an LCDR1 set forth in SEQ ID NO: 233, an LCDR2 set forth in SEQ ID NO: 243, an LCDR3 set forth in SEQ ID NO: 254, an HCDR1 set forth in SEQ ID NO: 265, an HCDR2 set forth in SEQ ID NO: 276, and an HCDR3 set forth in SEQ ID NO: 288;
(3) an LCDR1 set forth in SEQ ID NO: 234, an LCDR2 set forth in SEQ ID NO: 244, an LCDR3 set forth in SEQ ID NO: 255, an HCDR1 set forth in SEQ ID NO: 266, an HCDR2 set forth in SEQ ID NO: 277, and an HCDR3 set forth in SEQ ID NO: 289;
(4) an LCDR1 set forth in SEQ ID NO: 235, an LCDR2 set forth in SEQ ID NO: 245, an LCDR3 set forth in SEQ ID NO: 255, an HCDR1 set forth in SEQ ID NO: 267, an HCDR2 set forth in SEQ ID NO: 278, and an HCDR3 set forth in SEQ ID NO: 290;
(5) an LCDR1 set forth in SEQ ID NO: 236, an LCDR2 set forth in SEQ ID NO: 246, an LCDR3 set forth in SEQ ID NO: 256, an HCDR1 set forth in SEQ ID NO: 268, an HCDR2 set forth in SEQ ID NO: 279, and an HCDR3 set forth in SEQ ID NO: 291;
(6) an LCDR1 set forth in SEQ ID NO: 237, an LCDR2 set forth in SEQ ID NO: 247, an LCDR3 set forth in SEQ ID NO: 257, an HCDR1 set forth in SEQ ID NO: 269, an HCDR2 set forth in SEQ ID NO: 280, and an HCDR3 set forth in SEQ ID NO: 292;
(7) an LCDR1 set forth in SEQ ID NO: 238, an LCDR2 set forth in SEQ ID NO: 248, an LCDR3 set forth in SEQ ID NO: 258, an HCDR1 set forth in SEQ ID NO: 266, an HCDR2 set forth in SEQ ID NO: 281, and an HCDR3 set forth in SEQ ID NO: 293;
(8) an LCDR1 set forth in SEQ ID NO: 239, an LCDR2 set forth in SEQ ID NO: 249, an LCDR3 set forth in SEQ ID NO: 259, an HCDR1 set forth in SEQ ID NO: 270, an HCDR2 set forth in SEQ ID NO: 282, and an HCDR3 set forth in SEQ ID NO: 294;
(9) an LCDR1 set forth in SEQ ID NO: 233, an LCDR2 set forth in SEQ ID NO: 250, an LCDR3 set forth in SEQ ID NO: 260, an HCDR1 set forth in SEQ ID NO: 271, an HCDR2 set forth in SEQ ID NO: 283, and an HCDR3 set forth in SEQ ID NO: 295;
(10) an LCDR1 set forth in SEQ ID NO: 233, an LCDR2 set forth in SEQ ID NO: 251, an LCDR3 set forth in SEQ ID NO: 261, an HCDR1 set forth in SEQ ID NO: 272, an HCDR2 set forth in SEQ ID NO: 284, and an HCDR3 set forth in SEQ ID NO: 296;
(11) an LCDR1 set forth in SEQ ID NO: 240, an LCDR2 set forth in SEQ ID NO: 252, an LCDR3 set forth in SEQ ID NO: 262, an HCDR1 set forth in SEQ ID NO: 273, an HCDR2 set forth in SEQ ID NO: 285, and an HCDR3 set forth in SEQ ID NO: 297; and
(12) an LCDR1 set forth in SEQ ID NO: 241, an LCDR2 set forth in SEQ ID NO: 245, an LCDR3 set forth in SEQ ID NO: 263, an HCDR1 set forth in SEQ ID NO: 274, an HCDR2 set forth in SEQ ID NO: 286, and an HCDR3 set forth in SEQ ID NO: 298.

59. The pharmaceutical combination or the immune cell according to claim 58, wherein the light chain variable region and the heavy chain variable region are selected from the group consisting of:
(1) a light chain variable region set forth in SEQ ID NO: 299 and a heavy chain variable region set forth in SEQ ID NO: 311;
(2) a light chain variable region set forth in SEQ ID NO: 300 and a heavy chain variable region set forth in SEQ ID NO: 312;
(3) a light chain variable region set forth in SEQ ID NO: 301 and a heavy chain variable region set forth in SEQ ID NO: 313;
(4) a light chain variable region set forth in SEQ ID NO: 302 and a heavy chain variable region set forth in SEQ ID NO: 314;
(5) a light chain variable region set forth in SEQ ID NO: 303 and a heavy chain variable region set forth in SEQ ID NO: 315;
(6) a light chain variable region set forth in SEQ ID NO: 304 and a heavy chain variable region set forth in SEQ ID NO: 316;
(7) a light chain variable region set forth in SEQ ID NO: 305 and a heavy chain variable region set forth in SEQ ID NO: 317;
(8) a light chain variable region set forth in SEQ ID NO: 306 and a heavy chain variable region set forth in SEQ ID NO: 318;
(9) a light chain variable region set forth in SEQ ID NO: 307 and a heavy chain variable region set forth in SEQ ID NO: 319;
(10) a light chain variable region set forth in SEQ ID NO: 308 and a heavy chain variable region set forth in SEQ ID NO: 320;
(11) a light chain variable region set forth in SEQ ID NO: 309 and a heavy chain variable region set forth in SEQ ID NO: 321; and
(12) a light chain variable region set forth in SEQ ID NO: 310 and a heavy chain variable region set forth in SEQ ID NO: 322.

60. The pharmaceutical combination or the immune cell according to claim 58 or 59, wherein the anti-BCMA antibody is an scFv comprising an amino acid sequence selected from SEQ ID NOs: 323-334.

61. The pharmaceutical combination or the immune cell according to any one of claims 58-60, wherein the CAR targeting BCMA comprises an amino acid sequence selected from SEQ ID NOs: 335-346.

62. The pharmaceutical combination or the immune cell according to claim 49, wherein the CAR targeting CD19 comprises an anti-CD19 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 628, an LCDR2 set forth in SEQ ID NO: 629, an LCDR3 set forth in SEQ ID NO: 630, an HCDR1 set forth in SEQ ID NO: 631, an HCDR2 set forth in SEQ ID NO: 632, and an HCDR3 set forth in SEQ ID NO: 633.

63. The pharmaceutical combination or the immune cell according to claim 62, wherein the anti-CD19 antibody comprises a light chain variable region set forth in SEQ ID NO: 635 and a heavy chain variable region set forth in SEQ ID NO: 634.

64. The pharmaceutical combination or the immune cell according to claim 62 or 63, wherein the anti-CD19 antibody is an scFv comprising the amino acid sequence set forth in SEQ ID NO: 636.

65. The pharmaceutical combination or the immune cell according to any one of claims 62-64, wherein the CAR targeting CD19 comprises the amino acid sequence set forth in SEQ ID NO: 637.

66. The pharmaceutical combination or the immune cell according to claim 49, wherein the CAR targeting HER2 comprises an anti-HER2 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 618, an LCDR2 set forth in SEQ ID NO: 619, an LCDR3 set forth in SEQ ID NO: 620, an HCDR1 set forth in SEQ ID NO: 621, an HCDR2 set forth in SEQ ID NO: 622, and an HCDR3 set forth in SEQ ID NO: 623.

67. The pharmaceutical combination or the immune cell according to claim 66, wherein the anti-HER2 antibody comprises a light chain variable region set forth in SEQ ID NO: 624 and a heavy chain variable region set forth in SEQ ID NO: 625.

68. The pharmaceutical combination or the immune cell according to claim 66 or 67, wherein the anti-HER2 antibody is an scFv comprising the amino acid sequence set forth in SEQ ID NO: 626.

69. The pharmaceutical combination or the immune cell according to any one of claims 66-68, wherein the CAR targeting HER2 comprises the amino acid sequence set forth in SEQ ID NO: 627.

70. The pharmaceutical combination or the immune cell according to claim 49, wherein the CAR targeting IL13Ra2 comprises an anti-IL13Ra2 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 638, an LCDR2 set forth in SEQ ID NO: 639, an LCDR3 set forth in SEQ ID NO: 640, an HCDR1 set forth in SEQ ID NO: 641, an HCDR2 set forth in SEQ ID NO: 642, and an HCDR3 set forth in SEQ ID NO: 642.

71. The pharmaceutical combination or the immune cell according to claim 70, wherein the anti-IL13Ra2 antibody comprises a light chain variable region set forth in SEQ ID NO: 644 and a heavy chain variable region set forth in SEQ ID NO: 645.

72. The pharmaceutical combination or the immune cell according to claim 70 or 71, wherein the anti-IL13Ra2 antibody is an scFv comprising the amino acid sequence set forth in SEQ ID NO: 646.

73. The pharmaceutical combination or the immune cell according to any one of claims 70-72, wherein the CAR targeting II,13Ra2 comprises the amino acid sequence set forth in SEQ ID NO: 647.

74. The pharmaceutical combination or the immune cell according to claim 49, wherein the CAR targeting B7H3 comprises an anti-B7H3 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 648, an LCDR2 set forth in SEQ ID NO: 649, an LCDR3 set forth in SEQ ID NO: 650, an HCDR1 set forth in SEQ ID NO: 651, an HCDR2 set forth in SEQ ID NO: 652, and an HCDR3 set forth in SEQ ID NO: 653.

75. The pharmaceutical combination or the immune cell according to claim 74, wherein the anti-B7H3 antibody comprises a light chain variable region set forth in SEQ ID NO: 654 and a heavy chain variable region set forth in SEQ ID NO: 655.

76. The pharmaceutical combination or the immune cell according to claim 74 or 75, wherein the anti-B7H3 antibody is an scFv comprising the amino acid sequence set forth in SEQ ID NO: 656.

77. The pharmaceutical combination or the immune cell according to any one of claims 74-76, wherein the CAR targeting B7H3 comprises the amino acid sequence set forth in SEQ ID NO: 657.

78. A method for enhancing the killing effect of an immune cell on a target cell in a subject, comprising administering to the subject receiving the immune cell the humanized anti-CD28 antibody or the antigen-binding fragment thereof according to any one of claims 1-12 or the multispecific antibody according to any one of claims 13-37, the polynucleotide according to claim 38, the vector according to any one of claims 39-41, or the pharmaceutical composition according to claim 43.

79. The method according to claim 78, wherein the immune cell is a T cell, an NK cell, an NKT cell, a macrophage, a neutrophil, or a granulocyte.

80. The method according to claim 78 or 79, wherein the immune cell recombinantly expresses a chimeric antigen receptor (CAR), a T cell receptor (TCR), or a bispecific T cell engager (BiTE), wherein the CAR, the TCR, or the BiTE binds to a tumor antigen or a viral antigen.

81. The method according to any one of claims 78-80, wherein the immune cell is a CAR-T cell.

82. The method according to any one of claim 80 or 81, wherein the CAR targets mesothelin, CD123, BCMA, HER2, IL13Ra2, CD19, or B7H3.

83. The method according to claim 82, wherein the CAR targeting mesothelin comprises an anti-mesothelin antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 87, an LCDR2 set forth in SEQ ID NO: 102, an LCDR3 set forth in SEQ ID NO: 116, an HCDR1 set forth in SEQ ID NO: 131, an HCDR2 set forth in SEQ ID NO: 144, and an HCDR3 set forth in SEQ ID NO: 157;
(2) an LCDR1 set forth in SEQ ID NO: 88, an LCDR2 set forth in SEQ ID NO: 103, an LCDR3 set forth in SEQ ID NO: 117, an HCDR1 set forth in SEQ ID NO: 132, an HCDR2 set forth in SEQ ID NO: 145, and an HCDR3 set forth in SEQ ID NO: 158;
(3) an LCDR1 set forth in SEQ ID NO: 89, an LCDR2 set forth in SEQ ID NO: 104, an LCDR3 set forth in SEQ ID NO: 118, an HCDR1 set forth in SEQ ID NO: 133, an HCDR2 set forth in SEQ ID NO: 146, and an HCDR3 set forth in SEQ ID NO: 159;
(4) an LCDR1 set forth in SEQ ID NO: 90, an LCDR2 set forth in SEQ ID NO: 105, an LCDR3 set forth in SEQ ID NO: 119, an HCDR1 set forth in SEQ ID NO: 134, an HCDR2 set forth in SEQ ID NO: 147, and an HCDR3 set forth in SEQ ID NO: 160;
(5) an LCDR1 set forth in SEQ ID NO: 91, an LCDR2 set forth in SEQ ID NO: 106, an LCDR3 set forth in SEQ ID NO: 120, an HCDR1 set forth in SEQ ID NO: 135, an HCDR2 set forth in SEQ ID NO: 148, and an HCDR3 set forth in SEQ ID NO: 161;
(6) an LCDR1 set forth in SEQ ID NO: 92, an LCDR2 set forth in SEQ ID NO: 107, an LCDR3 set forth in SEQ ID NO: 121, an HCDR1 set forth in SEQ ID NO: 136, an HCDR2 set forth in SEQ ID NO: 149, and an HCDR3 set forth in SEQ ID NO: 162;
(7) an LCDR1 set forth in SEQ ID NO: 93, an LCDR2 set forth in SEQ ID NO: 108, an LCDR3 set forth in SEQ ID NO: 122, an HCDR1 set forth in SEQ ID NO: 137, an HCDR2 set forth in SEQ ID NO: 150, and an HCDR3 set forth in SEQ ID NO: 163;
(8) an LCDR1 set forth in SEQ ID NO: 94, an LCDR2 set forth in SEQ ID NO: 109, an LCDR3 set forth in SEQ ID NO: 123, an HCDR1 set forth in SEQ ID NO: 138, an HCDR2 set forth in SEQ ID NO: 151, and an HCDR3 set forth in SEQ ID NO: 164;
(9) an LCDR1 set forth in SEQ ID NO: 95, an LCDR2 set forth in SEQ ID NO: 110, an LCDR3 set forth in SEQ ID NO: 124, an HCDR1 set forth in SEQ ID NO: 139, an HCDR2 set forth in SEQ ID NO: 152, and an HCDR3 set forth in SEQ ID NO: 165;
(10) an LCDR1 set forth in SEQ ID NO: 96, an LCDR2 set forth in SEQ ID NO: 111, an LCDR3 set forth in SEQ ID NO: 125, an HCDR1 set forth in SEQ ID NO: 134, an HCDR2 set forth in SEQ ID NO: 147, and an HCDR3 set forth in SEQ ID NO: 166;
(11) an LCDR1 set forth in SEQ ID NO: 97, an LCDR2 set forth in SEQ ID NO: 112, an LCDR3 set forth in SEQ ID NO: 126, an HCDR1 set forth in SEQ ID NO: 140, an HCDR2 set forth in SEQ ID NO: 153, and an HCDR3 set forth in SEQ ID NO: 167;
(12) an LCDR1 set forth in SEQ ID NO: 98, an LCDR2 set forth in SEQ ID NO: 113, an LCDR3 set forth in SEQ ID NO: 127, an HCDR1 set forth in SEQ ID NO: 139, an HCDR2 set forth in SEQ ID NO: 152, and an HCDR3 set forth in SEQ ID NO: 168;
(13) an LCDR1 set forth in SEQ ID NO: 99, an LCDR2 set forth in SEQ ID NO: 114, an LCDR3 set forth in SEQ ID NO: 128, an HCDR1 set forth in SEQ ID NO: 141, an HCDR2 set forth in SEQ ID NO: 154, and an HCDR3 set forth in SEQ ID NO: 169;
(14) an LCDR1 set forth in SEQ ID NO: 100, an LCDR2 set forth in SEQ ID NO: 115, an LCDR3 set forth in SEQ ID NO: 129, an HCDR1 set forth in SEQ ID NO: 142, an HCDR2 set forth in SEQ ID NO: 155, and an HCDR3 set forth in SEQ ID NO: 170; and
(15) an LCDR1 set forth in SEQ ID NO: 101, an LCDR2 set forth in SEQ ID NO: 104, an LCDR3 set forth in SEQ ID NO: 130, an HCDR1 set forth in SEQ ID NO: 143, an HCDR2 set forth in SEQ ID NO: 156, and an HCDR3 set forth in SEQ ID NO: 171.

84. The method according to claim 83, wherein the light chain variable region and the heavy chain variable region are selected from the group consisting of:
(1) a light chain variable region set forth in SEQ ID NO: 172 and a heavy chain variable region set forth in SEQ ID NO: 187;
(2) a light chain variable region set forth in SEQ ID NO: 173 and a heavy chain variable region set forth in SEQ ID NO: 188;
(3) a light chain variable region set forth in SEQ ID NO: 174 and a heavy chain variable region set forth in SEQ ID NO: 189;
(4) a light chain variable region set forth in SEQ ID NO: 175 and a heavy chain variable region set forth in SEQ ID NO: 190;
(5) a light chain variable region set forth in SEQ ID NO: 176 and a heavy chain variable region set forth in SEQ ID NO: 191;
(6) a light chain variable region set forth in SEQ ID NO: 177 and a heavy chain variable region set forth in SEQ ID NO: 192;
(7) a light chain variable region set forth in SEQ ID NO: 178 and a heavy chain variable region set forth in SEQ ID NO: 193;
(8) a light chain variable region set forth in SEQ ID NO: 179 and a heavy chain variable region set forth in SEQ ID NO: 194;
(9) a light chain variable region set forth in SEQ ID NO: 180 and a heavy chain variable region set forth in SEQ ID NO: 195;
(10) a light chain variable region set forth in SEQ ID NO: 181 and a heavy chain variable region set forth in SEQ ID NO: 196;
(11) a light chain variable region set forth in SEQ ID NO: 182 and a heavy chain variable region set forth in SEQ ID NO: 197;
(12) a light chain variable region set forth in SEQ ID NO: 183 and a heavy chain variable region set forth in SEQ ID NO: 198;
(13) a light chain variable region set forth in SEQ ID NO: 184 and a heavy chain variable region set forth in SEQ ID NO: 199;
(14) a light chain variable region set forth in SEQ ID NO: 185 and a heavy chain variable region set forth in SEQ ID NO: 200; and
(15) a light chain variable region set forth in SEQ ID NO: 186 and a heavy chain variable region set forth in SEQ ID NO: 201.

85. The method according to claim 83 or 84, wherein the anti-mesothelin antibody is an scFv comprising an amino acid sequence selected from SEQ ID NOs: 202-216.

86. The method according to any one of claims 83-85, wherein the CAR targeting mesothelin comprises an amino acid sequence selected from SEQ ID NOs: 217-231.

87. The method according to claim 82, wherein the CAR targeting CD123 comprises an anti-CD123 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 349, an LCDR2 set forth in SEQ ID NO: 379, an LCDR3 set forth in SEQ ID NO: 407, an HCDR1 set forth in SEQ ID NO: 437, an HCDR2 set forth in SEQ ID NO: 458, and an HCDR3 set forth in SEQ ID NO: 482;
(2) an LCDR1 set forth in SEQ ID NO: 363, an LCDR2 set forth in SEQ ID NO: 391, an LCDR3 set forth in SEQ ID NO: 421, an HCDR1 set forth in SEQ ID NO: 435, an HCDR2 set forth in SEQ ID NO: 468, and an HCDR3 set forth in SEQ ID NO: 495;
(3) an LCDR1 set forth in SEQ ID NO: 353, an LCDR2 set forth in SEQ ID NO: 383, an LCDR3 set forth in SEQ ID NO: 412, an HCDR1 set forth in SEQ ID NO: 441, an HCDR2 set forth in SEQ ID NO: 462, and an HCDR3 set forth in SEQ ID NO: 486;
(4) an LCDR1 set forth in SEQ ID NO: 364, an LCDR2 set forth in SEQ ID NO: 378, an LCDR3 set forth in SEQ ID NO: 422, an HCDR1 set forth in SEQ ID NO: 446, an HCDR2 set forth in SEQ ID NO: 469, and an HCDR3 set forth in SEQ ID NO: 496;
(5) an LCDR1 set forth in SEQ ID NO: 369, an LCDR2 set forth in SEQ ID NO: 396, an LCDR3 set forth in SEQ ID NO: 428, an HCDR1 set forth in SEQ ID NO: 445, an HCDR2 set forth in SEQ ID NO: 475, and an HCDR3 set forth in SEQ ID NO: 503;
(6) an LCDR1 set forth in SEQ ID NO: 370, an LCDR2 set forth in SEQ ID NO: 397, an LCDR3 set forth in SEQ ID NO: 429, an HCDR1 set forth in SEQ ID NO: 452, an HCDR2 set forth in SEQ ID NO: 474, and an HCDR3 set forth in SEQ ID NO: 504;
(7) an LCDR1 set forth in SEQ ID NO: 354, an LCDR2 set forth in SEQ ID NO: 384, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(8) an LCDR1 set forth in SEQ ID NO: 350, an LCDR2 set forth in SEQ ID NO: 380, an LCDR3 set forth in SEQ ID NO: 408, an HCDR1 set forth in SEQ ID NO: 435, an HCDR2 set forth in SEQ ID NO: 456, and an HCDR3 set forth in SEQ ID NO: 480;
(9) an LCDR1 set forth in SEQ ID NO: 371, an LCDR2 set forth in SEQ ID NO: 398, an LCDR3 set forth in SEQ ID NO: 430, an HCDR1 set forth in SEQ ID NO: 453, an HCDR2 set forth in SEQ ID NO: 476, and an HCDR3 set forth in SEQ ID NO: 505;
(10) an LCDR1 set forth in SEQ ID NO: 372, an LCDR2 set forth in SEQ ID NO: 399, an LCDR3 set forth in SEQ ID NO: 431, an HCDR1 set forth in SEQ ID NO: 441, an HCDR2 set forth in SEQ ID NO: 462, and an HCDR3 set forth in SEQ ID NO: 506;
(11) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(12) an LCDR1 set forth in SEQ ID NO: 363, an LCDR2 set forth in SEQ ID NO: 392, an LCDR3 set forth in SEQ ID NO: 423, an HCDR1 set forth in SEQ ID NO: 447, an HCDR2 set forth in SEQ ID NO: 470, and an HCDR3 set forth in SEQ ID NO: 497;
(13) an LCDR1 set forth in SEQ ID NO: 373, an LCDR2 set forth in SEQ ID NO: 400, an LCDR3 set forth in SEQ ID NO: 432, an HCDR1 set forth in SEQ ID NO: 451, an HCDR2 set forth in SEQ ID NO: 477, and an HCDR3 set forth in SEQ ID NO: 507;
(14) an LCDR1 set forth in SEQ ID NO: 355, an LCDR2 set forth in SEQ ID NO: 385, an LCDR3 set forth in SEQ ID NO: 414, an HCDR1 set forth in SEQ ID NO: 443, an HCDR2 set forth in SEQ ID NO: 464, and an HCDR3 set forth in SEQ ID NO: 488;
(15) an LCDR1 set forth in SEQ ID NO: 356, an LCDR2 set forth in SEQ ID NO: 386, an LCDR3 set forth in SEQ ID NO: 415, an HCDR1 set forth in SEQ ID NO: 444, an HCDR2 set forth in SEQ ID NO: 465, and an HCDR3 set forth in SEQ ID NO: 489;
(16) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 451, an HCDR2 set forth in SEQ ID NO: 474, and an HCDR3 set forth in SEQ ID NO: 502;
(17) an LCDR1 set forth in SEQ ID NO: 351, an LCDR2 set forth in SEQ ID NO: 381, an LCDR3 set forth in SEQ ID NO: 409, an HCDR1 set forth in SEQ ID NO: 438, an HCDR2 set forth in SEQ ID NO: 459, and an HCDR3 set forth in SEQ ID NO: 483;
(18) an LCDR1 set forth in SEQ ID NO: 347, an LCDR2 set forth in SEQ ID NO: 376, an LCDR3 set forth in SEQ ID NO: 410, an HCDR1 set forth in SEQ ID NO: 439, an HCDR2 set forth in SEQ ID NO: 460, and an HCDR3 set forth in SEQ ID NO: 484;
(19) an LCDR1 set forth in SEQ ID NO: 357, an LCDR2 set forth in SEQ ID NO: 387, an LCDR3 set forth in SEQ ID NO: 416, an HCDR1 set forth in SEQ ID NO: 437, an HCDR2 set forth in SEQ ID NO: 466, and an HCDR3 set forth in SEQ ID NO: 490;
(20) an LCDR1 set forth in SEQ ID NO: 347, an LCDR2 set forth in SEQ ID NO: 377, an LCDR3 set forth in SEQ ID NO: 405, an HCDR1 set forth in SEQ ID NO: 435, an HCDR2 set forth in SEQ ID NO: 456, and an HCDR3 set forth in SEQ ID NO: 480;
(21) an LCDR1 set forth in SEQ ID NO: 365, an LCDR2 set forth in SEQ ID NO: 393, an LCDR3 set forth in SEQ ID NO: 424, an HCDR1 set forth in SEQ ID NO: 448, an HCDR2 set forth in SEQ ID NO: 471, and an HCDR3 set forth in SEQ ID NO: 498;
(22) an LCDR1 set forth in SEQ ID NO: 374, an LCDR2 set forth in SEQ ID NO: 401, an LCDR3 set forth in SEQ ID NO: 433, an HCDR1 set forth in SEQ ID NO: 454, an HCDR2 set forth in SEQ ID NO: 478, and an HCDR3 set forth in SEQ ID NO: 508;
(23) an LCDR1 set forth in SEQ ID NO: 358, an LCDR2 set forth in SEQ ID NO: 385, an LCDR3 set forth in SEQ ID NO: 417, an HCDR1 set forth in SEQ ID NO: 442, an HCDR2 set forth in SEQ ID NO: 463, and an HCDR3 set forth in SEQ ID NO: 491;
(24) an LCDR1 set forth in SEQ ID NO: 359, an LCDR2 set forth in SEQ ID NO: 388, an LCDR3 set forth in SEQ ID NO: 418, an HCDR1 set forth in SEQ ID NO: 441, an HCDR2 set forth in SEQ ID NO: 462, and an HCDR3 set forth in SEQ ID NO: 492;
(25) an LCDR1 set forth in SEQ ID NO: 347, an LCDR2 set forth in SEQ ID NO: 376, an LCDR3 set forth in SEQ ID NO: 403, an HCDR1 set forth in SEQ ID NO: 435, an HCDR2 set forth in SEQ ID NO: 456, and an HCDR3 set forth in SEQ ID NO: 480;
(26) an LCDR1 set forth in SEQ ID NO: 366, an LCDR2 set forth in SEQ ID NO: 394, an LCDR3 set forth in SEQ ID NO: 425, an HCDR1 set forth in SEQ ID NO: 449, an HCDR2 set forth in SEQ ID NO: 472, and an HCDR3 set forth in SEQ ID NO: 499;
(27) an LCDR1 set forth in SEQ ID NO: 360, an LCDR2 set forth in SEQ ID NO: 389, an LCDR3 set forth in SEQ ID NO: 413, an HCDR1 set forth in SEQ ID NO: 442, an HCDR2 set forth in SEQ ID NO: 463, and an HCDR3 set forth in SEQ ID NO: 487;
(28) an LCDR1 set forth in SEQ ID NO: 347, an LCDR2 set forth in SEQ ID NO: 376, an LCDR3 set forth in SEQ ID NO: 404, an HCDR1 set forth in SEQ ID NO: 435, an HCDR2 set forth in SEQ ID NO: 456, and an HCDR3 set forth in SEQ ID NO: 480;
(29) an LCDR1 set forth in SEQ ID NO: 348, an LCDR2 set forth in SEQ ID NO: 378, an LCDR3 set forth in SEQ ID NO: 406, an HCDR1 set forth in SEQ ID NO: 436, an HCDR2 set forth in SEQ ID NO: 457, and an HCDR3 set forth in SEQ ID NO: 481;
(30) an LCDR1 set forth in SEQ ID NO: 361, an LCDR2 set forth in SEQ ID NO: 390, an LCDR3 set forth in SEQ ID NO: 419, an HCDR1 set forth in SEQ ID NO: 445, an HCDR2 set forth in SEQ ID NO: 467, and an HCDR3 set forth in SEQ ID NO: 493;
(31) an LCDR1 set forth in SEQ ID NO: 375, an LCDR2 set forth in SEQ ID NO: 402, an LCDR3 set forth in SEQ ID NO: 434, an HCDR1 set forth in SEQ ID NO: 455, an HCDR2 set forth in SEQ ID NO: 479, and an HCDR3 set forth in SEQ ID NO: 509;
(32) an LCDR1 set forth in SEQ ID NO: 368, an LCDR2 set forth in SEQ ID NO: 395, an LCDR3 set forth in SEQ ID NO: 427, an HCDR1 set forth in SEQ ID NO: 450, an HCDR2 set forth in SEQ ID NO: 473, and an HCDR3 set forth in SEQ ID NO: 5015;
(33) an LCDR1 set forth in SEQ ID NO: 362, an LCDR2 set forth in SEQ ID NO: 388, an LCDR3 set forth in SEQ ID NO: 420, an HCDR1 set forth in SEQ ID NO: 441, an HCDR2 set forth in SEQ ID NO: 462, and an HCDR3 set forth in SEQ ID NO: 494;
(34) an LCDR1 set forth in SEQ ID NO: 352, an LCDR2 set forth in SEQ ID NO: 382, an LCDR3 set forth in SEQ ID NO: 411, an HCDR1 set forth in SEQ ID NO: 440, an HCDR2 set forth in SEQ ID NO: 461, and an HCDR3 set forth in SEQ ID NO: 485; and
(35) an LCDR1 set forth in SEQ ID NO: 367, an LCDR2 set forth in SEQ ID NO: 391, an LCDR3 set forth in SEQ ID NO: 426, an HCDR1 set forth in SEQ ID NO: 435, an HCDR2 set forth in SEQ ID NO: 468, and an HCDR3 set forth in SEQ ID NO: 500.

88. The method according to claim 87, wherein the light chain variable region and the heavy chain variable region are selected from the group consisting of:
(1) a light chain variable region set forth in SEQ ID NO: 510 and a heavy chain variable region set forth in SEQ ID NO: 545;
(2) a light chain variable region set forth in SEQ ID NO: 511 and a heavy chain variable region set forth in SEQ ID NO: 546;
(3) a light chain variable region set forth in SEQ ID NO: 512 and a heavy chain variable region set forth in SEQ ID NO: 547;
(4) a light chain variable region set forth in SEQ ID NO: 513 and a heavy chain variable region set forth in SEQ ID NO: 548;
(5) a light chain variable region set forth in SEQ ID NO: 514 and a heavy chain variable region set forth in SEQ ID NO: 549;
(6) a light chain variable region set forth in SEQ ID NO: 515 and a heavy chain variable region set forth in SEQ ID NO: 550;
(7) a light chain variable region set forth in SEQ ID NO: 516 and a heavy chain variable region set forth in SEQ ID NO: 551;
(8) a light chain variable region set forth in SEQ ID NO: 517 and a heavy chain variable region set forth in SEQ ID NO: 552;
(9) a light chain variable region set forth in SEQ ID NO: 518 and a heavy chain variable region set forth in SEQ ID NO: 553;
(10) a light chain variable region set forth in SEQ ID NO: 519 and a heavy chain variable region set forth in SEQ ID NO: 554;
(11) a light chain variable region set forth in SEQ ID NO: 520 and a heavy chain variable region set forth in SEQ ID NO: 555;
(12) a light chain variable region set forth in SEQ ID NO: 521 and a heavy chain variable region set forth in SEQ ID NO: 556;
(13) a light chain variable region set forth in SEQ ID NO: 522 and a heavy chain variable region set forth in SEQ ID NO: 557;
(14) a light chain variable region set forth in SEQ ID NO: 523 and a heavy chain variable region set forth in SEQ ID NO: 558;
(15) a light chain variable region set forth in SEQ ID NO: 524 and a heavy chain variable region set forth in SEQ ID NO: 559;
(16) a light chain variable region set forth in SEQ ID NO: 525 and a heavy chain variable region set forth in SEQ ID NO: 560;
(17) a light chain variable region set forth in SEQ ID NO: 526 and a heavy chain variable region set forth in SEQ ID NO: 561;
(18) a light chain variable region set forth in SEQ ID NO: 527 and a heavy chain variable region set forth in SEQ ID NO: 562;
(19) a light chain variable region set forth in SEQ ID NO: 528 and a heavy chain variable region set forth in SEQ ID NO: 563;
(20) a light chain variable region set forth in SEQ ID NO: 529 and a heavy chain variable region set forth in SEQ ID NO: 564;
(21) a light chain variable region set forth in SEQ ID NO: 530 and a heavy chain variable region set forth in SEQ ID NO: 565;
(22) a light chain variable region set forth in SEQ ID NO: 531 and a heavy chain variable region set forth in SEQ ID NO: 566;
(23) a light chain variable region set forth in SEQ ID NO: 532 and a heavy chain variable region set forth in SEQ ID NO: 567;
(24) a light chain variable region set forth in SEQ ID NO: 533 and a heavy chain variable region set forth in SEQ ID NO: 568;
(25) a light chain variable region set forth in SEQ ID NO: 534 and a heavy chain variable region set forth in SEQ ID NO: 569;
(26) a light chain variable region set forth in SEQ ID NO: 535 and a heavy chain variable region set forth in SEQ ID NO: 570;
(27) a light chain variable region set forth in SEQ ID NO: 536 and a heavy chain variable region set forth in SEQ ID NO: 571;
(28) a light chain variable region set forth in SEQ ID NO: 537 and a heavy chain variable region set forth in SEQ ID NO: 572;
(29) a light chain variable region set forth in SEQ ID NO: 538 and a heavy chain variable region set forth in SEQ ID NO: 573;
(30) a light chain variable region set forth in SEQ ID NO: 539 and a heavy chain variable region set forth in SEQ ID NO: 574;
(31) a light chain variable region set forth in SEQ ID NO: 540 and a heavy chain variable region set forth in SEQ ID NO: 575;
(32) a light chain variable region set forth in SEQ ID NO: 541 and a heavy chain variable region set forth in SEQ ID NO: 576;
(33) a light chain variable region set forth in SEQ ID NO: 542 and a heavy chain variable region set forth in SEQ ID NO: 577;
(34) a light chain variable region set forth in SEQ ID NO: 543 and a heavy chain variable region set forth in SEQ ID NO: 578; and
(35) a light chain variable region set forth in SEQ ID NO: 544 and a heavy chain variable region set forth in SEQ ID NO: 579.

89. The method according to claim 87 or 88, wherein the CD123 antibody is an scFv comprising an amino acid sequence selected from SEQ ID NOs: 583-617.

90. The method according to any one of claims 87-89, wherein the CAR targeting CD123 comprises an amino acid sequence selected from SEQ ID NOs: 580-582.

91. The method according to claim 82, wherein the CAR targeting BCMA comprises an anti-BCMA antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are selected from the group consisting of:
(1) an LCDR1 set forth in SEQ ID NO: 232, an LCDR2 set forth in SEQ ID NO: 242, an LCDR3 set forth in SEQ ID NO: 253, an HCDR1 set forth in SEQ ID NO: 264, an HCDR2 set forth in SEQ ID NO: 275, and an HCDR3 set forth in SEQ ID NO: 287;
(2) an LCDR1 set forth in SEQ ID NO: 233, an LCDR2 set forth in SEQ ID NO: 243, an LCDR3 set forth in SEQ ID NO: 254, an HCDR1 set forth in SEQ ID NO: 265, an HCDR2 set forth in SEQ ID NO: 276, and an HCDR3 set forth in SEQ ID NO: 288;
(3) an LCDR1 set forth in SEQ ID NO: 234, an LCDR2 set forth in SEQ ID NO: 244, an LCDR3 set forth in SEQ ID NO: 255, an HCDR1 set forth in SEQ ID NO: 266, an HCDR2 set forth in SEQ ID NO: 277, and an HCDR3 set forth in SEQ ID NO: 289;
(4) an LCDR1 set forth in SEQ ID NO: 235, an LCDR2 set forth in SEQ ID NO: 245, an LCDR3 set forth in SEQ ID NO: 255, an HCDR1 set forth in SEQ ID NO: 267, an HCDR2 set forth in SEQ ID NO: 278, and an HCDR3 set forth in SEQ ID NO: 290;
(5) an LCDR1 set forth in SEQ ID NO: 236, an LCDR2 set forth in SEQ ID NO: 246, an LCDR3 set forth in SEQ ID NO: 256, an HCDR1 set forth in SEQ ID NO: 268, an HCDR2 set forth in SEQ ID NO: 279, and an HCDR3 set forth in SEQ ID NO: 291;
(6) an LCDR1 set forth in SEQ ID NO: 237, an LCDR2 set forth in SEQ ID NO: 247, an LCDR3 set forth in SEQ ID NO: 257, an HCDR1 set forth in SEQ ID NO: 269, an HCDR2 set forth in SEQ ID NO: 280, and an HCDR3 set forth in SEQ ID NO: 292;
(7) an LCDR1 set forth in SEQ ID NO: 238, an LCDR2 set forth in SEQ ID NO: 248, an LCDR3 set forth in SEQ ID NO: 258, an HCDR1 set forth in SEQ ID NO: 266, an HCDR2 set forth in SEQ ID NO: 281, and an HCDR3 set forth in SEQ ID NO: 293;
(8) an LCDR1 set forth in SEQ ID NO: 239, an LCDR2 set forth in SEQ ID NO: 249, an LCDR3 set forth in SEQ ID NO: 259, an HCDR1 set forth in SEQ ID NO: 270, an HCDR2 set forth in SEQ ID NO: 282, and an HCDR3 set forth in SEQ ID NO: 294;
(9) an LCDR1 set forth in SEQ ID NO: 233, an LCDR2 set forth in SEQ ID NO: 250, an LCDR3 set forth in SEQ ID NO: 260, an HCDR1 set forth in SEQ ID NO: 271, an HCDR2 set forth in SEQ ID NO: 283, and an HCDR3 set forth in SEQ ID NO: 295;
(10) an LCDR1 set forth in SEQ ID NO: 233, an LCDR2 set forth in SEQ ID NO: 251, an LCDR3 set forth in SEQ ID NO: 261, an HCDR1 set forth in SEQ ID NO: 272, an HCDR2 set forth in SEQ ID NO: 284, and an HCDR3 set forth in SEQ ID NO: 296;
(11) an LCDR1 set forth in SEQ ID NO: 240, an LCDR2 set forth in SEQ ID NO: 252, an LCDR3 set forth in SEQ ID NO: 262, an HCDR1 set forth in SEQ ID NO: 273, an HCDR2 set forth in SEQ ID NO: 285, and an HCDR3 set forth in SEQ ID NO: 297; and
(12) an LCDR1 set forth in SEQ ID NO: 241, an LCDR2 set forth in SEQ ID NO: 245, an LCDR3 set forth in SEQ ID NO: 263, an HCDR1 set forth in SEQ ID NO: 274, an HCDR2 set forth in SEQ ID NO: 286, and an HCDR3 set forth in SEQ ID NO: 298.

92. The method according to claim 91, wherein the light chain variable region and the heavy chain variable region are selected from the group consisting of:
(1) a light chain variable region set forth in SEQ ID NO: 299 and a heavy chain variable region set forth in SEQ ID NO: 311;
(2) a light chain variable region set forth in SEQ ID NO: 300 and a heavy chain variable region set forth in SEQ ID NO: 312;
(3) a light chain variable region set forth in SEQ ID NO: 301 and a heavy chain variable region set forth in SEQ ID NO: 313;
(4) a light chain variable region set forth in SEQ ID NO: 302 and a heavy chain variable region set forth in SEQ ID NO: 314;
(5) a light chain variable region set forth in SEQ ID NO: 303 and a heavy chain variable region set forth in SEQ ID NO: 315;
(6) a light chain variable region set forth in SEQ ID NO: 304 and a heavy chain variable region set forth in SEQ ID NO: 316;
(7) a light chain variable region set forth in SEQ ID NO: 305 and a heavy chain variable region set forth in SEQ ID NO: 317;
(8) a light chain variable region set forth in SEQ ID NO: 306 and a heavy chain variable region set forth in SEQ ID NO: 318;
(9) a light chain variable region set forth in SEQ ID NO: 307 and a heavy chain variable region set forth in SEQ ID NO: 319;
(10) a light chain variable region set forth in SEQ ID NO: 308 and a heavy chain variable region set forth in SEQ ID NO: 320;
(11) a light chain variable region set forth in SEQ ID NO: 309 and a heavy chain variable region set forth in SEQ ID NO: 321; and
(12) a light chain variable region set forth in SEQ ID NO: 310 and a heavy chain variable region set forth in SEQ ID NO: 322.

93. The method according to claim 91 or 92, wherein the anti-BCMA antibody is an scFv comprising an amino acid sequence selected from SEQ ID NOs: 323-334.

94. The method according to any one of claims 91-93, wherein the CAR targeting BCMA comprises an amino acid sequence selected from SEQ ID NOs: 335-346.

95. The method according to claim 82, wherein the CAR targeting CD19 comprises an anti-CD19 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 628, an LCDR2 set forth in SEQ ID NO: 629, an LCDR3 set forth in SEQ ID NO: 630, an HCDR1 set forth in SEQ ID NO: 631, an HCDR2 set forth in SEQ ID NO: 632, and an HCDR3 set forth in SEQ ID NO: 633.

96. The method according to claim 95, wherein the anti-CD19 antibody comprises a light chain variable region set forth in SEQ ID NO: 635 and a heavy chain variable region set forth in SEQ ID NO: 634.

97. The method according to claim 95 or 96, wherein the anti-CD19 antibody is an scFv comprising the amino acid sequence set forth in SEQ ID NO: 636.

98. The method according to any one of claims 95-97, wherein the CAR targeting CD19 comprises the amino acid sequence set forth in SEQ ID NO: 637.

99. The method according to claim 82, wherein the CAR targeting HER2 comprises an anti-HER2 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 618, an LCDR2 set forth in SEQ ID NO: 619, an LCDR3 set forth in SEQ ID NO: 620, an HCDR1 set forth in SEQ ID NO: 621, an HCDR2 set forth in SEQ ID NO: 622, and an HCDR3 set forth in SEQ ID NO: 623.

100. The method according to claim 99, wherein the anti-HER2 antibody comprises a light chain variable region set forth in SEQ ID NO: 624 and a heavy chain variable region set forth in SEQ ID NO: 625.

101. The method according to claim 99 or 100, wherein the anti-HER2 antibody is an scFv comprising the amino acid sequence set forth in SEQ ID NO: 626.

102. The method according to any one of claims 99-101, wherein the CAR targeting HER2 comprises the amino acid sequence set forth in SEQ ID NO: 627.

103. The method according to claim 82, wherein the CAR targeting IL13Ra2 comprises an anti-IL13Ra2 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 638, an LCDR2 set forth in SEQ ID NO: 639, an LCDR3 set forth in SEQ ID NO: 640, an HCDR1 set forth in SEQ ID NO: 641, an HCDR2 set forth in SEQ ID NO: 642, and an HCDR3 set forth in SEQ ID NO: 642.

104. The method according to claim 103, wherein the anti-IL13Ra2 antibody comprises a light chain variable region set forth in SEQ ID NO: 644 and a heavy chain variable region set forth in SEQ ID NO: 645.

105. The method according to claim 103 or 105, wherein the anti-IL13Ra2 antibody is an scFv comprising the amino acid sequence set forth in SEQ ID NO: 646.

106. The method according to any one of claims 103-105, wherein the CAR targeting IL13Ra2 comprises the amino acid sequence set forth in SEQ ID NO: 647.

107. The method according to claim 82, wherein the CAR targeting B7H3 comprises an anti-B7H3 antibody as a binding domain thereof, and the anti-mesothelin antibody comprises a light chain variable region comprising an LCDR1, an LCDR2 and an LCDR3 and a heavy chain variable region comprising an HCDR1, an HCDR2 and an HCDR3, wherein the LCDR1, the LCDR2, the LCDR3, the HCDR1, the HCDR2, and the HCDR3 are an LCDR1 set forth in SEQ ID NO: 648, an LCDR2 set forth in SEQ ID NO: 649, an LCDR3 set forth in SEQ ID NO: 650, an HCDR1 set forth in SEQ ID NO: 651, an HCDR2 set forth in SEQ ID NO: 652, and an HCDR3 set forth in SEQ ID NO: 653.

108. The method according to claim 107, wherein the anti-B7H3 antibody comprises a light chain variable region set forth in SEQ ID NO: 654 and a heavy chain variable region set forth in SEQ ID NO: 655.

109. The method according to claim 107 or 108, wherein the anti-B7H3 antibody is an scFv comprising the amino acid sequence set forth in SEQ ID NO: 656.

110. The method according to any one of claims 107-109, wherein the CAR targeting B7H3 comprises the amino acid sequence set forth in SEQ ID NO: 657.

111. A method for killing a target cell in a subject, comprising administering to the subject the pharmaceutical combination or the immune cell according to any one of claims 43-77.

112. The method according to any one of claims 78-111, wherein the target cell is a tumor cell.

113. The method according to claim 112, wherein the tumor cell expresses mesothelin, CD123, BCMA, HER2, IL13Ra2, CD19, or B7H3.

114. A method for treating a disease in a subject in need thereof, comprising administering to the subject a therapeutically effective amount of the pharmaceutical combination or the immune cell according to any one of claims 44-77.

115. The method according to claim 114, wherein the disease is a tumor, a cancer, a viral infection, or an autoimmune disease.

116. The method according to claim 115, wherein the cancer expresses mesothelin, CD123, BCMA, HER2, IL13Ra2, or B7H3.

117. The method according to claim 115 or 116, wherein the cancer is a solid tumor or a hematologic cancer.

118. The method according to any one of claims 115-117, wherein the cancer is acute myeloid leukemia (AML), B-type acute lymphocytic leukemia (B-ALL), T-type acute lymphocytic leukemia (T-ALL), B-cell precursor acute lymphocytic leukemia (BCP-ALL), or blastic placytoid dendritic cell neoplasm (BPDCN), non-Hodgkin's lymphoma, chronic lymphocytic leukemia, acute lymphocytic leukemia, human B-cell precursor leukemia, multiple myeloma, or malignant lymphoma.

119. The method according to any one of claims 115-117, wherein the cancer is mesothelioma, pancreatic cancer, ovarian cancer, lung cancer, breast cancer, gastric cancer, cervical cancer, urothelial cancer, esophageal cancer, bladder cancer, colorectal cancer, endometrial cancer, renal cancer, head and neck cancer, sarcoma, glioblastoma, prostate cancer, thyroid cancer, or glioma.

120. The humanized anti-CD28 antibody or the antigen-binding fragment thereof according to any one of claims 1-12, wherein the heavy chain variable region of the humanized anti-CD28 antibody is fused to an immunoglobulin heavy chain constant region and/or the light chain variable region of the humanized anti-CD28 antibody is fused to an immunoglobulin light chain constant region.

121. The humanized anti-CD28 antibody or the antigen-binding fragment thereof according to claim 120, wherein the immunoglobulin heavy chain constant region comprises SEQ ID NO: 81 and the immunoglobulin light chain constant region comprises SEQ ID NO: 82.

122. A method for inducing T cell proliferation, comprising contacting the humanized anti-CD28 antibody or the antigen-binding fragment thereof according to any one of claims 1-12 or the multispecific antibody according to any one of claims 13-37, the polynucleotide according to claim 38, the vector according to any one of claims 39-41 or the pharmaceutical composition according to claim 43 with a T cell.

123. The method according to claim 122, further comprising introducing the polynucleotide or the vector into the T cell and allowing the T cell to express the humanized anti-CD28 antibody or the antigen-binding fragment thereof or the multispecific antibody.

124. The method according to claim 122 or 123, wherein the T cell expresses a chimeric antigen receptor (CAR).
